(19) Europäisches Patentamt
European Patent Office
Office européen des brevets

(11) EP 4 242 218 A1

(12) EUROPEAN PATENT APPLICATION
published in accordance with Art. 153(4) EPC

(43) Date of publication:
**13.09.2023 Bulletin 2023/37**

(21) Application number: **21882000.9**

(22) Date of filing: **19.10.2021**

(51) International Patent Classification (IPC):
***C07H 21/04*** (2006.01) ***A61K 31/7084*** (2006.01)
***A61P 35/00*** (2006.01) ***A61P 31/12*** (2006.01)

(52) Cooperative Patent Classification (CPC):
**A61K 31/7084; A61P 31/12; A61P 35/00; C07H 21/04**

(86) International application number:
**PCT/CN2021/124704**

(87) International publication number:
**WO 2022/083584 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.10.2020 CN 202011124067**
**15.10.2021 CN 202111201201**

(71) Applicant: **Tyligand Bioscience (Shanghai) Limited**
**Shanghai 201203 (CN)**

(72) Inventors:
- **ZHANG, Tony Yantao**
  **Fishers IN 46037 (US)**
- **QU, Li**
  **Shanghai 200082 (CN)**
- **LOU, Liang**
  **Pudong New District Shanghai 201323 (CN)**
- **ZHENG, Puji**
  **Shanghai 201210 (CN)**
- **LV, Fei**
  **Shanghai 200240 (CN)**

(74) Representative: **Vossius & Partner Patentanwälte Rechtsanwälte mbB**
**Siebertstraße 3**
**81675 München (DE)**

# (54) MULTIFUNCTIONAL CYCLIC DINUCLEOTIDE AND USE THEREOF

(57) The present invention relates to a multifunctional cyclic-dinucleotide compound of formula (X) and its derivatives, which can be used as a prodrug of an apoptosis inducer or a cytotoxic agent for inducing cell apoptosis or anti-virus, and can also regulate immunity pathway to generate a therapeutically beneficial immune response. The present disclosure further relates to pharmaceutical compositions and pharmaceutical combinations comprising the cyclic-dinucleotide compounds of the present invention, methods for synthesizing them, and medical uses thereof.

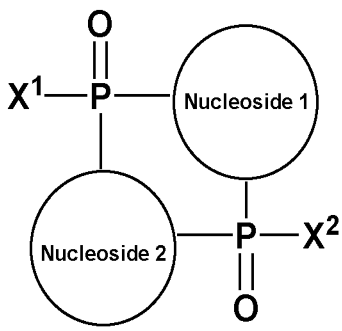

(x)

EP 4 242 218 A1

## Description

### Technical field

The present invention relates to multifunctional cyclic dinucleotide compounds and derivatives thereof, which function as pro-drugs of apoptosis-inducing agents or cytotoxic agents for inducing apoptosis of tumor cells or for combating viruses, and as immunomodulators for modulating immune pathways to generate therapeutically beneficial immune responses, particularly for activating STING-mediated immune pathways. The disclosure further relates to pharmaceutical compositions and pharmaceutical combinations comprising the cyclic-dinucleotide compounds of the present invention, methods for synthesizing the same, and medical uses thereof.

### Background

Cancer, as a malignant disease characterized biologically by abnormal cell differentiation and proliferation, uncontrolled growth, infiltrative and metastatic properties, has become one of the important causes of death in humans, and its incidence is continuously increasing across the world. Mmeanwhile, viral infections have also led to millions of human deaths worldwide.

Antimetabolic nucleoside analogs represent one of the major therapeutic approaches in the treatment strategies against cancer/tumors and viral infections. Specifically, antimetabolic nucleoside drugs do not act directly per se, but have to be converted into triphosphorylated forms in vivo by various cellular kinases, serve as pseudo metabolites to become active substrates of polymerases, then are embedded into DNA or RNA via nucleic acid biosynthesis pathway to inhibit the modification and extension of DNA or RNA or to inhibit the reverse transcriptases associated with DNA or RNA synthesis, thereby inducing apoptosis of tumor cells or preventing virus replication, i.e. exhibiting cytotoxicity, and can be used for the treatment of cancer/tumors or viral infections. Meanwhile, cell fragments resulting from the cytotoxicity of the nucleoside drugs can elicit immune response of host cells, thereby further inhibiting the growth and reproduction of tumor cells or viruses.

The anticancer nucleosides currently used in clinical practice include the following:

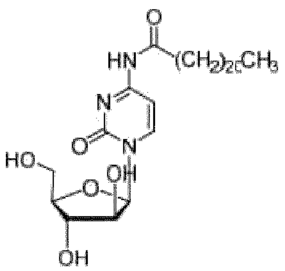

Enocitabine (sunrabin)
1983

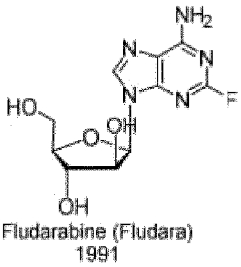

Fludarabine (Fludara)
1991

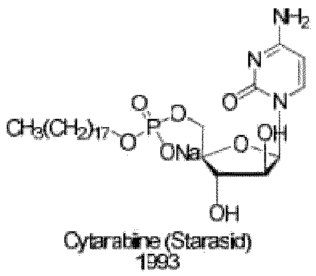

Cytarabine (Starasid)
1993

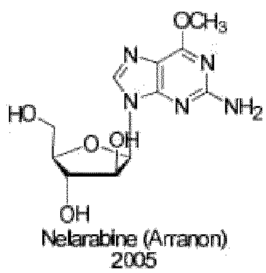

Nelarabine (Arranon)
2005

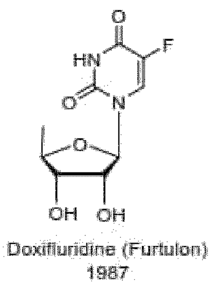

Doxifluridine (Furtulon)
1987

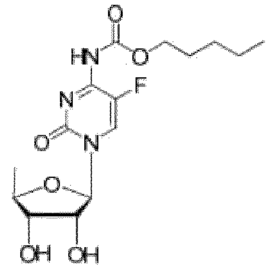

Capecitabine (Xeloda)
1998

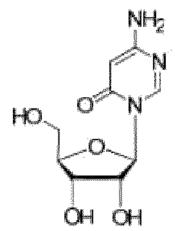

Azacitidine (Vidaza)
2004

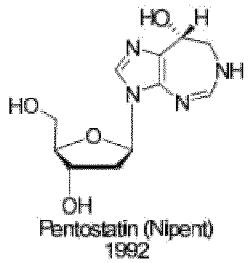
Pentostatin (Nipent)
1992

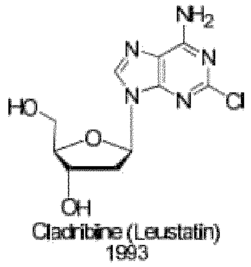
Cladribine (Leustatin)
1993

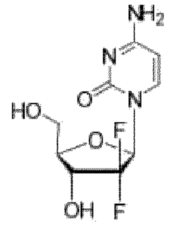
Gemcitabine (Gemzar)
1995

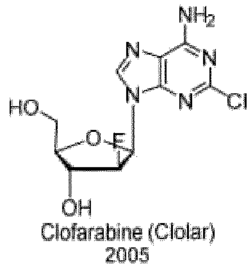
Clofarabine (Clolar)
2005

;

However, nucleoside drugs carry a plurality of active groups such as hydroxyl and amino groups on their molecular structures, which leads to low membrane permeation efficiency and poor stability and pharmacokinetic properties, so special or frequent administration is oftem required which brings lots of inconvenience to patients. Therefore, to seek new and improved nucleoside drug solutions has been an active area of drug development.

Endogenous cyclic dinucleotide (CDN) cGAMP is an important link in the key component of the innate immune system - - the cGAS-STING (cyclic GMP-AMP synthase-interferon gene stimulator) signal pathway. Specifically, cGAS interacts with DNA from tumor cells, dying cells, viruses, bacteria or mitochondria, and catalyzes the synthesis of cyclic dinucleotide (CDN) cGAMP from ATP and GTP. The produced endogenous cGAMP further binds to STING on the endoplasmic reticulum (ER), and the STING bound to cGAMP is activated, undergoes a conformational change, translocates to Golgi, then induces activation of the key transcription factors IRF-3 and NF-κB, and those activated transcription factors enter the nucleus and induce expression of type I interferons and proinflammatory cytokines such as IL-6, TNF-α and IFN-γ (Jiang et al, cGAS-STING, an antigenic pathway in cancer immunology, Journal of Hematology & Oncology, 2020, 13:81; Xiangling Cui et al, STING modulatories: Predictive significance in drug discovery, European Journal of chemical Chemistry 182 (2019) 111591).

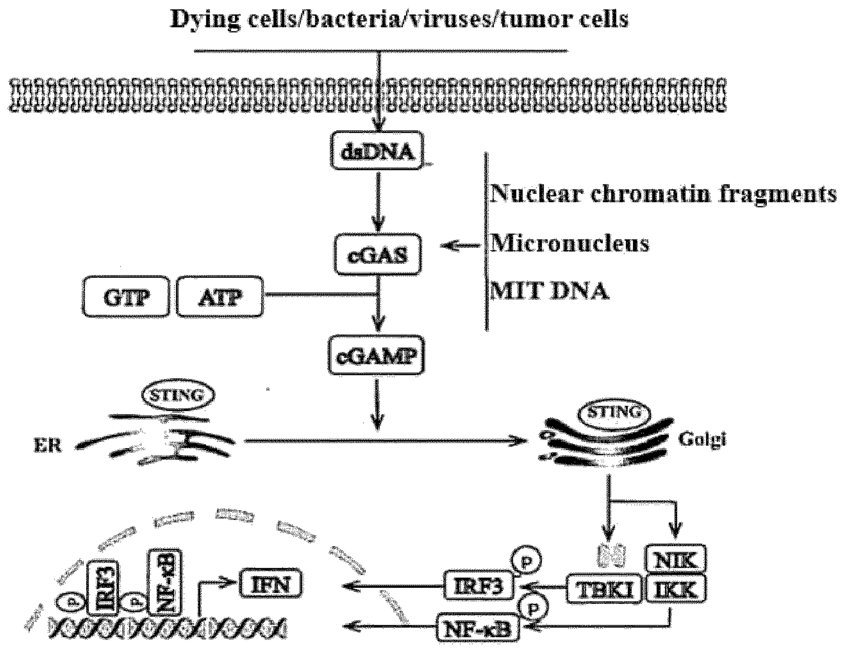


It is well known in the art that, type I interferons not only exhibit antiviral activity, but also directly inhibit human tumor cell proliferation, significantly enhance antitumor immune response by inducing the activation of adaptive and innate immune cells, and inhibit tumor invasion by modulating the expression of enzymes associated with tissue remodeling, and thus are also useful as anticancer agents.

Given that endogenous cyclic dinucleotide (CDN) cGAMP, as shown above, is a key mediator of innate immune system in response to viruses and tumors and ultimately contributing to interferon or pro-inflammatory cytokine production and thereby achieving therapeutic benefits, a series of CDN STING agonists have been synthesized and verified in laboratory for activity, examples of which are described in WO2014/189805, WO2017/027645, and WO2018/060323. However, existing CDN-based therapies still lack sufficient clinical therapeutic efficacy, and so improved CDN-based STING agonists are still needed to provide safer and more potent antiviral or anti-tumor effects.

According to the consensus in the art, the STING pathway can be activated by exogenous DNA (tumor or virus, etc.). Without the mediation of protein neoantigen, the downstream proinflammatory factors driven by interferon and the like in the STING pathway lack the targeting property, resulting in poor tolerance of autoimmune response and narrow treatment window. The first generation of single STING agonists combined with PD-1 antibodies still focused on activation of systemic immunity, and did not address the selectivity of adaptive immunity and provoke a response from the tumor microenvironment.

The compounds of the invention on the one hand act as the known highlyactive STING agonists by activating signal pathway, releasing interferons and other cytokines and activating immune system; secondly, their cytotoxic function is then activated to selectively kill tumors, releasing large amounts of tumor neoantigens and tumor DNA to establish adaptive immune recognition and train the targeting performance of the immune system; thirdly, the tumor neoantigens and the tumor DNA continue to activate the STING pathway and other immune systems to kill tumor cells; finally, the released tumor neoantigens are recognized by DC cells and interact with T cells to form immunological memory, thereby achieving long-term control of remote tumors and cancer cell migration.

Specifically, in searching for a new cytotoxic nucleoside drug with improved properties, the present invention creatively introduced a cytotoxic nucleoside drug as a building unit into the molecular structure of CDN, namely introducing a hidden cytotoxic pharmacophore at the molecular level of CDN. The resulting novel CDN drug molecules first of all can activate STING and induce the production of type I interferons, thereby achieving antiviral or anti-tumor immunotherapy effects. More innovatively, the product formed by the decomposition of molecules in vivo, namely the cytotoxic nucleoside drug, can specifically interfere with nucleic acid metabolism, prevent cell division and reproduction, induce tumor cell death or prevent virus replication, release tumor DNA to continuously activate STING, and release tumor neoantigens to establish adaptive immune recognition function and train the targeting performance of the immune system, thereby the defects of low membrane penetration efficiency, poor stability and poor pharmacokinetic properties and requiring frequent or special drug administration for single nucleoside drugs have been overcome, and simultaneously overcomed is the source of exogenous DNA (such as tumor DNA) required for continuous activation of STING pathway. More importantly, the CDN structural backbone of the molecules disclosed herein can activate STING, thereby inducing type I interferon production, and achieving the antiviral or antitumor immunotherapeutic effects. On the other hand, the cytotoxic pharmacophore hidden in the novel CDN molecule can be timely released so as to generate apoptotic fragments, provide antigens against tumors or viruses for the immune system, and generate antibody-antigen response under the coordination of immune leukocyte subsets, thereby providing the ability of "immunological memory" or persistent immunity against the antigens.

Therefore, the invention provides a class of novel CDN compounds, which, by the drug combination at submolecular level, can simultaneously realize the anti-metabolic treatment and immunosuppression of viral infections or tumors, and can provide enhanced and even synergistic effects as compared with single cytotoxic drug or single CDN STING agonist.

It should be noted that, the above discussion of the background of the invention is provided merely to help understand the present invention and should not be interpreted as an admission of prior art description or as constituting prior art for the present invention.

## Summary of the Invention

The invention aims to provide a group of novel cyclic dinucleotide-based antiviral or antitumor compounds.

In one aspect, the invention provides a group of cyclic dinucleotide compounds of the following formula, stereoisomers, tautomers, stable isotopic variants, pharmaceutically acceptable salts or solvates thereof,

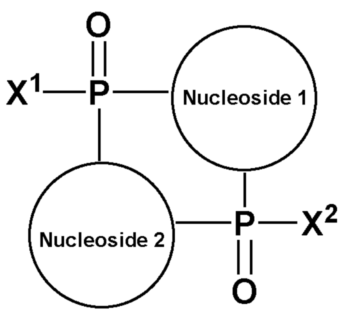

(X),

e.g.,

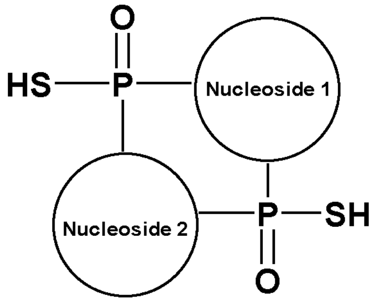

wherein $X_1$ and $X_2$ are each independently selected from -OH and-SH; and at least one of the two nucleosides of the cyclic dinucleotide has cytotoxic or antiviral effect. The compound of such a structure on one hand can generate a single cytotoxic or antiviral nucleoside compound after being decomposed in vivo, and upon the triphosphorylation activation by nucleoside kinases in cells, the compound can prevent the division and reproduction of cells by specifically interfering with nucleic acid metabolism, thereby inhibiting tumor cell proliferation or virus replication. On the other hand, the molecule of the invention maintains the immune activation function of cyclic dinucleotide, namely activating the target STING and finally inducing type I interferon production through STING signal conduction cascade, thereby generating tumor immune activity to inhibit the growth and metastasis of tumors, or exerting antiviral activity. Furthermore, apoptotic tissue fragments provide the immune system with antigens that are not naturally expressed in the host, produce an antigen-antibody response, and thereby providing the ability of "memory" or persistent immunity against the antigens.

Specifically, the invention in this aspect provides a cyclic dinucleotide compound of formula (Y),

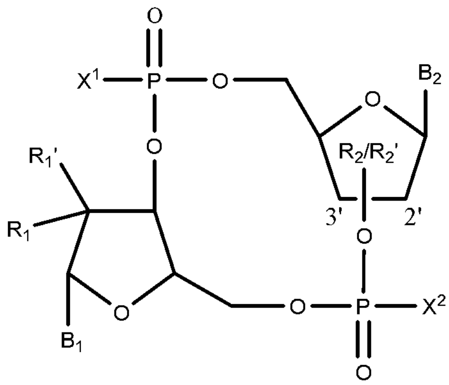

(Y)

wherein $X^1$, $X^2$, $B_1$, $B_2$, $R^1$, $R^{1'}$, $R^2$, $R^{2'}$ are as defined herein; a stereoisomer, tautomers, a stable isotopic variant, a pharmaceutically acceptable salt, a prodrug or a solvate thereof.

More specifically, the present invention in this aspect provides cyclic dinucleotide compounds of formulas (I), (II), (III) and (IV) and their respective subformulas; more specifically, the present invention in this aspect provides cyclic dinucleotide compounds of each subformula of formulas (I), (II), (III) and (IV); and respective specific embodiments thereof, as described below.

In another aspect, the invention provides methods for the preparation of the compounds of the invention described herein, and also provides compounds of the invention described herein obtainable by the methods described herein.

In another aspect, the present invention provides a pharmaceutical composition comprising a compound of the present invention described herein, a stereoisomer, tautomer, stable isotopic variant, pharmaceutically acceptable salt, pro-drug or solvate thereof and one or more pharmaceutically acceptable excipients.

In another aspect, the invention provides the compounds of the invention as described herein or the pharmaceutical composition as described herein, for use as an active agent for the treatment or prevention of diseases associated with or mediated by an immune response, in particular for the treatment or prevention of diseases associated with or mediated by STING, more particularly for the treatment or prevention of inflammation, allergic or autoimmune diseases, infectious diseases or cancer, especially for antiviral or antitumor use, or for use as vaccine adjuvants.

In another aspect, the present invention provides the compounds of the invention as described herein or the pharmaceutical composition as described herein, for use as a cytotoxic agent, especially an antineoplastic agent, for the treatment or prevention of hyperproliferative diseases.

In another aspect, the invention provides the compounds of the invention as described herein or the pharmaceutical composition as described herein, for use as a cytotoxic agent for the treatment or prophylaxis of viral infections.

In another aspect, the present invention provides a use of the compounds of the invention as described herein or the pharmaceutical composition as described herein for the treatment or prevention of diseases associated with or mediated by an immune response, for example use as a STING agonist specifically for the treatment or prevention of diseases associated with or mediated by STING, more specifically for the treatment or prevention of inflammation, allergic or autoimmune diseases, infectious diseases or cancers, especially tumors or viral infections; or use as a vaccine adjuvant.

In another aspect, the present invention provides a use of the compounds of the invention described herein or the pharmaceutical composition described herein as a cytotoxic agent in the treatment or prevention of hyperproliferative diseases, especially tumors; or as a cytotoxic agent in the treatment or prevention of viral infections.

In another aspect, the invention provides a method for the treatment or prevention of diseases associated with or mediated by an immune response, specifically diseases associated with or mediated by STING, more specifically inflammation, allergic or autoimmune diseases, infectious diseases or cancers, especially tumors or viral infections in a subject, comprising administering to a human or animal the compound of the invention as described herein or the pharmaceutical composition as described herein.

In another aspect, the present invention provides a method for the treatment or prevention of hyperproliferative diseases, especially tumors, in a subject, comprising administering to a human or animal the compound of the invention as described herein or the pharmaceutical composition as described herein.

In another aspect, the invention provides a method for the treatment or prevention of viral infections in a subject, comprising administering to a human or animal the compound of the invention described herein or the pharmaceutical composition described herein.

In another aspect, the present invention provides a use of the compounds of the present invention as described herein or the pharmaceutical composition as described herein in the manufacture of a medicament for the treatment or prevention of diseases associated with or mediated by an immune response, specifically diseases associated with or mediated by STING, more specifically inflammation, allergic or autoimmune diseases, infectious diseases or cancers, especially tumors or viral infections, or for use as a vaccine adjuvant.

In another aspect, the present invention provides a use of the compounds of the invention as described herein or the pharmaceutical composition as described herein for the manufacture of a medicament for the treatment or prevention of hyperproliferative diseases, especially tumors.

In another aspect, the present invention provides a use of the compounds of the invention described herein or the pharmaceutical composition described herein for the manufacture of a medicament for the treatment or prevention of viral infections.

In another aspect, the present invention provides the compounds of the invention as described herein or the pharmaceutical composition as described herein for use as a multifunctional agent having both immunotherapeutic and cytotoxic therapeutic activities, including for use of exerting anti-tumor and anti-viral replication functions by activating STING signaling pathway and activating the immune system, for use of causing tumor cell death or preventing viral replication by releasing cytotoxic agents, and then for use of killing tumor cells by releasing tumor DNA and continuously activating STING, and for use of providing the ability of "immunological memory" or persistent immunity to tumors by releasing tumor neoantigens to produce antibody-antigen responses. In this aspect, the present invention also provides the use of the compounds of the present invention described herein or the pharmaceutical composition described herein for realizing the above-mentioned multiple functions, for example, specifically for the treatment or prevention of viral infections or tumors; the method of treating or preventing through the above-mentioned multiple functions diseases associated with or mediated by immune responses, specifically diseases associated with or mediated by STING in a subject, more specifically inflammation, allergic or autoimmune diseases, infectious diseases or cancers, especially tumors or viral infections, comprising administering to a human or an animal a compound of the invention as described herein or a pharmaceutical combination as described herein; and the use of the compounds of the present invention described herein or the pharmaceutical composition described herein in the manufacture of a medicament for realizing the above-mentioned multiple functions, for example, the medicament is specifically used for treating or preventing viral infections or tumors.

In another aspect, the present invention provides a pharmaceutical combination comprising a compound of the present invention described herein, a stereoisomer, tautomer, stable isotopic variant, pharmaceutically acceptable salt or solvate thereof, and at least one other therapeutic agent.

In another aspect, the invention provides a pharmaceutical composition comprising a compound of the invention described herein, stereoisomer, tautomer, stable isotopic variant, pharmaceutically acceptable salt or solvate thereof, at least one other therapeutic agent, and one or more pharmaceutically acceptable excipients.

In another aspect, the invention provides a pharmaceutical combination as described herein comprising a compound of the invention and at least one other therapeutic agent for use in the treatment or prevention of hyperproliferative diseases, viral infections or diseases associated with or mediated by STING, more specifically inflammation,

allergic or autoimmune diseases, infectious diseases or cancers, especially tumors or viral infections.

In another aspect, the invention provides a use of the pharmaceutical combination as described herein comprising a compound of the invention and at least one other therapeutic agent for the treatment or prevention of hyperproliferative diseases, viral infections or diseases associated with or mediated by STING, more specifically inflammation, allergic or autoimmune diseases, infectious diseases or cancers, especially tumors or viral infections.

In another aspect, the present invention provides a method for the treatment or prevention of hyperproliferative diseases, viral infections or diseases associated with or mediated by STING, more specifically inflammation, allergic or autoimmune diseases, infectious diseases or cancers, especially tumors or viral infections, comprising administering to a human or animal the pharmaceutical combination as described herein comprising a compound of the invention and at least one other therapeutic agent.

## Brief Description of the Drawings

Figure 1 illustrates the interferon-stimulating activity of representative compounds of the present invention in THP-1 cells.

Figure 2 illustrates the tumor growth inhibitory activity of representative compounds of the present invention in the transplanted CT26 colon cancer model in mice. 2A: Changes in tumor volume in treated tumors; 2B: Changes in tumor volume in untreated tumors; 2C: Changes in body weight of mice; 2D: Changes in tumor volume in mice with/without immunization.

Figure 3 illustrates the immune memory of representative compounds of the present invention in mice with/without immunization. 3A: Immune memory in immunocompetent mice; 3B: Immune memory in immunodeficient mice.

Figure 4 illustrates the hepatocyte metabolic properties of representative compounds of the invention. 4A: Metabolic stability in hepatocytes; 4B: Identification of metabolites in hepatocytes.

## Description of the Invention

### Definitions

Unless otherwise stated, all technical and scientific terms used herein have the same meaning as commonly understood by one of ordinary skill in the art to which this invention belongs.

Unless otherwise stated, the nomenclature used in this application is based on the IUPAC systematic nomenclature. OpenEye Lexichem version 1.2.0, PerkinElmer E-notebook for Chemistry or Insight for Excel 2017 R2 have been used to generate the IUPAC chemical names.

Unless otherwise stated, any open valences appearing on carbon, oxygen, sulfur or nitrogen atoms in the structures herein indicate the presence of a hydrogen atom.

The term "immune system" has the ordinary meaning understood by those skilled in the art, and refers to molecules, substances (such as body fluids), anatomical structures (such as cells, tissues or organs), and physiological processes as a whole or any one or more components, which are related to preventing infections in vivo, protecting the body during infections or diseases, and/or helping the body recover after infections or diseases.

The term "disease associated with or mediated by an immune response" refers to a disease associated with or mediated by a defense response of the body's immune system against exotic components or mutated autogenous components. For the purposes of the present invention, "disease associated with or mediated by an immune response" specifically refers to a state in a human or animal or a disease state caused by it where the function of the immune system is weakened, inactivated or otherwise compromised, or where the function of one or more immune components is weakened, inactivated or otherwise compromised, especially a disease which can be alleviated by inducing an immune response through STING pathway.

The term "STING" is an abbreviation for "stimulator of interferon genes". STING is a transmembrane protein receptor in human, and activation of STING by cyclic dinucleotides (CDNs) leads to activation of IRF3 and NF-κB pathways, thus leading to the induction of type I interferons and pro-inflammatory cytokines, respectively. The term "STING agonist" refers to any substance that activates STING in vitro or in vivo to elicit a physiological response.

The term "disease associated with or mediated by STING" refers to a disease which can be alleviated by inducing an immune response through STING pathway, i.e., a disease in which activation of STING will reduce its incidence and decrease or eliminate disease symptoms, including but not limited to inflammation, allergic or autoimmune

diseases, infectious diseases or cancers etc. For the purposes of the present invention, "diseases associated with or mediated by STING" is preferably selected from tumors or cancers.

The term "hyperproliferative disease", "tumor" or "cancer" refers to a physiological condition in a subject characterized by uncontrolled or unregulated cell growth or death, including solid tumors and hematogenous tumors, whether malignant or benign, including but not limited to brain, skin, bladder, ovary, breast, stomach, pancreas, prostate, colon, blood, lung, and bone cancers. Examples of the above cancer types include neuroblastoma, intestinal cancers such as rectal cancer, colon cancer, familial adenomatous polyposis carcinoma, and hereditary nonlymphocytic colorectal cancer, esophageal cancer, lip cancer, laryngeal cancer, nasopharyngeal carcinoma, oral cancer, salivary gland cancer, peritoneal cancer, soft tissue sarcoma, urothelial carcinoma, hidradenocarcinoma, gastric cancer, adenocarcinoma, medullary thyroid cancer, papillary thyroid cancer, renal carcinoma, renal parenchymal carcinoma, ovarian cancer, cervical cancer, corpus carcinoma, endometrial cancer, pancreatic cancer, prostate cancer, testicular cancer, breast cancer (including HER2-negative breast cancer), urinary cancer, melanoma, brain tumors such as glioblastoma, astrocytoma, meningioma, medullary tumor, and peripheral neuroectodermal tumor, Hodgkin lymphoma, non-Hodgkin lymphoma, Burkitt's lymphoma, acute lymphoblastic leukemia (ALL), chronic lymphocytic leukemia (CLL), chronic myelogenous leukemia (CLL), and lymphocytic carcinoma, acute myelogenous leukaemia (AML), myelogenous leukemia (chronic myelogenous leukaemia (CML), adult T-cell lymphoma, diffuse lymphoma (DLBCL), hepatoma, multiple myeloma, seminoma, osteosarcoma, chondrosarcoma, anal canal carcinoma, adrenocortical carcinoma, chordoma, fallopian tube carcinoma, gastrointestinal stromal tumor, myeloproliferative disorder, mesothelioma, biliary tract carcinoma, Ewing's sarcoma, and other rare tumor types.

The term "therapeutic agent" refers to one or more substances administered to a human or animal to obtain certain therapeutic effects, including substances that prevent, cure or alleviate the effects of diseases and improve health conditions. Therapeutic agents of the present invention include not only the provided CDN compounds themselves, but also therapeutic agents that can be used in combination with the provided CDN compounds, including but not limited to chemotherapeutic agents, immune agents (especially immuno-oncology agents), vaccines, adjuvants and radiation therapy.

The term "chemotherapeutic agent" refers to one or more chemical substances that are administered to a human or animal to kill tumors, or to slow or arrest the growth of tumors, and/or to slow or arrest the division of cancer cells, and/or to prevent or retarting metastasis.

The term "immune agent" refers to any endogenous or exogenous substance that can interact with any one or more components of the immune system, including antibodies, antigens, vaccines and their components, nucleic acids, synthetic drugs, natural or synthetic organic compounds, cytokines, natural or modified cells, synthetic analogs thereof and/or fragments thereof.

The term "immunotherapy" refers to any medical treatment in which one or more components of the immune system of a human or animal is deliberately modulated in order to directly or indirectly obtain certain therapeutic benefits, including systemic and/or local effects as well as prophylactic and/or therapeutic effects. Immunotherapy may administer one or more immunizing agents, alone or in any combination, whether systemically, topically, or in combination, to human and animal subjects by any route, e.g., oral, intravenous, dermal, injectable, inhaled etc.

The term "vaccine" refers to a biological preparation administered to a human or animal to elicit or enhance a specific immune system response and/or protect against one or more antigens in the human or animal.

The term "adjuvant" refers to a secondary therapeutic substance administered in any order with the primary therapeutic substance to achieve certain complementary, synergistic or other beneficial effects that cannot be achieved with the primary therapeutic substance alone. Adjuvants can be used with vaccines, chemotherapies or other therapeutic substances to enhance the efficacy of the primary therapeutic substance, reduce the toxic side effects of the primary therapeutic substance, or provide certain protection to subjects receiving the primary therapeutic substance, such as but not limited to improving the function of the immune system.

The term "cytotoxic agent" or "apoptosis inducer" or similar expressions as used herein refers to an active agent useful in the treatment of abnormal and uncontrolled development and growth of cells. For the purposes of the present invention, "cytotoxic agent" especially refers to nucleoside anti-metabolic cytotoxic agents or antiviral agents, including but not limited to cytarabine, azacitidine, floxuridine, deoxyuridine, enocitabine, doxifluridine, pentostatin, fludarabine, cladribine, gemcitabine, capecitabine, clofarabine, nelarabine, trifluorothymidine, 8-chloroadenosine, triciribine, forodesine, 5-fluorodeoxycytidine, ribavirin or acadesine.

The term "multifunctional active agent" used herein refers to the compounds of the present invention which can exert multiple functions in subjects based on their unique structural design, namely exhibit both immunotherapeutic activity and cytotoxic therapeutic activity, including but not limited to activating the immune system by activating STING signaling pathway so as to exert anti-tumor and anti-viral replication functions, causing tumor cell death or preventing viral replication by releasing cytotoxic agents, continuously activating STING by releasing tumor DNA so as to kill tumor cells, releasing tumor neoantigens to induce an antibody-antigen response so as to provide the ability of "immunological memory" or persistent immunity to tumors.

The term "treatment" or "treating" of a disease includes inhibiting the disease state, i.e. arresting the development of the disease state or its clinical symptoms, or ameliorating the disease state, i.e. causing temporary or permanent regression of the disease state or its clinical symptoms.

The term "prevention" or "preventing" of a disease means preventing the development of clinical symptoms of the disease state in subjects who may be exposed to or susceptible to the disease state but have not yet experienced or exhibited the symptoms of the disease state.

The term "therapeutically effective amount" means an amount of the compound or molecule of the invention that (i) treats or prevents a particular disease, condition or disorder, (ii) alleviates, ameliorates or eliminates one or more symptoms of the particular disease, condition or disorder, or (iii) preventing or delaying the onset of one or more symptoms of the particular disease, condition or disorder described herein. A therapeutically effective amount will vary depending on the compound, the condition being treated, the severity of the condition being treated, the age and relative health of the subject, the route and form of administration, the judgment of the attending physician or veterinarian and other factors.

The term "subject", "individual" or "patient" as used herein refers to a vertebrate animal. In certain embodiments, the vertebrate is a mammal. Mammals include but are not limited to, farm animals (such as cows), sport animals, pets (such as guinea pigs, cats, dogs, rabbits, and horses), primates, mice, and rats. In a preferred embodiment, the mammal is a human.

The terms "pharmaceutical composition" and "pharmaceutical formulation" (or "formulation") are used interchangeably and refers to a mixture or solution comprising a therapeutically effective amount of active pharmaceutical ingredient(s) and pharmaceutically acceptable excipient(s) to be administered to a mammal (e.g. a human) in need thereof.

The term "pharmaceutical combination" means that the compounds of the present invention can be combined with other active agents to achieve the purposes of the present invention. The other active agent may be one or more additional compounds of the invention, or may be a second or additional (e.g. a third) compound which is compatible with the compounds of the invention, i.e. does not adversely affect each other, or has complementary activities. Such active agents are suitably present in combination in amounts effective to achieve the intended purpose. The other active agent may be co-administered with the compound of the present invention in a single pharmaceutical composition, or administered separately from the compound of the present invention in separate discrete units, either simultaneously or sequentially when administered separately.

The term "pharmaceutically acceptable" means the attribute of a material useful in the preparation of a pharmaceutical composition, which is generally safe, nontoxic, neither biologically nor otherwise undesirable, and acceptable for veterinary as well as human pharmaceutical use.

The terms "pharmaceutically acceptable excipient", "pharmaceutically acceptable carrier" and "therapeutically inert excipient" can be used interchangeably and refer to any pharmaceutically acceptable ingredient in a pharmaceutical composition which is not therapeutically active and non-toxic to the subject to which it is administered, such as disintegrants, binders, fillers, solvents, buffers, tonicity agents, stabilizers, antioxidants, surfactants, carriers, diluents or lubricants for the formulation of pharmaceutical products.

The term "pharmaceutically acceptable salt" as used herein refers to a salt of a compound of the present invention that is pharmaceutically acceptable and possesses the desired pharmacological activity of the parent compound. Specifically, such salts are non-toxic and may be inorganic acid addition salts or organic acid addition salts and base addition salts, including but not limited to: (1) acid addition salts formed with inorganic acids, such as hydrochloric acid, hydrobromic acid, sulfuric acid, nitric acid, phosphoric acid, etc.; or acid addition salts formed with organic acids such as acetic acid, propionic acid, caproic acid, glycolic acid, pyruvic acid, lactic acid, malonic acid, butanedioic acid, malic acid, maleic acid, fumaric acid, tartaric acid, citric acid, benzoic acid, cinnamic acid, mandelic acid, methanesulfonic acid, ethanesulfonic acid, benzenesulfonic acid, 2-naphthalenesulfonic acid, 4-toluenesulfonic acid, camphorsulfonic acid, glucoheptanoic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, etc.; or (2) salts formed when the acidic protons present in the parent compound are replaced by metal ions such as alkali metal ions, alkaline earth metal ions or aluminum ions, or coordinated with organic bases such as ethanolamine, diethanolamine, triethanolamine, N-methylglucamine. General principles and techniques for preparing pharmaceutically acceptable salts are known to those skilled in the art, such as those described in Berge et al., Pharm ScL, 66, 1-19. (1977).

The term "pharmaceutically acceptable prodrug" as used herein refers to a compound that has a cleavable group and becomes a pharmaceutically active compound of the present invention in vivo by solvolysis or under physiological conditions, specifically, prodrugs include those that can be oxidized, reduced, ammoniated, deaminated, hydroxylated, dehydroxylated, hydrolyzed, dehydrolyzed, alkylated, dealkylated, acylated, deacylated, phosphorylated or dephosphorylated to yield the active compound, including derivatives of the compounds of the invention. Various forms of prodrugs are well known in the art and suitable prodrug moieties are described, for example, in "Prodrugs and Targeted Delivery", J. Rautico, Ed., John Wiley & Sons, 2011.

Prodrugs of the CDN compounds described herein generally can increase the activity, bioavailability or stability

of the compounds. Generally, alkylation, acylation or other lipophilic modification of the phosphate moiety or use of other analogs of the nucleotide will help to increase the stability of the nucleotide.

The term "solvate" as used herein refers to a solvent addition form comprising stoichiometric or non-stoichiometric solvents, including, for example, solvates with water, such as hydrates, or with organic solvents, such as methanol, ethanol or acetonitrile, i.e. as methanolate, ethanolate or acetonitrile solvate, respectively; or in any polymorphic form. It should be understood that such solvates of the compounds of the invention also include solvates of the pharmaceutically acceptable salts of the compounds of the invention.

The term "isotopic variant" as used herein refers to a compound in which one or more atoms constituting the compound are replaced by an atom having an atomic mass or mass number different from that normally found in nature. Examples of isotopes that may be incorporated on one or more atoms of the compounds of the invention include, for example, $^{2}H$, $^{3}H$, $^{13}C$, $^{14}C$, $^{15}N$, $^{17}O$, $^{18}O$, $^{31}P$, $^{32}P$, $^{35}S$, and $^{18}F$, so as to form the isotopic variants of the compounds of the invention, which, whether radioactive or not, are intended to be encompassed within the scope of the present invention. In some embodiments, the incorporated isotope is 2H (deuterium); in other embodiments, the incorporated isotope is 3H (tritium).

The term "stereoisomer" as used herein refers to isomers formed due to at least one asymmetric center. In compounds with one or more (e.g., 1, 2, 3 or 4) asymmetric centers, racemic mixtures, single enantiomers, diastereomeric mixtures and single diastereoisomers may exist. Similarly, the compounds of the present invention may exist as mixtures of two or more different structural forms in rapid equilibrium (commonly referred to as tautomers). It should be understood that the scope of this application encompasses all such isomers or mixtures thereof in any ratio (e.g., 60%, 65%, 70%, 75%, 80%, 85%, 90%, 95%, 96%, 97%, 98%, 99%).

The compounds of the present invention may possess one or more asymmetric centers and thus may be prepared as (R)-or (S)-stereoisomers individually or as mixtures thereof. The " 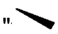 " or " 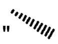 " used in the structural formulas or structural fragments of the compounds herein indicates the configuration of the asymmetric center, i.e., the chiral center. Correspondingly, in the naming of the compounds or intermediates provided by the present invention, R or S represents the configuration of the chiral center. Those skilled in the art will appreciate that the phosphorothioate linkages in the compounds of the present invention are inherently chiral and may each exist in R or S configuration, so that it is possible for the two phosphorothioate linkages to be in the form of R,R, S,S, S,R and R,S. The present invention encompasses compounds of the invention and embodiments thereof in substantially pure form or in mixtures, and compounds containing two phosphorothioate linkages are preferably in substantially pure form of R,R, S,S, S,R and R,S stereoisomers, particularly preferably substantially pure R,R stereoisomers, i.e. both the phosphorus atoms have the R configuration. Their absolute configuration assignments can be carried out according to methods in literatures (Zhao et al. Nucleosides, Nucleotides and Nucleic Acids 2009, 289, 352-378; Knouse et al. Science 2018, 361, 1234). It should be noted that the erroreous designation of the configuration due to the error in literature methods does not affect the actual configuration of the compounds of the present invention.

The term "substantially pure" as used herein with respect to CDN, refers to that a certain stereoconfiguration form is at least 75% pure relative to other possible stereochemical configurations at the chiral centers indicated in the above figure. In preferred embodiments, a substantially pure CDN is at least 85% pure, at least 90% pure, at least 95% pure, at least 97% pure, and at least 99% pure. Substantially pure CDN preparations of the present invention are "stereochemically pure", in the sense that all CDNs within the preparation have a specific stereochemical configuration at these chiral centers, and are not intended to indicate all CDNs with a particular stereochemical configuration at the centers within the preparation are otherwise identical. For example, a substantially pure CDN R,R cGAMP phosphorothioate formulation may comprise a combination of R,R c-di-GMP phosphorothioate and R,R c-di-AMP phosphorothioate and still be substantially pure cyclic purine dinucleotide preparations.

The term "protecting group" as used herein refers to a group that selectively blocks a reactive site in a polyfunctional compound such that the chemical reaction can be selectively performed on another unprotected reactive site in the sense usually associated with it in synthetic chemistry. Protecting groups can be removed at an appropriate timing. Exemplary protecting groups include amino protecting groups, including but not limited to, TBS (tert-butyldimethylsilyl), DMTr (bis(4-methoxyphenyl)benzyl), Bz (benzoyl), $^{i}$-BuCO (isobutyryl), benzyl, benzyloxycarbonyl (carbonylbenzyloxy, CBZ), Fmoc (9-fluorenylmethoxycarbonyl), p-methoxybenzyloxycarbonyl, p-nitrobenzyloxycarbonyl, tert-butoxycarbonyl (BOC) and trifluoroacetyl; and hydroxyl protecting groups, including but not limited to ester and ether forming groups, especially tetrahydropyranyloxy, Bz (benzoyl), $^{i}$-BuCO (isobutyryl), DMTr (bis(4-methoxyphenyl)benzyl), acetoxy, carbamoyloxy, benzyl and silyl ethers such as TBS (tert-butyldimethylsilyl), TBDPS (tert-butyldiphenylsilyl). Additional examples of these groups are found in T.W. Greene and P.G.M. Wuts, "Greene's protective groups in organic synthesis", 5th edition. John Wiley & Sons., Inc., Hoboken, New Jersey, 2014.

The term "deprotection" refers to the process of removing a protecting group after a selective reaction is complete. Deprotecting agents include acids, bases or hydrogen, especially potassium or sodium carbonate, lithium hydroxide in alcohol, zinc in methanol, acetic acid, trifluoroacetic acid, palladium catalysts or boron tribromide.

The term "alkyl" as used herein refers to a straight or branched aliphatic hydrocarbon group having the designated number of carbon atoms. Specifically, the alkyl group can have 1 to 14, 1 to 12, 1 to 10, 1 to 8, 1 to 7, 1 to 6, 1 to 5, 1 to 4, 1 to 3 or 1 to 2 carbon atoms. Examples of suitable $C_{1-14}$ alkyl groups include but are not limited to, methyl, ethyl, n-propyl, isopropyl, n-butyl, isobutyl, sec-butyl, tert-butyl, n-pentyl, isopentyl, neopentyl, n-hexyl, isohexyl, dimethylmethyl, dipropylmethyl, ethylbutylmethyl, diethylmethyl, methylethylmethyl, ethylpropylmethyl, diethylethyl, diethylpropyl, dipropylethyl, etc. Particular alkyl groups have 1 to 7 carbon atoms, e.g., 1 to 6 carbon atoms, 1 to 4 carbon atoms.

The term "halo" or "halogen" as used herein refers to fluorine (F), chlorine (Cl), bromine (Br) and iodine (I). Particular halo is fluoro or chloro.

For clarity, the term "guanine

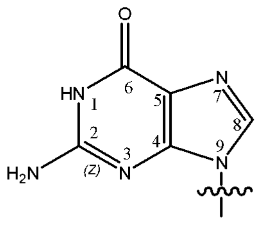

”

as used herein can also be expressed as

“ 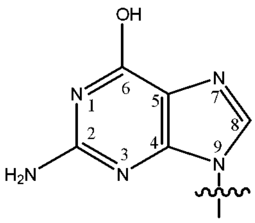 ”.

Phosphorothioate groups used in the formulas of compounds of the invention can be displayed as

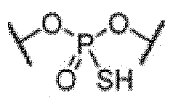 or 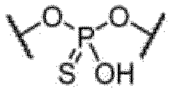 .

**Compounds of the invention**

Unless otherwise indicated, the terms "compounds described herein", "compounds of the invention" and "compounds of the present invention", etc., as used throughout this application, encompass the compounds of formula (X), formula (Y) and specific embodiments formulas (I), (II), (III) and (IV), their respective subformula embodiments and their specific or preferred embodiments, their stereoisomers, tautomers, racemates, stable isotopic variants, pharmaceutically acceptable salts or solvates, and pharmaceutically acceptable prodrugs, each as described in the definition section above. The compounds of the present invention may be isolated as a mixture of isomers or as individual isomers which may be prepared by resolution of racemates, e.g., by chromatography or fractional crystallization, or synthesized from optically active starting materials. Similarly, the reference to "intermediate" herein, no matter whether it is claimed or not, is intended to encompass its free form and the above-mentioned derivative forms, if the context allows.

Preferably, the compounds of the present invention are in free form or pharmaceutically acceptable salts or solvates thereof; most preferably in free form or pharmaceutically acceptable salts thereof.

Certain compounds of the present invention may exist in polymorphic or amorphous forms which are also within the scope of the present invention. When in solid crystalline form, the compounds of the invention may be in the form of co-crystals with another chemical entity, and this specification includes all such co-crystals.

Specifically, in one aspect, the present invention provides a cyclic dinucleotide compound of the following formula, a stereoisomer, a tautomer, a stable isotopic variant, a pharmaceutically acceptable salt, a prodrug or a solvate thereof:

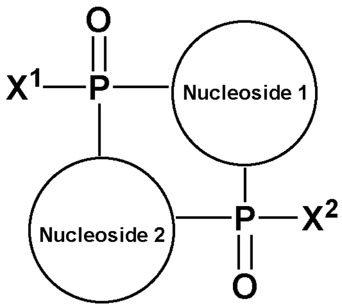

(X),

e.g.,

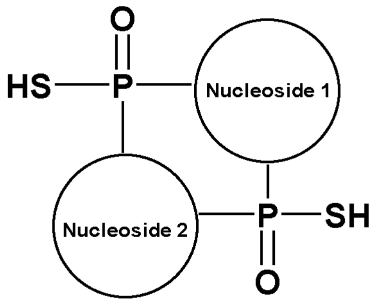

wherein $X_1$ and $X_2$ are each independently selected from -OH and-SH; and at least one of the two nucleosides of the cyclic dinucleotide has cytotoxic or antiviral effect, preferably those antimetabolic anticancer drugs, namely nucleoside anticancer drugs or their derivatives, including but not limited to enocitabine, fludarabine, cytarabine, nelarabine, doxifluridine, capecitabine, azacytidine, pentostatin, cladribine, gemcitabine and clofarabine, more preferably cladribine, gemcitabine and clofarabine or derivatives thereof. The nucleoside anticancer drug derivatives mentioned here refer to the compounds retaining anti-cancer cytotoxicity and derived from the substitution of hydrogen atoms or atomic groups in the compound structure with other atoms or atomic groups.

In specific embodiments, the present invention provides a cyclic dinucleotide compound of the following formula (Y),

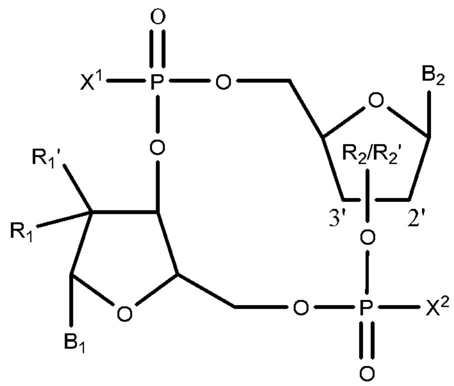

(Y)

wherein $X^1$ and $X^2$ are each independently selected from -OH and -SH;

$B_1$ is adenine

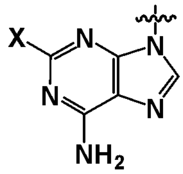

substituted by X, wherein X is selected from Cl, F or -NHC$_{1-6}$ alkyl; or cytosine C$_{1-14}$ alkyl;

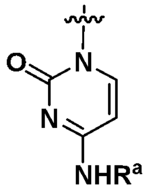

optionally substituted by $R^a$, wherein $R^a$ is selected from H or -C(O)-C$_{1-14}$ alkyl;
$R_1$ and $R_1$' are each independently selected from H, F or -OH;
$B_2$ is selected from adenine

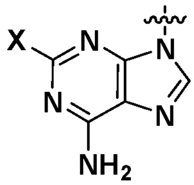

optionally substituted by X, wherein X is selected from H, F or Cl; cytosine

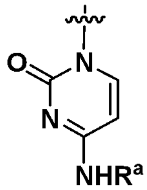

optionally substituted by $R^a$, wherein $R^a$ is selected from H or -C(O)-C$_{1-14}$ alkyl; or guanine

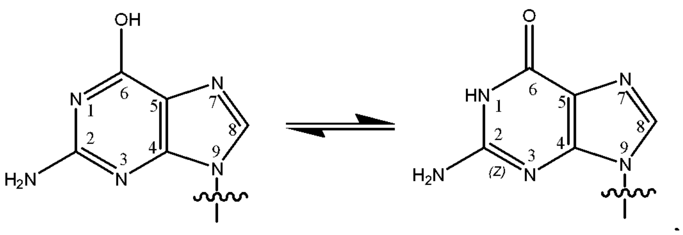

wherein OH is optionally substituted by C$_{1-6}$ alkyl;

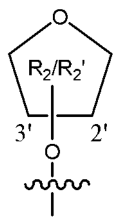

denotes the phosphate linkage is connected to the 2' position or the 3' position of the pentose, and the position not cyclized with the phosphate is substituted by $R^2$ and $R_2$'; and

$R_2$ and $R_2$' are each independently selected from H, -OH or F;

with the proviso that when one of $B_1$ or $B_2$ is cytosine optionally substituted by $R^a$, the carbon atom adjacent to it on the pentose ring to which it is attached is substituted by two F;

or a stereoisomer, a tautomer, a stable isotopic variant, a pharmaceutically acceptable salt, a prodrug or a solvate thereof.

In specific embodiments, the compound of formula (Y) may be of

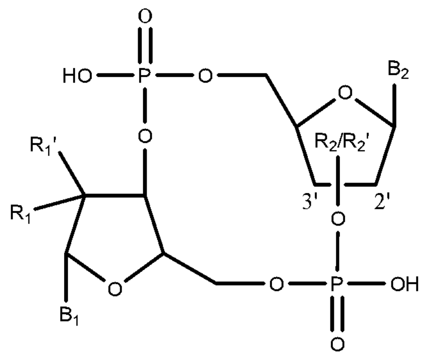
(I),

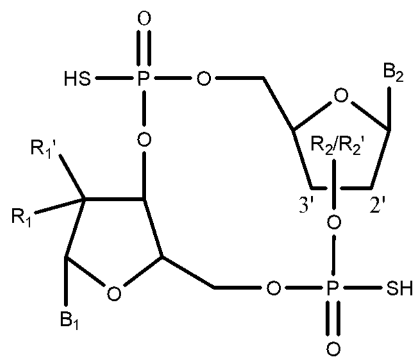
(II)

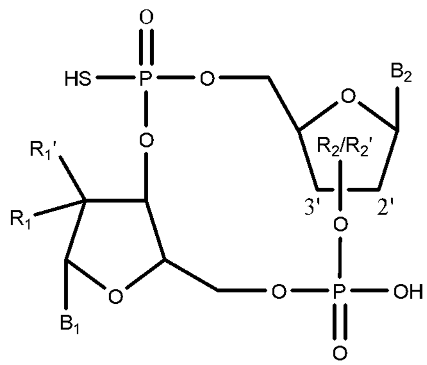
(III) or

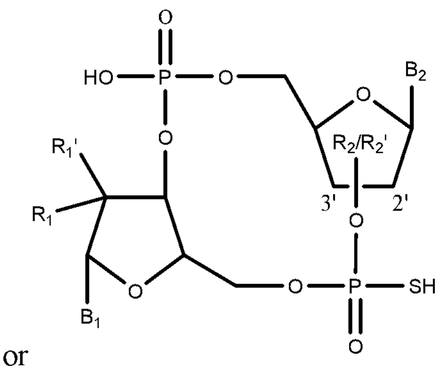
(IV),

wherein $B_1$, $B_2$, $R_1$, $R_1$', $R_2$, $R_2$' have the meanings as defined above for the compound of formula (Y).

In specific embodiments, the two phosphorothioate linkages (when present) in the compounds of the invention are in the R,R, S,S, S,R or R,S configuration or mixtures thereof. In a preferred embodiment, the two phosphorothioate linkages (when present) in the compounds of the invention are present in substantially pure form in the R,R, S,S, S,R or R,S configuration, particularly preferably in substantially pure in the R,R configuration.

In specific embodiments, the single phosphorothioate linkage present in the compounds of the invention may be present in the R or S configuration, preferably in the R configuration.

In one embodiment of the compound of formula (I), (II), (III) or (IV), $B_1$ is adenine

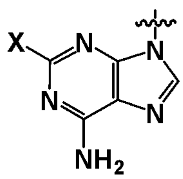

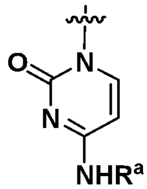

substituted by X, wherein X is selected from Cl or F, preferably Cl, or cytosine optionally substituted by $R^a$, wherein $R^a$ is selected from H or -C(O)-$C_{1-14}$ alkyl.

In one embodiment of the compound of formula (I), (II), (III) or (IV), $B_1$ is adenine

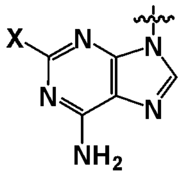

substituted by X, wherein X is -NH$C_{1-6}$ alkyl, for example -$NHCH_3$, -$NHCH_2CH_3$, -$NHCH_2CH_2CH_3$, preferably -$NHCH_3$.

In one embodiment of the compound of formula (I), (II), (III) or (IV), $B_1$ is cytosine

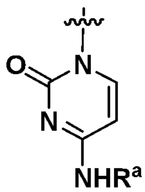

optionally substituted by $R^a$, wherein $R^a$ is selected from H or -C(O)-$C_{1-14}$ alkyl.

In one embodiment of the compound of formula (I), (II), (III) or (IV), $R_1$ and $R_1$' are both H, or one is H and the other is F, or both are F.

In one embodiment of the compound of formula (I), (II), (III) or (IV), $B_2$ is adenine

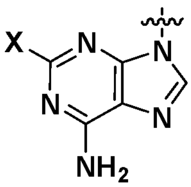

optionally substituted by X, wherein X is selected from H, F or Cl, preferably H or Cl.

In one embodiment of the compound of formula (I), (II), (III) or (IV), $B_2$ is cytosine

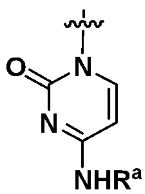

optionally substituted by $R^a$, wherein $R^a$ is selected from H or -C(O)-$C_{1-14}$ alkyl.

In one embodiment of the compound of formula (I), (II), (III) or (IV), $B_2$ is guanine

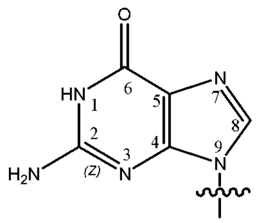

In one embodiment of the compound of formula (I), (II), (III) or (IV), $R_2$ and $R_2$' are both H, or one is H and the other is F, or one is H and the other is OH, or both are F.

In one embodiment of the compound of formula (I), (II), (III) or (IV), when $R^a$ is -C(O)$C_{1-14}$ alkyl, it can be, for example, -C(O)$C_{1-10}$ alkyl, -C(O)$C_{1-9}$ alkyl, -C(O)$C_{1-8}$ alkyl, -C(O)$C_{1-7}$ alkyl, -C(O)CH($C_{1-4}$ alkyl)$_2$, -C(O)CH($C_{1-3}$ alkyl)$_2$, -C(O)$CH_2$CH($C_{1-4}$ alkyl)$_2$, -C(O)$CH_2$CH($C_{1-3}$ alkyl)$_2$.

The compound of formula (I), (II), (III) or (IV) of the present invention also encompasses any combination of the above-mentioned embodiments and their preferred or exemplary embodiments.

In further specific embodiments, the present invention provides a cyclic dinucleotide compound of formula (Y) having the following general formula,

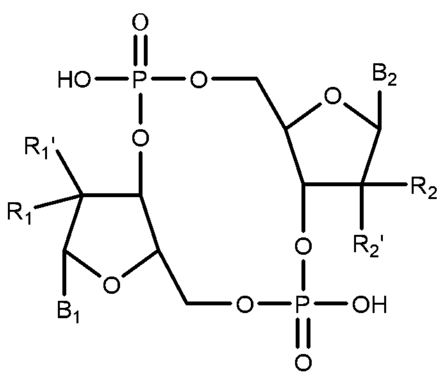
(I-a)

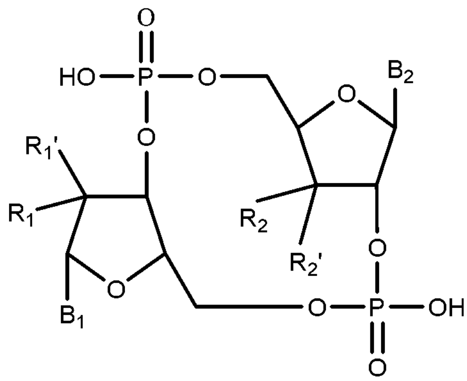
(I-b)

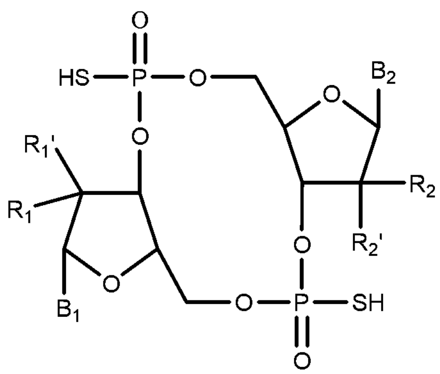
(II-a)

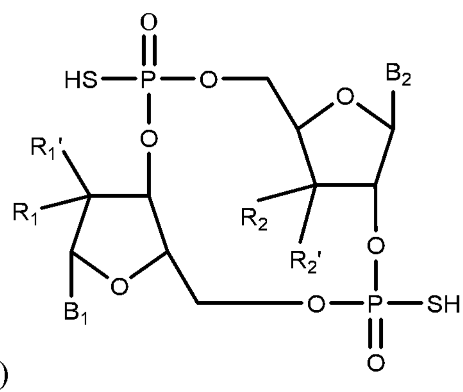
(II-b)

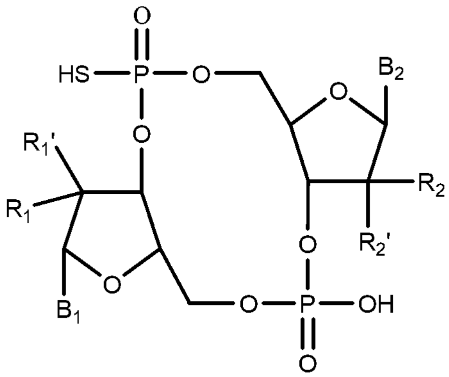

(III-a)

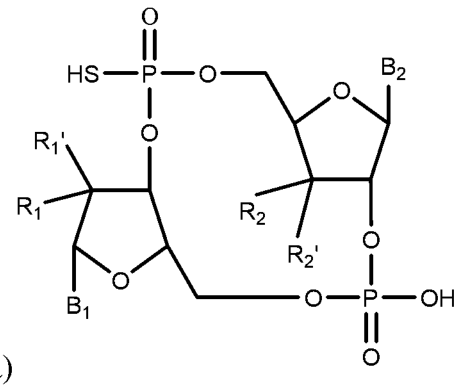

(III-b)

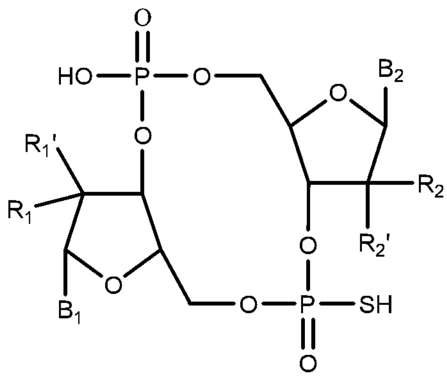

(IV-a)

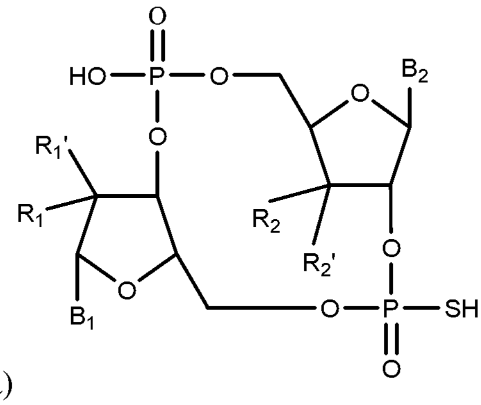

(IV-b)

wherein $B_1$, $B_2$, $R_1$, $R_1$', $R_2$, $R_2$' have the meanings as defined above for the compound of formula (I), (II), (III) or (IV) and specific embodiments thereof.

In one embodiment of the compound of formula (II-a) or formula (II-b), especially the compound of formula (II-a), $B_1$ is

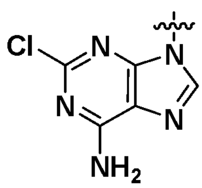

,

and $B_2$ is

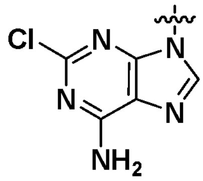

,

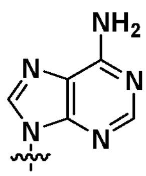

or

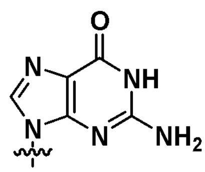

;

in s specific such embodiment, both $R_1$ and $R_1$' are H; in another specific such embodiment, one of $R_1$ and $R_1$' is H, and the other is F. Further, in each of such specific embodiments, both $R_2$ and $R_2$' are H, or one of them is H and the other is F, or one of them is H and the other is OH.

In one embodiment of the compound of formula (II-a) or formula (II-b), especially the compound of formula (II-a), both $B_1$ and $B_2$ are cytosine embodiment, $R_1$, $R_1$', $R_2$ and $R_2$' are all F.

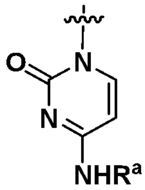

optionally substituted by $R^a$; in a specific such

In one embodiment of the compound of formula (II-a) or formula (II-b), especially the compound of formula (II-b), $B_1$ is

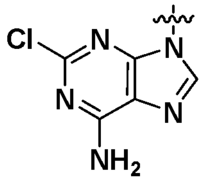

,

and $B_2$ is

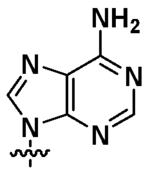

or

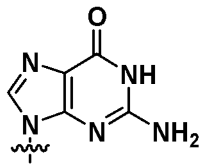

;

in a specific such embodiment, both $R_1$ and $R_1$' are H; in another specific such embodiment, one of $R_1$ and $R_1$' is H and the other is F. Further, in each of such embodiments, $R_2$ and $R_2$' are both H, or one is H and the other is F, or one is H and the other is OH, preferably one is H and the other is OH.

In one embodiment of the compound of formula (II-a) or formula (II-b), especially the compound of formula (II-b), $B_1$ is cytosine

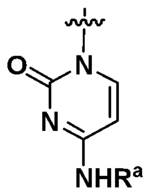

optionally substituted by $R^a$, and $B_2$ is

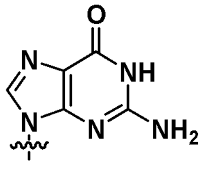

;

in a specific such embodiment, $R_1$ and $R_1$' are both F. Further, in each of such embodiments, $R_2$ and $R_2$' are both H, or one is H and the other is F, or one is H and the other is OH, preferably one is H and the other is OH.

In one embodiment of the compound of formula (II-a) or formula (II-b), $R^a$ is H, or $-C(O)C_{1-10}$ alkyl, for example $-C(O)C_{1-9}$ alkyl, $-C(O)C_{1-8}$ alkyl, $-C(O)C_{1-7}$ alkyl, $-C(O)CH(C_{1-4}$ alkyl$)_2$, $-C(O)CH(C_{1-3}$ alkyl$)_2$, $-C(O)CH_2CH(C_{1-4}$ alkyl$)_2$, $-C(O)CH_2CH(C_{1-3}$ alkyl$)_2$.

In one embodiment of the compound of formula (II-a) or formula (II-b), $B_1$ is

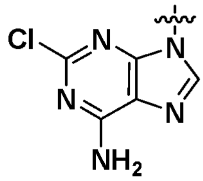

,

$R_1$ and $R_1$' are both H.

In one embodiment of the compound of formula (II-a) or formula (II-b), $B_1$ is

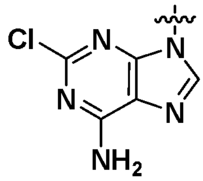

,

one of $R_1$ and $R_1$' is H, and the other is F; in a specific such embodiment, F and $B_1$ are on the same side of the ribose.

In one embodiment of the compound of formula (II-a) or formula (II-b), especially the compound of formula (II-b), $B_1$ is

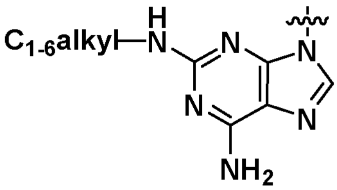

,

preferably

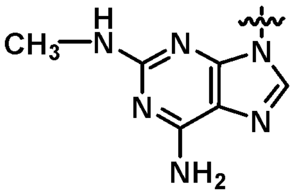

,

and $R_1$ and $R_1$' are both H, or one of $R_1$ and $R_1$' is H and the other is F, preferably one of $R_1$ and $R_1$' is H and the other is F.

In one embodiment of the compound of formula (II-a) or formula (II-b), $B_1$ is cytosine

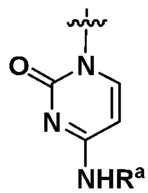

optionally substituted by $R^a$, wherein $R^a$ is selected from H, $R_1$ and $R_1$' are both F.

In one embodiment of the compound of formula (II-a) or formula (II-b), B is cytosine

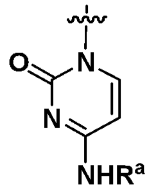

optionally substituted by $R^a$, wherein $R^a$ is -C(O)$C_{1-10}$ alkyl, preferably -C(O)CH($C_{1-4}$ alkyl)$_2$, both $R_1$ and $R_1$' are F.

In exemplary embodiments, $R^a$ is -C(O)CH($CH_3$)$_2$, -C(O)CH($CH_2CH_3$)$_2$, -C(O)CH($CH_3$)($CH_2CH_3$), -C(O)CH($CH_2CH_2CH_3$)$_2$, -C(O)CH($CH_2CH_3$)($CH_2CH_2CH_3$), -C(O)$CH_2$CH($CH_2CH_3$)($CH_2CH_2CH_3$), C(O)$CH_2$CH($CH_2CH_2CH_3$)$_2$, preferably $R^a$ is C(O)CH($CH_2CH_2CH_3$)$_2$.

In one embodiment of the compound of formula (II-a) or formula (II-b), $B_2$ is guanine

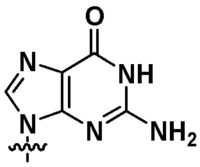

or adenine

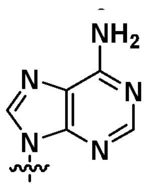

,

and one of $R_2$ and $R_2$' is H, and the other is selected from - OH or F.

In one embodiment of the compound of formula (II-a), $B_2$ is guanine

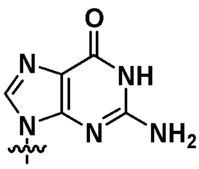

,

one of $R_2$ and $R_2$' is H, and the other is selected from -OH or F.

In one embodiment of the compound of formula (II-a), $B_2$ is adenine

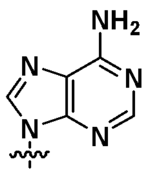

,

one of $R_2$ and $R_2$' is H, and the other is selected from -OH or F.

In one embodiment of the compound of formula (II-a), $B_2$ is adenine

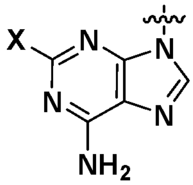

substituted by halogen X, wherein X is Cl; one of $R_2$ and $R_2$' is H, and the other is F, preferably F and $B_2$ is on the same side as the ribose.

In one embodiment of the compound of formula (II-a), $B_2$ is adenine

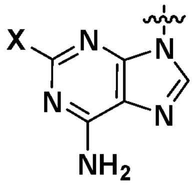

substituted by halogen X, wherein X is Cl; $R_2$ and $R_2$' are both H.

In one embodiment of the compound of formula (II-a), $B_2$ is cytosine

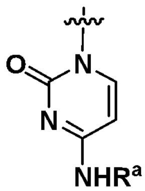

optionally substituted by $R^a$, wherein $R^a$ is selected from H, and both $R_1$ and $R_1$' are F.

In one embodiment of the compound of formula (II-a), $B_2$ is cytosine

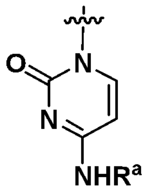

substituted by $R^a$, wherein $R^a$ is $-C(O)C_{1-10}$ alkyl, preferably $-C(O)CH(C_{1-4}$ alkyl$)_2$, $R_1$ and $R_1$' are all F.

In exemplary embodiments, $R^a$ is $-C(O)C(CH_3)_2$, $-C(O)CH(CH_2CH_3)_2$, $-C(O)CH(CH_3)(CH_2CH_3)$, $-C(O)CH(CH_2CH_2CH_3)_2$, $-C(O)CH(CH_2CH_3)(CH_2CH_2CH_3)$, $-C(O)CH_2CH(CH_2CH_3)(CH_2CH_2CH_3)$, $C(O)CH_2CH(CH_2CH_2CH_3)_2$, preferably $R^a$ is $C(O)CH(CH_2CH_2CH_3)_2$.

In one embodiment of the compound of formula (II-b), $B_2$ is guanine

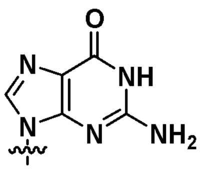

or adenine

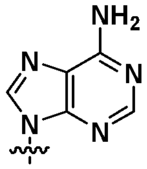
,

one of $R_2$ and $R_2$' is H, and the other is -OH.

In the above-mentioned compound of formula (II-a) or formula (II-b) and various specific embodiments thereof, the two phosphorothioate linkages are in R,R, S,S, S,R or R,S configuration or in mixture thereof; preferably in the R,R, S,S, S,R or R,S configuration in substantially pure form, particularly preferably in the substantially pure R,R configuration.

The specific embodiments and their preferred or exemplary embodiments given above for the compound of formula (II-a) or formula (II-b), are also applicable to the compound of formula (I-a) or formula (I-b), the compound of formula (III-a) or formula (III-b), and the compound of formula (IV-a) or formula (IV-b) respectively, that is, the present invention also encompasses the compound of formula (I-a) or formula (I-b), the compound of formula (III-a) or formula (III-b), the compound of formula (IV-a) or formula (IV-b) wherein each specific substituent takes the specific definitions given above for the compound of formula (II-a) or formula (II-b) or its specific embodiments respectively.

For example, in one embodiment of the compound of formula (I-a) or formula (I-b), formula (III-a) or formula (III-b), or formula (IV-a) or formula (IV-b), especially formula (I-b), formula (III-b) or formula (IV-b), $B_1$ is

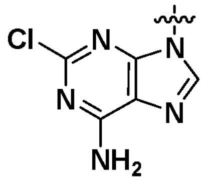
,

and $B_2$ is

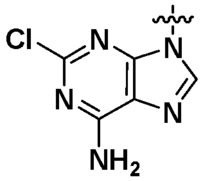
, 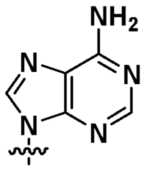 or 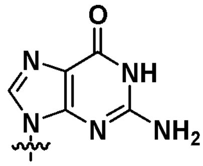 ,

preferably

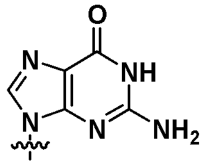
;

in a specific such embodiment, one of $R_1$ and $R_1$' is H and the other is F. Further, in each of these embodiments, one of $R_2$ and $R_2$' is H, and the other is OH.

The compound of formula (I-a)/(I-b), (II-a)/(II-b), (III-a)/(III-b) or (IV-a)/(IV-b) of the present invention, also encompasses any combination of the above-mentioned embodiments and their preferred or exemplary embodiments.

In still further specific embodiments, the present invention provides a cyclic dinucleotide compound of the following subformula,

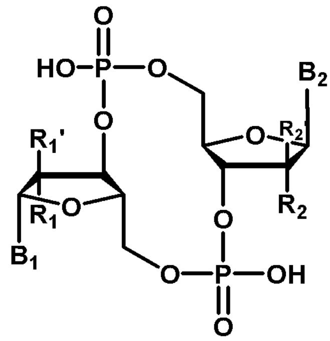 (I-a')

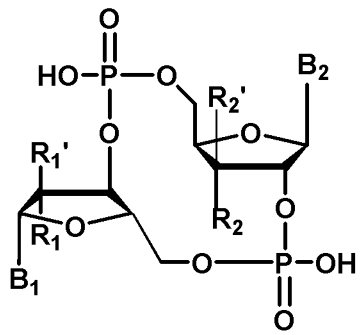 (I-b')

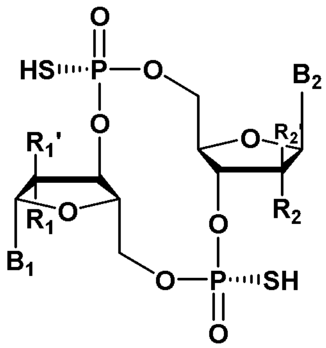 (II-a')

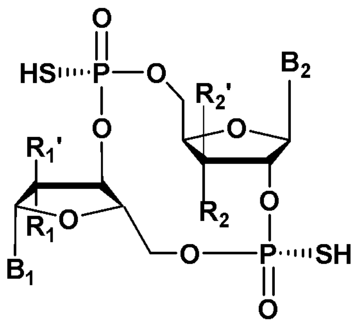 (II-b')

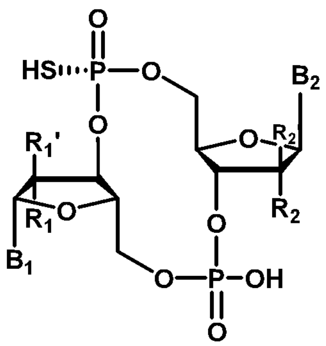 (III-a')

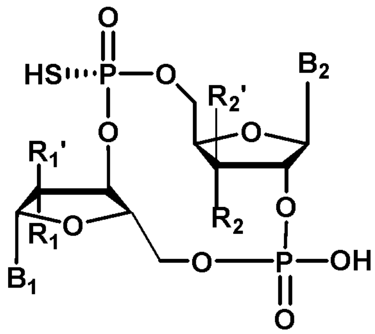 (III-b')

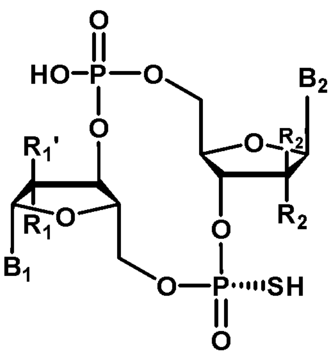 (IV-a')

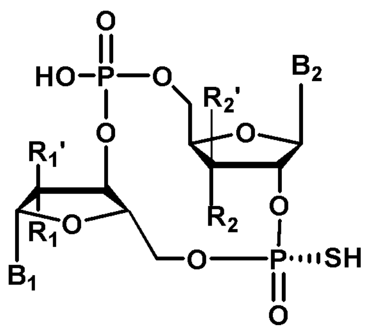 (IV-b')

wherein $B_1$, $B_2$, $R_1$, $R_1$', $R_2$, $R_2$' have the meanings as defined above for the compound of formula (I), (II), (III) or (IV) and specific embodiments thereof; more specifically have the meanings as defined above for the compound of formula (Ia)/(Ib), (II-a)/(II-b), (III-a)/(III-b) or (IV-a)/(IV-b) and specific embodiments thereof.

In one embodiment of the compound of formula (II-a') or formula (II-b'), especially the compound of formula (II-a'), $B_1$ is

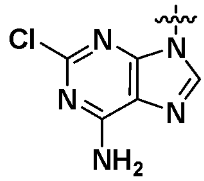

,

and $B_2$ is

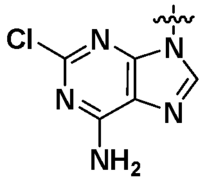

, 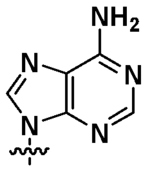 or 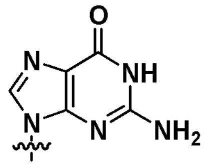 ;

In a specific such embodiment, both $R_1$ and $R_1$' are H; in another specific such embodiment, $R_1$ is F and $R_1$' is H, that is, F and $B_1$ are on the same side of ribose. Further, in each of the specific embodiments, $R_2$ and $R_2$' are both H, or $R_2$ is H and R2' is F, or $R_2$ is F and $R_2$' is H, or $R_2$ is OH and $R_2$' is H.

In one embodiment of the compound of formula (II-a') or formula (II-b'), especially the compound of formula (II-a'), both $B_1$ and $B_2$ are cytosine

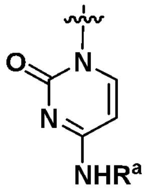

optionally substituted by $R^a$; in a specific such embodiment, $R_1$, $R_1$', $R_2$, $R_2$' are all F.

In one embodiment of the compound of formula (II-a') or formula (II-b'), especially the compound of formula (II-b'), $B_1$ is

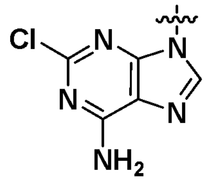

,

and $B_2$ is

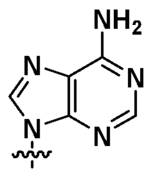

or 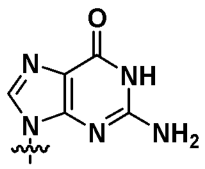 ;

in a specific such embodiment, both $R_1$ and $R_1$' are H; in another specific such embodiment, $R_1$ is F and $R_1$' is H, that is, F and $B_1$ are on the same side of ribose. Further, in each of these embodiments, $R_2$ and $R_2$' are both H, or $R_2$ is H and $R_2$' is F, or $R_2$ is F and $R_2$' is H, or $R_2$ is OH and $R_2$' is H; preferably $R_2$ is OH and $R_2$' is H.

In one embodiment of the compound of formula (II-a') or formula (II-b'), especially the compound of formula (II-b'), $B_1$ is cytosine

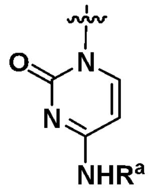

optionally substituted by $R^a$, and $B_2$ is

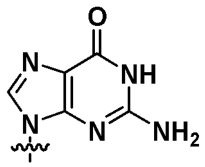

;

in a specific such embodiment, $R_1$ and $R_1$' are both F. Further, in each of these embodiments, $R_2$ and $R_2$' are both H, or $R_2$ is H and $R_2$' is F, or $R_2$ is F and $R_2$' is H, or $R_2$ is OH and $R_2$' is H; preferably $R_2$ is OH and $R_2$' is H.

In one embodiment of the compound of formula (II-a') or formula (II-b'), $R^a$ is H, or $-C(O)C_{1-10}$ alkyl, for example $-C(O)C_{1-9}$ alkyl, $-C(O)C_{1-8}$ alkyl, $-C(O)C_{1-7}$ alkyl, $-C(O)CH(C_{1-4}$ alkyl$)_2$, $-C(O)CH(C_{1-3}$ alkyl$)_2$, $-C(O)CH_2CH(C_{1-4}$ alkyl$)_2$, $-C(O)CH_2CH(C_{1-3}$ alkyl$)_2$.

In one embodiment of the compound of formula (II-a') or formula (II-b'), $B_1$ is

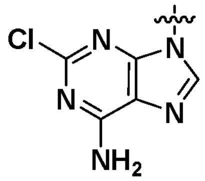

,

$R_1$ and $R_1$' are both H, thus the $B_1$ together with the ribose to which it is attached form the nucleoside antineoplastic drug cladribine.

In one embodiment of the compound of formula (II-a') or formula (II-b'), $B_1$ is

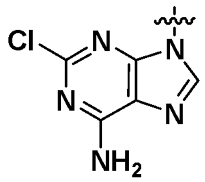

,

$R_1$ is F, $R_1$' is H, that is, F and $B_1$ are on the same side of ribose, thus the $B_1$ together with the ribose to which it is attached form the nucleoside antineoplastic drug clofarabine.

In one embodiment of the compound of formula (II-a') or formula (II-b'), especially the compound of formula (II-b'), $B_1$ is

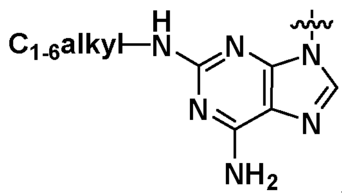

,

preferably

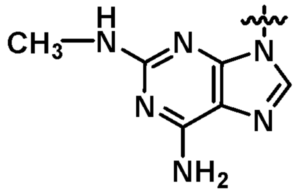

,

and $R_1$ and $R_1$' are both H, or $R_1$ is F and $R_1$' is H, preferably $R_1$ is F and $R_1$' is H.

In one embodiment of the compound of formula (II-a') or formula (II-b'), $B_1$ is cytosine

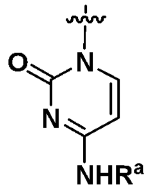

optionally substituted by $R^a$, wherein $R^a$ is selected from H, both $R_1$ and $R_1$' are F, thus this $B_1$ together with the ribose to which it is attached form the nucleoside antineoplastic drug gemcitabine.

In one embodiment of the compound of formula (II-a') or formula (II-b'), $B_1$ is cytosine

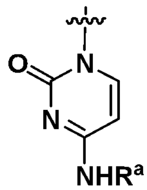

optionally substituted by $R^a$, wherein $R^a$ is $-C(O)C_{1-10}$ alkyl, preferably $-C(O)CH(C_{1-4}$ alkyl$)_2$, $R_1$ and $R_1$' are both F, thus this $B_1$ together with the ribose it is attached form the alkanoylated derivatives of the nucleoside antineoplastic drug gemcitabine. In exemplary embodiments, $R^a$ is $-C(O)CH(CH_3)_2$, $-C(O)CH(CH_2CH_3)_2$, $-C(O)CH(CH_3)(CH_2CH_3)$, $-C(O)CH(CH_2CH_2CH_3)_2$, $-C(O)CH(CH_2CH_3)(CH_2CH_2CH_3)$, $-C(O)CH_2CH(CH_2CH_3)(CH_2CH_2CH_3)$, $-C(O)CH_2CH(CH_2CH_2CH_3)_2$, preferably $R^a$ is $-C(O)CH(CH_2CH_2CH_3)_2$.

In one embodiment of the compound of formula (II-a'), $B_2$ is guanine

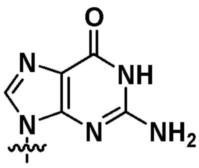

,

$R_2$' is H, and $R_2$ is selected from -OH or F.

In one embodiment of the compound of formula (II-a'), $B_2$ is adenine

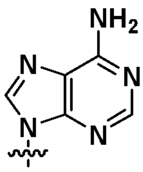

,

$R_2$' is H, and $R_2$ is selected from -OH or F.

In one embodiment of the compound of formula (II-a'), $B_2$ is adenine

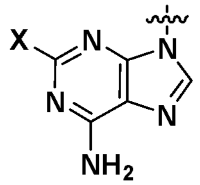

substituted by halogen X, wherein X is Cl, $R_2$ is H, $R_2$' is F, and F and $B_2$ are on the same side of the ribose, thus the $B_2$ together with the ribose it is attached form the nucleoside antineoplastic drug clofarabine.

In one embodiment of the compound of formula (II-a'), $B_2$ is adenine

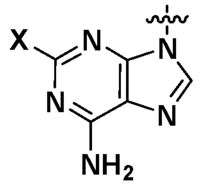

substituted by halogen X, wherein X is Cl, $R_2$ and $R_2$' are both H, thus the $B_2$ together with the ribose it is attached form the nucleoside antineoplastic drug cladribine.

In one embodiment of the compound of formula (II-a'), $B_2$ is cytosine

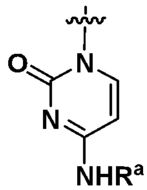

optionally substituted by $R^a$, wherein $R^a$ is selected from H, $R_1$ and $R_1$' are both F, thus the $B_2$ together with the ribose it is attached form the nucleoside antineoplastic drug gemcitabine.

In one embodiment of the compound of formula (II-a'), $B_2$ is cytosine

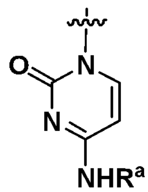

substituted by $R^a$, wherein $R^a$ is $-C(O)C_{1-10}$ alkyl, preferably $-C(O)CH(C_{1-4}$ alkyl$)_2$, $R_1$ and $R_1$' are both F, thus the $B_2$ together with the ribose it is attached form the alkanoylated derivatives of the nucleoside antineoplastic drug gemcitabine. In exemplary embodiments, $R^a$ is $-C(O)CH(CH_3)_2$, $-C(O)CH(CH_2CH_3)_2$, $-C(O)CH(CH_3)(CH_2CH_3)$, $-C(O)CH(CH_2CH_2CH_3)_2$, $-C(O)CH(CH_2CH_3)(CH_2CH_2CH_3)$, $-C(O)CH_2CH(CH_2CH_3)(CH_2CH_2CH_3)$, $-C(O)CH_2CH(CH_2CH_2CH_3)_2$, preferably $R^a$ is $-C(O)CH(CH_2CH_2CH_3)_2$.

In one embodiment of the compound of formula (II-b'), $B_2$ is guanine

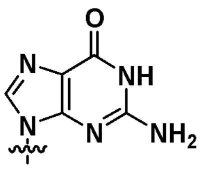

or adenine

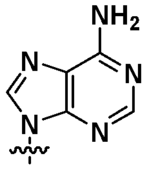

,

$R_2$' is H, $R_2$ is -OH.

The specific embodiments and their preferred or exemplary embodiments given above for the compound of formula (II-a') or formula (II-b'), are also applicable to the compound of formula (I-a') or formula (I-b'), the compound of formula (III-a') or formula (III-b'), and the compound of formula (IV-a') or formula (IV-b') respectively, that is, the present invention also encompasses the compound of formula (I-a') or formula (I-b'), the compound of formula (III-a') or formula (III-b'), the compound of formula (IV-a') or formula (IV-b') wherein each specific substituent takes the specific definitions given above for the compound of formula (II-a') or formula (II-b') or its specific embodiments respectively.

For example, in one embodiment of the compound of formula (I-a') or formula (I-b'), formula (III-a') or formula (III-b'), or formula (IV-a') or formula (IV-b'), especially formula (I-b'), formula (III-b') or formula (IV-b'), $B_1$ is

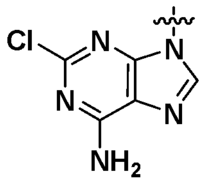

,

and $B_2$ is

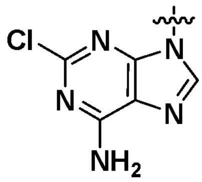

,

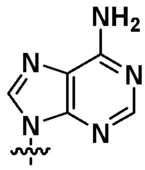

or

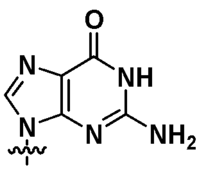

,

preferably

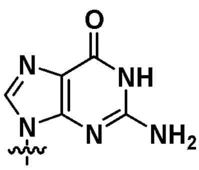

;

in a specific such embodiment, $R_1$ is F and $R_1$' is H.

Further, in each of these embodiments, one of $R_2$ is OH and $R_2$' is H.

It should be noted that, the compound of the present invention encompasses each of the above specific embodiments, and encompasses the embodiments resulting from any combination or subcombination of the above-mentioned specific embodiments, also the embodiments resulting from any combination of any of the above preferred or

exemplified embodiments.

Preferred specific embodiments of the compounds of the present invention include the following compounds, their stereoisomers, tautomers, stable isotopic variants, pharmaceutically acceptable salts or solvates,

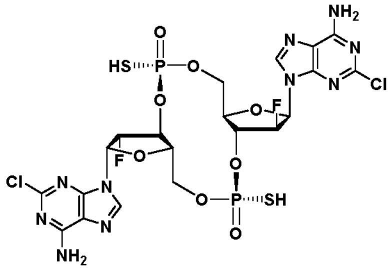

compound 1

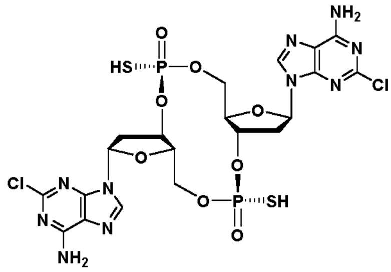

compound 2

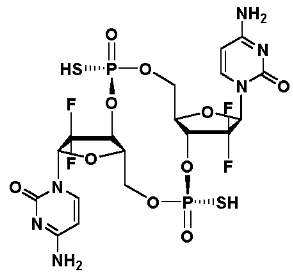

compound 3

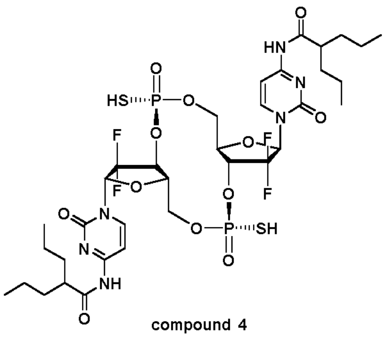

compound 4

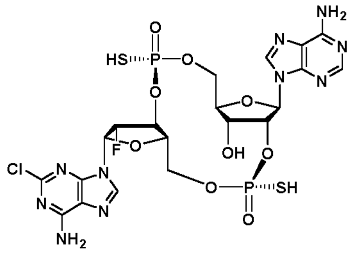

compound 5

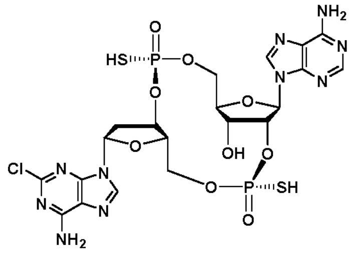

compound 6

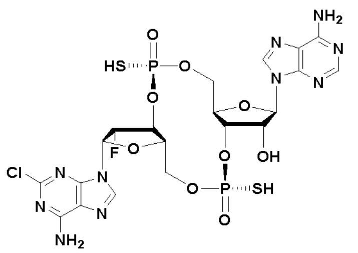

compound 7

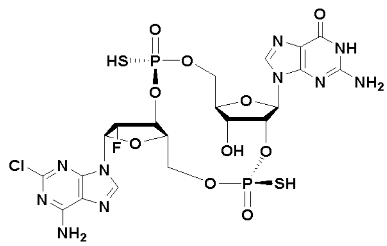

compound 8

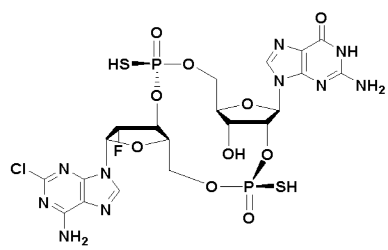

compound 9

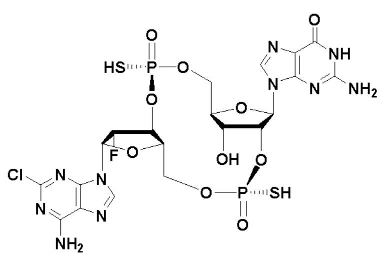

compound 10

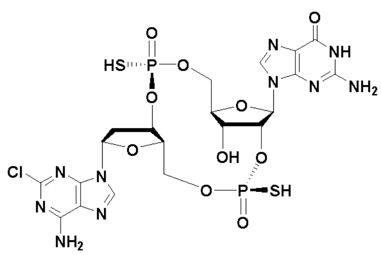

compound 11

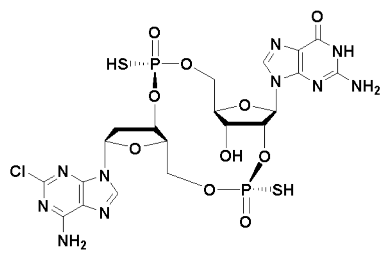

compound 12

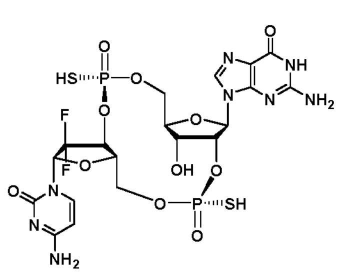

compound 13

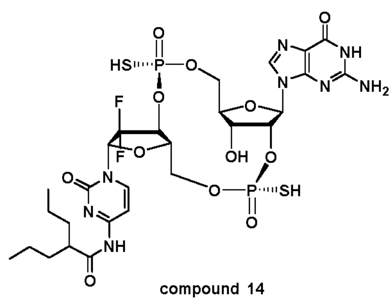

compound 14

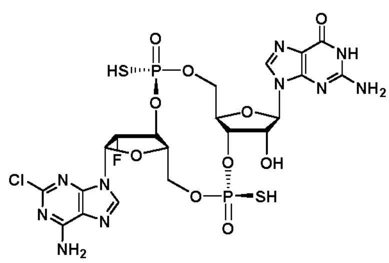

compound 15

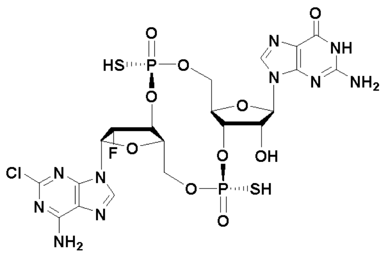

compound 16

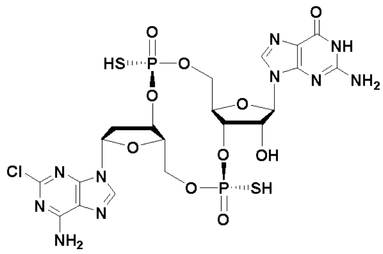

compound 17

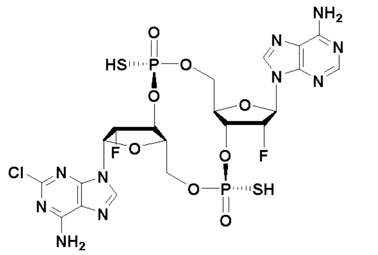

compound 18

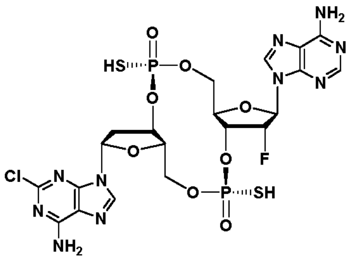

compound 19

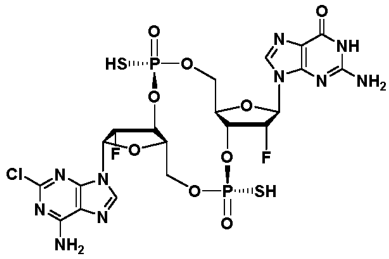

compound 20

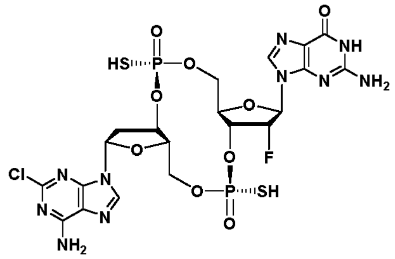

compound 21

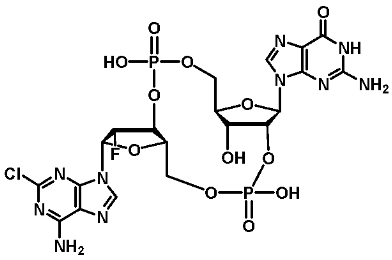

compound 22

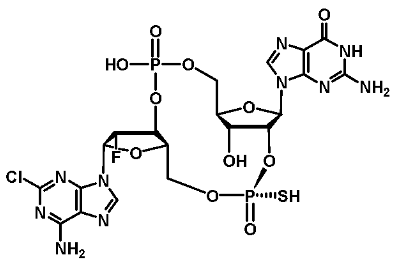

compound 23

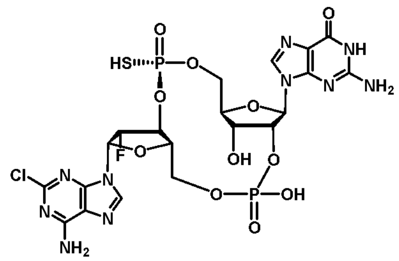

compound 24

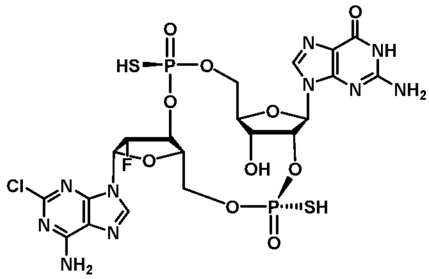

compound 25

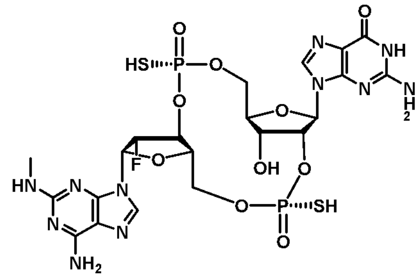

compound 26

**Pharmacological Activities and Beneficial Effects of the Compounds of the Invention**

It has been found through research that, the cyclic dinucleotide compounds provided by the present invention have the following pharmacological activities and beneficial effects:

- Effectively stimulating THP-1 cells to secrete IFN-β, indicating that they have a high affinity for STING receptor, can effectively activate STING, and induce the production of type I interferon;

- Effectively inhibiting the in vitro growth of mouse colorectal cancer cell line CT26, showing a toxic effect on tumor cells, preventing tumor cell division and proliferation;

- Showing an anti-tumor activity in the bilateral implanted tumor model of immune-competent CT26 syngeneic mice that is superior to that in the immune-deficient mouse model, indicating that the compouns have both the tumor immune activity by activating STING and the cytotoxicity which have produced additive and even synergistic antitumor

effect;

- Showing the immunological memory function in CT26 syngeneic mouse implanted tumor model, which can effectively prevent tumor recurrence;
- Demonstrating favorable pharmacokinetic properties in hepatocyte metabolism studies, such as relatively long $t_{1/2}$ and low clearance, allowing for longer dosing intervals and better patient compliance; and
- Allowing topical administration on the lesion, with precise effect and reduced dosage; showing limited toxicity and goog safety because the high polarity of the molecules limits their spreading outside the lesion.

**Pharmaceutical compositions**

In another aspect, the invention provides a pharmaceutical composition comprising a compound of the invention and one or more pharmaceutically acceptable excipients, and a method of using the compound of the invention for the preparation of said composition.

Compositions or dosage forms are formulated, dosed and administered in a manner consistent with good medical practice. Factors considered in this context include the particular condition being treated, the particular mammal being treated, the clinical condition of the individual patient, the cause of the condition, the site of delivery of the agent, the method of administration, the timing of administration, and others known to the medical practitioner.

A typical pharmaceutical composition or dosage form is prepared by mixing a compound of the invention with a carrier or excipient. Suitable carriers and excipients are well known to those skilled in the art and are described in detail, for example, in Gennaro AR et al., Remington: The Science and Practice of Pharmacy (2000) Lippincott, Williams & Wilkins, Philadelphia. The formulation may also include one or more buffers, stabilizers, surfactants, wetting agents, lubricants, emulsifiers, suspending agents, preservatives, antioxidants, opacifiers, glidants, processing aids, colorants agents, sweeteners, fragrances, flavoring agents, diluents and other known additives to provide the elegant appearance of the drug (i.e. the compound of the present invention or its pharmaceutical composition) or to contribute to the preparation of drug product (i.e. the medicament).

The compounds of the present invention may be administered by any suitable modes, including oral, topical (including buccal and sublingual), rectal, vaginal, transdermal, parenteral, subcutaneous, intraperitoneal, intrapulmonary, intradermal, intrathecal and epidural and intranasal, and if needed for topical treatment and intralesional administration. Parenteral infusions include intramuscular, intravenous, intraarterial, intraperitoneal or subcutaneous administration.

In a preferred embodiment, there is provided a pharmaceutical composition comprising one or more compounds of the invention, wherein said pharmaceutical composition is suitable for intravenous, intratumoral, peritumoral or subcutaneous administration. Intratumoral (directly into the tumor mass) or peritumoral (around the tumor mass) administration of the compound of the present invention can directly activate local infiltrating dendritic cells, directly promote the apoptosis of tumor cells or sensitize tumor cells to cytotoxic agents.

The compounds of the present invention can be administered in any convenient administration form, such as tablets, powders, capsules, sterile injectable preparations, solutions, dispersions, suspensions, syrups, sprays, suppositories, gels, emulsions, patches, etc. Such compositions may contain conventional components in pharmaceutical formulations, such as diluents, carriers, pH regulators, preservatives, solubilizers, stabilizers, wetting agents, emulsifiers, sweeteners, colorants, flavoring agents, salts for modifying osmotic pressure, buffers, masking agents, antioxidants and other active agents. They may also contain other therapeutically valuable substances. Various preparation forms can be prepared according to conventional methods in the field of formulation, for example, see Gennaro AR et al., Remington: The Science and Practice of Pharmacy (2000) Lippincott, Williams & Wilkins, Philadelphia or various national pharmacopoeias.

In a preferred embodiment, there is provided a pharmaceutical composition comprising one or more compounds of the invention in the form of a parenteral formulation, especially a sterile injectable formulation, such as a sterile injectable solution or suspension in a non-toxic and parenterally acceptable diluent or solvent, or prepared as lyophilized powders. Among the acceptable vehicles or solvents, for example, water, 1,3-butanediol, Ringer's solution or isotonic sodium chloride solution can be adopted; in addition, sterile fixed oils are conventionally employed as solvents or suspending mediums, for this purpose any mild fixed oil may be employed including, for example, synthetic mono-or diglycerides, fatty acids, and the like.

The administered dosage of the compounds of the invention can vary within wide ranges and, of course, can be adjusted to the individual requirements in each particular case. Typically, an effective amount for systemic administration of a compound of the invention is from about 0.1 μg/kg/day to about 50 mg/kg/day, for example 0.5 μg/kg/day to about 10 mg/kg/day, 1 μg /kg/day to about 1 mg/kg/day. Each dosage unit may conveniently contain from 0.001 μg to 10 mg, for example 0.01 μg to 1 mg, for example 50 μg to 500 μg. The effective amount can be given in one or more

doses, i.e. 1, 2 or more administrations, and can be administered multiple times at equal or different time intervals, including one or more per day, once or multiple per week, or following an administration regimen of every several days/weeks.

**Uses and methods**

In view of the fact that the compounds of the present invention can activate STING, induce the expression of type I interferon and pro-inflammatory cytokines such as IL-6, TNF-α and IFN-y, etc., and meanwhile has cytotoxic activity, another aspect of the present invention provides therapeutic uses and methods of the compounds of the present invention.

In one aspect, the compounds or pharmaceutical compositions described herein can be used as therapeutic substances for the treatment or prevention of diseases associated with or mediated by immune responses, in particular for the treatment or prevention of diseases associated with or mediated by STING, including inflammation, allergic or autoimmune diseases, infectious diseases or cancers, or as a vaccine adjuvant.

In a preferred embodiment, the compounds or compositions of the present invention are used as cytotoxic agents for the treatment or prevention of hyperproliferative diseases, especially tumors. In another preferred embodiment, the compounds or compositions of the present invention are used for treating recurrent tumors, or for preventing tumor recurrence.

In a preferred embodiment, the compounds or compositions of the present invention are used as cytotoxic agents for the treatment or prevention of viral infections.

In another aspect, the present invention accordingly provides a method of inducing, stimulating or assisting an immune response in an individual comprising administering to the individual a compound or pharmaceutical composition of the invention. In one embodiment, the compounds of the invention are administered to an individual as immunotherapy to induce in vivo production of a wide variety of cytokines that are therapeutically useful in humans or animals, including type I interferons and pro-inflammatory cytokines such as IL-6, TNF-α and IFN-γ to modulate the immune system of the human or animal to achieve certain therapeutic benefits.

In another aspect, the present invention accordingly provides a method for the treatment or prevention of diseases associated with or mediated by immune responses, specifically diseases associated with or mediated by STING, including inflammation, allergic or autoimmune diseases, infectious diseases or cancers, comprising administering to a subject in need thereof a therapeutically effective amount of a compound or pharmaceutical composition of the present invention.

In a preferred embodiment, the present invention provides a method for the treatment or prevention of hyperproliferative diseases, especially tumors, comprising administering a therapeutically effective amount of a compound or pharmaceutical composition of the present invention to a subject in need thereof. In another preferred embodiment, the present invention provides a method for treating recurrent tumors or preventing tumor recurrence, comprising administering a therapeutically effective amount of a compound or pharmaceutical composition of the present invention to a subject in need thereof.

In a preferred embodiment, the present invention provides a method of the treatment or prevention of viral infections, comprising administering a therapeutically effective amount of a compound or pharmaceutical composition of the present invention to a subject in need thereof.

In another aspect, the present invention accordingly provides a use of the compound or pharmaceutical composition of the invention for the manufacture of a medicament for the treatment or prevention of diseases associated with or mediated by immune responses, specifically diseases associated with or mediated by STING, including inflammation, allergic or autoimmune diseases, infectious diseases or cancers.

In another aspect, the present invention accordingly also provides a use of the compound or pharmaceutical composition of the present invention in the manufacture of vaccine adjuvants.

In a preferred embodiment, the present invention provides a use of the compound or pharmaceutical composition of the present invention in the manufacture of a medicament for the treatment or prevention of hyperproliferative diseases, especially tumors. In another preferred embodiment, the present invention provides a use of a compound or pharmaceutical composition of the present invention in the manufacture of a medicament for treating recurrent tumors or preventing tumor recurrence.

In a preferred embodiment, the present invention provides a use of the compound or pharmaceutical composition of the present invention in the manufacture of a medicament for treating or preventing viral infections.

For the above uses and methods, the inflammation may be of acute or chronic, involving any organ or tissue of the body, including musculoskeletal inflammation, vascular inflammation, neuroinflammation, digestive system inflammation, eye inflammation, inflammation in genital system or other inflammations, of course also autoimmune disorders as well as allergic disorders such as contact dermatitis, urticaria and respiratory allergies of an inflammatory nature.

For the above uses and methods, the autoimmune disease refers to diseases in which the body's immune

response to autoantigens results in damages to its own tissues, including but not limited to systemic lupus erythematosus, psoriasis, insulin-dependent diabetes, dermatomyositis, Sjgren's syndrome, chronic fatigue syndrome, aplastic anemia, autoimmune hepatitis, multiple sclerosis, optic neuritis, pemphigus, rheumatoid arthritis, ulcerative colitis, crohn's disease, morphea, scleroderma, etc.

For the above uses and methods, the hyperproliferative disease refers to a physiological condition in a subject characterized by uncontrolled or unregulated cell growth or death, especially tumors or cancers, including solid tumors and hematogenous tumors, including but not limited to brain cancer, skin cancer, bladder cancer, ovarian cancer, breast cancer, stomach cancer, pancreatic cancer, prostate cancer, colon cancer, blood cancer, lung cancer and bone cancer. Examples of the above cancer types include neuroblastoma, intestinal cancers such as rectal cancer, colon cancer, familial adenomatous polyposis carcinoma and hereditary nonlymphocytic colorectal cancer, esophageal cancer, lip cancer, laryngeal cancer, nasopharyngeal carcinoma, oral cancer, salivary gland cancer, peritoneal cancer, soft tissue sarcoma, urothelial carcinoma, hidradenocarcinoma, gastric cancer, adenocarcinoma, medullary thyroid cancer, papillary thyroid cancer, renal carcinoma, renal parenchymal carcinoma, ovarian cancer, cervical cancer, corpus carcinoma, endometrial cancer, pancreatic cancer, prostate cancer, testicular cancer, breast cancer (including HER2-negative breast cancer), urinary cancer, melanoma, brain tumors such as glioblastoma, astrocytoma, meningioma, medullary tumor and peripheral neuroectodermal tumor, Hodgkin lymphoma, non-Hodgkin lymphoma, Burkitt's lymphoma, acute lymphoblastic leukemia (ALL), chronic lymphocytic leukemia (CLL), chronic myelogenous leukemia (CLL), and lymphocytic carcinoma, acute myelogenous leukaemia (AML), myelogenous leukemia (chronic myelogenous leukaemia (CML), adult T-cell lymphoma, diffuse lymphoma (DLBCL), hepatoma, multiple myeloma, seminoma, osteosarcoma, chondrosarcoma, anal canal carcinoma, adrenocortical carcinoma, chordoma, fallopian tube carcinoma, gastrointestinal stromal tumor, myeloproliferative disorder, mesothelioma, biliary tract carcinoma, Ewing's sarcoma, and other rare tumor types, and recurrent forms of the above tumors.

In a preferred embodiment, the hyperproliferative disease for the above uses and methods is small cell lung cancer, non-small cell lung cancer, colorectal cancer, liver cancer, breast cancer, ovarian cancer, gastric cancer, prostate cancer, melanoma, renal cell carcinoma, head and neck cancer, pancreatic cancer, Hodgkin's lymphoma, leukemia, or bladder cancer.

For the above uses and methods, the virus infection refers to the process in which viruses invade the body through various channels and multiply in susceptible host cells. The viruses involved include but are not limited to double-stranded DNA viruses and single-stranded DNA viruses, positive-sense single-stranded RNA viruses, negative-sense single-stranded RNA viruses and double-stranded RNA viruses and retroviruses, examples are hepatitis B virus, TTV virus, adenovirus, papilloma virus, herpes zoster virus, smallpox virus and vaccinia virus, influenza virus, swine fever virus, hepatitis A virus, hepatitis C virus, hepatitis D virus, hepatitis E virus, hepatitis G virus, Rabies virus, Ebola virus, enterovirus and human immunodeficiency virus, etc. The therapeutic uses and methods provided by the present invention can be used for the above-mentioned viral infections and the diseases caused therefrom.

For the above use of the compounds or compositions of the present invention as a vaccine adjuvant and the use in the manufacture of a vaccine adjuvant, it refers to that the compounds or compositions of the present invention can be used as an adjuvant in a therapeutic or preventive strategy using a vaccine, that is, the compounds or compositions of the invention are used together with one or more vaccines selected to stimulate an immune response to one or more predetermined antigens, the vaccines comprising inactivated or attenuated bacteria or viruses, e.g. comprising inactivated tumor cells expressing and secreting one or more of GM-CSF, CCL-20, CCL3, IL-12p70, FLT-3 ligand, and cytokines.

**Pharmaceutical combinations**

In view of the favorable pharmacological activities of the compounds of the present invention, in addition to their use alone in the above-mentioned therapeutic uses or methods, they may also be used in combination with at least one other therapeutic agent or therapy to provide further therapeutic benefits.

Accordingly, in another aspect, the present invention also provides a pharmaceutical combination comprising the cyclic dinucleotide compound described herein, a stereoisomer, tautomer, stable isotopic variant, pharmaceutically acceptable salt, prodrug or solvate thereof or the pharmaceutical composition thereof, and at least one other therapeutic agent, or consist of both.

In another aspect, the present invention provides a pharmaceutical composition comprising a pharmaceutical combination as described herein and one or more pharmaceutically acceptable excipients.

In another aspect, the present invention provides a use of a pharmaceutical combination as described herein or a pharmaceutical composition comprising it for the treatment or prevention of diseases, or in the manufacture of a medicament for the treatment or prevention of diseases, including hyperproliferative diseases, viral infections or diseases associated with or mediated by STING, more specifically inflammation, allergic or autoimmune diseases, infectious diseases or cancers. In a preferred embodiment, the diseases involved in the use are tumors or viral infections.

In another aspect, the present invention also provides a method of treatment wherein a compound of the

invention is administered with one or more other therapeutic agents.

The inflammation, autoimmune diseases, hyperproliferative diseases and viral infections for the use of the pharmaceutical combination of the invention and the pharmaceutical composition comprising it are as described above for the uses and methods of the invention.

The compounds of the invention may also be administered in conjunction with surgery, radiation therapy, transplantation (e.g., stem cell transplantation, bone marrow transplantation), immunooncology agents, and the like.

The other therapeutic agent used in combination with the present invention may be administered simultaneously, separately or sequentially with the compound of the present invention by the same or a different route of administration. The additional therapeutic agent may be co-administered with the compound of the present invention in a single pharmaceutical composition, or administered separately in separate discrete units from the compound of the present invention, such as a combination product, preferably in the form of a kit, which when administered separately may be performed simultaneously or sequentially, the sequential administrations may be close or distant in time. They may be prepared and/or formulated by the same or different manufacturers. Furthermore, the compound of the invention and other therapeutic agent may be added to the combination therapy (i) prior to sending the combination product to the physician (for example, in the case of a kit comprising the compound of the invention and the other agent); (ii) immediately prior to administration by the physician himself (or under the direction of the physician); (iii) by the patient himself, e.g., during the sequential administration of the compound of the invention and the other therapeutic agent.

Therefore, in another aspect, the present invention also provides a kit comprising two or more separate pharmaceutical compositions, at least one of which comprises a cyclic dinucleotide compound of the present invention, a stereoisomer, tautomer, stable isotopic variant, pharmaceutically acceptable salt, prodrug or solvate thereof, and the remaining co-existing pharmaceutical compositions comprising at least one other therapeutic agent, and devices respectively containing said compositions, such as containers, dispenser bottles or discrete foil packs, such as blister packs for tablets, capsules, etc. The kit of the invention is particularly suitable for administering different dosage forms, such as oral and parenteral dosage forms, or for administering different compositions at different dosage intervals.

The other therapeutic agent may be one or more additional compounds of the invention, or may be a second or additional (e.g., the third) therapeutic agent that is compatible (namely without adverse effects on each other) with the compound of the present invention, or complementary in activity ot has another activity.

In specific embodiments, other therapeutic agents that may be used in combination with the compounds of the invention include, but are not limited to, vaccines, adjuvants, immune checkpoint inhibitors, T cell receptor agonists, TLR agonists, therapeutic antibodies, lipids, liposomes, chemotherapeutic agents, immunomodulatory cell lines, etc.

In a specific embodiment, the adjuvants used in combination with the compounds of the invention are useful, by their nature, to stimulate or otherwise harness the immune system to respond to cancer antigens present on tumor cells, including but not limited to lipids, liposomes, inactivated bacteria that induce innate immunity, compounds that mediate activation of innate immunity, etc.

In a specific embodiment, the immune checkpoint inhibitors used in combination with the compounds of the present invention are, for example, selected from CTLA-4 pathway antagonists, PD-1 pathway antagonists, Tim-3 pathway antagonists, Vista pathway antagonists, BTLA Pathway antagonists, LAG-3 pathway antagonists or TIGIT pathway antagonists.

In a specific embodiment, the T cell receptor agonists used in combination with the compounds of the present invention includes but are not limited to CD28 agonists, OX40 agonists, GITR agonists, CD137 agonists, CD27 agonists or HVEM agonists.

In a specific embodiment, the TLR agonists used in combination with the compounds of the present invention include but are not limited to Pam3Cys, CFA, MALP2, Pam2Cys, FSL-1, Hib-OMPC, polyadenosine-polyuridine (polyAU), LPS, bacterial flagellin, monophosphoryl lipid A (MPL), imiquimod, resiquimod, loxoribine, etc.

In a specific embodiment, the chemotherapeutic agents used in combination with the compounds of the invention include but are not limited to alkylating agent-based antineoplastics, platinum-based antineoplastics, antimetabolites, antimicrotubule agents, antimitotic agents, topoisomerase inhibitors and antitumor antibiotics, etc.

In a specific embodiment, the therapeutic antibodies used in combination with the compounds of the present invention include but are not limited to, Muromonab-CD3, Infliximab (Remicade®) and adalimumab (Humira®), Omalizumab (Xolair®), Daclizumab (Zenapax®), Rituximab (trade name = Rituxan®), Ibritumomab (trade name = Zevalin®), Tositumomab (Bexxar®), Cetuximab (Erbitux®), Trastuzumab (Herceptin®), Adcetris®, Alemtuzumab (Campath-1H®), Lym-1 (Oncolym®), Ipilimumab (Yervoy®), Vitaxin, Bevacizumab (Avastin®), Abciximab (ReoPro®). Other therapeutic antibodies that can be used in combination include prolactin receptor inhibitors, HER3 inhibitors, EGFR2 and/or EGFR4 inhibitors, M-CSF inhibitors, anti-APRIL antibodies or anti-SIRPa or anti-CD47 antibodies.

In other embodiments, the compounds of the present invention can also be combined with PKC inhibitors, BCR-ABL inhibitors, HSP90 inhibitors, PI3K and/or mTOR inhibitors, FGFR inhibitors, cytochrome P450 inhibitors, HDM2 inhibitors, aromatase inhibitor, p53 and/or p53/Mdm2 interaction inhibitors or CSF-1R tyrosine kinase inhibitors.

Examples of the above-mentioned various therapeutic agents and other therapeutic agents that can be used

in combination with the compounds of the present invention can be found in WO2016/145102 and WO2018/060323, the corresponding contents of which are incorporated herein.

For the above-mentioned compounds, pharmaceutical compositions, methods, uses, pharmaceutical combinations and kits of the present invention, the cyclic dinucleotide compounds of the present invention, their stereoisomers, tautomers, stable isotopic variants, pharmaceutically acceptable salts, prodrugs or solvates or pharmaceutical compositions thereof in the various preferred embodiments described above, are preferred, more preferably the compounds defined in the specific embodiments, that is, the compounds of Examples 1-26, most preferably those compounds that show excellent activity in the activity examples.

For the above-mentioned compounds, pharmaceutical compositions, methods, uses, pharmaceutical combinations and kits of the present invention, it is preferred to use the free form or pharmaceutically acceptable salts or prodrugs of the cyclic dinucleotide compounds defined herein, preferably the free form or pharmaceutically acceptable salts or prodrugs of substantially pure cyclic dinucleotide compounds as defined herein.

For the therapeutic uses and methods of the invention described above, it is preferred that the subject of uses or treatments is a mammal, preferably a human.

When dosages of a drug, or a pharmaceutically acceptable salt thereof, are described herein, it is understood that such dosages are based on the weight of the free base, excluding any hydrates or solvates thereof, unless otherwise indicated.

**Preparation methods of the compounds of the present invention**

**General Synthetic Method**

The compounds of the present invention, their stereoisomers, tautomers, stable isotopic variants, pharmaceutically acceptable salts or solvates can be prepared by a variety of methods well known in the art of organic synthesis, including those given below, the methods given in the examples or similar methods understood by those skilled in the art.

The general synthetic schemes for the synthesis of compounds of the invention are illustrated below. For the individual reaction steps, suitable reaction conditions are known to the person skilled in the art or can be determined routinely. In particular, the process steps for the synthesis of the compounds of the present invention may be carried out under reaction conditions known per se, including those specifically mentioned, in the absence or usually in the presence of solvents or diluents (including, for example, solvents or diluents which are inert for the reagents used and can dissolve the reagents used), in the absence or presence of catalysts, condensing agents or neutralizing agents (such as ion exchangers, such as cation exchangers, e.g. in the H+ form), depending on the nature of the reaction and/or reactants at reduced, normal or elevated temperatures (e.g. from about -100°C to about 190°C, including for example from about -78°C to about 150°C, for example from about 0°C to about 125°C, room temperature, -20 to 40°C or reflux temperature), at atmospheric pressure or in a closed vessel, under pressure when appropriate, and/or under an inert atmosphere such as oxygen or nitrogen.

The starting materials and reagents used in the preparation of these compounds are generally commercially available, or can be prepared by the methods below, methods analogous to those given below, or methods known in the art. Starting materials and intermediates in the synthetic reaction schemes can be isolated and purified, if necessary, by conventional techniques including but not limited to filtration, distillation, crystallization, chromatography, and the like. The materials can be characterized using conventional methods including physical constants and spectral data.

Solvents suitable for any particular reaction, unless otherwise stated in the description of a method, include: those specifically mentioned, or, for example, water; esters, such as lower fatty acid lower alkyl esters, such as ethyl acetate; ethers, such as aliphatic ethers, such as diethyl ether, or cyclic ethers, such as tetrahydrofuran or dioxane; liquid aromatic hydrocarbons, such as benzene or toluene; alcohols, such as methanol, ethanol or 1- or 2-propanols, such as acetonitrile; halogenated hydrocarbons, such as dichloromethane or chloroform; amides, such as *N,N*-dimethylformamide or *N,N*-dimethylacetamide; bases, such as heterocyclic nitrogen bases, such as pyridine; carboxylic anhydrides, such as lower aliphatic chain carboxylic anhydrides, such as acetic anhydride; cyclic, linear or branched hydrocarbons, such as cyclohexane, hexane or isopentane; or mixtures of these solvents, such as aqueous solutions. Such solvent mixtures can also be used for work-up, for example by chromatography or partitioning.

Those skilled in the art will recognize the presence of stereocenters in the compounds of formula I. At all stages of the reaction, the mixture of isomers formed can be separated into individual isomers, such as diastereomers or enantiomers, or into any desired mixture of isomers, such as racemates or mixtures of diastereomers, see e.g. EL Eliel, SH Wilen and LN Mander "Stereochemistry of Organic Compounds" (Wiley-Interscience, 1994).

Scheme 1 below illustrates a general synthetic route that can be used to prepare the compounds of formula II and various embodiments thereof as defined herein. The variables of the general formulas in the following schemes have the same meanings as in the compounds defined herein or in their respective embodiments, unless otherwise stated.

## Scheme 1

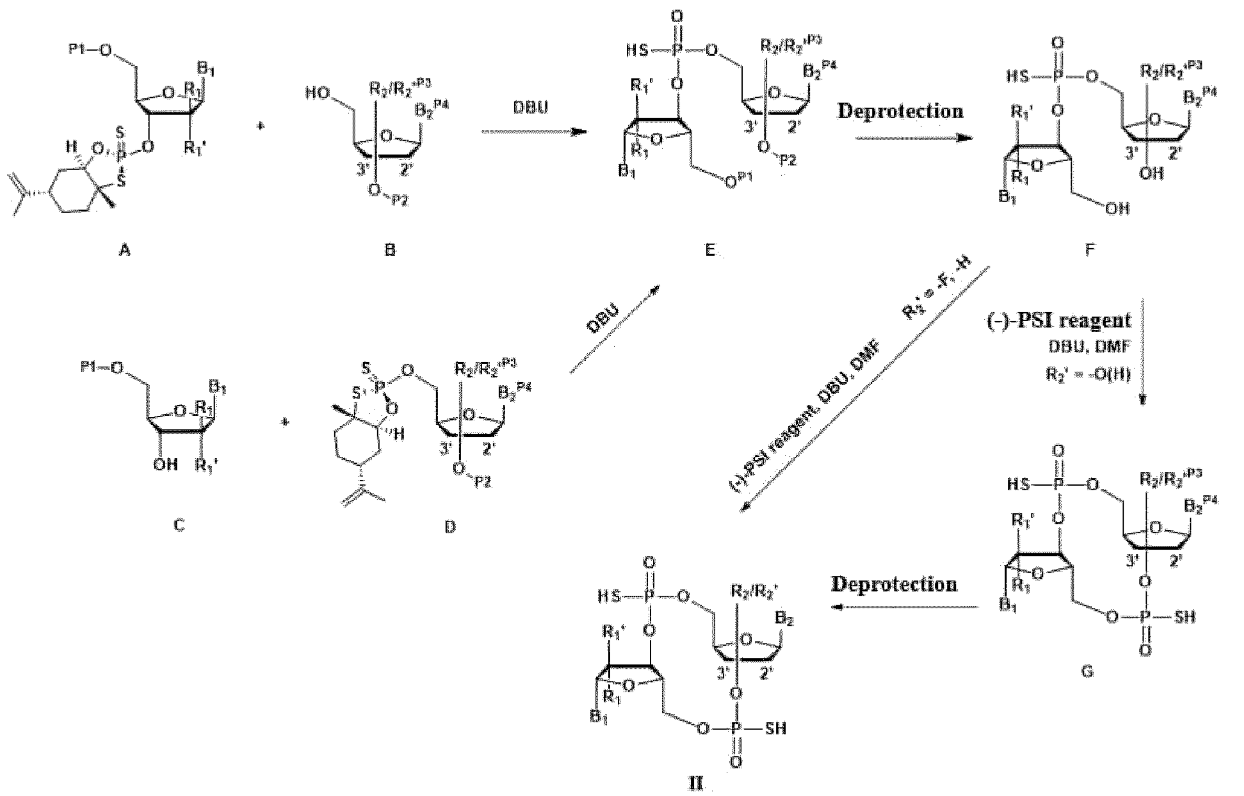


wherein $P_1$ and $P_2$ are suitable hydroxyl protecting groups, $P_3$ and $P_4$ are suitable hydroxyl or amino protecting groups, including but not limited to TBS (tert-butyldimethylsilyl), DMTr (bis(4-methyloxyphenyl)benzyl), Bz (benzoyl), $^{i}$-BuCO (isobutyryl). The deprotection used in the synthesis scheme of the compounds of the present invention is under acidic conditions (including but not limited to acetic acid/water, trifluoroacetic acid/water, etc.), basic conditions (including but not limited to ammonia water, ammonia/methanol solution, etc.) or in the presence of fluoride anion-containing compounds (including but not limited to such as tetrabutylammonium fluoride, triethylamine trihydrofluoride, etc.).

Scheme 2 illustrates the synthetic route of the compound of formula A and formula C in Scheme 1, and the synthesis of the intermediates further used therein.

## Scheme 2

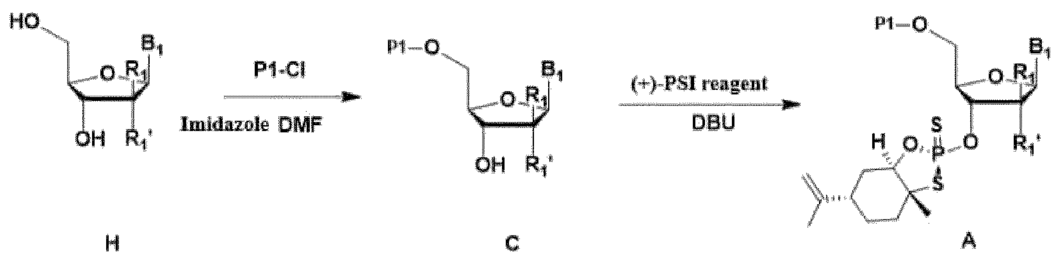


Scheme 3 illustrates the synthetic route when the compound of formula H is the compound of formula H-1 (gemcitabine prodrug LY2334737).

## Scheme 3

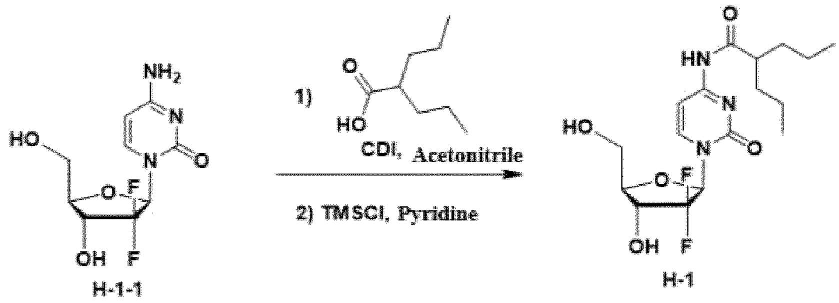


Schemes 4-7 illustrate the synthetic route of the compound of formula B in Scheme 1, and the synthesis of intermediates further used therein.

## Scheme 4

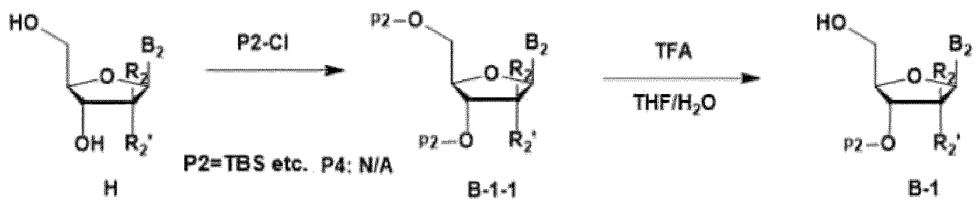


## Scheme 5

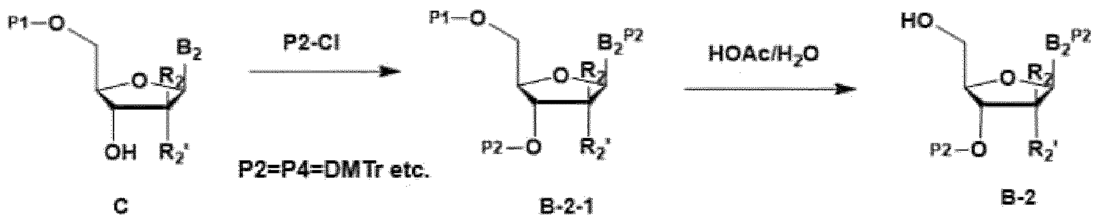


## Scheme 6

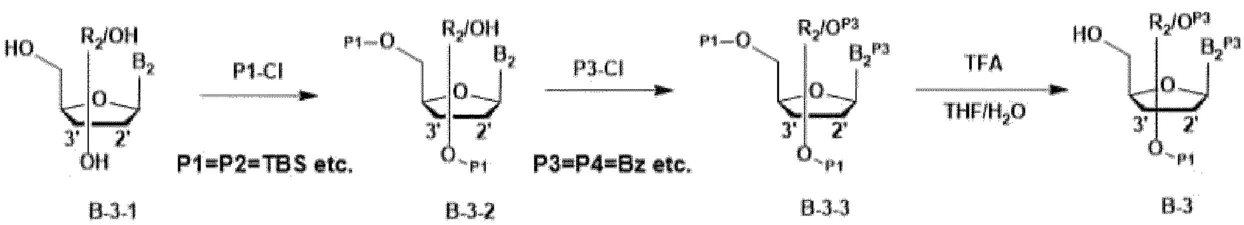

## Scheme 7

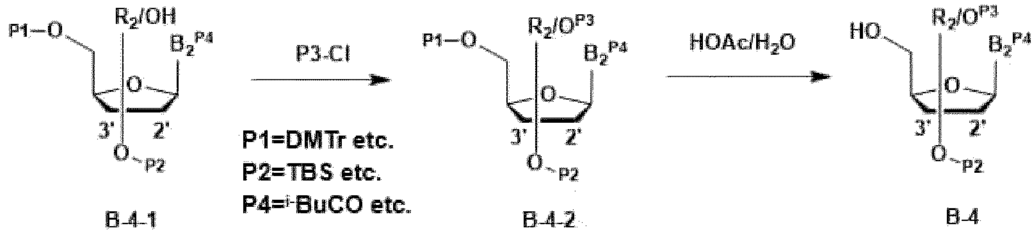


B-4-1 B-4-2 B-4

Scheme 8 illustrates the synthetic route of the compound of formula D.

## Scheme 8

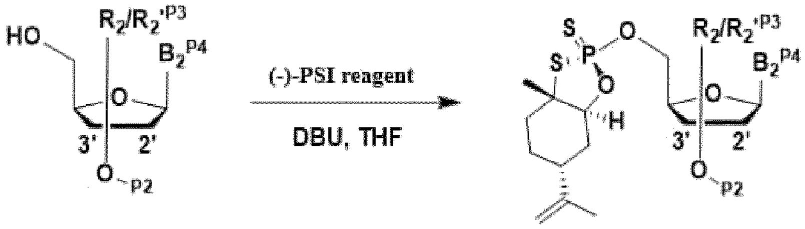


B D

Specifically, the present invention provides the preparation method of the above-mentioned compounds of the present invention, which comprises:

reacting a compound of formula A,

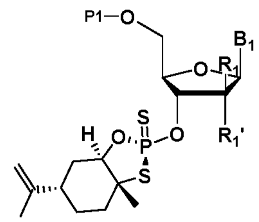

A

wherein $B_1$, $R_1$, $R_1$' have the meanings defined above for the compound of formula (II) of the present invention or its various specific embodiments; P1 is a suitable hydroxyl protecting group, such as but not limited to TBS (tert-butyldimethylsilyl), DMTr (bis(4-methoxyphenyl)benzyl), Bz (benzoyl), $^i$BuCO (isobutyryl);

with a compound of formula B in the presence of a base, such as DBU,

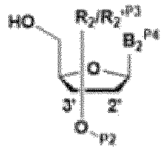

B

wherein $B_2$, $R_2$, $R_2$' have the meanings defined above for the compound of formula (II) of the present invention or its various embodiments; P2 is a suitable hydroxyl protecting group, and P3 and P4 are each suitable hydroxyl or

amino protecting group such as but not limited to TBS (tert-butyldimethylsilyl), DMTr (bi(4-methoxyphenyl)benzyl), Bz (benzoyl), $^{i}$-BuCO (isobutyryl);

or reacting a compound of formula C,

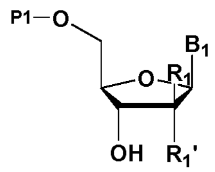

C

wherein $B_1$, $R_1$, $R_1$' and $P_1$ are as defined above for the compound of formula A; with a compound of formula D in the presence of a base, such as DBU,

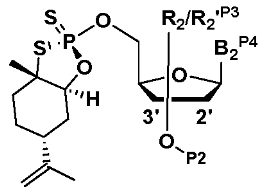

D

wherein $B_2$, $R_2$, $R_2$', P2, P3 and P4 are as defined above for the compound of formula B; to obtain the compound of formula E,

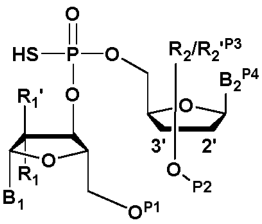

E

which is selectively deprotected, for example, under the conditions of trifluoroacetic acid/water, tetrabutylammonium fluoride or triethylamine trihydrofluoride, to obtain the compound of formula F,

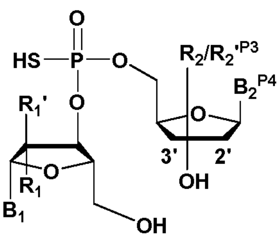

F

wherein each group has the meaning defined above;

a) when Rz'=-O(H) and the protecting group P3 is benzoyl, the compound of formula F is cyclized with (-)-PSI reagent in the presence of a base, such as DBU, to obtain the cyclic dinucleotide compound of formula G,

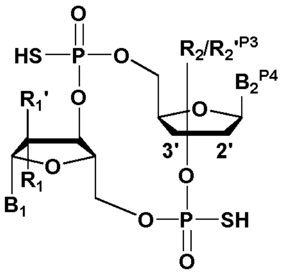

G

then deprotecting the benzoyl in ammonia water or ammonia methanol solution, to obtain the cyclic dinucleotide compound of formula II,

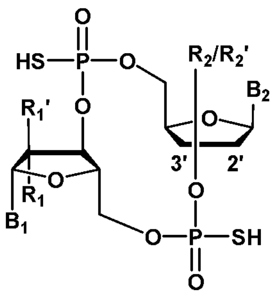

II

wherein Rz'=-O(H), and each other group has the meaning defined above for the compound of formula II or its various specific embodiments;

or b) when $R_2$' = -F or -H, the compound of formula F is cyclized with (-)-PSI reagent in the presence of a base, such as DBU, to obtain the cyclic dinucleotide compound of formula II,

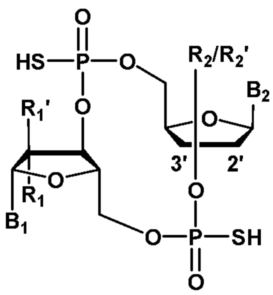

II

wherein $R_2$' = -F or -H, and each other group has the meanings defined above for the compound of formula II or its various specific embodiments.

The compounds of formula A and formula C can be prepared as follows:

selectively protecting the primary alcohol of the compound of formula H in the presence of a base such as imidazole,

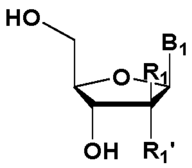

H

to obtain the compound of formula C,

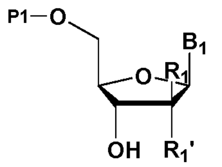

C

which is reacted with (+)-PSI reagent in the presence of a base, such as DBU, to obtain the compound of formula A,

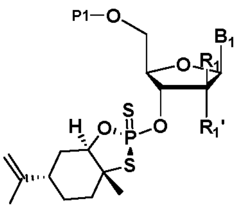

A

wherein $B_1$, $R_1$, $R_1$' and P1 are as defined above for the compound of formula A.

When the compound of formula H is the compound of formula H-1 (gemcitabine prodrug LY2334737),

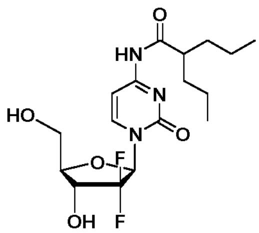

H-1

it is prepared with the compound of formula H-1-1 and 2-propylpentanoic acid,

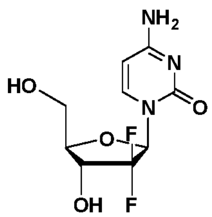

H-1-1

The compounds of formula B can be prepared in the form of formula B-1 as follows:

protecting the two hydroxyl groups of the compound of formula H in the presence of a base such as imidazole,

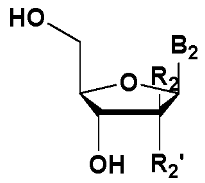

H

to obtain the compound of formula B-1-1,

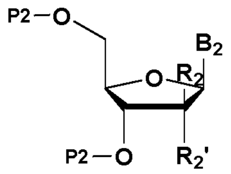

B-1-1

selectively removing the protecting group on the primary alcohol of the compound of formula B-1-1 under conditions such as trifluoroacetic acid/water, to obtain the compound of formula B-1,

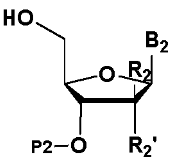

B-1

wherein $B_2$, $R_2$, $R_2$' and P2 are as defined above for the compound of formula B.

The compounds of formula B can also be prepared in the form of formula B-2 as follows:

protecting the secondary alcohol of the compound of formula C in the presence of a base such as DBU/pyridine,

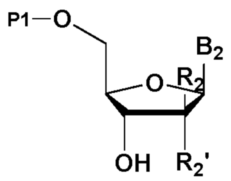

C

to obtain the compound of formula B-2-1,

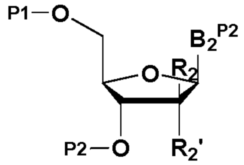

B-2-1

selectively removing the protecting group on the primary alcohol of the compound of formula B-2-1 under conditions

such as acetic acid/water, to obtain the compound of formula B-2,

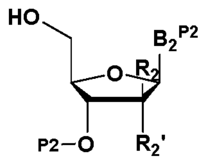

B-2

wherein $B_2$, $R_2$, $R_2$', P1, P2 are as defined above for the compound of formula A or formula B.

The compounds of formula B can also be prepared in the form of formula B-3 as follows:

selectively protecting the primary alcohol and one of the secondary alcohols of the compound of formula B-3-1 in the presence of a base such as imidazole,

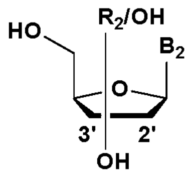

B-3-1

to obtain the compound of formula B-3-2,

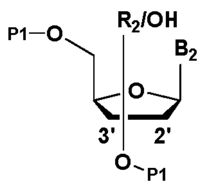

B-3-2

reacting the unprotected secondary alcohol and the amino group on the base of the compound of the above formula B-3-2 with a protecting group such as benzoyl chloride, in the presence of a base such as N-methylimidazole, to obtain the compound of formula B-3-3,

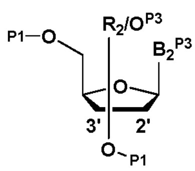

B-3-3

selectively removing the protecting group on the primary alcohol of the compound of formula B-3-3 under conditions such as trifluoroacetic acid/water, to obtain the compound of formula B-3,

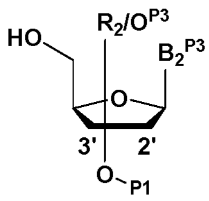

B-3

wherein $B_2$, $R_2$, $R_2$', $P_1$, $P_2$ and P3 are as defined above for the compound of formula A or formula B.

The compound of formula B can also be prepared in the form of formula B-4 as follows:

reacting the unprotected secondary alcohol of the compound of the formula B-4-1 with a protecting group such as benzoyl chloride, in the presence of a base such as N-methylimidazole,

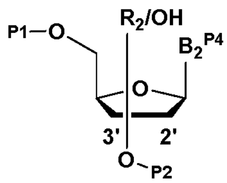

B-4-1

to obtain the compound of formula B-4-2,

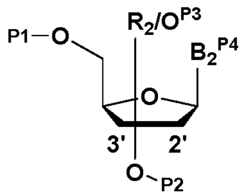

B-4-2

selectively removing the protecting group on the primary alcohol of the compound of formula B-4-2 under conditions such as acetic acid/water, to obtain the compound of formula B-4,

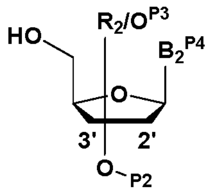

B-4

wherein $B_2$, $R_2$, $R_2$', P1, P2 and P4 are as defined above for the compound of formula A or formula B.

The compound of formula D can be prepared as follows:

reacting the compound of formula B with a (-)-PSI reagent in the presence of a base, such as DBU,

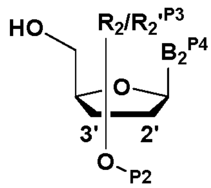

B

to obtain the compound of formula D,

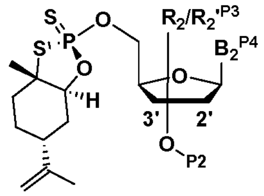

D

wherein $B_2$, $R_2$, $R_2$', $P_2$, $P_3$ and $P_4$ are as defined above for the compound of formula B.

Scheme 9 below illustrates a general synthetic route that can be used to prepare the compounds of formula (Ib), (II-b), (III-b), (IV-b) and various embodiments thereof as defined herein. The variables of the general formulas in the following schemes have the same meanings as in the compounds or in their respective embodiments as defined herein, unless otherwise stated.

**Scheme 9**

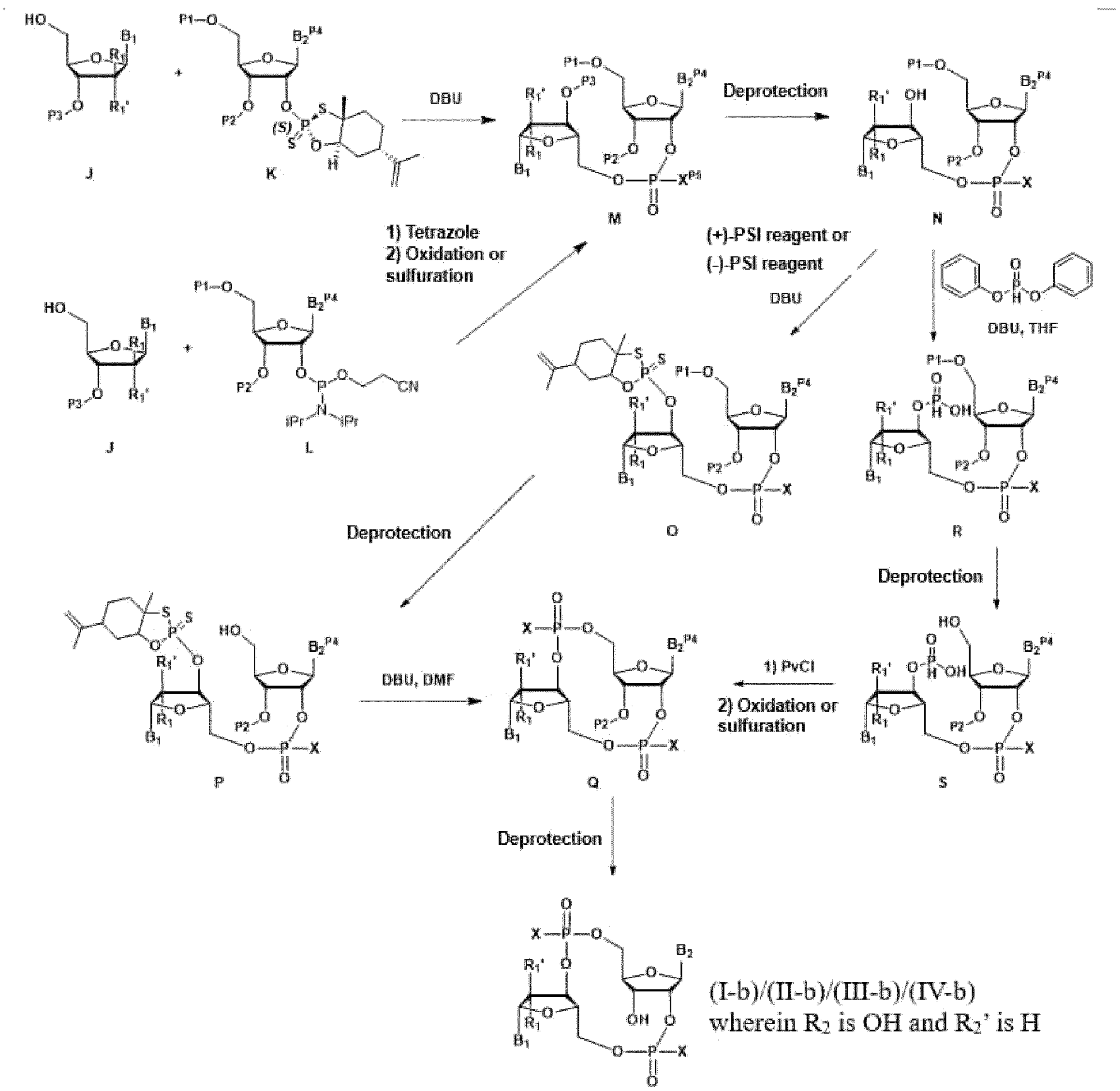


(I-b)/(II-b)/(III-b)/(IV-b) wherein $R_2$ is OH and $R_2$' is H

wherein X is each independently hydroxyl or mercapto; $R_1$, $R_1$', $B_1$ and $B_2$ are each as defined above for formulas (Ib), (II-b), (III-b), (IV-b) and each of their sub-general formulas and specific embodiments; $P_1$ and $P_2$ are suitable hydroxyl protecting groups, $P_3$ and $P_4$ are suitable hydroxyl or amino protecting groups, including but not limited to TBS (tert-butyldimethylsilyl), DMTr (bis(4-methoxyphenyl)benzyl), Bz (benzoyl), $^{i}$BuCO (isobutyryl). $P_5$ is a suitable hydroxyl or mercapto protecting group on the phosphoric acid/phosphate, including but not limited to cyanoethyl.

The deprotection used in the above synthesis schemes of the compounds of the present invention is performed under acidic conditions (including but not limited to such as acetic acid/water, trifluoroacetic acid/water, etc.), basic conditions (including but not limited to such as ammonia water, ammonia/methanol solution, methylamine/ethanol solution, lithium hydroxide, etc.) or in the presence of fluorine anion-containing compounds (including but not limited to such as tetrabutylammonium fluoride, triethylamine trihydrofluoride, ammonium fluoride, etc.). The oxidation used in the synthesis schemes of the compounds of the present invention is performed under a condition including but not limited to in the presence of iodine, and the sulfurization is perfomed under a considtion including but not limited to in the presence of 3H-1,2-benzodisulfonol-3-one.

Scheme 10 illustrates the synthetic route of the compound of formula J in Scheme 9, and the synthesis of intermediates further used therein.

## Scheme 10

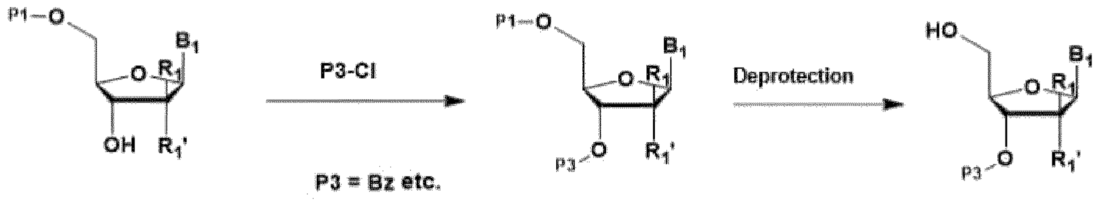


C J-1 J

Scheme 11 illustrates the synthetic route of the compound of formula K, and the synthesis of intermediates further used therein.

## Scheme 11

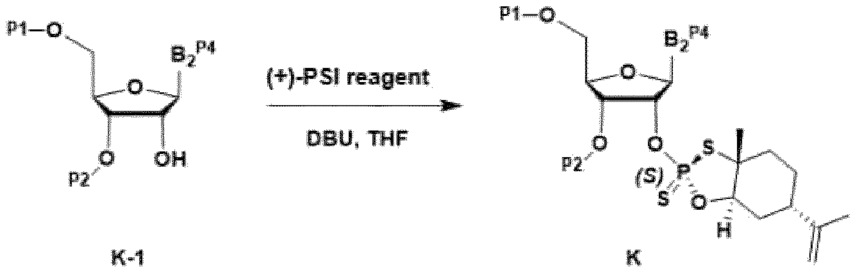


K-1 K

Specifically, the present invention provides the preparation method of the above-mentioned compounds of the present invention, which comprises: reacting the compound of formula J,

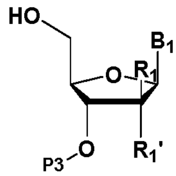

J

wherein $B_1$, $R_1$, $R_1$' have the meanings defined above for the compounds of formula (Ib), (II-b), (III-b), (IV-b) or their specific embodiments of the present invention; P3 is a suitable hydroxyl protecting group such as but not limited to TBS (tert-butyldimethylsilyl), DMTr (bis(4-methoxyphenyl)benzyl), Bz (benzoyl), $^{i}$-BuCO (isobutyryl),

with the compound of formula K in the presence of a base (such as DBU),

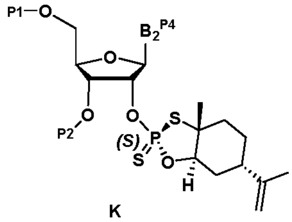

K

wherein $B_2$ has the meaning defined above for the compounds of formula (Ib), (II-b), (III-b), (IV-b) or their specific embodiments of the present invention; P1, P2 are suitable hydroxyl protecting groups, P4 is a suitable hydroxyl or amino protecting group, such as but not limited to TBS (tert-butyldimethylsilyl), DMTr (bis(4-methoxyphenyl)benzyl), Bz (benzoyl), $^{i}$-BuCO (isobutyryl),

or, reacting the compound of formula J with the compound of formula L in the presence of tetrazole, and oxidizing or sulfurizing, wherein the oxidation conditions include but not limited to the use of iodine, tert-butyl hydroperoxide, etc., and the sulfurization conditions include but not limited to the use of *N,N*-dimethyl-*N'*-(3-thioxo-3H-1,2,4-dithiazol-5-yl)formamidine (DDTT), 3H-1,2-benzodisulfonol-3-one, etc.; the introduction and removal of protecting groups are carried out according to standard methods well known to those skilled in the art,

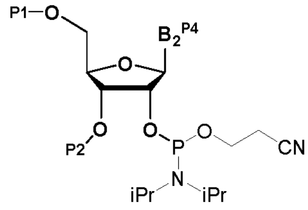

**L**

wherein $B_2$, P1, P2 and P4 are as defined above for the compound of formula K; to obtain the compound of formula M,

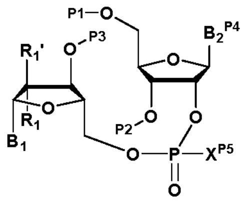

**M**

wherein X is OH or SH, and $P_5$ is a suitable hydroxyl or mercapto protecting group on the phosphoric acid/phosphate, such as but not limited to cyanoethyl;

selectively deprotecting the compound of formula M, for example, under the condition of lithium hydroxide, to obtain the compound of formula N,

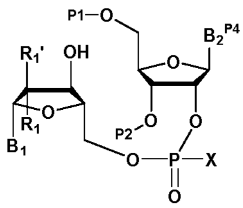

**N**

wherein each group has the meaning as defined above;

a) reacting the compound of formula N with a (+)-PSI reagent or (+)-PSI reagent in the presence of a base, such as DBU, to obtain the compound of formula O,

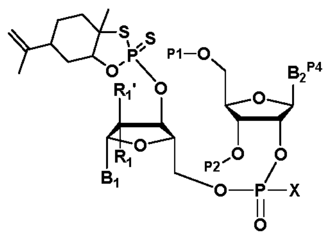

**O**

then removing P1 protection (such as DMTr) in acetic acid/water solution, to obtain the compound of formula

P,

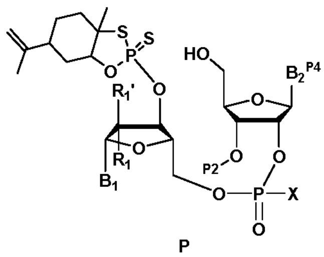

P

cyclizing the compound of formula P in the presence of a base, such as DBU, to obtain a compound of cyclic dinucleotide formula Q,

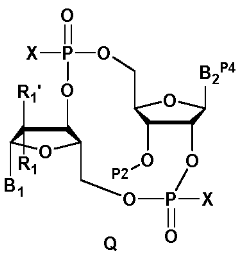

Q

removing P4 protection (such as TBS) under conditions such as ammonium fluoride, to obtain a cyclic dinucleotide compound of formula (Ib), (II-b), (III-b) or (IV-b), wherein $R_2$ is OH and $R_2$' is H,

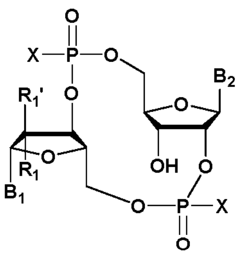

(Ib)/(II-b)/(III-b)/(IV-b)

wherein each group has the meaning as defined above;

b) or, reacting the compound of formula N with diphenyl phosphite in the presence of a base, such as DBU, to obtain the compound of formula R,

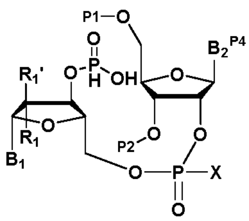

R

then removing P1 protection (such as DMTr) in acetic acid/water solution, to obtain the compound of formula S,

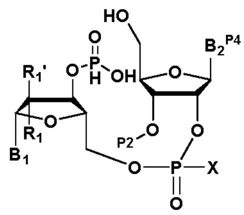

S

cyclizing the compound of formula S in the presence of an activating reagent, such as pivaloyl chloride, and oxidizing or sulfurizing, to obtain the cyclic dinucleotide compound of formula Q,

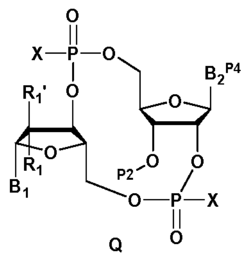

Q

then removing P4 protection (such as TBS) under conditions such as ammonium fluoride, to obtain a cyclic dinucleotide compound of formula (Ib), (II-b), (III-b) or (IV-b), wherein $R_2$ is OH and $R_2$' is H,

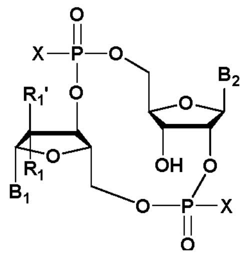

(Ib)/(II-b)/(III-b)/(IV-b)

wherein each group has the meaning as defined above.

The compound of formula J can be prepared as follows:

protecting the secondary alcohol of the compound of formula C in the presence of a base, such as N-methylimidazole,

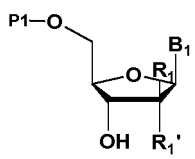

C

to obtain the compound of formula J-1,

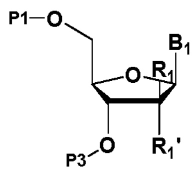

J-1

selectively deprotecting the compound of formula J-1, to obtain the compound of formula J,

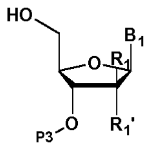

J

wherein each group is as defined above.

The compound of formula K can be prepared as follows:

reacting the compound of formula K-1 with a (+)-PSI reagent in the presence of a base, such as DBU,

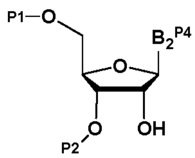

K-1

to obtain the compound of formula K,

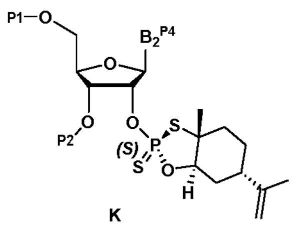

K

wherein each group is as defined above.

The experimental materials and reagents used in the above synthetic methods and schemes, unless otherwise specified, can be obtained from commercially, prepared according to methods of the prior art, or prepared according to methods similar to those disclosed in the present disclosure. The synthetic conditions used in the above synthetic methods and schemes can be routinely determined by those skilled in the art unless otherwise specified.

The present invention also relates to preparation methods in which compounds obtainable as intermediates at any step in the various preparation methods and schemes described herein are used as starting materials and the remaining process steps are carried out, or in which the starting materials are formed in situ under the reaction conditions or used in the form of derivatives, for example in protected form or in salt form, or compounds obtainable by the methods according to the invention are produced under the process conditions and are further processed in situ.

## Examples

The present invention will be further illustrated below by referring to the examples. It should be noted that the following examples are not intended to limit the protection scope of the present invention.

Unless there is an obvious error in the structural formula, when the chemical name of any compound of the present invention is inconsistent with the given structural formula, the structural formula shall prevail.

The experimental methods for which specific conditions are not indicated in the following examples are usually in accordance with the conventional conditions of this type of reaction, or in accordance with the conditions suggested by the manufacturer. The experimental materials and reagents used in the following examples can be obtained from commercial sources, prepared according to methods in the prior art or prepared according to methods similar to those disclosed in the present application, unless otherwise specified.

Percentages and parts are by weight; and ratios of liquids are by volume, unless otherwise indicated; all temperatures are given in degrees Celsius, unless otherwise indicated.

In the following examples, $^1H$ NMR spectrum $^{31}P$ NMR spectrum were usually recorded by Bruker 400 MHz NMR and 500 MHz NMR nuclear magnetic resonance instrument, and the chemical shift is expressed in $\delta$ (ppm); the mass spectrum was recorded by Agilent 1290 liquid chromatography + 6120B mass spectrometer LCMS liquid chromatography-mass spectrometry. Silica gel column purification was carried out by Biotage SelektSEL-2SV or ISO-1SV; preparative liquid chromatography purification was carried out by Gilson 281 (Column: Waters Xbridge 19 x 250 mm, 5 $\mu$m or WELCH C18, 21.2 x 250 mm, 10 $\mu$m; Mobile phase: A: water (10 mM $NH_4HCO_3$ or 0.05% formic acid), B: acetonitrile (or containing 0.05% formic acid); Flow rate: 20-30 mL/min; Detection wavelength: 214 nm/254 nm), or otherwise stated.

The following abbreviations are used in the following synthesis examples, and each abbreviation not listed has the meaning generally understood by those skilled in the art.

## List of abbreviations

| | |
|---|---|
| $CDCl_3$ | Deuterated chloroform |
| DMSO-*d*$_6$ | Deuterated dimethyl sulfoxide |
| MHz | Megahertz |
| MS-ESI | Electrospray mass spectrometry |
| DBU | 1,8-Diazabicycloundec-7-ene |
| DMF | *N*,*N*-Dimethylformamide |
| CDI | *N*,N'-Carbonyldiimidazole |
| TMSCl | Trimethylchlorosilane |
| TFA | Trifluoroacetic acid |
| THF | Tetrahydrofuran |
| $H_2O$ | Water |
| HOAc | Acetic acid |
| BzCl | Benzoyl chloride |
| IFN | Interferon |
| FBS | Fetal bovine serum |
| PBS | Phosphate buffered solution |
| ELISA | Enzyme-linked immunosorbent assay |
| (+)-PSI reagent | (*2R*, *3aR*, *6S*, *7aR*)-3a-Methyl-2-((perfluorophenyl)thio)-6-(propyl-1-en-2-yl)hexahydrobenzo[d][1,3,2]oxathiophospholane-2-sulfide) (CAS: 2245335-71-9) |

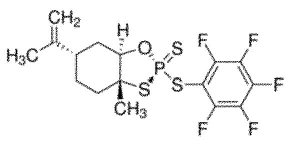

| | |
|---|---|
| (-)-PSI reagent | (*2S,3aS,6R,7aS*)-3a-Methyl-2-((perfluorophenyl)thio)-6-(propyl-1-en-2-yl)hexahydrobenzo[d][1,3,2]oxathiophospholane -2-sulfide (CAS: 2245335-70-8) |

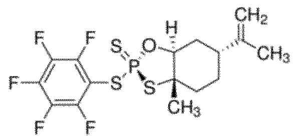

## Synthetic examples

**Preparation of Intermediate 1: 2-Chloro-5'-O-tert-butyldimethylsilyl-2'-deoxy-2'-fluoro-beta-adenosine**

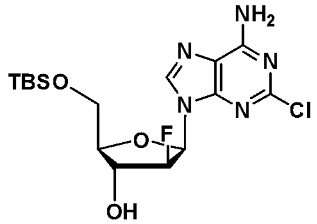

To a DMF solution (15 mL) of **2-chloro-2'-deoxy-2'-fluoro-beta-adenosine** (2.50 g, 8.25 mmol) was sequentially added imidazole (1.12 g, 16.5 mmol) and tert-butyldimethyl chlorosilane (1.31 g, 8.66 mmol), and the resulting mixture was stirred at 20-25°C for 16 hours. The reaction mixture was poured into water (150 mL), and extracted twice with ethyl acetate (100 mL each). The organic phases were combined, washed twice with saturated brine (100 mL each) and dried over anhydrous sodium sulfate. The crude product obtained by concentration was purified by a silica gel column, eluting with petroleum ether : ethyl acetate (2:3) to give a white solid (2.53 g).

$^1$H NMR (400 MHz, DMSO-$d_6$) δ 8.18 (s, 1H), 7.89 (brs, 2H), 6.33 (dd, *J* = 12.0, 4.8 Hz, 1H), 6.00 (d, *J* = 4.8 Hz, 1H), 5.28 (dt, *J* = 52, 4.8 Hz, 1H), 4.50-4.30 (m, 1H), 3.98-3.73 (m, 3H), 0.89 (s, 9H), 0.07 (s, 6H).

**Preparation of Intermediate 2: (*2S,3aR,6S,7aR*)-2-((*2R,3R,4S,SR*)-5-(6-amino-2-chloro-9H-purin-9-yl)-2-((tert-butyldimethylsilyl)oxy)methyl)-4-fluorotetrahydrofuran-3-yl)oxy)-3a-methyl-6-(isopropyl-1-ene-2-yl)hexahydrobenzo[d][1,3,2]oxathiophospholane 2-sulfide**

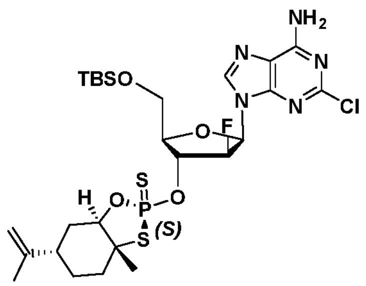

To a solution (10 mL) of **Intermediate 1** (1.00 g, 2.40 mmol) and **(+)-PSI reagent** (1.39 g, 3.12 mmol) in THF at 0°C was added 1,8-diazabicycloundeca-7-ene (474 mg, 3.12 mmol), and the resulting mixture was stirred at 0-5°C for 1 hour. The reaction mixture was diluted with ethyl acetate (50 mL). The organic phase was washed successively with 10% sodium dihydrogen phosphate aqueous solution (50 mL) and saturated brine (50 mL), and dried over anhydrous sodium sulfate. The crude product obtained by concentration was purified by silica gel column eluting with petroleum ether : ethyl acetate (3:7) to give a white solid (1.50 g).

$^1$H NMR (400 MHz, $CDCl_3$) δ 8.09 (s, 1H), 6.52-6.40 (m, 1H), 6.23 (brs, 2H), 5.55-5.40 (m, 1H), 5.30-5.06 (m, 2H), 4.92 (s, 1H), 4.52-4.40 (m, 1H), 4.20-4.12 (m, 1H), 4.08-3.90 (m, 2H), 2.62-2.52 (m, 1H), 2.36-2.23 (m, 1H), 2.20-2.08 (m, 1H), 2.02-1.80 (m, 3H), 1.80-1.62 (m, 7H), 0.93 (s, 9H), 0.12 (s, 6H). $^{31}$P NMR (162 MHz, $CDCl_3$) δ 101.9.

**Preparation of Intermediate 3: 2-Chloro-5'-O-tert-butyldimethylsilyl-2'-deoxyadenosine**

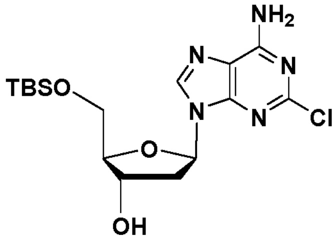

**Intermediate 3** was obtained from **2'-deoxyadenosine** via the route of **Intermediate 1.**

$^1$H NMR (400 MHz, DMSO-$d_6$) δ 8.29 (s, 1H), 7.82 (brs, 2H), 6.26 (t, *J* = 6.4 Hz, 1H), 5.37 (d, *J* = 4.0 Hz, 1H), 4.45-4.32 (m, 1H), 3.90-3.76 (m, 1H), 3.73-3.60 (m, 2H), 2.75-2.61 (m, 1H), 2.37-2.25 (m, 1H), 0.84 (s, 9H), 0.02 (s, 6H).

**Preparation of Intermediate 4: (*2S,3aR,6S,7aR*)-2-((*2R,3S,5R)*-5-(6-amino-2-chloro-9H-purin-9-yl)-2-((tert-butyldimethylsilyl)oxy)methyl)tetrahydrofuran-3-yl)oxy)-3a-methyl-6-(propyl-1-en-2-yl)hexahydrobenzo[d][1,3,2] oxathiophospholane 2-sulfide**

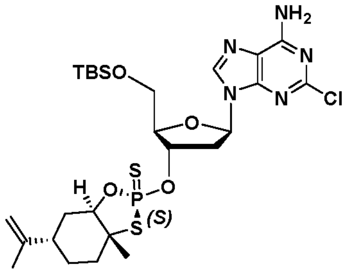

**Intermediate 4** was obtained from **Intermediate 3** and **(+)-PSI reagent** via the route of **Intermediate 2.**

$^1$H NMR (400 MHz, $CDCl_3$) δ 8.65 (s, 1H), 6.59-6.52 (m, 1H), 5.49-5.40 (m, 1H), 5.09 (s, 1H), 4.92 (s, 1H), 4.52-4.40 (m, 2H), 4.25-4.12 (m, 2H), 3.96 (s, 2H), 2.90-2.81 (m, 1H), 2.69-2.56 (m, 2H), 2.20-2.08 (m, 2H), 2.02-1.80 (m, 3H), 1.80-1.62 (m, 7H), 0.91 (s, 9H), 0.14 (s, 6H). $^{31}$P NMR (162 MHz, $CDCl_3$) δ 100.6.

**Preparation of Intermediate 5: 5'-O-tert-butyldimethylsilyl-2'-deoxy-2',2'-difluorocytidine**

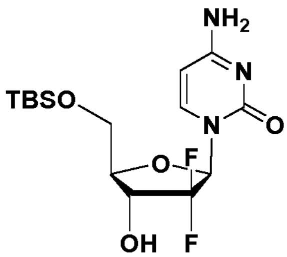

**Intermediate 5** was obtained from **2'-deoxy-2',2'-difluorocytidine** via the route of **Intermediate 1.**

$^1$H NMR (400 MHz, DMSO-$d_6$) δ 7.63 (d, *J* = 7.6 Hz, 1H), 7.37 (brs, 2H), 6.29 (d, *J* = 6.8 Hz, 1H), 6.14 (t, *J* = 7.6 Hz, 1H), 5.75 (d, *J* = 7.6 Hz, 1H), 4.15-4.02 (m, 1H), 3.98-3.73 (m, 3H), 0.90 (s, 9H), 0.09 (s, 6H).MS-ESI [M+H]$^+$: 378.2.

**Preparation of Intermediate 6: 4-Amino-1-((*2R*,*4R*,*5R*)-5-((tert-butyldimethylsilyl(oxy)methyl)-3,3-difluoro-4-(((*2S*,*3aR*,*6S*,*7aR)*-3a-methyl-6-(propyl-1-en-2-yl)-2-thiohexahydrobenzo[d][1,3,2]oxythiophospholan-2-yl)oxy) tetrahydrofuran-2-yl)pyrimidin-2(1H)-one**

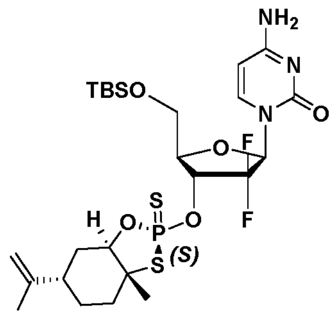

**Intermediate 6** was obtained from **Intermediate 5** and **(+)-PSI reagent** via the route of **Intermediate.**

$^1H$ NMR (400 MHz, DMSO-$d_6$) δ 7.73 (d, *J* = 8.0 Hz, 1H), 6.34 (t, *J* = 8.0 Hz, 1H), 6.21 (d, *J* = 8.0 Hz, 1H), 5.36-5.24 (m, 1H), 5.04 (s, 1H), 4.87 (s, 1H), 4.55-4.43 (m, 1H), 4.17-4.06 (m, 1H), 4.05-3.86 (m, 2H), 2.66-2.52 (m, 1H), 2.30-2.05 (m, 2H), 2.02-1.80 (m, 3H), 1.80-1.62 (m, 7H), 0.92 (s, 9H), 0.13 (s, 3H), 0.12 (s, 3H).

$^{31}P$ NMR (162 MHz, $CDCl_3$) δ 103.0. MS-ESI $[M+H]^+$: 624.2.

**Preparation of Intermediate 7: N4-(2-propylpentanoyl)-2'-deoxy-2',2'-difluorocytidine**

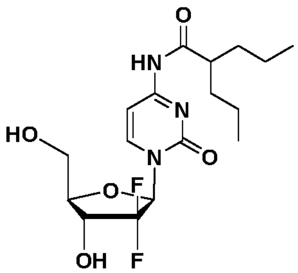

To a solution of **2'-deoxy-2',2'-difluorocytidine hydrochloride** (2.00 g, 6.67 mmol) in pyridine (20 mL) at 0°C was added dropwise trimethylchlorosilane (3.61 g, 33.4 mmol), and the resulting mixture was stirred at 0-5°C for 2 hours. Meanwhile, carbonyldiimidazole (1.19 g, 7.33 mmol) was added in portions to a solution of 2-propylvaleric acid (1.06 g, 7.33 mmol) in acetonitrile (20 mL), and the resulting mixture was stirred at 25°C for 2 hours. Then, the obtained acetonitrile mixture was added dropwise to the previous 0°C pyridine mixture, and the resulting mixture was stirred at 45°C for 16 hours. Ethanol (20 mL) was added to the above mixture, and stirred at 45°C for 0.5 hours. Then water (20 mL) was added to the above mixture and stirred at 45°C for 1 hour. The resulting reaction solution was rotary evaporated to dry and diluted with water (50 mL) . The pH of the above mixture was adjusted to 2-3 with 2*N* aqueous hydrochloric acid, and extracted twice with ethyl acetate (50 mL each). The organic phases were combined, washed twice with water (50 mL each) and dried over anhydrous sodium sulfate. The crude product obtained by concentration was purified by a silica gel column eluting with petroleum ether: ethyl acetate (2:3) to give a white solid (1.20 g) .

$^1H$ NMR (400 MHz, DMSO-$d_6$) δ 11.06 (brs, 1H), 8.25 (d, *J* = 8.0 Hz, 1H), 7.33 (d, *J* = 8.0 Hz, 1H), 6.32 (d, *J* = 8.0 Hz, 1H), 6.17 (t, *J* = 8.0 Hz, 1H), 5.32-5.25 (m, 1H), 4.25-4.10 (m, 1H), 3.97-3.75 (m, 2H), 3.70-3.58 (m, 1H), 2.70-2.55 (m, 1H), 1.56-1.42 (m, 2H), 1.38-1.10 (m, 6H), 0.85 (t, *J* = 7.6 Hz, 6H). MS-ESI $[M+H]^+$: 390.2.

**Preparation of Intermediate 8: N4-(2-propylpentanoyl)-5'-O-tert-butyldimethylsilyl-2'-deoxy-2',2'-difluorocytidine**

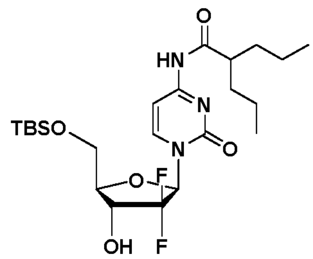

**Intermediate 8** was obtained from **Intermediate 7** via the route of **Intermediate 1.**

$^1H$ NMR (400 MHz, $CDCl_3$) δ 9.07 (brs, 1H), 8.27 (brs, 1H), 7.51 (d, *J* = 8.0 Hz, 1H), 6.45-6.30 (m, 1H), 4.46-4.33 (m, 1H), 4.12-4.00 (m, 2H), 3.91 (dd, *J* = 8.0, 2.4 Hz, 1H), 2.25-2.12 (m, 1H), 1.71-1.60 (m, 2H), 1.56-1.42 (m, 2H), 1.40-1.25 (m, 4H), 1.05-0.85 (m, 15H), 0.13 (s, 6H).

**Preparation of Intermediate 9: *N*-(1-((*2R,4R,5R*)-5-((tert-butyldimethylsilyl)oxy) methyl)-3,3-difluoro-4-(((*2S,3aR,6S,7aR)*-3a-methyl-6-(propyl-1-en-2-yl)-2-thiohexahydrobenzo[d][1,3,2]oxythiophospholan-2-yl)oxytetrahydrofuran-2-yl)-2-oxo-1,2-dihydropyrimidin-4-yl)-2-propylpentanamide**

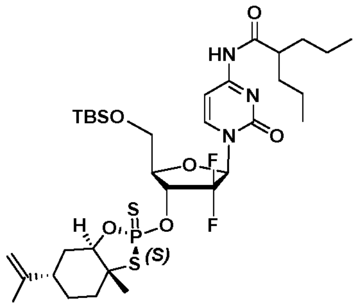

**Intermediate 9** was obtained from **Intermediate 8** and **(+)-PSI reagent** via the route of **Intermediate 2.**

$^1H$ NMR (400 MHz, $CDCl_3$) δ 9.63 (brs, 1H), 8.29-8.15 (m, 1H), 7.60-7.48 (m, 1H), 6.48-6.39 (m, 1H), 5.40-5.25 (m, 1H), 5.04 (s, 1H), 4.88 (s, 1H), 4.55-4.45 (m, 1H), 4.28-4.05 (m, 1H), 4.00-3.88 (m, 2H), 2.65-2.55 (m, 1H), 2.46-2.35 (m, 1H), 2.33-2.23 (m, 1H), 2.20-1.80 (m, 5H), 1.79-1.60 (m, 8H), 1.58-1.45 (m, 2H), 1.45-1.25 (m, 4H), 0.98-0.85 (m, 15H), 0.15 (d, *J* = 4.0 Hz, 6H).

**Preparation of Intermediate 10: N6-bis(4-methoxyphenyl)benzyl-2-chloro-5'-O-tert-butyldimethylsilyl-3'-bis(4-methoxyphenyl)benzyl-2'-deoxy-2'-fluoro-beta-adenosine**

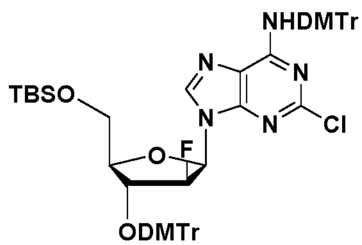

To a solution of **Intermediate 1** (1.53 g, 3.67 mmol) in pyridine (10 mL) was added bis(4-methoxyphenyl)benzyl chloride (1.49 g, 4.40 mmol), and the resulting mixture was stirred at 25°C for 2 hours. Then bis(4-methoxyphenyl)benzyl chloride (497 mg, 1.47 mmol) and 1,8-diazabicycloundec-7-ene (669 mg, 4.40 mmol) were added successively, and the resulting mixture was stirred at 20-25°C for 16 hours. Then bis(4-methoxyphenyl)benzyl chloride (1.49 g, 4.40 mmol) and 1,8-diazabicycloundec-7-ene (669 mg, 4.40 mmol) were added successively, and the resulting mixture was stirred at 20-25°C for 24 hours. The reaction mixture was concentrated and diluted with ethyl acetate (100 mL). The organic

phase was washed twice with saturated brine (100 mL each) and dried over anhydrous sodium sulfate. The crude product obtained by concentration was purified by silica gel column eluting with petroleum ether: ethyl acetate (5:1) to give a white solid (2.60 g).

$^1$H NMR (400 MHz, DMSO-$d_6$) δ 8.00 (s, 1H), 7.95 (s, 1H), 7.50-7.10 (m, 18H), 6.90-6.78 (m, 8H), 6.33-6.20 (m, 1H), 4.46-4.30 (m, 2H), 4.25-4.15 (m, 1H), 3.76-3.68 (m, 12H), 3.55-3.49 (m, 2H), 0.76 (s, 9H),-0.08 (s, 3H), -0.11 (s, 3H).

**Preparation of Intermediate 11: N6-bis(4-methoxyphenyl)benzyl-2-chloro-3'-bis (4-methoxyphenyl)benzyl-2'-deoxy-2'-fluoro-beta-adenosine**

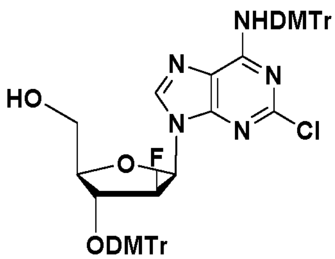

To a solution of **Intermediate 10** (2.60 g, 2.55 mmol) in tetrahydrofuran (15 mL) was added tetrabutylammonium fluoride (1.33 g, 5.10 mmol), and the resulting mixture was stirred at 25°C for 1 hour. The reaction mixture was concentrated and diluted with ethyl acetate (30 mL). The organic phase was washed with water (30 mL) and dried over anhydrous sodium sulfate. The crude product obtained by concentration was purified by a silica gel column eluting with petroleum ether : ethyl acetate (2:3) to give a white solid (1.20 g).

$^1$H NMR (400 MHz, DMSO-$d_6$) δ 8.17 (d, *J* = 2.4 Hz, 1H), 7.97 (s, 1H), 7.50-7.10 (m, 18H), 6.90-6.78 (m, 8H), 6.30-6.18 (m, 1H), 4.86 (t, *J* = 6.0 Hz, 1H), 4.30-4.10 (m, 3H), 3.76-3.68 (m, 12H), 3.49-3.30 (m, 2H).

**Preparation of Intermediate 12: N6-bis(4-methoxyphenyl)benzyl-2-chloro-5'-O-tert-butyldimethylsilyl-3'-bis(4-methoxyphenyl) base) benzyl-2'-deoxyadenosine**

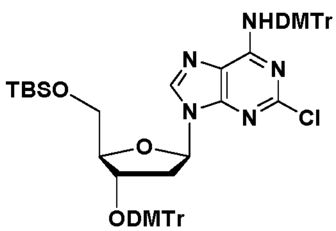

To a solution of **Intermediate 3** (1.80 g, 4.51 mmol) in pyridine (10 mL) was added bis(4-methoxyphenyl)benzyl chloride (3.81 g, 11.3 mmol) and 1,8- diazabicycloundec -7-ene (743 mg, 11.3 mmol), and the resulting mixture was stirred at 20-25°C for 16 hours. The reaction mixture was concentrated, poured into water (100 mL), and extracted twice with ethyl acetate (50 mL each). The organic phases were combined, washed twice with saturated brine (50 mL each) and dried over anhydrous sodium sulfate. The crude product obtained by concentration was purified by silica gel column eluting with petroleum ether : ethyl acetate (3:7) to give a white solid (2.50 g).

$^1$H NMR (400 MHz, DMSO-$d_6$) δ 8.19 (s, 1H), 7.83 (s, 1H), 7.50-7.10 (m, 18H), 6.95-6.78 (m, 8H), 6.28-6.18 (m, 1H), 4.36-4.28 (m, 1H), 4.00-3.88 (m, 1H), 3.80-3.65 (m, 12H), 3.50-3.41 (m, 1H), 3.40-3.30 (m, 1H), 2.33-2.25 (m, 1H), 1.88-1.76 (m, 1H), 0.71 (s, 9H), -0.14 (s, 6H).

**Preparation of Intermediate 13: N6-bis(4-methoxyphenyl)benzyl-2-chloro-3'-bis (4-methoxyphenyl)benzyl-2'-deoxyadenosine**

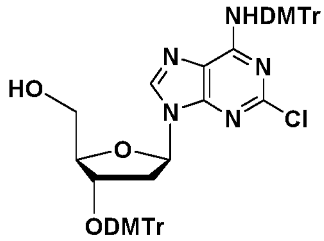

**Intermediate 13** was obtained from **Intermediate 12** via **the route** of **Intermediate 11.**

$^1$H NMR (400 MHz, DMSO-$d_6$) δ 8.32 (s, 1H), 7.87 (s, 1H), 7.50-7.10 (m, 18H), 6.95-6.77 (m, 8H), 6.24 (t, *J* = 6.8 Hz, 1H), 4.83 (brs, 1H), 4.39-4.28 (m, 1H), 3.85-3.65 (m, 13H), 3.22-3.12 (m, 1H), 2.35-2.21 (m, 1H), 1.88-1.70 (m, 1H).

**Preparation of Intermediate 14: 3'-5'-Di-O-tert-butyldimethylsilyl-2'-deoxy-2' ,2'-difluorocytidine**

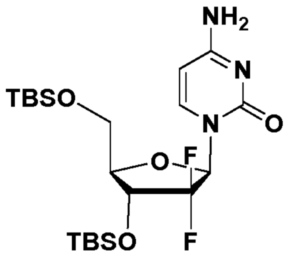

To a solutionof **2'-deoxy-2',2'-difluorocytidine hydrochloride** (3.00 g, 10.0 mmol) in DMF (50 mL) was sequentially added imidazole (3.41 g, 50.0 mmol) and tert-butyldimethylsilyl chloride (4.53 g, 30.0 mmol), and the resulting mixture was stirred at 20-25°C for 2 hours, then at 60°C for 16 hours. The reaction mixture was poured into a mixture of ethyl acetate (100 mL) and water (100 mL). The organic phase was separated, washed successively with water (100 mL) and saturated brine (100 mL), and dried over anhydrous sodium sulfate. The crude product obtained by concentration was purified by a silica gel column eluting with dichloromethane : methanol (9:2) to give a white solid (2.70 g).

$^1$H NMR (400 MHz, DMSO-$d_6$) δ 7.53 (d, *J* = 7.6 Hz, 1H), 7.40 (brs, 2H), 6.17 (t, *J* = 8.0 Hz, 1H), 5.78 (d, *J* = 7.6 Hz, 1H), 4.40-4.22 (m, 1H), 3.98-3.83 (m, 2H), 3.78-3.70 (m, 1H), 1.00-0.75 (m, 18H), 0.15-0.00 (m, 12H).

**Preparation of Intermediate 15: 3'-O-tert-butyldimethylsilyl-2'-deoxy-2',2'-difluorocytidine**

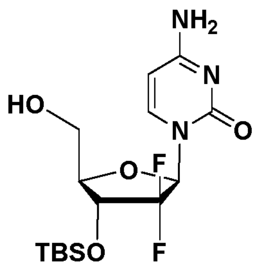

**Intermediate 14** (1.00 g, 2.04 mmol) was dissolved in a mixture of tetrahydrofuran (10 mL), trifluoroacetic acid (5 mL) and water (5 mL) at 0°C and stirred at 0-5°C for 2 hours. The pH of the reaction mixture was adjusted to about 9 with saturated sodium bicarbonate aqueous solution, then poured into a mixture of ethyl acetate (50 mL) and water (50 mL). The organic phase was separated, washed with saturated brine (50 mL) and dried over anhydrous sodium sulfate. The crude product obtained by concentration was purified by silica gel column eluting with ethyl acetate : methanol (17:3) to give a white solid (620 mg).

$^1$H NMR (400 MHz, DMSO-$d_6$) δ 7.67 (d, *J* = 7.6 Hz, 1H), 7.50-7.35 (m, 2H), 6.14 (t, *J* = 8.0 Hz, 1H), 5.79 (d, *J* = 7.6 Hz, 1H), 5.26 (d, *J* = 7.6 Hz, 1H), 4.40-4.22 (m, 1H), 3.86-3.72 (m, 2H), 3.68-3.50 (m, 1H), 0.88 (s, 9H), 0.11 (s,

3H), 0.10 (s, 3H). MS-ESI [M+H]+:378.2.

**Preparation of Intermediate 16: N4-(2-propyl-pentanoyl)-3'-5'-di-O-tert-butyldimethylsilyl-2'-deoxy-2',2'-difluoro cytidine**

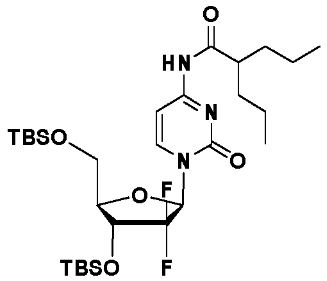

To a solution of 2-propylvaleric acid (322 mg, 2.24 mmol) in acetonitrile (5 mL) was added carbonyldiimidazole (363 mg, 2.24 mmol) at 25°C, and the resulting mixture was stirred at 25°C for 2 hours. The resulting mixture was added dropwise to a solution of **Intermediate 14** (1.0 g, 2.03 mmol) in pyridine (10 ml) at 0°C. The resulting mixture was stirred at 45°C for 16 hours, the resulting reaction solution was rotary evaporated to dry, diluted with water (20 mL), and extracted twice with ethyl acetate (30 mL each). The organic phases were combined, washed twice with water (50 mL each) and dried over anhydrous sodium sulfate. The crude product obtained by concentration was purified by silica gel column with petroleum ether : ethyl acetate (1:1) to give a white solid (430 mg).

$^1$H NMR (400 MHz, $CDCl_3$) δ 8.09 (d, *J* = 7.6 Hz, 1H), 7.49 (d, *J* = 7.6 Hz, 1H), 6.33 (d, *J* = 10.0 Hz, 1H), 4.39-4.29 (m, 1H), 4.02 (d, *J* = 12.0 Hz, 1H), 3.96 (d, *J* = 7.6 Hz, 1H), 3.81 (d, *J* = 11.2 Hz, 1H), 2.50-2.30 (m, 1H), 1.49-1.44 (m, 2H), 1.33 (q, J = 7.6 Hz, 6H), 0.99-0.80 (m, 24H), 0.13 (s, 9H), 0.10 (s, 3H). MS-ESI [M+H]+: 619.4.

**Preparation of Intermediate 17: N4-(2-propyl-pentanoyl)-3'-O-tert-butyldimethylsilyl-2'-deoxy-2',2'-difluorocytidine**

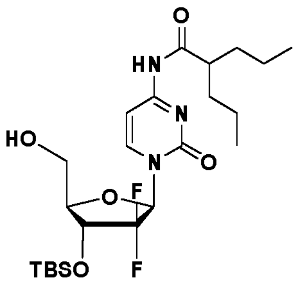

**Intermediate 17** was obtained from **Intermediate 16** via the route of **Intermediate 15.**

$^1$H NMR (400 MHz, $CDCl_3$) δ 8.38 (s, 1H), 8.00 (d, *J* = 7.6 Hz, 1H), 7.50 (d, *J* = 7.6 Hz, 1H), 6.25 (t, *J* = 7.6 Hz, 1H), 4.49-4.40 (m, 1H), 4.08 (d, *J* = 12.4 Hz, 1H), 4.02-3.90 (m, 1H), 3.88-3.75 (m, 1H), 2.51 (s, 1H), 2.40-2.30 (m, 1H), 1.75-1.55 (m, 2H), 1.52-1.41 (m, 2H), 1.38-1.28 (m, 4H), 0.95-0.85 (m, 15H), 0.14 (d, *J*= 2.8 Hz, 6H). MS-ESI [M+H]+:504.2.

**Intermediate 18: 5'-O-tert-butyldimethylsilyl-2'-tert-butyldimethylsilyl-adenosine, and**

**Intermediate 19: 5'-O-tert-butyldimethylsilyl-3'-tert-butyldimethylsilyl-adenosine**

To a solution of adenosine (3.35 g, 12.5 mmol) in tetrahydrofuran (40 mL) was added successively triethylenediamine (7.0 g, 62.5 mmol) and silver nitrate (5.08 g, 28.8 mmol) at room temperature (10°C). The mixture was stirred at room temperature (10°C) for 30 min, then tert-butyldimethylsilyl chloride (4.71 g, 30 mmol) was added, and the resulting mixture was stirred at 10°C for 16 hours. The reaction mixture was filtered through celite and the filtrate was quenched with water (50 mL), then extracted twice with ethyl acetate (50 mL each). The organic phases were combined, washed

twice with saturated brine (100 mL each) and dried over anhydrous sodium sulfate. The crude product obtained by concentration was purified by silica gel column eluting with petroleum ether : ethyl acetate (1:1) to give **Intermediate 18** (1.0 g, white solid). Elution with ethyl acetate afforded **Intermediate 19** (4.4 g, white solid).

**Intermediate 18:**

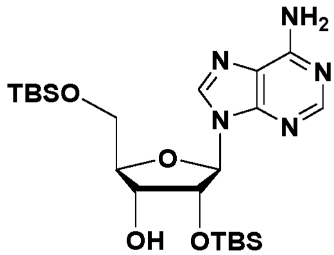

$^1$H NMR (400 MHz, $CDCl_3$) δ 8.35 (s, 1H), 8.24 (s, 1H), 6.11 (d, *J* =4.8 Hz, 1H), 5.72 (brs, 2H), 4.64 (t, *J* = 4.8 Hz, 1H), 4.31-4.26 (m, 1H), 4.24-4.20 (m, 1H), 4.02 (dd, *J* = 11.6, 2.8 Hz, 1H), 3.87 (dd, *J* = 11.6, 2.8 Hz, 1H), 2.76 (d, *J* = 3.6 Hz, 1H), 0.96 (s, 9H), 0.84 (s, 9H), 0.18-0.11 (m, 6H), -0.05 (s, 3H), -0.12 (s, 3H).

**Intermediate 19:**

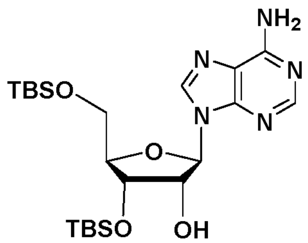

$^1$H NMR (400 MHz, $CDCl_3$) δ 8.35 (s, 1H), 8.10 (s, 1H), 6.03 (d, *J* = 3.6 Hz, 1H), 5.75 (brs, 2H), 4.56 (d, *J* = 1.6 Hz, 1H), 4.12 (d, *J* = 2.8 Hz, 1H), 3.92 (dd, *J* = 11.6, 3.6 Hz, 1H), 3.77 (dd, *J* = 11.2, 2.8 Hz, 1H), 3.31 (brs, 1H), 0.95 (s, 9H), 0.90 (s, 9H), 0.17 (s, 6H), 0.06 (s, 3H), -0.01 (s, 3H).

**Preparation of Ontermediate 20: N4-benzoyl-2'-5'-di-O-tert-butyldimethylsilyl-3'-benzoyl-adenosine**

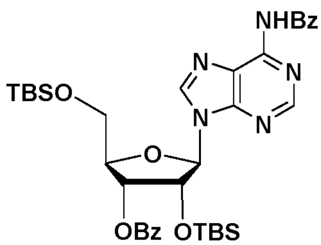

To a solution of **Intermediate 18** (500 mg, 1.01 mmol) and *N*-methylimidazole (248 mg, 3.03 mmol) in dichloromethane (10 mL) was added dropwise benzoyl chloride (354.4 mg, 2.52 mmol) at room temperature (25°C). The resulting mixture was reacted at room temperature for 16 hours. The reaction solution was quenched with water (20 mL), the organic phase was separated, and the aqueous phase was extracted twice with dichloromethane (20 mL each). The combined organic phases were washed once with saturated brine (20 mL), and then dried over anhydrous sodium sulfate. The crude product obtained by concentration was purified by silica gel column eluting with petroleum ether :

ethyl acetate (1:1) to give a white solid (400 mg).

$^1$H NMR (400 MHz, $CDCl_3$) δ 9.36 (s, 1H), 8.85 (s, 1H), 8.48 (s, 1H), 8.15-8.10 (m, 2H), 8.08-8.05 (m, 2H), 7.70-7.55 (m, 2H), 7.55-7.47 (m, 4H), 6.33 (d, J = 6.0 Hz, 1H), 5.63-5.50 (m, 1H), 4.89 (d, J = 1.2 Hz, 1H), 4.48 (d, *J* = 2.4 Hz, 1H), 4.05 (d, *J* = 2.4 Hz, 1H), 4.00 (d, *J*= 2.4 Hz, 1H), 0.99 (s, 9H), 0.65 (s, 9H), 0.19 (d, J = 4.4 Hz, 6H), -0.10 (s, 3H), -0.26 (s, 3H). MS-ESI $[M+H]^+$:704.3.

**Preparation of Intermediate 21: N4-benzoyl-2'-O-tert-butyldimethylsilyl-3'-benzoyl-adenosine**

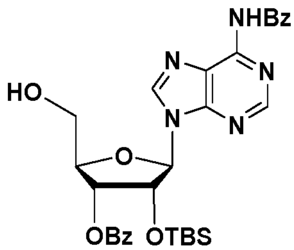

**Intermediate 21** was obtained from **Intermediate 20** via the route of **Intermediate 15.**

$^1$H NMR (500 MHz, $CDCl_3$) δ 8.86 (s, 1H), 8.17-8.09 (m, 3H), 8.08-8.03 (m, 2H), 7.66 -7.60 (m, 2H), 7.55 (t, *J* = 7.6 Hz, 2H), 7.50 (t, *J* = 7.6 Hz, 2H), 5.97 (d, *J* = 7.6 Hz, 1H), 5.75 (d, *J* = 5.2 Hz, 1H), 5.27 (dd, J = 7.6, 5.2 Hz, 1H), 4.51 (s, 1H), 4.08-4.03 (m, 1H), 3.93 (d, *J* = 12.8 Hz, 1H), 0.62 (s, 9H), -0.10 (s, 3H), -0.43 (s, 3H). MS-ESI $[M+H]^+$:591.2.

**Preparation of Intermediate 22: _N4-benzoyl-5'-O-tert-butyldimethylsilyl-3'-tert-butyldimethylsilyl-2'-benzoyl-adenosine**

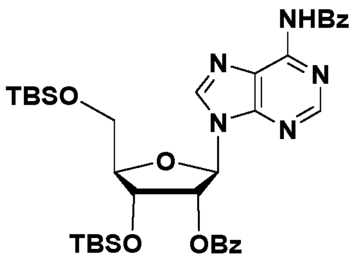

**Intermediate 22** was obtained from **Intermediate 19** and benzoyl chloride via the route of **Intermediate 20.**

$^1$H NMR (400 MHz, $CDCl_3$) δ 9.02 (brs, 1H), 8.83 (s, 1H), 8.41 (s, 1H), 8.08-7.99 (m, 4H), 7.64-7.49 (m, 4H), 7.47-7.39 (m, 2H), 6.54 (d, *J* = 6.0 Hz, 1H), 5.81 (t, *J* = 5.6 Hz, 1H), 4.84 (dd, *J* = 4.8, 3.6 Hz, 1H), 4.26 (dd, *J* = 6.0, 3.2 Hz, 1H), 4.02 (dd, *J* = 11.6, 3.2 Hz, 1H), 3.85 (dd, *J* = 11.6, 2.8 Hz, 1H), 0.95 (s, 9H), 0.84 (s, 9H), 0.18-0.11 (m, 6H), 0.03--0.05 (m, 6H).

**Preparation of Intermediate 23: N4-benzoyl-3'-tert-butyldimethylsilyl-2'-benzoyl-adenosine**

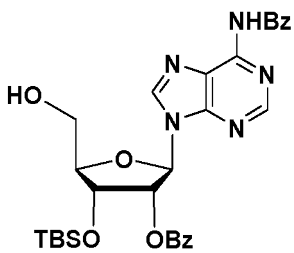

**Intermediate 23** was obtained from **Intermediate 22** via the route of **Intermediate 15.**

$^1$H NMR (400 MHz, $CDCl_3$) δ 9.08 (brs, 1H), 8.84 (s, 1H), 8.18 (s, 1H), 8.08-7.98 (m, 4H), 7.67-7.41 (m, 6H), 6.32 (d, *J* = 7.6 Hz, 1H), 6.01 (dd, *J* = 7.6, 5.2 Hz, 1H), 4.94 (d, *J* = 5.2 Hz, 1H), 4.35 (d, *J* = 0.8 Hz, 1H), 4.07 (dd, *J* = 13.2, 1.6 Hz, 1H), 3.84 (d, *J* = 12.4 Hz, 1H), 0.89 (s, 9H), 0.11 (s, 3H), 0.00 (s, 3H).

**Preparation of Intermediate 24: N2-isobutyryl-5'-O-bis(4-methoxyphenyl) benzyl-3'-O-benzoyl-2'-O-tert-butyldimethylsilyl-guanosine**

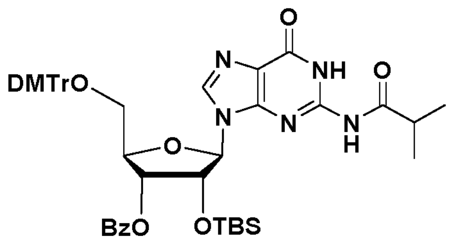

**Intermediate 24** was obtained from N2-isobutyryl-5'-O-bis(4-methoxyphenyl)benzyl-2'-O-tert-butyldimethylsilyl-guanosine via the route of **Intermediate 20.** MS-ESI [M+H]$^+$ :874.3.

**Preparation of Intermediate 25: N2-isobutyryl-3'-O-benzoyl-2'-O-tert-butyldimethylsilyl-guanosine**

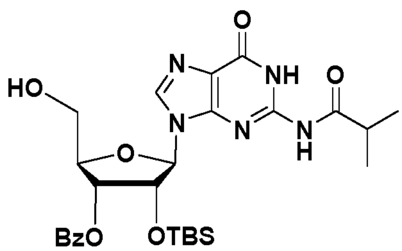

**Intermediate 24** (5.50 g, 6.30 mmol) was dissolved in a mixture of glacial acetic acid (60 mL) and water (10 mL), stirred at 25°C **for** 2 hours. The reaction mixture was rotary evaporated to dry and co-evaporated twice with ethyl acetate (25 mL each). The crude product was purified by silica gel column eluting with ethyl acetate to give a white solid (2.2 g).

$^1$H NMR (400 MHz, $CDCl_3$) δ 12.15 (brs, 1H), 8.35 (s, 1H), 8.15-8.02 (m, 2H), 7.74 (s, 1H), 7.68-7.56 (m, 1H), 7.55-7.42 (m, 2H), 5.80 (d, *J* = 7.6 Hz, 1H), 5.66 (dd, *J* = 5.6, 1.2 Hz, 1H), 5.52 (d, *J* = 9.2 Hz, 1H), 4.98 (dd, *J* = 7.2, 5.6 Hz, 1H), 4.45 (d, *J* = 1.2 Hz, 1H), 4.06-3.82 (m, 2H), 2.78-2.62 (m, 1H), 1.35-1.25 (m, 6H), 0.66 (s, 9H), -0.09 (s, 3H), -0.33 (s, 3H). MS-ESI [M+H]$^+$:572.3.

**Preparation of Intermediate 26: N2-isobutyryl-5'-O-bis(4-methoxyphenyl) benzyl-3'-tert-butyldimethylsilyl-2'-benzoyl-guanosine**

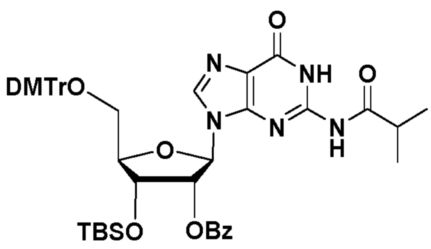

**Intermediate 26** was obtained from N2-isobutyryl-5'-O-bis(4-methoxyphenyl)benzyl-3'-O-tert-butyldimethylsilyl-**g**uanosine via the route of **Intermediate 20.** MS-ESI [M+H] $^{+}$ :874.3.

**Preparation of Intermediate 27: N2-isobutyryl-3'-tert-butyldimethylsilyl-2'-benzoyl-guanosine**

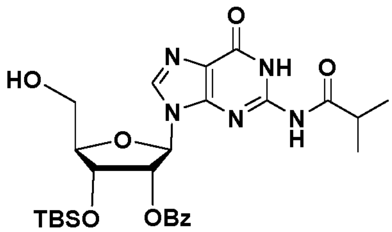

**Intermediate 27** was obtained from **Intermediate 26** via the route of **Intermediate 25.**

$^{1}$H NMR (400 MHz, $CDCl_3$) δ 12.06 (brs, 1H), 8.24 (brs, 1H), 8.01-7.96 (m, 2H), 7.83 (s, 1H), 7.62-7.55 (m, 1H), 7.43 (d, *J* = 8.0 Hz, 2H), 6.13 (d, *J* = 7.2 Hz, 1H), 5.81 (dd, *J* = 7.2, 5.6 Hz, 1H), 4.80 (dd, *J* = 4.8, 1.6 Hz, 1H), 4.30-4.25 (m, 1H), 4.02 (dd, *J* = 12.8, 2.0 Hz, 1H), 3.78 (d, *J* = 12.4 Hz, 1H), 2.71-2.60 (m, 1H), 1.30-1.28 (m, 6H), 0.83 (s, 9H), 0.07 (s, 3H), 0.00 (s, 3H).

**Preparation of Intermediate 28: 2'-5'-Di-O-tert-butyldimethylsilyl-guanosine**

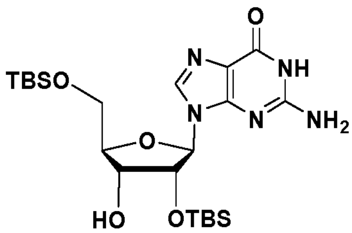

**Intermediate 28** was obtained from **guanosine** via the route of **Intermediate 18.** MS-ESI [M+H]$^{+}$:513.3.

**Preparation of Intermediate 29: 2'-5'-Di-O-tert-butyldimethylsilyl-3'-benzoyl-guanosine**

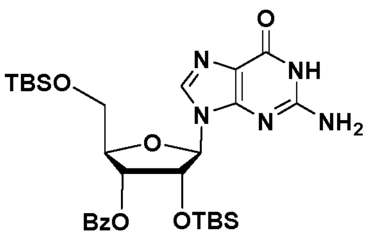

**Intermediate 29** was obtained from **Intermediate 28** via the route of **Intermediate 20.**

$^{1}$H NMR (400 MHz, $CDCl_3$) δ 12.04 (brs, 1H), 8.14-8.09 (m, 2H), 7.94 (s, 1H), 7.61 (t, *J* = 7.6 Hz, 1H), 7.49 (t, *J* = 7.6 Hz, 2H), 6.09 (s, 2H), 6.00 (d, *J* = 6.0 Hz, 1H), 5.53 (s, 1H), 4.76 (t, *J* = 5.6 Hz, 1H), 4.41 (d, *J* = 2.8 Hz, 1H), 4.01 (d, *J* = 11.6 Hz, 1H), 3.93 (dd, *J* = 11.6, 2.4 Hz, 1H), 0.97 (s, 9H), 0.70 (s, 9H), 0.16 (d, *J* = 3.2 Hz, 6H), -0.09 (s, 3H), -0.16 (s, 3H). MS-ESI [M-H]$^{-}$:615.3.

Preparation of Intermediate 30: 2'-O-tert-butyldimethylsilyl-3'-benzoyl-guanosine

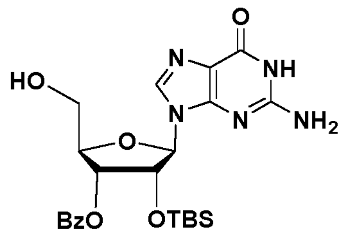

**Intermediate 30** was obtained from **Intermediate 29** via the route of **Intermediate 15.**

$^1$H NMR (400 MHz, DMSO-$d_6$) δ 10.71 (brs, 1H), 8.05 (d, *J* = 7.2 Hz, 3H), 7.75-7.68 (m, 1H), 7.59 (t, J = 7.6 Hz, 2H), 6.51 (s, 2H), 5.84 (d, J = 7.6 Hz, 1H), 5.58 (dd, J = 5.6, 1.6 Hz, 1H), 5.42 (t, J = 5.6 Hz, 1H), 4.96 (dd, J = 7.6, 5.2 Hz, 1H), 4.39-4.25 (m, 1H), 4.20-4.00 (m, 1H), 0.57 (s, 9H), -0.12 (s, 3H), -0.29 (s, 3H). MS-ESI [M-H]$^-$:501.3.

Preparation of Intermediate 31: 3'-5'-Di-O-tert-butyldimethylsilyl-2'-deoxy-2'-fluoroadenosine

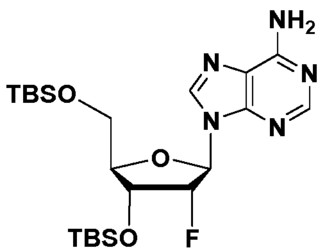

To a solution of **2'-deoxy-2'-fluoroadenosine** (1.10 g, 4.09 mmol) in DMF (10 mL) was added imidazole (1.81 g, 26.6 mmol) and 4-dimethylaminopyridine (50 mg, 0.41 mmol), and then tert-butyldimethylsilyl chloride (2.16 g, 14.3 mmol) was added, and the resulting mixture was stirred at 15-20°C for 16 hours. The reaction mixture was poured into water (100 mL), extracted twice with ethyl acetate (100 mL each). The organic phases were combined, washed twice with saturated brine (100 mL) and dried over anhydrous sodium sulfate. The crude product obtained by concentration was purified by silica gel column eluting with petroleum ether : ethyl acetate (3:2) to give a white solid (1.90 g).

$^1$H NMR (400 MHz, $CDCl_3$) δ 8.35 (s, 1H), 8.12 (s, 1H), 6.25 (dd, *J* = 16.0, 2.4 Hz, 1H), 5.66 (brs, 2H), 5.48-5.20 (m, 1H), 4.79-4.62 (m, 1H), 4.18-3.95 (m, 2H), 3.85-3.72 (m, 1H), 1.00-0.80 (m, 18H), -0.05-0.18 (m, 12H). MS-ESI [M+H]$^+$:498.4.

Preparation of Intermediate 32: 3'-O-tert-butyldimethylsilyl-2'-deoxy-2'-fluoroadenosine

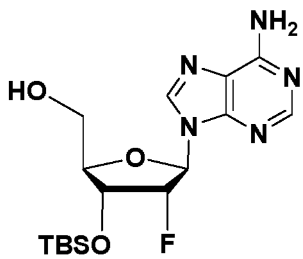

**Intermediate 31** was obtained from **Intermediate 31** via the route of **Intermediate 15.**

$^1$H NMR (400 MHz, DMSO-$d_6$) δ 8.37 (s, 1H), 8.14 (s, 1H), 7.38 (brs, 2H), 6.22 (dd, *J* = 16.4, 3.6 Hz, 1H), 5.70-5.40 (m, 1H), 5.29 (t, *J* = 5.6 Hz, 1H), 4.80-4.62 (m, 1H), 4.02-3.90 (m, 1H), 3.79-3.68 (m, 1H), 3.63-3.48 (m, 1H),

0.90 (s, 9H), 0.14 (s, 3H), 0.13 (s, 3H).

**Preparation of Intermediate 33: (*2R,3aS,6R,7aS)*-2-((*2R,3R,4R,5R*)-5-(6-amino-9H-purin-9-yl)-3-((tertbutyldimethylsilyl)oxy)-4-fluorotetrahydrofuran-2-yl)methoxy)-3a-methyl-6-(propyl-1-en-2-yl)hexahydrobenzo[d][1,3,2] oxathiophospholane 2-sulfide**

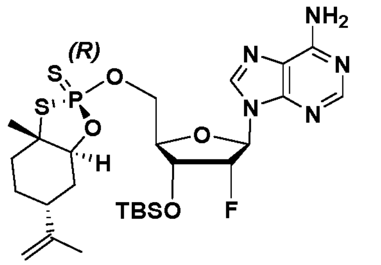

**Intermediate 33** was obtained from **Intermediate 32** and **(-)-PSI reagent** via the route of **Intermediate 2.**

$^{1}$H NMR (400 MHz, $CDCl_3$) δ 8.36 (s, 1H), 8.05 (s, 1H), 6.30-6.18 (m, 1H), 5.58 (brs, 2H), 5.50-5.22 (m, 1H), 4.80-4.62 (m, 3H), 4.50-4.25 (m, 4H), 2.59-2.48 (m, 1H), 2.33-2.20 (m, 1H), 2.15-2.02 (m, 1H), 1.99-1.76 (m, 3H), 1.75-1.55 (m, 7H), 0.93 (s, 9H), 0.19 (s, 3H), 0.15 (s, 3H).

**Preparation of Intermediate 34: 3'-5'-Di-O-tert-butyldimethylsilyl-2'-deoxy-2'-fluoroguanosine**

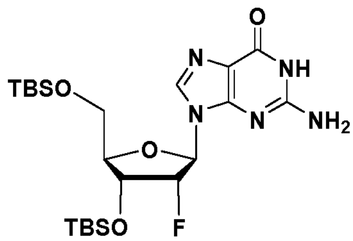

**Intermediate 34** was obtained from **2'-deoxy-2'-fluoroguanosine** via the route of **Intermediate 31.**

1H NMR (400 MHz, DMSO-$d_6$) δ 10.69 (brs, 1H), 7.84 (s, 1H), 6.52 (brs, 2H), 6.00 (d, *J* = 16.8 Hz, 1H), 5.36 (d, *J* = 52.0 Hz, 1H), 4.62-4.45 (m, 1H), 3.98-3.86 (m, 2H), 3.74 (d, *J* = 11.6 Hz, 1H), 0.93 -0.85 (m, 18H), 0.20-0.00 (m, 12H). MS-ESI $[M+H]^+$:515.3.

**Preparation of Intermediate 35: 3'-O-tert-butyldimethylsilyl-2'-deoxy-2'-fluoro-guanosine**

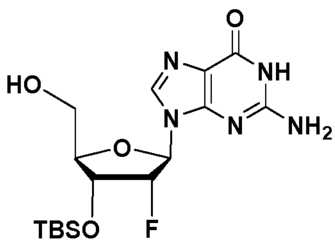

**Intermediate 35** was obtained from **Intermediate 34** via the route of **Intermediate 15.**

$^{1}$H NMR (400 MHz, DMSO-$d_6$) δ 10.71 (brs, 1H), 7.97 (s, 1H), 6.56 (s, 2H), 5.36 (dd, *J* = 52.5, 5.0 Hz, 1H), 5.20 (s, 1H), 4.52 (dd, *J* = 12.6, 6.4 Hz, 1H), 3.96-3.89 (m, 1H), 3.70 (d, *J* = 12.0 Hz, 1H), 0.89 (s, 9H), 0.12 (s, 6H). MS-ESI $[M+H]^+$:401.2.

**Preparation of Intermediate 36: 2-Amino-9-((*2R*,*3R*,*4R*,*5R*)-4-((tert-butyldimethylsilyl)oxy)-3-fluoro-5-((((2R,*3aS*,*6R*,*7aS*)-3a-methyl-6-(propyl-1-en-2-yl)-2-thiohexahydrobenzo[d][1,3,2]oxathiophospholane-2-yl)oxy)methyl) tetrahydrofuran-2-yl)-1,9-dihydro-6H-purin-6-one**

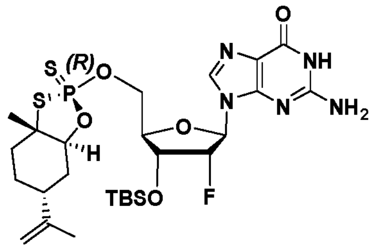

**Intermediate 36** was obtained from **Intermediate 35** and **(-)-PSI reagent** in DMF via the route of **Intermediate 2.** MS-ESI [M+H] $^+$ :646.4.

**Preparation of Intermediate 37: [3'-O-phosphorothioate-diester-2-chloro-5'-O-tert-butyldimethylsilyl-2'-deoxy-2'-fluoro-beta-adenosine]-(3',5')-[N6-bis(4-methoxyphenyl)benzyl-2-chloro-3'-bis(4-methoxyphenyl)benzyl-2'-deoxy-2'-fluoro-beta-adenosine]**

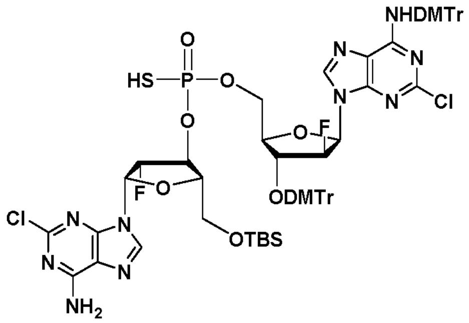

To a solution of **Intermediate 2** (280 mg, 0.422 mmol) and **Intermediate 11** (383 mg, 0.422 mmol) in acetonitrile (3 mL) was added 1,8-diazabicycloundec-7-ene (192 mg, 1.27 mmol), and the resulting mixture was stirred at 25°C for 1 hour. The reaction mixture was diluted with ethyl acetate (25 mL). The organic phase was washed successively with water (25 mL) and saturated brine (25 mL) and dried over anhydrous sodium sulfate. The crude product obtained by concentration was purified by silica gel column eluting with ethyl acetate : methanol (7:3) to give a white solid (260 mg).

$^1$HNMR (400 MHz, DMSO-$d_6$) δ 8.26 (s, 1H), 8.08 (s, 1H), 7.91 (brs, 3H), 7.45-7.10 (m, 18H), 6.95-6.76 (m, 8H), 6.32-6.15 (m, 2H), 5.40-5.18 (m, 2H), 5.00-4.80 (m, 2H), 4.40-4.02 (m, 3H), 3.85-3.62 (m, 15H), 0.84 (s, 9H), 0.01 (s, 6H).

**Preparation of Intermediate 38: [3'-O-phosphorothioate-diester-2-chloro-5'-O-tert-butyldimethylsilyl-2'-deoxyadenosine]-(3',5')-[N6-bis(4-methoxyphenyl)benzyl-2-chloro-3'-bis(4-methoxyphenyl)benzyl-2'-deoxyadenosine]**

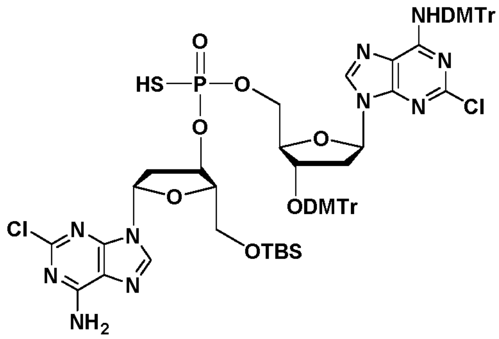

**Intermediate 38** was obtained from **Intermediate 4** and **Intermediate 13** in a mixed solution of tetrahydrofuran and acetonitrile via the route of **Intermediate 37.**

$^1$H NMR (400 MHz, DMSO-$d_6$) δ 8.68 (s, 1H), 8.22 (s, 1H), 7.82 (brs, 2H), 7.75 (s, 1H), 7.42 (d, $J$ = 8.0 Hz, 2H), 7.39-7.10 (m, 16H), 6.95-6.78 (m, 8H), 6.32-6.10 (m, 2H), 4.90-4.80 (m, 1H), 4.43-4.36 (m, 1H), 4.15-4.08 (m, 1H), 3.85-3.40 (m, 17H), 2.50-2.20 (m, 4H), 0.81 (s, 9H), 0.00 (s, 3H), -0.02 (s, 3H).

**Preparation of Intermediate 39: [3'-O-phosphorothioate-diester-5'-O-tert-butyldimethylsilyl-2'-deoxy-2',2'-difluorocytidine]-(3',5')-[3'-O-tert-butyldimethylsilyl-2'-deoxy-2',2'-difluorocytidine]**

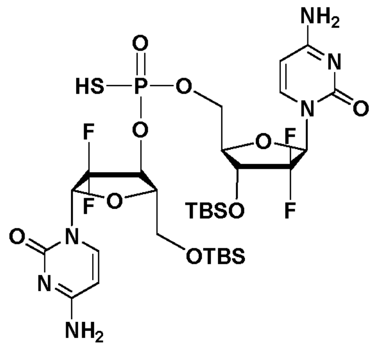

**Intermediate 39** was obtained from **Intermediate 6** and **Intermediate 15** in a mixed solution of tetrahydrofuran and acetonitrile via the route of **Intermediate 37.**

$^1$H NMR (400 MHz, DMSO-$d_6$) δ 9.58 (brs, 1H), 7.79-7.60 (m, 2H), 7.48-7.25 (m, 4H), 6.28-6.05 (m, 2H), 5.76 (dd, $J$ = 7.6, 1.6 Hz, 2H), 5.00-4.85 (m, 1H), 4.38-4.25 (m, 1H), 4.15-3.80 (m, 6H), 0.95-0.80 (m, 18H), 0.18-0.00 (m, 12H).

**Preparation of Intermediate 40: [3'-O-phosphorothioate-diester-N4-(2-propylpentanoyl)-5'-O-tert-butyldimethylsilyl-2'-deoxy-2',2'-difluorocytidine]-(3',5')-[N4-(2-propylpentanoyl)-3'-O-tert-butyldimethylsilyl-2'-deoxy-2',2'-difluorocytidine]**

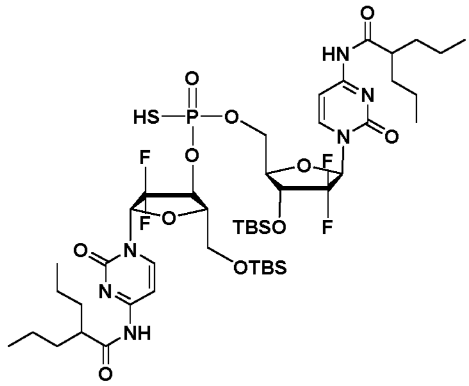

**Intermediate 40** was obtained from **Intermediate 17** and **Intermediate 9** via the route of **Intermediate 37.**

$^1$H NMR (500 MHz, DMSO-$d_6$) δ 11.07 (brs, 1H), 11.02 (brs, 1H), 8.32 (d, *J* = 7.6 Hz, 1H), 8.24 (d, *J* = 7.6 Hz, 1H), 7.36-7.30 (m, 2H), 6.28-6.15 (m, 2H), 4.97 (t, *J* = 10.4 Hz, 1H), 4.40-4.30 (m, 1H), 4.17 (s, 1H), 4.08 (q, *J* = 9.1, 8.5 Hz, 2H), 4.04*3.99 (m, 2H), 3.93 (t, *J* = 7.8 Hz, 1H), 2.62 (d, *J* = 3.2 Hz, 2H), 1.60-1.45 (m, 4H), 1.42-1.27 (m, 4H), 1.25-1.20 (m, 9H), 1.00-0.75 (m, 30H), 0.11 (d, *J* = 3.0 Hz, 6H), 0.05 (s, 3H), 0.02 (s, 3H).

**Preparation of Intermediate 41: [3'-O-phosphorothioate-diester-2-chloro-5'-O-tert-butyldimethylsilyl-2'-deoxy-2'-fluoro-beta-adenosine]-(3',5')-[N6-benzoyl-2'-O-tert-butyldimethylsilyl-3'-O-benzoyl-adenosine]**

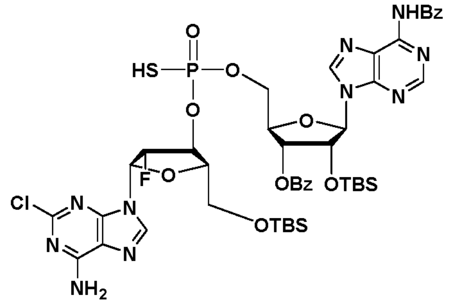

**Intermediate 41** was obtained from **Intermediate 21** and **Intermediate 2** via the route of **Intermediate 37.**

$^1$H NMR (400 MHz, DMSO-$d_6$) δ 11.19 (brs, 1H), 9.10 (brs, 1H), 8.76 (s, 1H), 8.12 (d, *J* = 2.4 Hz, 1H), 8.05 (dd, *J* = 7.6, 2.0 Hz, 4H), 7.89 (s, 2H), 7.75-7.61 (m, 2H), 7.60-7.45 (m, 4H), 6.33 (dd, *J* = 18.0, 4.0 Hz, 1H), 6.23 (d, *J* = 7.2 Hz, 1H), 5.77 (d, *J* = 5.2 Hz, 1H), 5.30 (dd, *J* = 7.6, 5.2 Hz, 1H), 5.04 (s, 1H), 4.53 (s, 1H), 4.21 (t, *J* = 10.0 Hz, 1H), 4.18-4.08 (m, 3H), 4.00-3.92 (m, 1H), 3.92-3.85 (m, 1H), 0.85 (s, 9H), 0.49 (s, 9H), 0.05 (s, 6H), -0.12 (s, 3H), -0.42 (s, 3H). MS-ESI [(M+2H)/2]$^+$: 543.7.

**Preparation of Intermediate 42: [3'-O-phosphorothioate-diester-2-chloro-5'-O-tert-butyldimethylsilyl-2'-deoxyadenosine]-(3',5')-[N6-benzoyl-2'-O-tert-butyldimethylsilyl-3'-O-benzoyl-adenosine]**

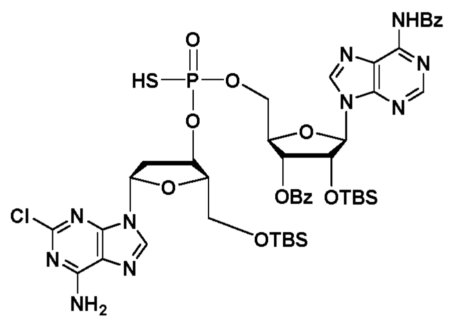

**Intermediate 42** was obtained from **Intermediate 21** and **Intermediate 4** via the route of **Intermediate 37.** MS-ESI [(M+H)] + : 1067.2.

**Preparation of Intermediate 43: [3'-O-phosphorothioate-diester-2-chloro-5'-O-tert-butyldimethylsilyl-2'-deoxy-2'-fluoro-beta-adenosine]-(3',5')-[N6-benzoyl-3'-O-tert-butyldimethylsilyl-2'-O-benzoyl-adenosine]**

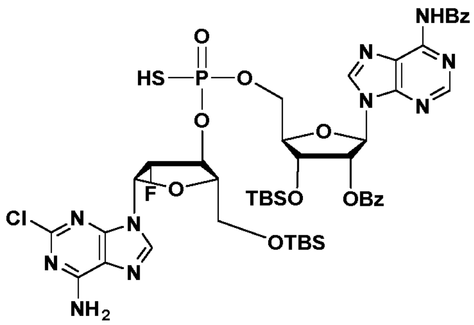

**Intermediate 43** was obtained from **Intermediate 23** and **Intermediate 2** in *N,N'*-dimethylformamide via the route of **Intermediate 37.**

$^1$H NMR (400 MHz, DMSO-$d_6$) δ 11.23 (brs, 1H), 8.96 (s, 1H), 8.76 (s, 1H), 8.11 (d, *J* = 2.4 Hz, 1H), 8.02 (d, *J* = 7.6 Hz, 2H), 7.88 (d, *J* = 7.6 Hz, 4H), 7.64 (dd, *J* = 6.8, 6.0 Hz, 2H), 7.65-7.45 (m, 4H), 6.51 (d, *J* = 5.6 Hz, 1H), 6.32 (dd, *J* = 18.8, 3.6 Hz, 1H), 5.92 (t, *J* = 5.6 Hz, 1H), 5.55-5.30 (m, 1H), 5.11-4.98 (m, 1H), 4.83 (t, *J* = 4.0 Hz, 1H), 4.29 (s, 1H), 4.28-4.15 (m, 1H), 4.15-4.05 (m, 1H), 4.00-3.89 (m, 2H), 3.87-.80 (m, 1H), 0.85 (s, 9H), 0.76 (s, 9H), 0.08 (s, 3H), 0.04 (s, 6H), -0.09 (s, 3H). MS-ESI [M+H]+: 1085.3.

**Preparation of Intermediate 44: [3'-O-phosphorothioate-diester-2-chloro-5'-O-tert-butyldimethylsilyl-2'-deoxy-2'-fluoro-beta-adenosine]-(3',5')-[N2-isobutyryl-3'-0-benzoyl-2'-O-tert-butyldimethylsilyl-guanosine]**

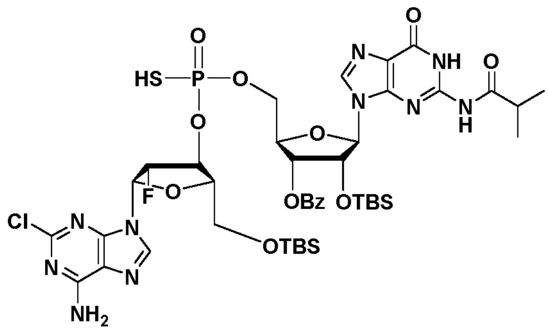

**Intermediate 44** was obtained from **Intermediate 2** and **Intermediate 25** in DMF via the route of **Intermediate 37.**

$^1$H NMR (400 MHz, DMSO-$d_6$) δ 12.59 (brs, 1H), 12.11 (brs, 1H), 8.37 (s, 1H), 8.14 (d, *J* = 2.8 Hz, 1H), 8.08-7.85 (m, 4H), 7.80-7.50 (m, 3H), 6.35 (dd, *J* = 18.0, 4.0 Hz, 1H), 5.92 (d, *J* = 8.0 Hz, 1H), 5.68 (d, *J* = 5.2 Hz, 1H), 5.55-5.38 (m, 2H), 5.15-4.98 (m, 1H), 4.52-4.40 (m, 1H), 4.20-3.95 (m, 5H), 2.90-2.80 (m, 1H), 1.25-1.05 (m, 6H), 0.87 (s, 9H), 0.53 (s, 9H), 0.07 (s, 6H), -0.13 (s, 3H), -0.38 (s, 3H). MS-ESI [(M-2H)/2]-: 532.2.

**Preparation of Intermediate 45: [3'-O-phosphorothioate-diester-2-chloro-5'-O-tert-butyldimethylsilyl-2'-deoxyadenosine]-(3', 5')-[N2-isobutyryl-3'-O-benzoyl-2'-O-tert-butyldimethylsilyl-guanosine]**

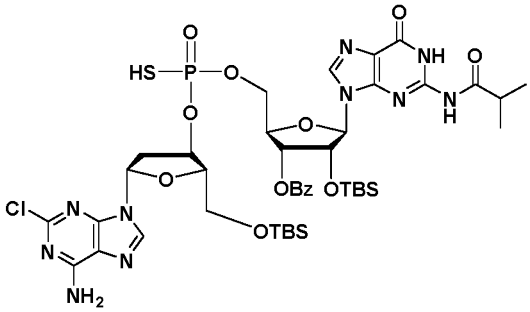

**Intermediate 45** was obtained from **Intermediate 4** and **Intermediate 25** in DMF via the route of **Intermediate 37.**

$^1$H NMR (400 MHz, DMSO-$d_6$) δ 12.76 (brs, 1H), 12.12 (brs, 1H), 8.37 (s, 1H), 8.27 (s, 1H), 8.03 (d, *J* = 7.6 Hz, 2H), 7.81 (brs, 2H), 7.70-7.50 (m, 3H), 6.29 (t, *J* = 6.8 Hz, 1H), 5.92 (d, *J* = 7.6 Hz, 1H), 5.76-5.65 (m, 1H), 5.55-5.45 (m, 1H), 5.10-4.96 (m, 1H), 4.47 (s, 1H), 4.30-4.20 (m, 1H), 4.10-3.75 (m, 3H), 2.98-2.83 (m, 1H), 2.81-2.55 (m, 2H), 1.20-1.05 (m, 6H), 0.84 (s, 9H), 0.54 (s, 9H), 0.03 (s, 6H), -0.11 (s, 3H), -0.36 (s, 3H). MS-ESI [(M+2H)/2]$^+$: 525.3.

**Preparation of Intermediate 46: [3'-O-phosphorothioate-diester-5'-O-tert-butyldimethylsilyl-2'-deoxy-2',2'-difluorocytidine]-(3',5')-[N2-isobutyryl-3'-O-benzoyl-2'-O-tert-butyldimethylsilyl-guanosine]**

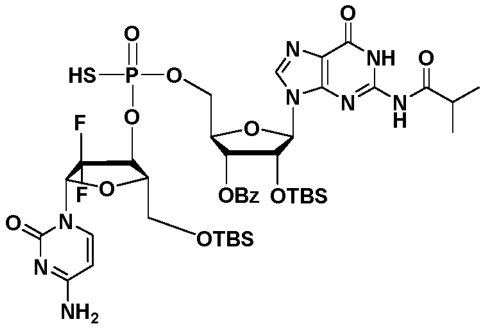

**Intermediate 46** was obtained from **Intermediate 6** and **Intermediate 25** in DMF via the route of **Intermediate 37.**

$^1$H NMR (400 MHz, DMSO-$d_6$) δ 12.49 (brs, 1H), 12.09 (brs, 1H), 8.39 (brs, 1H), 8.05 (d, *J* = 7.2 Hz, 2H), 7.78-7.50 (m, 4H), 7.40 (brs, 2H), 6.19 (t, *J* = 8.4 Hz, 1H), 5.91 (d, *J* = 8.0 Hz, 1H), 5.78 (d, *J* = 7.6 Hz, 1H), 5.66 (d, *J* = 4.8 Hz, 1H), 5.49-5.35 (m, 1H), 5.10-4.90 (m, 1H), 4.45 (s, 1H), 4.20-3.80 (m, 5H), 2.95-2.77 (m, 1H), 1.12 (d, *J* = 6.8 Hz, 6H), 0.89 (s, 9H), 0.54 (s, 9H), 0.15-0.05 (m, 6H), -0.10 (s, 3H), -0.37 (s, 3H). MS-ESI [(M+2H)/2]$^+$: 514.3.

**Preparation of Intermediate 47: [3'-O-phosphorothioate-diester-N4-(2-propylpentanoyl)-5'-O-tert-butyldimethylsilyl-2'-deoxy-2',2'-difluorocytidine]-(3',5')-[2'-O-tert-butyldimethylsilyl-3'-O-benzoyl-guanosine]**

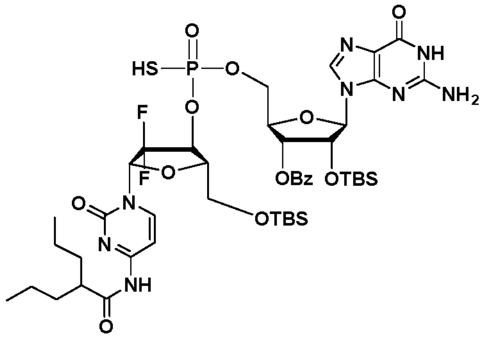

**Intermediate 47** was obtained from **Intermediate 30** and **Intermediate 9** in DMF via the route of **Intermediate 37.**

$^1$H NMR (400 MHz, DMSO-$d_6$) δ 11.10 (brs, 1H), 10.60 (brs, 1H), 8.22 (d, *J* = 7.6 Hz, 1H), 8.16 (s, 1H), 8.02 (d, *J* = 7.6 Hz, 2H), 7.68 (t, *J* = 7.6 Hz, 1H), 7.56 (t, *J* = 7.6 Hz, 2H), 7.35 (d, *J* = 7.6 Hz, 1H), 6.51 (brs, 2H), 6.23 (t, *J* = 7.6 Hz, 1H), 5.82 (d, *J* = 7.6 Hz, 1H), 5.67 (d, *J* = 5.2 Hz, 1H), 5.09 (dd, *J* = 7.6, 5.2 Hz, 1H),5.02-4.95 (m, 1H), 4.41 (d, *J* = 3.6 Hz, 1H), 4.18-3.92 (m, 4H), 2.70-2.55 (m, 1H), 1.65-1.50 (m, 2H), 1.45-1.30 (m, 2H), 1.28-1.15 (m, 4H), 0.96-0.88 (m, 12H), 0.87-0.80 (m, 9H), 0.53 (s, 9H), -0.11 (s, 3H), -0.31 (s, 3H). MS-ESI [(M-2H)/2]$^-$: 540.3.

**Preparation of Intermediate 48: [3'-O-phosphorothioate-diester-2-chloro-5'-O-tert-butyldimethylsilyl-2'-deoxy-2'-fluoro-beta-adenosine]-(3',5')-[N2-isobutyryl-3'-tert-butyldimethylsilyl-2'-benzoyl-guanosine]**

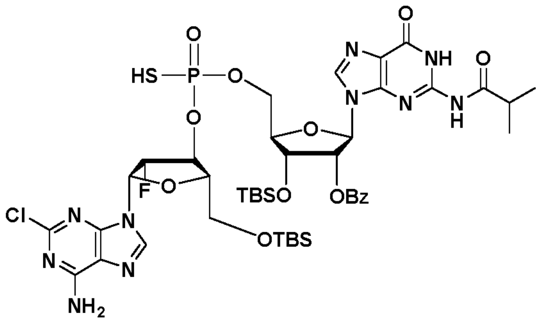

**Intermediate 48** was obtained from **Intermediate 27** and **Intermediate 2** in DMF via the route of **Intermediate 37.**

$^1$H NMR (400 MHz, DMSO-$d_6$) δ 12.53 (brs, 1H), 12.13 (brs, 1H), 8.36 (s, 1H), 8.14 (d, *J*= 2.8 Hz, 1H), 7.98-7.80 (m, 4H), 7.64 (t, *J* = 7.6 Hz, 1H), 7.48 (t, *J* = 8.0 Hz, 2H), 6.36 (dd, *J* = 18.0, 4.0 Hz, 1H), 6.22 (d, *J* = 7.2 Hz, 1H), 5.88 (dd, J= 7.2, 5.2 Hz, 1H), 5.60--5.43 (m, 1H), 5.19-5.05 (m, 1H), 4.72 (d, *J* = 4.8 Hz, 1H), 4.29-4.22 (m, 1H), 4.22-4.09 (m, 2H), 4.08-3.91 (m, 3H), 2.96-2.86 (m, 1H), 1.18-1.09 (m, 6H), 0.87 (s, 9H), 0.72 (s, 9H), 0.07 (s, 6H), 0.00 (s, 3H), -0.02 (s, 3H).

**Preparation of Intermediate 49: [3'-O-phosphorothioate-diester-2-chloro-5'-O-tert-butyldimethylsilyl-2'-deoxy-adenosine]-(3',5')-[N2-isobutyryl-3'-tert-butyl-dimethylsilyl-2'-benzoyl-guanosine]**

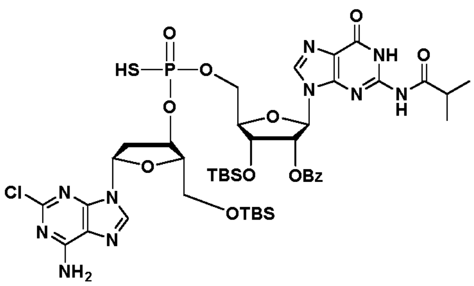

**Intermediate 49** was obtained from **Intermediate 27** and **Intermediate 4** in DMF via the route of **Intermediate 37.**

$^1$H NMR (400 MHz, DMSO-$d_6$) δ 12.55 (brs, 1H), 12.14 (brs, 1H), 8.37 (s, 1H), 8.28 (s, 1H), 7.94-7.76 (m, 4H), 7.65 (t, *J* = 7.6 Hz, 1H), 7.49 (t, *J* = 8.0 Hz, 2H), 6.33-6.21 (m, 2H), 5.95-5.85 (m, 1H), 5.10-5.02 (m,1H), 4.75 (d, *J* = 4.8 Hz, 1H), 4.31-4.23 (m, 2H), 4.22-4.15 (m, 1H), 4.14-4.09 (m, 1H), 3.91-3.78 (m, 2H), 2.93-2.86 (m, 1H), 2.76-2.71 (m, 1H), 2.68-2.60 (m, 1H),1.20-1.10 (m, 6H), 0.84 (s, 9H), 0.74 (s, 9H), 0.07-0.00 (m, 9H), -0.10 (s, 3H).

**Preparation of Intermediate 50: [3'-O-phosphorothioate-diester-2-chloro-5'-O-tert-butyldimethylsilyl-2'-deoxy-2'-fluoro-beta-adenosine]-(3',5')-[3'-O-tert-butyl-dimethylsilyl-2'-deoxy-2'-fluoroadenosine]**

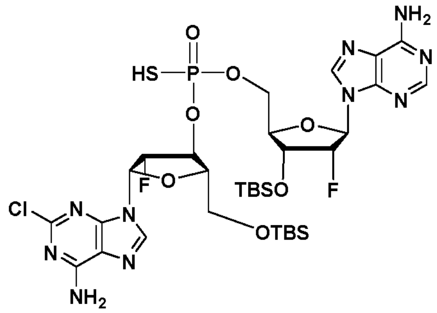

**Intermediate 50** was obtained from **Intermediate 32** and **Intermediate 2** in DMF via the route of **Intermediate 37.**

$^1$H NMR (400 MHz, DMSO-$d_6$) δ 8.49 (s, 1H), 8.18-8.08 (m, 2H), 7.92 (brs, 2H), 7.34 (brs, 2H), 6.32-6.20 (m, 2H), 5.50 (dt, *J* = 52.0, 4.4 Hz, 1H), 5.30 (d, *J* = 52.0 Hz, 1H), 5.05-4.94 (m, 1H), 4.78-4.65 (m, 1H), 4.14-4.06 (m, 3H), 3.95-3.70 (m, 3H), 0.90-0.75 (m, 18H), 0.10 (d, *J* = 8.8 Hz, 6H), 0.04 (s, 6H).

**Preparation of Intermediate 51: [3'-O-phosphorothioate-diester-2-chloro-5'-O-tert-butyldimethylsilyl-2'-deoxyadenosine]-(3',5')-[3'-O-tert-butyldimethylsilyl-2'-deoxy-2'-fluoroadenosine]**

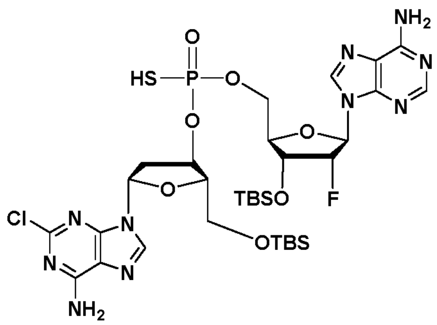

**Intermediate 51** was obtained from **Intermediate 3** and **Intermediate 33** via the route of **Intermediate 37.**

$^1$H NMR (400 MHz, DMSO-$d_6$) δ 8.51 (s, 1H), 8.25 (s, 1H), 8.13 (s, 1H), 7.82 (brs, 2H), 7.31 (brs, 2H), 6.30-6.15 (m, 2H), 5.69 (dt, *J* = 52.4, 4.0 Hz, 1H), 5.00-4.86 (m, 1H), 4.80-4.65 (m, 1H), 4.19-4.00 (m, 3H), 3.99-3.85 (m, 1H), 3.78-3.62 (m, 2H), 2.75-2.62 (m, 1H), 2.60-2.50 (m, 1H), 0.88 (s, 9H), 0.81 (s, 9H), 0.14 (s, 3H), 0.11 (s, 3H), 0.00 (s, 3H), -0.01 (s, 3H).

**Preparation of Intermediate 52: [3'-O-phosphorothioate-diester-2-chloro-5'-O-tert-butyldimethylsilyl-2'-deoxy-2'-fluoro-beta-adenosine]-(3',5')-[3'-O-tert-butyl-dimethylsilyl-2'-deoxy-2'-fluoroguanosine]**

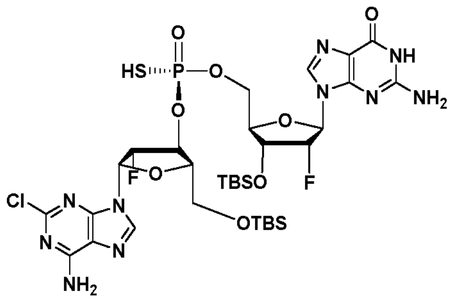

**Intermediate 52** was obtained from **Intermediate 1** and **Intermediate 36** in DMF via the route of **Intermediate 37.**

$^1$H NMR (400 MHz, DMSO-$d_6$) δ 10.64 (brs, 1H), 8.12 (s, 1H), 8.05 (s, 1H), 7.93-7.82 (m, 3H), 6.54 (brs, 2H), 6.36-6.25 (m, 1H), 6.00 (dd, *J* = 14.8, 4.8 Hz, 1H), 5.58-5.28 (m, 2H), 5.15-4.98 (m, 1H), 4.60-4.50 (m, 1H), 4.15-4.00 (m, 3H), 3.98-3.88 (m, 3H), 0.86 (s, 18H), 0.10-0.04 (m, 12H).

**Preparation of Intermediate 53: [3'-O-phosphorothioate-diester-2-chloro-5'-O-tert-butyldimethylsilyl-2'-deoxyadenosine]-(3',5')-[3'-O-tert-butyldimethylsilyl-2'-deoxy-2'-fluoroguanosine]**

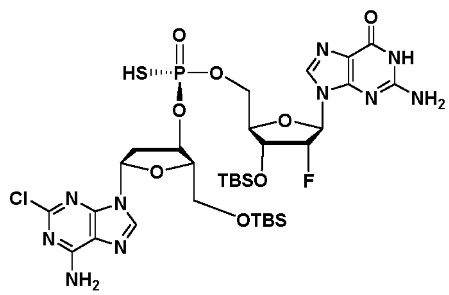

**Intermediate 53** was obtained from **Intermediate 3** and **Intermediate 36** in DMF via the route of **Intermediate 37.**

$^1$H NMR (400 MHz, DMSO-$d_6$) δ 10.65 (brs, 1H), 8.25 (s, 1H), 8.05 (s, 1H), 7.80 (brs, 2H), 6.55 (brs, 2H), 6.25 (t, *J* = 7.2 Hz, 1H), 6.03-5.95 (m, 1H), 5.47 (d, *J* = 52.0 Hz, 1H), 4.97 (s, 1H), 4.65-4.50 (m, 1H), 4.19 (s, 1H), 4.15-4.00 (m, 3H), 3.99-3.85 (m, 1H), 3.82 (d, *J* = 11.2 Hz, 1H), 3.76-3.69 (m, 1H), 2.75-2.60 (m, 1H), 0.88 (s, 9H), 0.80 (s, 9H), 0.11 (s, 3H), 0.10 (s, 3H), 0.02 (s, 6H). MS-ESI [(M-H)]$^-$: 875.4.

**Preparation of Intermediate 54: [3'-O-phosphorothioate-diester-2-chloro-2'-deoxy-2'-fluoro-beta-adenosine]-(3',5')-[2-chloro-2'-deoxy-2'-fluoro-beta-adenosine]**

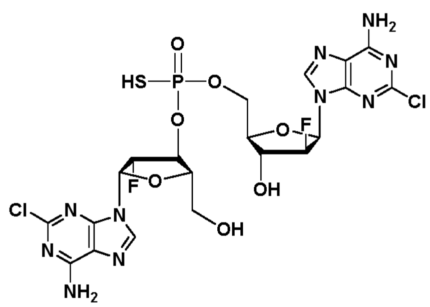

**Intermediate 37** (260 mg, 0.188 mmol) was dissolved in a mixture of tetrahydrofuran (2 mL), trifluoroacetic acid (2 mL) and water (1 mL), stirred at 25°C for 2 hours. The reaction mixture was rotary evaporated to dry and diluted

with methanol (5 mL), and the pH of the above mixture was adjusted to about 8-9 with saturated aqueous sodium bicarbonate. After rotary evaporation to dry, it was diluted with methanol (5 mL) again and filtered. The filtrate was concentrated and the resulting crude product was purified by silica gel column eluting with ethyl acetate : methanol (7:3) to give a white solid (120 mg).

$^1$H NMR (400 MHz, DMSO-$d_6$) δ 8.30 (s, 1H), 8.23 (s, 1H), 7.88 (brs, 4H), 6.40-6.18 (m, 2H), 6.06 (d, *J* = 5.2 Hz, 1H), 5.45-5.12 (m, 2H), 5.04 (t, *J* = 6.0 Hz, 1H), 5.00-4.85 (m, 1H), 4.50-4.40 (m, 1H), 4.18-3.90 (m, 4H), 3.75-3.58 (m, 2H). MS-ESI [M-H]$^-$:682.8.

**Preparation of Intermediate 55: [3'-O-phosphorothioate-diester-2-chloro-2'-deoxyadenosine]-(3',5')-[2-chloro-2'-deoxyadenosine]**

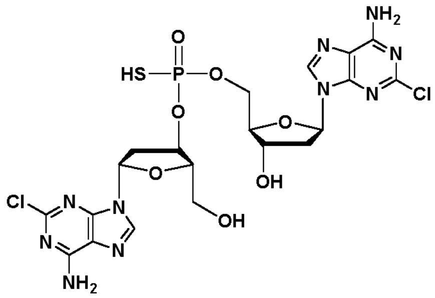

To a solution of **Intermediate 38** (150 mg, 0.110 mmol) in tetrahydrofuran (5 mL) was added tetrabutylammonium fluoride (34 mg, 0.132 mmol), and the resulting mixture was stirred at 25°C for 3 hours. The reaction mixture was rotary evaporated to dry, dissolved in a mixed solution of glacial acetic acid (2 mL) and water (0.5 mL), and stirred at 25°C for 0.5 hours. The reaction mixture was rotary evaporated to dry, and the obtained crude product was purified by silica gel column eluting with ethyl acetate : methanol (3:1) to give a white solid (45 mg). MS-ESI [MH]-:647.0.

**Preparation of Intermediate 56: [3'-O-phosphorothioate-diester-2'-deoxy-2',2'-difluorocytidine]-(3',5')-[2'-deoxy-2',2'-difluorocytidine]**

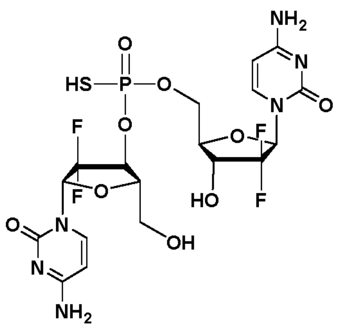

To a solution of **Intermediate 39** (350 mg, 0.421 mmol) in tetrahydrofuran (5 mL) was added tetrabutylammonium fluoride (329 mg, 1.26 mmol), and the resulting mixture was stirred at 25°C for 2 hours. The reaction mixture was rotary evaporated to dry, and the obtained crude product was purified by silica gel column eluting with ethyl acetate : methanol (3:1) to give a white solid (130 mg).

$^1$H NMR (400 MHz, DMSO-$d_6$) δ 7.77 (d, *J* = 7.2 Hz, 1H), 7.65 (d, *J* = 7.6 Hz, 1H), 7.45-7.25 (m, 4H), 6.30 (d, *J* = 6.4 Hz, 1H), 6.20-6.05 (m, 2H), 5.89-5.70 (m, 2H), 5.13 (t, *J* = 6.0 Hz, 1H), 4.90-4.75 (m, 1H), 4.20-4.00 (m, 2H), 4.00-3.60 (m, 5H).

**Preparation of Intermediate 57: [3'-O-phosphorothioate-diester-N4-(2-propylpentanoyl)-2'-deoxy-2',2'-difluoro-cytidine]-(3',5')-[N4-(2-propylpentanoyl)-2'-deoxy-2',2'-difluorocytidine]**

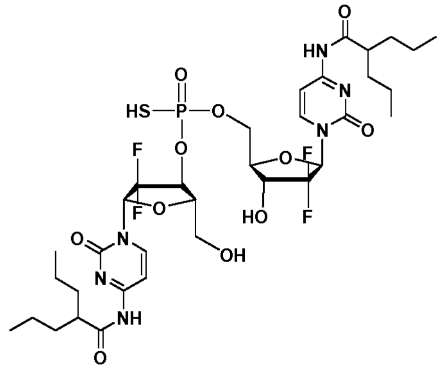

**Intermediate 57** was obtained from **Intermediate 40** via the route of **Intermediate** 56. MS-ESI [M+H] + :858.2, [(M+2H)/2] +: 429.1.

**Preparation of Intermediate 58: [3'-O-phosphorothioate-diester-2-chloro-2'-deoxy-2'-fluoro-beta-adenosine]-(3',5')-[N6-benzoyl-3'-O-benzoyl-adenosine]**

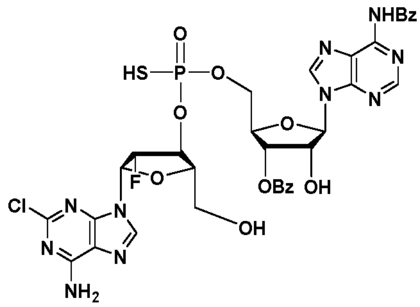

**Intermediate 58** was obtained from **Intermediate 41** via the route of **Intermediate 56.** MS-ESI [M+H] +:857.0.

**Preparation of Intermediate 59: [3'-O-phosphorothioate-diester-2-chloro-2'-deoxyadenosine]-(3',5')-[N6-benzoyl-3'-O-benzoyl-adenosine]**

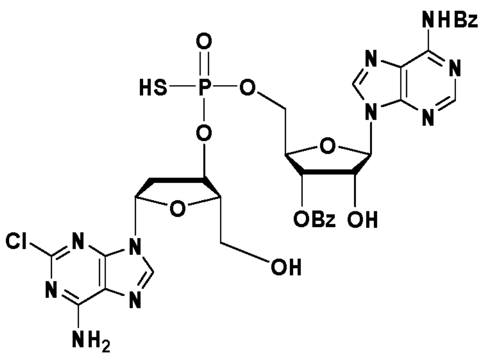

**Intermediate 59** was obtained from **Intermediate 42** via the route of **Intermediate 56.** MS-ESI [M+H] +:839.0.

**Preparation of Intermediate 60: [3'-O-phosphorothioate-diester-2-chloro-2'-deoxy-2'-fluoro-beta-adenosine]-(3',5')-[N6-benzoyl-2'-O-benzoyl-adenosine]**

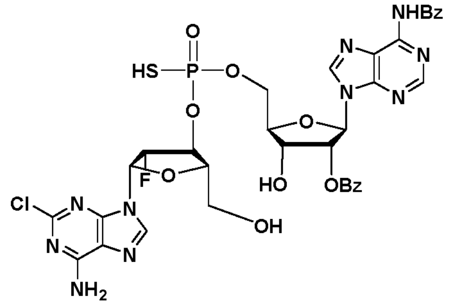

To a solution of **Intermediate 43** (250 mg, 0.23 mmol) in tetrahydrofuran (5 mL) was added triethylamine trihydrofluoride (223 mg, 1.38 mmol), and stirred at 30°C for 16 hours. The reaction mixture was neutralized to pH 7-8 with triethylamine. The resulting mixture was rotary evaporated to dry, and the obtained crude product was purified by silica gel column eluting with ethyl acetate : methanol (8:2) to give a white solid (160 mg). MS-ESI $[M+H]^+$ :857.1.

**Preparation of Intermediate 61: [3'-O-phosphorothioate-diester-2-chloro-2'-deoxy-2'-fluoro-beta-adenosine]-(3',5')-[N2-isobutyryl-3'-O-benzoyl-guanosine]**

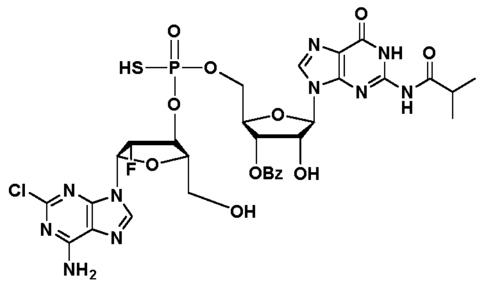

**Intermediate 61** was obtained from **Intermediate 44** via the route of **Intermediate 60.**

$^1$H NMR (400 MHz, DMSO-$d_6$) δ 12.56 (brs, 1H), 12.11 (brs, 1H), 8.35-8.20 (m, 2H), 8.10-8.00 (m, 2H), 7.91 (brs, 2H), 7.75-7.45 (m, 3H), 6.38-6.18 (m, 1H), 6.00-5.75 (m, 2H), 5.70-5.21 (m, 2H), 5.16-4.90 (m, 2H), 4.50-4.36 (m, 1H), 4.28-4.00 (m, 2H), 3.90-3.58 (m, 2H), 3.00-2.82 (m, 1H), 1.36-1.05 (m, 6H). MS-ESI $[M-H]^-$: 837.0.

**Preparation of Intermediate 62: [3'-O-phosphorothioate-diester-2-chloro-2'-deoxyadenosine]-(3',5')-[N2-isobutyryl-3'-O-benzoyl-guanosine]**

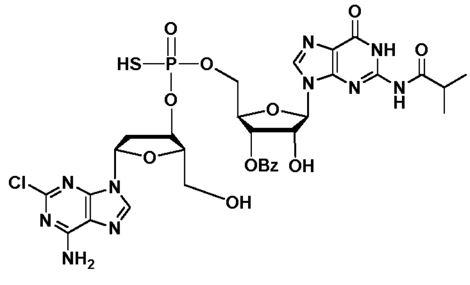

**Intermediate 62** was obtained from **Intermediate 45** via the route of **Intermediate 60.**

$^1$H NMR (400 MHz, DMSO-$d_6$) δ 12.72 (brs, 1H), 12.13 (brs, 1H), 8.34 (s, 1H), 8.32 (s, 1H), 8.03 (d, *J* = 7.6

Hz, 1H), 7.98-7.81 (m, 3H), 7.71-7.62 (m, 1H), 7.60-7.43 (m, 1H), 6.38-6.20 (m, 1H), 5.99-5.70 (m, 2H), 5.70-5.57 (m, 1H), 5.40-5.28 (m, 1H), 5.18-4.93 (m, 2H), 4.42 (s, 1H), 4.32-4.00 (m, 3H), 3.75-3.52 (m, 2H), 2.93-2.81 (m, 1H), 2.80-2.62 (m, 1H), 1.20-1.05 (m, 6H). MS-ESI $[M+H]^+$: 821.2.

**Preparation of Intermediate 63: [3'-O-phosphorothioate-diester-2'-deoxy-2',2'-difluorocytidine]-(3',5')-[N2-isobutyryl-3'-O-benzoyl-guanosine]**

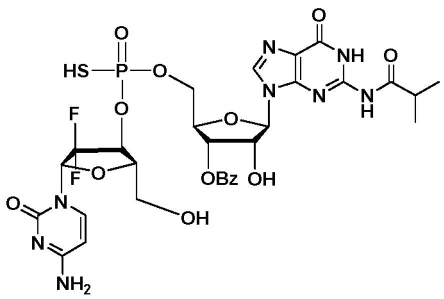

**Intermediate 63** was obtained from **Intermediate 46** via the route of **Intermediate 60.**

$^1$H NMR (400 MHz, DMSO-$d_6$) δ 12.42 (brs, 1H), 12.12 (brs, 1H), 8.35 (s, 1H), 8.08 (d, *J* = 7.6 Hz, 2H), 7.80-7.53 (m, 4H), 7.48-7.34 (m, 2H), 6.24-6.12 (m, 1H), 5.97-5.87 (m, 1H), 5.86-5.76 (m, 1H), 5.57 (d, *J* = 5.2 Hz, 1H), 5.30-5.18 (m, 1H), 5.17-5.05 (m, 1H), 4.95-4.75 (m, 1H), 4.41 (s, 1H), 4.25-3.70 (m, 6H), 2.90-2.77 (m, 1H), 1.20-1.05 (m, 6H).

**Preparation of Intermediate 64: [3'-O-phosphorothioate-diester-N4-(2-propylpentanoyl)-2'-deoxy-2',2'-difluorocytidine]-(3',5')-[3'-O-benzoyl-guanosine]**

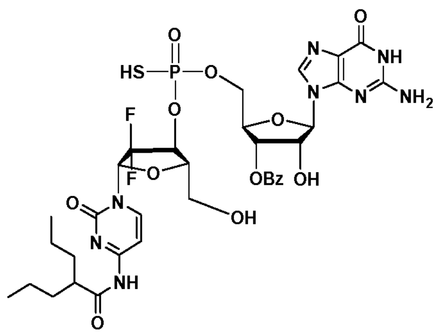

**Intermediate 64** was obtained from **Intermediate 47** via the route of **Intermediate 56.** MS-ESI $[(M+2H)/2]^+$ :428.0.

**Preparation of Intermediate 65: [3'-O-phosphorothioate-diester-2-chloro-2'-deoxy-2'-fluoro-beta-adenosine]-(3',5')-[N2-isobutyryl-2'-benzoyl-guanosine]**

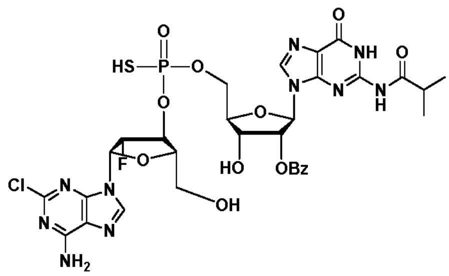

**Intermediate 65** was obtained from **Intermediate 48** via the route of **Intermediate 56.** MS-ESI[MH]-:837.0.

**Preparation of Intermediate 66: [3'-O-phosphorothioate-diester-2-chloro-2'-deoxy-adenosine]-(3',5')-[N2-isobutyryl-2'-benzoyl-guanosine]**

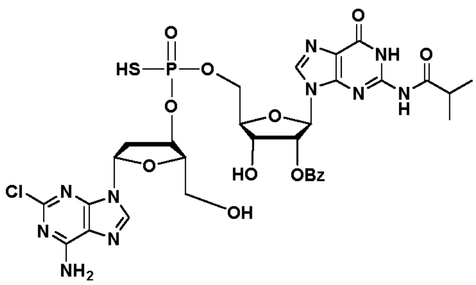

**Intermediate 66** was obtained from **Intermediate 49** via the route of **Intermediate 56.** MS+ESI $[M+H]^+$:821.1.

**Preparation of Intermediate 67: [3'-O-phosphorothioate-diester-2-chloro-2'-deoxy-2'-fluoro-beta-adenosine]-(3',5')-[2'-deoxy-2'-fluoroadenosine]**

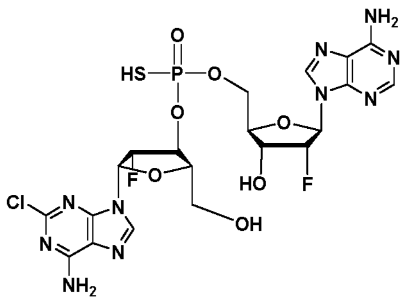

**Intermediate 67** was obtained from **Intermediate 50** via the route of **Intermediate 60.**

$^1$H NMR (400 MHz, DMSO-$d_6$) δ 8.47 (s, 1H), 8.24 (s, 1H), 8.15 (s, 1H), 7.91 (brs, 2H), 7.35 (brs, 2H), 6.32-6.20 (m, 2H), 5.88 (brs, 1H), 5.55-5.30 (m, 2H), 5.18-4.87 (m, 2H), 4.53 (d, $J$ = 16.4 Hz, 1H), 4.18-4.08 (m, 2H), 4.10-4.02 (m, 1H), 4.01-3.92 (m, 1H), 3.75-3.59 (m, 2H). MS-ESI $[M+H]^+$:651.0.

**Preparation of Intermediate 68: [3'-O-phosphorothioate-diester-2-chloro-2'-deoxyadenosine]-(3',5')-[2'-deoxy-2'-fluoroadenosine]**

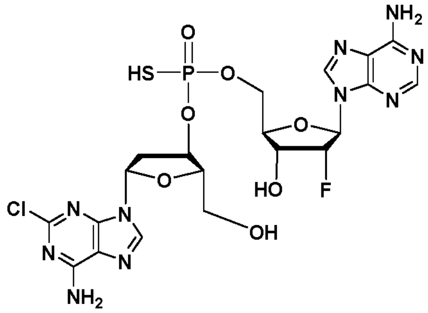

**Intermediate 68** was obtained from **Intermediate 51** via the route of **Intermediate 56.**

$^1$H NMR (400 MHz, DMSO-$d_6$) δ 8.50 (s, 1H), 8.34 (s, 1H), 8.15 (s, 1H), 7.84 (brs, 2H), 7.31 (brs, 2H), 6.30-6.18 (m, 2H), 5.90 (d, *J* = 5.6 Hz, 1H), 5.45 (dt, *J* = 52.0, 3.6 Hz, 1H), 5.12-4.90 (m, 2H), 4.60-4.45 (m, 1H), 4.18-4.00 (m, 3H), 3.99-3.85 (m, 1H), 3.65-3.50 (m, 2H), 2.70-2.55 (m, 1H), 2.50-2.40 (m, 1H).

**Preparation of Intermediate 69: [3'-O-phosphorothioate-diester-2-chloro-2'-deoxy-2'-fluoro-beta-adenosine]-(3',5')-[2'-Deoxy-2'-fluoroguanosine]**

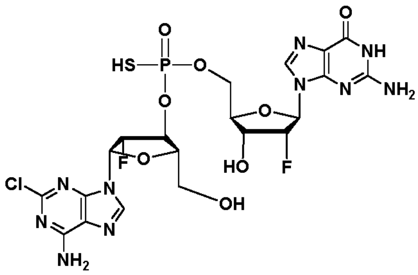

**Intermediate 69** was obtained from **Intermediate 52** via the route of **Intermediate 60.**

$^1$H NMR (400 MHz, DMSO-$d_6$) δ 10.64 (s, 1H), 8.24 (s, 1H), 8.00 (s, 1H), 7.88 (brs, 2H), 6.54 (brs, 2H), 6.28 (dd, *J* = 18.0, 3.6 Hz, 1H), 6.02 (dd, *J* = 16.0, 3.6 Hz, 1H), 5.80 (d, *J* = 5.4 Hz, 1H), 5.44 (d, *J* = 4.4 Hz, 1H), 5.30 (d, *J* = 7.6 Hz, 1H), 5.04 (d, *J* = 6.0 Hz, 1H), 4.95 (t, *J* = 14.8 Hz, 1H), 4.42 (dt, *J* = 10.4, 5.6 Hz, 1H), 4.11-3.99 (m, 3H), 3.98-3.89 (m, 1H), 3.77-3.60 (m, 2H). MS-ESI [M+H]$^+$:667.2.

**Preparation of Intermediate 70: [3'-O-phosphorothioate-diester-2-chloro-2'-deoxyadenosine]-(3',5')-[2'-deoxy-2'-fluoroguanosine]**

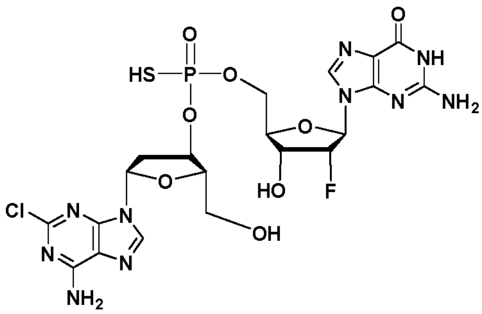

**Intermediate 69** was obtained from **Intermediate 53** via the route of **Intermediate 60.**

$^1$H NMR (400 MHz, DMSO-$d_6$) δ 10.67 (brs, 1H), 8.37 (s, 1H), 8.04 (s, 1H), 7.85 (brs, 2H), 6.58 (brs, 2H), 6.26 (t, *J* = 7.2 Hz, 1H), 6.03 (d, *J* = 15.6 Hz, 1H), 5.85 (d, *J* = 5.6 Hz, 1H), 5.38 (d, *J* = 52.8 Hz, 1H), 5.10 (s, 1H), 4.97 (s, 1H), 4.46 (d, *J* = 14.8 Hz, 1H), 4.20-4.00 (m, 4H), 4.00-3.90 (m, 1H), 3.70-3.50 (m, 2H), 2.78-2.60 (m, 1H). MS-ESI [M-H]$^-$:647.2.

**Preparation of Intermediate 71: (2',3')-cyclo-[2'-O-phosphorothioate-diester-N6-benzoyl-3'-benzoyl-adenosine]-[3'-O-phosphorothioate-diester-2-chloro-2'-deoxy-2'-fluoro-beta-adenosine]**

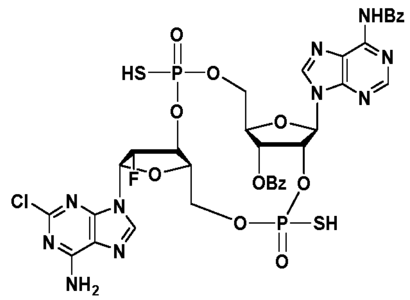

To a solutionof **Intermediate 58** (95 mg, 0.11 mmol) and **(-)-PSI reagent** (150 mg, 0.33 mmol) in DMF (10 mL) was added 1,8-diazabicycloundec-7-ene (253 mg, 1.66 mmol), and the resulting mixture was stirred at 25°C for 1 hour. The reaction mixture was rotary evaporated to dry and reprecipitated with ethyl acetate (20 mL). The obtained solid was slurried with ethyl acetate (20 mL), filtered and dried to give a crude product (200 mg), which was directly used in the next reaction. MS-ESI $[M+2+H]^+$: 937.0.

**Preparation of Intermediate 72: (2',3')-cyclo-[2'-O-phosphorothioate-diester-N6-benzoyl-3'-O-benzoyl-adenosine]-[3'-O-phosphorothioate-diester-2-chloro-2'-deoxyadenosine]**

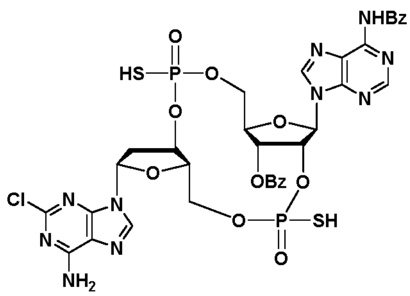

To a solution of **Intermediate 59** (210 mg, 0.25 mmol) and **(-)-PSI reagent** (335 mg, 0.75 mmol) in DMF (10 mL) was added 1,8-diazabicycloundec-7-ene (570 mg, 3.75 mmol), and the resulting mixture was stirred at 25°C for 1 hour. The reaction mixture was rotary evaporated to dry and reprecipitated with ethyl acetate (20 mL). The obtained solid was slurried with ethyl acetate (20 mL), filtered and dried to give a crude product. The crude product was purified by preparative liquid chromatography (10 mM ammonium bicarbonate as an additive), and a white solid (15.8 mg) was obtained after lyophilization, which was directly used in the next reaction. MS-ESI $[M+H]^+$: 917.0.

**Preparation of Intermediate 73: (3',3')-cyclo-bis-[3'-O-phosphorothioate-diester-2-chloro-2'-deoxy-2'-fluoro-beta-adenosine]-[3'-O-phosphorothioate-diester-N6-benzoyl-2'-O-benzoyl-adenosine]**

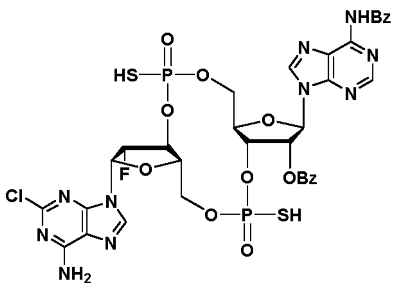

**Intermediate 73** was obtained from **Intermediate 60** and **(-)-PSI reagent** via the route of **Intermediate 71.** MS-ESI [MH]-: 932.8.

**Preparation of Intermediate 74: (2',3')-cyclo-[2'-O-phosphorothioate-diester-3'-O-benzoylguanosine]-[3'-O-phosphorothioate-diester-2-chloro-2'-deoxy-2'-fluoro-beta-adenosine]-Isomer 1 and Intermediate 75: (2',3')-cyclo-[2'-O-phosphorothioate-diester-3'-O-benzoyl-guanosine]-[3'-O-phosphorothioate-diester-2-chloro-2'-deoxy-2'-fluoro-beta-adenosine]-Isomer 2**

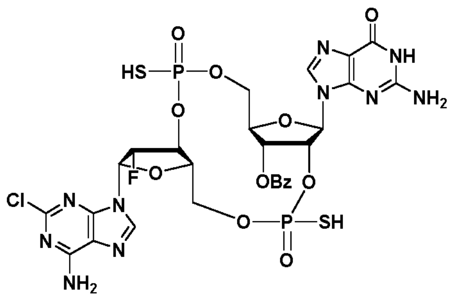

**Intermediate 74** and **Intermediate 75** were obtained from **Intermediate 61** and **(-)-PSI reagent** via the route of **Intermediate 72.**

**Intermediate 74:** MS-ESI [MH]-: 844.8, retention time: 4.96 min;

**Intermediate 75:** MS-ESI [MH] - : 844.9, retention time: 5.20 min. Analytical LCMS: Agilent 1100+G1946D LCMS, 4.6 x 150 mm Waters XBridge C18 3.5 μm analytical column, mobile phase A: 10 mM $NH_4HCO_3$ aqueous solution, B: acetonitrile, flow rate 1.0 ml/min, dual wavelength UV absorption monitoring at 214 and 254 nm, gradient elution: 0-0.1 min, 5%B; 0.1-8 min, 5-95%B; 8-15 min, 95%B.

**Preparation of Intermediate 76: (2',3')-cyclo-[2'-O-phosphorothioate-diester-N2-isobutyryl-3'-O-benzoyl-guanosine]-[3'-O-phosphorothioate-diester-2-chloro-2'-deoxy-adenosine]-Isomer 1, and Intermediate 77: (2',3')-cyclo-[2'-O-phosphorothioate-diester-N2-isobutyryl-3'-O-benzoylguanosine]-[3'-O-Phosphorothioate-diesters-2-chloro-2'-deoxy-adenosine]-Isomer 2**

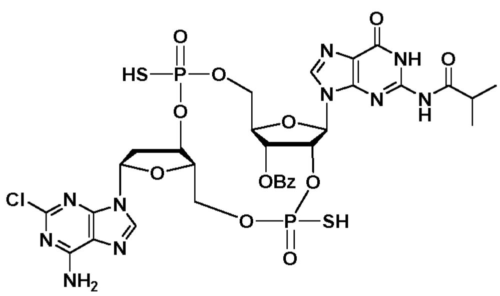

**Intermediate 76** and **Intermediate** 77 were obtained from **Intermediate 62** and **(-)-PSI reagent** via the route of **Intermediate 72.**

**Intermediate 76:** MS-ESI [MH]-: 897.2, retention time: 1.69 min;

**Intermediate 77:** MS-ESI [MH] - : 897.2, retention time: 1.74 min. Analytical LCMS: Agilent 1100+G1946D LCMS, 4.6 x 50 mm Waters XBridge C18 3.5 μm analytical column, mobile phase A: 10 mM $NH_4HCO_3$ aqueous solution, B: acetonitrile, flow rate 1.8 min/min, dual wavelength UV absorption monitoring at 214 and 254 nm, gradient elution: 0-0.1 min, 5%B; 0.1-2.5 min, 5-95%B; 2.5-5 min, 95%B.

**Preparation of Intermediate 78: (2',3')-cyclo-[2'-O-phosphorothioate-diester-N2-isobutyryl-3'-O-benzoyl-guanosine]-[3'-O-phosphorothioate-diester-2'-deoxy-2',2'-difluorocytidine]**

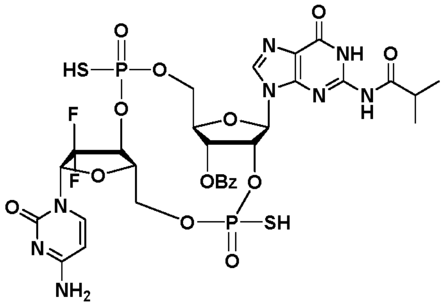

**Intermediate 78** was obtained from **Intermediate 63** and **(-)-PSI reagent** via the route of **Intermediate 72. Intermediate 78:** MS-ESI [(M-2H)/2]-: 437.0.

**Preparation of Intermediate 79: (2',3')-cyclo-[2'-O-phosphorothioate-diester-3'-O-benzoyl-guanosine]-[3'-O-phosphorothioate-diester-N4-(2-propylpentanoyl)-2'-deoxy-2',2'-difluorocytidine]**

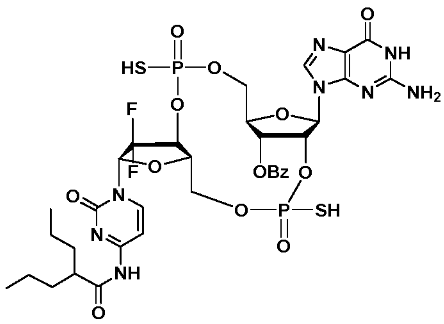

**Intermediate 79** was obtained from **Intermediate 64** and the **(-)-PSI reagent** via the route of **Intermediate 72.** MS-ESI [MH]-: 931.0.

**Intermediate 80: (3',3')-cyclo-[3'-O-phosphorothioate-diester-N2-isobutyryl-2'-benzoyl-guanosine]-[3'-O-phosphorothioate-diester-2-chloro-2'-deoxy-2'-fluoro-beta-adenosine]-Isomer 1, and**

**Intermediate 81: (3',3')-cyclo-[3'-O-phosphorothioate-diester-N2-isobutyryl-2'-benzoyl-guanosine]-[3'-O-phosphorothioate-diester-2-chloro-2'-deoxy-2'-fluoro-beta-adenosine]-Isomer 2**

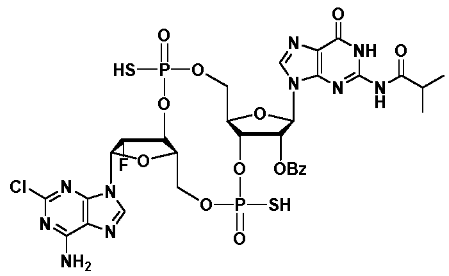

**Intermediates 80** and **81** were obtained from **Intermediate 65** and **(-)-PSI reagent** via the route of **Intermediate 72.**

**Intermediate 80:** MS-ESI [MH]-: 915.0, retention time: 1.50 min;

**Intermediate 81:** MS-ESI [MH] - : 915.0, retention time: 1.53 min. Analytical LCMS: Agilent 1100+G1946D LCMS, 4.6 x 50 mm Waters XBridge C18 3.5 μm analytical column, mobile phase A: 10 mM $NH_4HCO_3$ aqueous solution, B: acetonitrile, flow rate 1.8 ml/min, dual wavelength UV absorption monitoring at 214 and 254 nm, gradient elution: 0-0.2 min, 5%B; 0.2-1.5 min, 5-95%B; 1.5-3 min, 95%B.

**Preparation of Intermediate 82: (3',3')-cyclo-[3'-O-phosphorothioate-diester-N2-isobutyryl-2'-benzol-uanosine-3'-O-hoshorothioate-diester-2-chloro-2'-deox-adenosine**

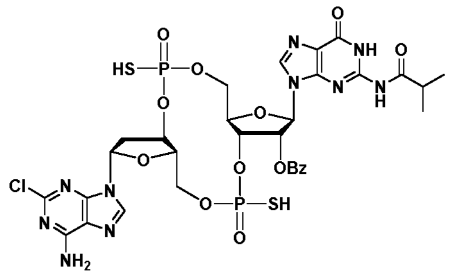

**Intermediate 82** was obtained from **Intermediate 66** and **(-)-PSI reagent** via the route of **Intermediate 72.** MS-ESI [MH]-: 896.8.

**Preparation of Intermediate 83: 2-Chloro-5'-O-tert-butyldimethylsilyl-3'-O-benzoyl-2'-deoxy-2'-fluoro-beta-adenosine**

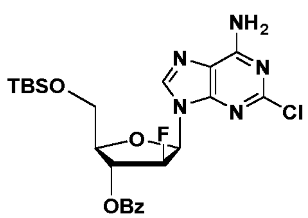

**Intermediate 83** was obtained from **Intermediate 1** via the route of **Intermediate 20.**

$^1H$ NMR (400 MHz, DMSO-$d_6$) δ 8.21 (d, *J* = 2.4 Hz, 1H), 8.08 (d, *J* = 7.2 Hz, 2H), 7.95 (brs, 2H), 7.72 (t, *J* = 7.6 Hz, 1H), 7.58 (t, *J* = 7.6 Hz, 2H), 6.50 (dd, *J* = 16.6, 4.0 Hz, 1H), 5.80-5.77 (m, 1H), 5.75-5.66 (m, 1H), 4.32 (q, *J* = 4.8 Hz, 1H), 3.97 (d, *J* = 4.4 Hz, 2H), 0.86 (s, 9H), 0.06 (s, 6H).

**Preparation of Intermediate 84: 2-Chloro-3'-O-benzoyl-2'-deoxy-2'-fluoro-beta-adenosine**

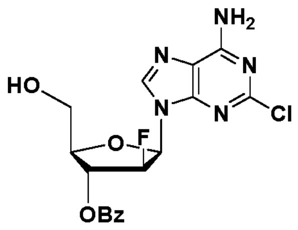

**Intermediate 84** was obtained from **Intermediate 83** via the route of **Intermediate 60.**

$^1$H NMR (400 MHz, DMSO-$d_6$) δ 8.40 (d, *J*= 2.4 Hz, 1H), 8.12 (d, *J*= 7.6 Hz, 2H), 7.99 (brs, 2H), 7.74 (t, *J* = 7.2 Hz, 1H), 7.60 (t, *J* = 7.6 Hz, 2H), 6.55 (dd, *J* = 18.2, 3.6 Hz, 1H), 5.81-5.78 (m, 1H), 5.77-5.66 (m, 1H), 4.35 (q, *J* = 4.4 Hz, 1H), 3.89-3.80 (m, 2H).

**Preparation of Intermediate 85: N-(9-((*2R,3R,4R,5R*)-5-((bis(4-methoxyphenyl)(phenyl)methoxy)methyl)-4-((tert-butyldimethylsilyl)oxy)-3-((*2S,3aR,6S,7aR*)-3a-methyl-6-(prop-1-en-2-yl)-2-thio-hexahydrobenzo[d][1,3,2] oxathiophosphorolan-2-yl)oxy)tetrahydrofuran-2-yl)-6-oxy-6,9-dihydro-1H-purin-2-yl)isobutyramide**

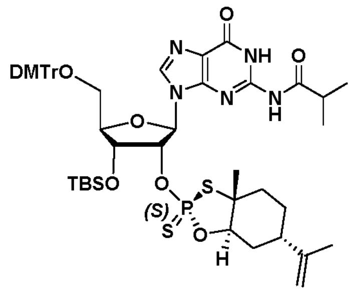

**Intermediate 85** was obtained from **N2-isobutyryl-5'-O-bis(4-methoxyphenyl)benzyl-3'-O-tert-butyldimethylsilyl-guanosine and (+)-PSI reagent** via the route of **Intermediate 2.**

$^1$H NMR (400 MHz, DMSO-$d_6$) δ 12.11 (s, 1H), 11.62 (s, 1H), 8.14 (s, 1H), 7.38 (d, *J* = 8.0 Hz, 2H), 7.31-7.22 (m, 7H), 6.86 (d, *J* = 8.4 Hz, 4H), 6.00 (d, *J* = 6.8 Hz, 1H), 5.67-5.59 (m, 1H), 4.89 (s, 1H), 4.75 (s, 1H), 4.42 (d, *J* = 4.0 Hz, 1H), 4.22 (d, *J* = 12.0 Hz, 1H), 4.06-4.02 (m, 1H), 3.73 (s, 6H), 3.41-3.35 (m, 2H), 3.24 (dd, *J*= 12.0 Hz, 1H), 2.82-2.73 (m, 1H), 2.07 (d, *J*= 12.0 Hz, 1H), 1.93-1.83 (m, 2H), 1.76-1.65 (mf, 6H), 1.54 (s, 3H), 1.12 (d, *J*= 6.8 Hz, 6H), 0.83 (s, 9H), 0.08 (s, 3H), 0.04 (s, 3H).

**Preparation of Intermediate 86: [2'-O-*Rp*-phosphorothioate-diester-N2-isobutyryl-5'-O-bis(4-methoxyphenyl)benzyl-3'-O-tert-butyldimethylsilyl-guanosine]-(2',5')-[2-chloro-3'-O-benzoyl-2'-deoxy-2'-fluoro-beta-adenosine]**

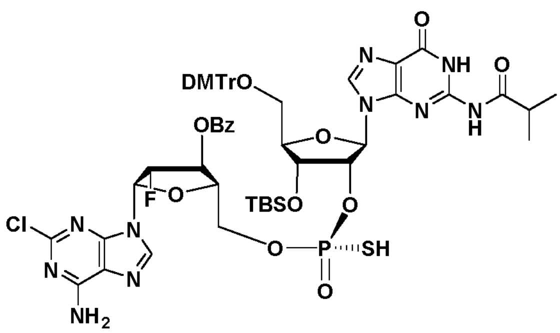

**Intermediate 86** was obtained from **Intermediate 84** and **Intermediate 85** in DMF via the route of **Intermediate 37.** MS-ESI [M+H]+: 1255.0.

**Preparation of Intermediate 87: [2'-O-*Rp*-phosphorothioate-diester-N2-isobutyryl-5'-O-bis(4-methoxyphenyl)benzyl-3'-O-tert-butyldimethylsilyl-guanosine]-(2',5')-[2-chloro-2'-deoxy-2'-fluoro-beta-adenosine]**

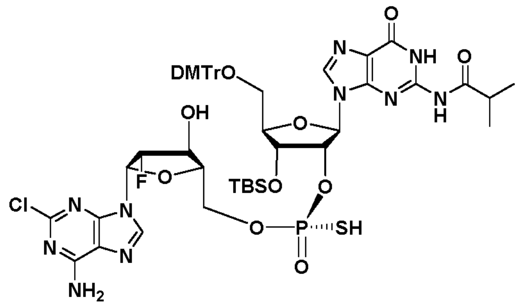

**Intermediate 86** (1.0 g, 0.8 mmol) was added to a mixed solution (**10** mL) of tetrahydrofuran/water/methanol 8:4:1, cooled to 0°C, and **lithium hydroxide monohydrate** (54 mg, 1.6 mmol) was added, and the resulting mixture was stirred at 0°C for 20 min. The reaction mixture was poured into water (50 mL), extracted twice with ethyl acetate (50 mL each). The organic phases were combined, washed twice with saturated brine (100 mL each) and dried over anhydrous sodium sulfate. The crude product obtained by concentration was purified by silica gel column eluting with ethyl acetate : methanol (10:1) to give a white solid (733 mg).

$^1$H NMR (400 MHz, DMSO-$d_6$) δ 12.02 (s, 1H), 11.67 (s, 1H), 8.21 (t, *J* = 5.2 Hz, 1H), 8.14 (s, 1H), 7.84 (s, 2H), 7.37 (d, *J* = 7.6 Hz, 2H), 7.29-7.14 (m, 7H), 6.87-6.77 (m, 4H), 6.23 (dd, *J* = 13.9, 4.5 Hz, 1H), 6.08-5.98 (m, 2H), 5.49-5.38 (m, 1H), 5.23-5.04 (m, 1H), 4.66-4.59 (m, 1H), 4.35-4.24 (m, 1H), 3.99-3.95 (m, 1H), 3.77-3.71 (m, 2H), 3.71-3.63 (m, 7H), 3.22-3.00 (m, 2H), 2.79-2.68 (m, 1H), 1.12-1.07 (m, 6H), 0.82 (s, 9H), 0.13 (d, J = 13.4 Hz, 6H).

**Preparation of Intermediate 88: O-((*2R,3R,4S,5R*)-5-(6-amino-2-chloro-9H-purin-9-yl)-4-fluoro-3-((*2R,3aS*,6R,*7aS*)-3a-Methyl-6-(prop-1-en-2-yl)-2-thiohexahydrobenzo[d][1,3,2]oxathiophospholane-2-yl)methyl)-O-((*2R,3R,4R,5R*)-5-((bis(4-methoxyphenyl)(phenyl)methoxy)methyl)-4-((tert-butyldimethylsilyl)oxy)-2-(2-isobutylamino-6-oxy-1,6-dihydro-9H-purin-9-yl)tetrahydrofuran-3-yl)-(*R)*-phosphorothioate-diester**

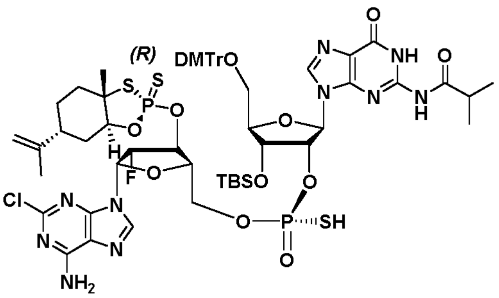

**Intermediate 88** was prepared as a crude product from **Intermediate 87** and **(-)-PSI reagent** in tetrahydrofuran via the route of **Intermediate 2,** and was directly used in the next reaction. MS-ESI [M+H-DMTr] +: 1095.0.

**Preparation of Intermediate 89: O-((*2R,3R,4S,5R*)-5-(6-amino-2-chloro-9H-purin-9-yl)-4-fluoro-3-((*2R,3aS,6R,7aS*)-3a-methyl-6-(prop-1-en-2-yl)-2-thiohexahydrobenzo[d][1,3,2]oxathiophospholane-2-yl)methyl)-O-((*2R,3R,4R,5R*)-4-((tert-butyldimethylsilyl)oxy)-2-(2-isobutylamino-6-oxy-1,6-dihydro-9H-purin-9-yl)tetrahydrofuran-3-yl)-(*R)*-phosphorothioate-diester**

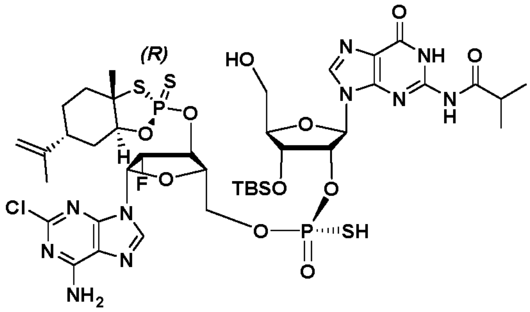

**Intermediate 89** was obtained from **Intermediate 88** via the route of **Intermediate 25.** MS-ESI $[M+H]^+$: 1095.0.

**Preparation of Intermediate 90: (2',3')-cyclo-(*Rp,Sp)*-[2'-O-phosphorothioate-diester-N2-isobutyryl-3'-O-tert-butyldimethylsilyl-guanosine]-[3'-O-phosphorothioate-diester-2-chloro-2'-deoxy-2'-fluoro-beta-adenosine]**

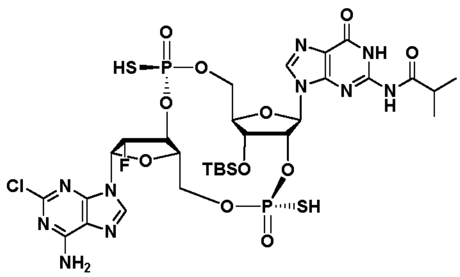

**Intermediate 89** (90 mg, 0.082 mmol) was dissolved in anhydrous DMF (7 mL), added with 4A molecular sieves (90 mg), stirred and dried at room temperature (28°C) under nitrogen for 0.5 hours. Then DBU (75.0 mg, 0.492 mmol) was added with a syringe, stirred at room temperature (28°C) for 0.5 hours, and celite was added for filration. Th filtrate was rotary evaporated to dry, to give the crude product **Intermediate 90** (100 mg, crude product) which was directly used in the next reaction.

MS-ESI $[M+H]^+$:926.8.

**Preparation of Intermediate 91: (*2R,3R,4S,5R*)-5-(6-Amino-2-chloro-9H-purin-9-yl)-2-((((((*2R,3R,4R,5R*)-5-((bis(4-methoxyphenyl)(phenyl)methoxy)methyl)-4-((tert-butyldimethylsilyl)oxy)-2-(2-isobutylamino-6-oxy-1,6-dihydro-9H-purin-9-yl)tetrahydrofuran-3-yl)oxy)(2-cyanoethoxy)phosphoryl)oxy)methyl)-4-fluoro-tetrahydrofuran-3-yl benzoate**

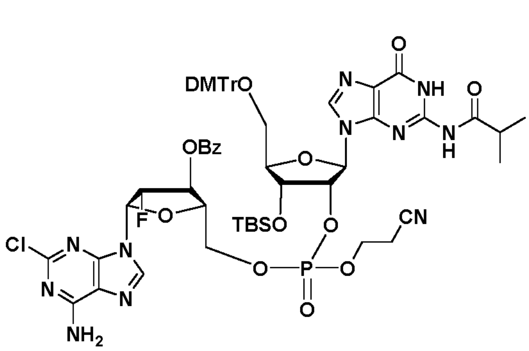

**Intermediate 84** (500 mg, 1.23 mmol) and ***(2R,3R,4R,5R)-5-((bis(4*-methoxyphenyl)(phenyl)methoxy)methyl)-4-((tert-butyldimethylsilyl)oxy)-2-(2-isobutylamino-6-oxy-1,6-dihydro-9H-purin-9-yl)tetrahydrofuran-3-yl (2-cyanoethyl)diisopropyl phosphoramide** (1.78 g, 1.84 mmol) were dissolved in a mixed solution of acetonitrile/tetrahydrofuran (8 mL/8 mL), added with 4A molecular sieves (500 mg), stirred at room temperature (28°C) under nitrogen

protection for 0.5 hours. Then a solution of tetrazole in acetonitrile (8.17 mL, 0.45 M) was added by syringe; after stirring at room temperature (28°C) for 2 hours, added with 70% tert-butanol peroxide (520.9 mg, 4.05 mmol, 0.544 mL), stirred at room temperature (28°C) for 20 min, and quenched with 50% aqueous sodium thiosulfate pentahydrate (5 mL); celite was added for filtration, and the filtrate was diluted with ethyl acetate (30 mL), washed with water (20 mL, twice), and the organic phase was dried over anhydrous sodium sulfate. The crude product obtained by concentration was purified by silica gel column eluting with petroleum ether : ethyl acetate (10:1-1:10) to give a mixture (1.9 g) which was directly used in the next step. MS-ESI [M+H]$^+$: 1296.0.

**Preparation of Intermediate 92: [2'-O-phosphodiester-5'-O-bis(4-methoxyphenyl) benzyl-3'-O-tert-butyldimethylsilyl-guanosine]-(2',5')-[2-chloro-2'-deoxy-2'-fluoro-beta-adenosine]**

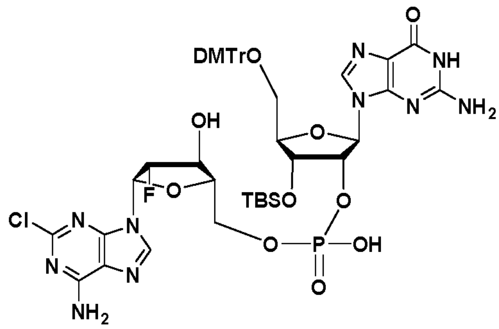

To **Intermediate 91** (1 g, 0.775 mmol) was added 33% methylamine in ethanol (8 mL), and stirred at room temperature for 3 hours. The reaction solution was rotary evaporated to dry and purified with a C18 reverse-phase column, eluting with water (containing 0.1% ammonium bicarbonate): acetonitrile (3:2), and a pink solid (550 mg) was obtained after lyophilization.

$^1$H NMR (400 MHz, DMSO-$d_6$) δ 10.65 (s, 1H), 8.18 (d, *J* = 2.0 Hz, 1H), 7.93-7.77 (m, 2H), 7.38-7.15 (m, 10H), 7.13-6.92 (m, 2H), 6.90-6.77 (m, 4H), 6.49-6.20 (m, 4H), 5.92 (d, *J* = 5.6 Hz, 1H), 5.26-5.09 (m, 2H), 4.61-4.24 (m, 3H), 3.98-3.82 (m, 3H), 3.79-3.67 (m, 7H), 0.79 (s, 9H), 0.14-0.02 (m, 6H). MS-ESI [M+H]$^+$: 1064.8.

**Preparation of Intermediate 93: [2'-O-phosphodiester-5'-O-bis(4-methoxyphenyl) benzyl-3'-O-tert-butyldimethylsilyl-guanosine]-(2',5')-[2-chloro-3'-O-phosphite-monoester-2'-deoxy-2'-fluoro-beta-adenosine]**

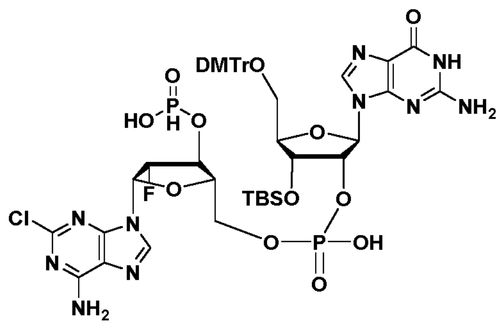

**Diphenyl phosphite** (137 mg, 0.47 mmol) was added to a solution of **Intermediate 92** (250 mg, 0.235 mmol), DBU (143 mg, 0.94 mmol) and 4A molecular sieves in pyridine (8 mL) at 0°C under nitrogen protection, and the resulting mixture was stirred at room temperature for 2 hours. The reaction solution was filtered and rotary evaporated to dry, and used directly for the next reaction. MS-ESI [M+H]$^+$: 1129.8.

**Preparation of Intermediate 94: [2'-O-phosphodiester-3'-O-tert-butyldimethylsilyl-guanosine]-(2',5')-[2-chloro-3'-O-phosphite-monoester-2'-deoxy-2'-fluoro-beta-adenosine]**

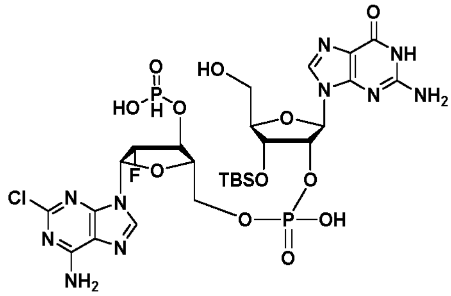

**Intermediate 94** was obtained from **Intermediate 93** via the route of **Intermediate 25.**

$^1$H NMR (400 MHz, DMSO-$d_6$) δ 8.26 (s, 1H), 7.99 (s, 1H), 7.88 (s, 2H), 6.55 (s, 2H), 6.27 (dd, *J* = 17.2, 4.0 Hz, 1H), 6.00-5.70 (m, 3H), 5.44-5.21 (m, 1H), 5.09-4.98 (m, 1H), 4.87-4.71 (m, 1H), 4.47 (d, *J* = 4.0 Hz, 1H), 4.07-3.97 (m, 1H), 3.91-3.73 (m, 3H), 3.56-3.49 (m, 5H), 0.89 (s, 9H), 0.14 (s, 6H). $^{31}$P NMR (162 MHz, DMSO-$d_6$) δ 1.72, -1.58. MS-ESI [M+H]$^+$: 826.8.

**Preparation of Intermediate 95: (2',3')-cyclo-[2'-O-phosphodiester-3'-O-tert-butyldimethylsilyl-guanosine]-[3'-O-phosphodiester-2-chloro-2'-deoxy-2'-fluoro-beta-adenosine]**

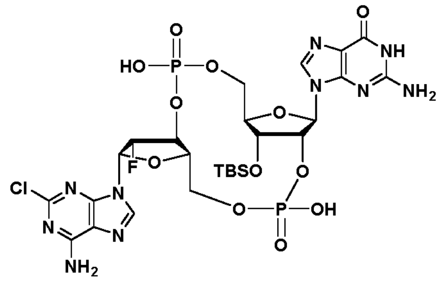

To a solution of **Intermediate 94** (55 mg, 0.067 mmol) in pyridine (15 mL) was added pivaloyl chloride (48 mg, 0.40 mmol) at 0°C, and the resulting reaction solution was stirred at room temperature for 3 hours. Then, a mixed solution of iodine (26 mg, 0.10 mmol) in acetonitrile (0.5 mL) and water (0.1 mL) was added thereto, and the resulting reaction solution was stirred at room temperature for 16 hours. The reaction solution was quenched with 10% sodium thiosulfate aqueous solution (5 mL) and then roatry evaporated to dry, purified on a C18 reverse-phase column eluting with water (containing 0.1% ammonium bicarbonate): acetonitrile (4:1), and freeze-dried to give a white solid (20 mg). MS-ESI [M+H] $^+$ : 825.0.

**Preparation of Intermediate 96: (2',3')-cyclo-[2'-O-Rp-phosphorothioate-diester-N2-isobutyryl-3'-O-tert-butyldimethylsilyl-guanosine]-[3'-O-phosphodiester-2-chloro-2'-deoxy-2'-fluoro-beta-adenosine]**

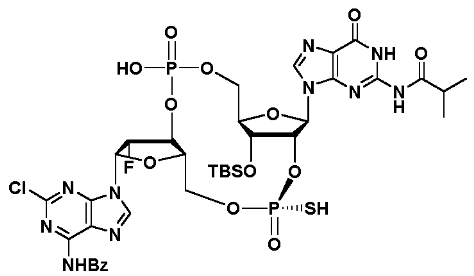

**Intermediate 96 was obtained from [2'-O-phosphodiester-N2-isobutyryl-3'-O-tert-butyldimethylsilyl-guanosine]-(2',5')-[N6-benzoyl-2-chloro-3'-O-phosphite- monoester-2'-deoxy-2'-fluoro-beta-adenosine]** via the route of **Intermediate 95.**

$^1$H NMR (400 MHz, DMSO-$d_6$) δ 12.92 (s, 1H), 12.12 (s, 1H), 11.54 (s, 1H), 8.55 (d, *J* = 2.0 Hz, 1H), 8.23 (s, 1H), 8.06 (d, *J* = 7.2 Hz, 2H), 7.71-7.61 (m, 1H), 7.59-7.51 (m, 3H), 6.47 (dd, *J* = 21.6, 2.4 Hz, 1H), 5.97-5.74 (m, 2H), 5.60-5.40 (m, 1H), 5.12-5.00 (m, 1H), 4.41 (d, *J* = 3.2 Hz, 1H), 4.27-4.15 (m, 1H), 4.13-3.94 (m, 5H), 2.97-2.84 (m, 1H), 1.65-1.54 (m, 1H), 1.09 (d, *J* = 7.2 Hz, 3H), 0.98 (d, *J* = 6.8 Hz, 3H), 0.93 (s, 9H), 0.20 (d, *J* = 8.6 Hz, 6H).

**Preparation of Intermediate 97: (2',3')-cyclo-[2'-O-phosphodiester-3'-O-tert-butyldimethylsilyl-guanosine]-[3'-O-*Rp*-phosphorothioate-diester-2-chloro-2'-deoxy-2'-fluoro-beta-adenosine]**

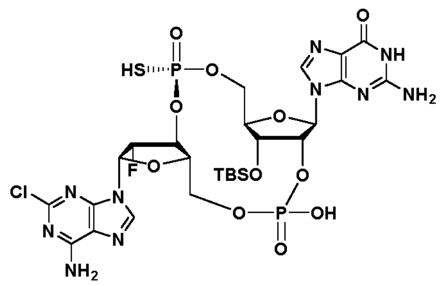

**Intermediate 97** was obtained via the route of **Intermediate 95** by reacting **Intermediate 92** with **(+)-PSI reagent,** followed by deprotection and cyclization, and purification with preparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate is 25 ml/min, dual-wavelength UV absorption monitoring at 214 and 254 nm, gradient elution: 0-2 min, 20%B; 2-14 min, 20-40%B; 14-14.2 min, 40-95%B; 14.2-18 min, 95%B; the compound's retention time: 12.5 min).

$^1$H NMR (400 MHz, DMSO-$d_6$) δ 10.59 (s, 1H), 8.18 (d, *J* = 2.4 Hz, 1H), 8.11 (s, 1H), 7.45 - 6.98 (m, 5H), 6.33 - 6.23 (m, 1H), 5.82 (d, *J* = 8.6 Hz, 1H), 5.42 (s, 1H), 5.32 - 5.23 (m, 2H), 4.33 (d, *J* = 4.0 Hz, 1H), 4.19 - 3.70 (m, 9H), 0.91 (s, 9H), 0.15 (s, 6H). $^{19}$F NMR (376 MHz, DMSO) δ -196.51 (s). $^{31}$P NMR (162 MHz, DMSO) δ 53.76 (s), -0.49 (s). MS-ESI $[M+H]^+$: 841.0.

**Preparation of Intermediate 98: (2',3')-cyclo-(*Rp*,*Rp*)-[2'-O-phosphorothioate-diester-N2-isobutyryl-3'-O-tert-butyldimethylsilyl-guanosine]-[3'-O-phosphorothioate-diester-2-chloro-2'-deoxy-2'-fluoro-beta-adenosine]**

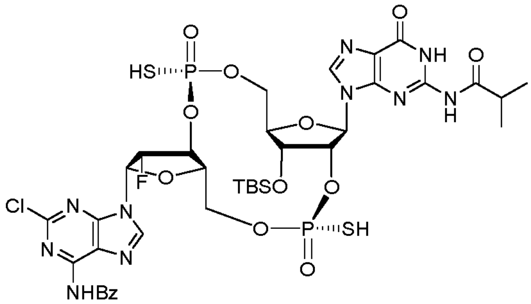

**Intermediate 98** was obtained via the synthesis route of **Intermediate 90** by reacting **Intermediate 87** with **(+)-PSI reagent,** followed by deprotection and cyclization.

$^1$H NMR (400 MHz, DMSO-d6) δ 12.41 (brs, 1H), 11.90 (s, 1H), 11.31 (s, 1H), 8.35 (d, *J* = 2.0 Hz, 1H), 8.03 (s, 1H), 7.85 (d, *J* = 7.2 Hz, 2H), 7.45 (t, *J* = 7.2 Hz, 1H), 7.35 (t, *J* = 7.6 Hz, 2H), 6.88 (t, *J* = 48 Hz, 6H), 6.25 (dd, *J* = 20.0, 4.0 Hz, 1H), 5.65 (d, *J* = 8.4 Hz, 1H), 5.59-5.46 (m, 1H), 5.32 (d, *J* = 50.0 Hz, 1H), 5.02 (t, *J* = 11.2 Hz, 1H), 4.15 (d, *J* = 4.0 Hz, 1H), 4.07-4.01 (m, 1H), 3.97-3.64 (m, 5H), 2.74-2.62 (m, 1H), 0.86 (d, *J* = 6.4 Hz, 3H), 0.81-0.66 (m, 12H), -0.01 (d, *J* = 9.6 Hz, 6H). MS+ESI--[M+H] : 1030.8.

**Example 1: (3',3')-cyclo-(*Rp*,*Rp*)-bis-[3'-O-phosphorothioate-diester-2-chloro-2'-deoxy-2'-fluoro-beta-adenosine]1.5-ammonium-0.5-1,8-diazabicycloundec-7-ene-salt**

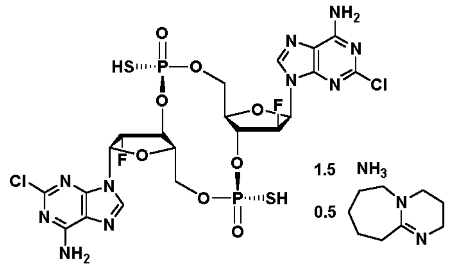

To a solution (8 mL) of **Intermediate 54** (120 mg, 0.150 mmol) and **(-)-PSI reagent** (201 mg, 0.450 mmol) in DMF was added 1,8-diazabicycloundec-7-ene (342 mg, 2.25 mmol), and the resulting mixture was stirred at 25°C for 1 hour. The reaction mixture was rotary evaporated to dry and reprecipitated with ethyl acetate (20 mL). The solid was collected, and the obtained crude product was purified with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-30%B; 14-14.3 min, 30-95%B; 14.3-20 min, 95%B; the compound' retention time: 13.5 min), and freeze-dried to give a white solid (20 mg).

$^1$H NMR (400 MHz, DMSO-$d_6$) δ 8.37 (s, 1H), 8.22 (s, 1H), 7.90 (brs, 4H), 7.12 (brs, 6H), 6.33-6.20 (m, 2H), 5.56-5.30 (m, 2H), 5.20-5.00 (m, 2H), 4.22-3.85 (m, 6H), 3.55-3.40 (m, 2H), 3.35-3.15 (m, 1H), 2.70-2.55 (m, 1H), 1.96-1.85 (m, 1H), 1.73-1.52 (m, 3H). $^{31}$P NMR (162 MHz, DMSO-$d_6$) δ 53.47. MS-ESI [M-H]$^-$: 760.9.

**Example 2: (3',3')-cyclo-(*Rp*,*Rp*)-bis-[3'-O-phosphorothioate-diester-2-chloro-2'-deoxyadenosine]**

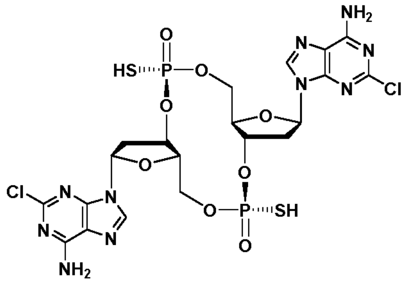

**Example 2** was obtained via the route of **Example 1** by reacting **Intermediate 55** and (-)-PSI **reagent,** and then purifying with preparative liquid chromatography (Gilson 281 Preparative HPLC, 19 x 250 mm Welch 10 μm preparative column, mobile phase A: 0.05% formic acid aqueous solution, B: 0.05% formic acid in acetonitrile, flow rate 25 ml/min, dual-wavelength UV absorption monitoring at 214 and 254 nm, gradient elution: 0-3 min, 0-5%B; 3-14 min, 5-50%B; 14-14.3 min, 50-95%B; 14.3-20 min, 95%B; the compound's retention time: 12 min).

$^{1}$H NMR (400 MHz, DMSO-$d_6$) δ 8.53 (s, 1H), 8.38 (s, 1H), 7.84 (brs, 4H), 6.33-6.18 (m, 2H), 5.20-4.75 (m, 2H), 4.30-3.75 (m, 6H), 3.00-2.70 (m, 2H), 2.70-2.50 (m, 2H).

$^{31}$P NMR (162 MHz, DMSO-$d_6$) δ 53.68, 52.69. MS-ESI [M-H]$^-$: 724.9.

**Example 3: (3',3')-cyclo-(*Rp,Rp*)-bis-[3'-O-phosphorothioate-diester-2'-deoxy-2',2'-difluorocytidine]**

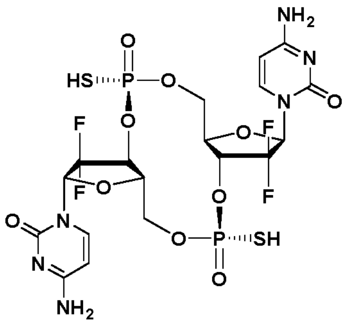

**Example** 3 was obtained via the route of **Example 1** by reacting **Intermediate 56** and **(-)-PSI reagent,** and then purifying with preparative liquid chromatography (Gilson 281 Preparative HPLC, 19 x 250 mm Welch 10 μm preparative column, mobile phase A: 0.05% formic acid aqueous solution, B: 0.05% formic acid in acetonitrile, flow rate 25 ml/min, dual-wavelength UV absorption monitoring at 214 and 254 nm, gradient elution: 0-3 min, 0-5%B; 3-14 min, 5-30%B; 14-14.3 min, 30-95%B; 14.3-20 min, 95%B; the compound's retention time: 18 min).

$^{1}$H NMR (400 MHz, DMSO-$d_6$) δ 9.20-8.80 (m, 2H), 8.31 (d, *J* = 6.4 Hz, 1H), 8.28-8.00 (m, 2H), 7.90 (d, *J* = 6.0 Hz, 1H), 6.20-6.05 (m, 3H), 6.00 (d, *J* = 6.4 Hz, 1H), 4.95-4.68 (m, 2H), 4.30-4.05 (m, 4H), 3.90-3.70 (m, 2H). $^{31}$P NMR (162 MHz, DMSO-$d_6$) δ 53.27, 52.67. MS-ESI [M+H]$^+$: 683.0.

**Example 4: (3',3')-cyclo-(*Rp,Rp*)-bis-[3'-O-phosphorothioate-diester-N4-(2-propylpentanoyl)-2'-deoxy-2',2'-difluorocytidine] diammonium salt**

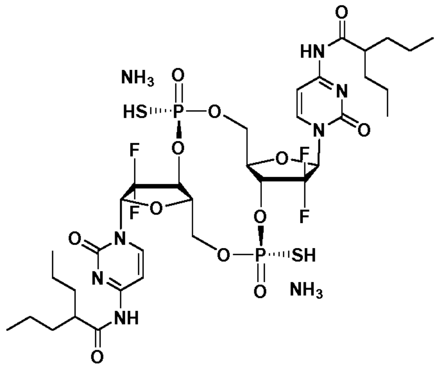

**Example** 4 was obtained via the route of **Example** 1 by reacting **Intermediate** 57 and **(-)-PSI reagent,** and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-35%B; 3-14 min, 35-70 %B; 14-14.3 min, 70-95%B; 14.3-20 min, 95%B; the compound's retention time: 14 min).

1H NMR (400 MHz, DMSO-$d_6$) δ 11.05 (s, 1H), 11.00 (s, 1H), 8.39 (d, *J* = 7.6 Hz, 1H), 8.16 (d, *J* = 7.6 Hz, 1H), 7.40 (d, *J* = 7.6 Hz, 1H), 7.34 (d, *J* = 7.6 Hz, 1H), 7.12 (t, *J* = 52.0 Hz, 8H), 6.30-6.18 (m, 2H), 5.02-4.75 (m, 2H), 4.35-4.10 (m, 4H), 3.83 (t, *J* = 11.2 Hz, 2H), 2.70-2.52 (m, 2H), 1.62-1.45 (m, 4H), 1.40-1.17 (m, 12H), 0.85 (t, *J* = 7.2 Hz, 12H). 31P NMR (162 MHz, DMSO) δ 54.54, 53.29. MS-ESI $[M+H]^+$: 935.1.

**Example 5: (2',3')-cyclo-(*Rp,Rp*)-[2'-O-phosphorothioate-diester-adenosine]-[3'-O-phosphorothioate-diester-2-chloro-2'-deoxy-2'-fluoro-beta-adenosine] diammonium salt**

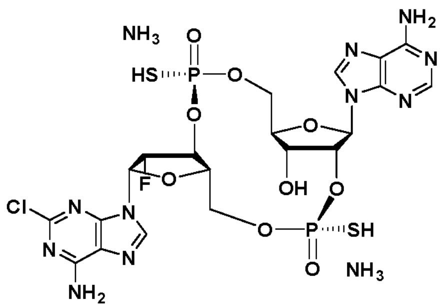

To **Intermediate 71** (200 mg, 0.214 mmol) was added 7 M ammonia in methanol (2 mL), and the resulting mixture was stirred at 25°C for 6 hours. The crude product was obtained after the reaction mixture was rotary evaporated to dry. The obtained crude product was purified with preparative liquid chromatography (Gilson 281 Preparative HPLC, 19 x 250 mm Welch 10 μm preparative column, mobile phase A: 0.05% formic acid aqueouse solution, B: 0.05% formic acid in acetonitrile, flow rate 25 ml/min, dual-wavelength UV absorption monitoring at 214 and 254 nm, gradient elution: 0-3 min, 0-5%B; 3-14 min, 5-30%B; 14-14.3 min, 30-95%B; 14.3-20 min, 95%B; the compound's retention time: 14 min), and freeze-dried to give a white solid (4.0 mg).

$^1$H NMR (500 MHz, DMSO-$d_6$) δ 8.63 (s, 1H), 8.23 (s, 1H), 8.17 (s, 1H), 7.92 (brs, 2H), 7.29 (brs, 2H), 6.33-6.28 (m, 1H), 6.12 (d, *J* = 8.5 Hz, 1H), 5.40-5.15 (m, 2H), 5.05-4.95 (m, 1H), 4.55 (t, *J* = 3.5 Hz, 1H), 4.36-4.24 (m, 2H), 4.19 (s, 1H), 4.10-3.95 (m, 2H), 3.90-3.78 (m, 1H), 3.72 (d, *J* = 12.0 Hz, 1H). $^{31}$P NMR (162 MHz, DMSO-$d_6$) δ 53.08, 48.81. MS-ESI $[M-H]^-$: 724.8.

**Example 6: (2',3')-cyclo-(*Rp,Rp)*-[2'-O-phosphorothioate-diester-adenosine]-[3'-O-phosphorothioate-diester-2-chloro-2'-deoxyadenosine] diammonium salt**

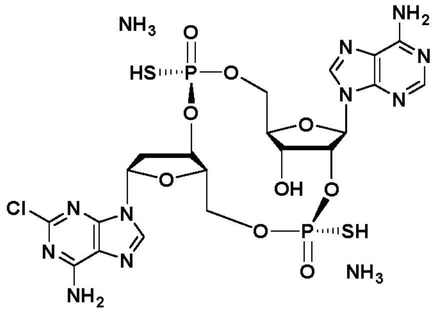

**Example** 6 was obtained via the route of **Example 5** by reacting **Intermediate** 72 with 28% ammonia water, and then purifying with preparative liquid chromatography (10 mM ammonium bicarbonate as an additive). Analytical LCMS: Agilent 1100+G1946D LCMS, 4.6 x 50 mm Waters XBridge C18 3.5 μm analytical column, mobile phase A: 10 mM ammonium bicarbonate aqueous solution, B: acetonitrile, flow rate 1.8 ml/min, dual-wavelength UV absorption monitoring at 214 and 254 nm, gradient elution: 0-1.5 min, 5-95%B; 1.5-3 min, 95%B. The compound's retention time: 0.35 min.

$^1$H NMR (400 MHz, DMSO-$d_6$) δ 8.61 (s, 1H), 8.40 (s, 1H), 8.17 (s, 1H), 7.86 (brs, 2H), 7.41-7.06 (m, 10H), 6.30-6.18 (m, 1H), 6.09 (d, *J* = 8.4 Hz, 1H), 5.42-5.30 (m, 2H), 5.30-5.20 (m, 1H), 4.27 (d, *J* = 4.4 Hz, 1H), 4.20-4.05 (m, 2H), 4.04-3.90 (m, 2H), 3.72-3.62 (m, 2H), 2.85-2.55 (m, 2H). 31P NMR (162 MHz, DMSO-$d_6$) δ 56.62, 53.65. MS-ESI [M-H]$^-$: 707.0.

**Example 7: (3',3')-cyclo-(*Rp,Rp*)-[3'-O-phosphorothioate-diester-adenosine]-[3'-O-phosphorothioate-diester-2-chloro-2'-deoxy-2'-fluoro-beta-adenosine] diammonium salt**

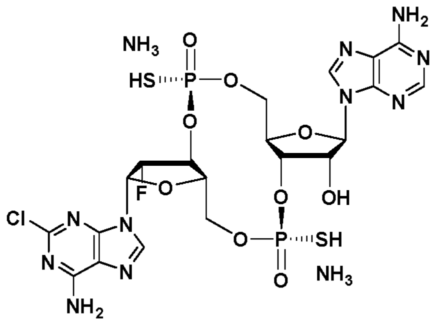

**Example** 7 was obtained via the route of **Example 5** by reacting **Intermediate 73** with 28% ammonia water, and then purifying with preparative liquid chromatography (10 mM ammonium bicarbonate as an additive). Analytical LCMS: Agilent 1100+G1946D LCMS, 4.6 x 150 mm Waters XBridge C18 3.5 μm analytical column, mobile phase A: 10 mM ammonium bicarbonate aqueous solution, B: acetonitrile, flow rate 1 ml/min, dual-wavelength UV absorption monitoring at 214 and 254 nm, gradient elution: 0-8 min, 5-95%B; 8-15 min, 95%B. The compound's retention time: 4.1 min.

$^1$H NMR (400 MHz, DMSO-$d_6$) δ 8.62 (s, 1H), 8.23 (s, 1H), 8.19 (s, 1H), 7.95 (brs, 2H), 7.65 (brs, 2H), 7.14 (t, *J* = 52.0 Hz, 8H), 6.30-6.22 (m, 1H), 6.12 (d, *J* = 8.4 Hz, 1H), 5.50-5.32 (m, 2H), 5.30-5.15 (m, 1H), 5.08-4.92 (m, 1H), 4.30-4.00 (m, 5H), 3.95-3.86 (m, 1H), 3.68 (d, *J* = 12.0 Hz, 1H). $^{31}$P NMR (162 MHz, DMSO-$d_6$) δ 57.14, 54.13. MS-ESI [(M-2H)/2]$^-$: 362.0.

**Example 8: (2',3')-cyclo-(*Sp,Rp*)-[2'-O-phosphorothioate-diester-guanosine]-[3'-O-phosphorothioate-diester-2-chloro-2'-deoxy-2'-fluoro-beta-adenosine] diammonium salt, and**

**Example 9: (2',3')-cyclo-(*Sp,Sp*)-[2'-O-phosphorothioate-diester-guanosine]-[3'-O-phosphorothioate-diester-2-chloro-2'-deoxy-2'-fluoro-beta-adenosine] diammonium salt**

**Example 8** and **Example 9** were obtained via the route of **Example 5** by reacting **Intermediate 74** and 28% ammonia water, and then purifying with preparative liquid chromatography (Gilson 281 Preparative HPLC, 19 x 250 mm

Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-2%B; 3-14 min, 2-37%B; 14-14.3 min, 37-95%B; 14.3-20 min, 95%B; Compound 8's retention time: 13 min, and Compound 9's retention time: 15 min).

**Example 8:**

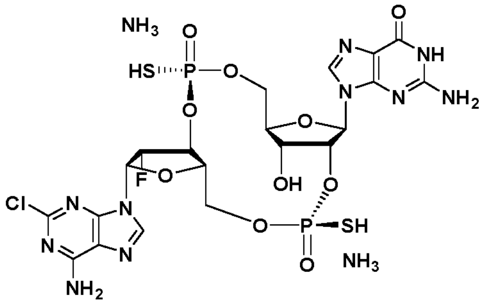

$^1$H NMR (400 MHz, DMSO-$d_6$) δ 10.62 (brs, 1H), 8.19 (s, 1H), 8.11 (s, 1H), 7.94 (brs, 2H), 7.10 (t, *J* = 50.8 Hz, 8H), 6.63 (brs, 2H), 6.25 (dd, *J* = 24.0, 2.8 Hz, 1H), 5.85 (d, *J* = 8.8 Hz, 1H), 5.50-5.15 (m, 4H), 4.30-4.13 (m, 2H), 4.12-3.92 (m, 4H), 3.72 (d, *J* = 12.0 Hz, 1H). $^{31}$P NMR (162 MHz, DMSO-$d_6$) δ 56.84, 54.05. MS-ESI [M-H]$^-$: 741.0.

**Example 9:**

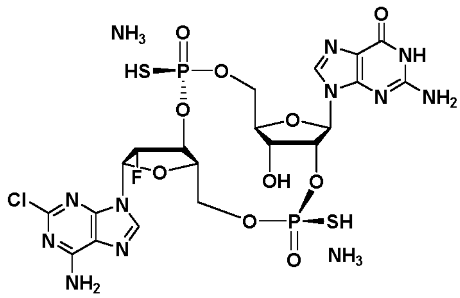

$^1$H NMR (400 MHz, DMSO-$d_6$) δ 10.67 (brs, 1H), 8.23 (s, 1H), 8.08 (s, 1H), 7.93 (brs, 2H), 7.10 (t, *J* = 51.2 Hz, 8H), 6.67 (brs, 2H), 6.30 (dd, *J* = 18.8, 3.6 Hz, 1H), 5.77 (d, *J* = 8.0 Hz, 1H), 5.58-5.38 (m, 1H), 5.02-4.92 (m, 1H), 4.86-4.78 (m, 1H), 4.69-4.60 (m, 1H), 4.33-3.75 (m, 7H). $^{31}$P NMR (162 MHz, DMSO-$d_6$) δ 59.03, 54.74. MS-ESI [M-H]$^-$: 740.9.

**Example 10: (2',3')-cyclo-*(Rp,Rp)*-[2'-O-phosphorothioate-diester-guanosine]-[3'-O-phosphorothioate-diester-2-chloro-2'-deoxy-2'-fluoro-beta-adenosine] diammonium salt**

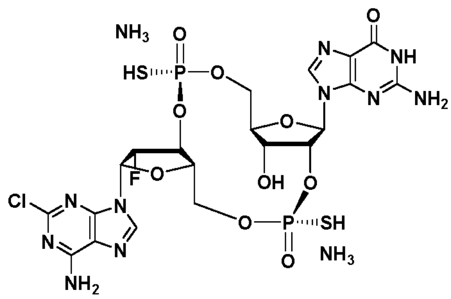

**Example 10** was obtained via the route of **Example 5** by reacting **Intermediate 75** and 28% ammonia water, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-38%B; 14-14.3 min, 38-95%B; 14.3-20 min, 95%B; the compound's retention time: 13 min). **Example 10 was** also obtained via the route of **Example 25 by** deprotecting **Intermediate 98,** and purifying with preparative liquid chromatography. Two different synthetic routes led to exactly the same product.

$^{1}$H NMR (400 MHz, DMSO-$d_6$) δ 10.63 (brs, 1H), 8.22 (s, 1H), 8.18 (s, 1H), 7.93 (brs, 2H), 7.12 (t, *J* = 51.2 Hz, 8H), 6.69 (brs, 2H), 6.28 (dd, *J* = 24.0, 2.4 Hz, 1H), 5.88 (d, *J* = 8.4 Hz, 1H), 5.42-4.80 (m, 4H), 4.45 (d, *J* = 4.0 Hz, 1H), 4.39-4.10 (m, 3H), 4.08-3.94 (m, 1H), 3.93-3.70 (m, 2H). $^{31}$P NMR (162 MHz, DMSO-$d_6$) δ 54.07, 50.13. MS-ESI [M-H]$^-$: 741.0.

**Example 11: (2',3')-cyclo-(*Sp*,*Rp*)-[2'-O-phosphorothioate-diester-guanosine]-[3'-O-phosphorothioate-diester-2-chloro-2'-deoxy-adenosine] diammonium salt**

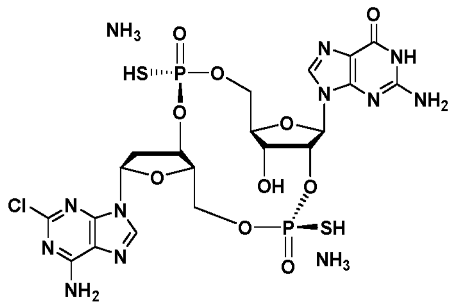

**Example 11** was obtained via the route of **Example 5** by reacting **Intermediate 76** with 7 M ammonia methanol solution, and then purifying with preparative liquid chromatography (10 mM ammonium bicarbonate as an additive). Analytical LCMS: Agilent 1100+G1946D LCMS, 4.6 x 150 mm Waters XBridge C18 3.5 μm analytical column, mobile phase A: 10 mM ammonium bicarbonate aqueous solution, B: acetonitrile, flow rate 1 ml/min, dual-wavelength UV absorption monitoring at 214 and 254 nm, gradient elution: 0-8 min, 5-95%B; 8-15 min, 95%B; the compound's retention time: 3.3 min.

$^{1}$H NMR (400 MHz, DMSO-$d_6$) δ 10.63 (brs, 1H), 8.39 (s, 1H), 8.21 (s, 1H), 7.83 (brs, 2H), 7.10 (t, *J* = 50.8 Hz, 8H), 6.67 (brs, 2H), 6.30-6.18 (m, 1H), 5.87 (d, *J* = 8.4 Hz, 1H), 5.35-5.20 (m, 2H), 4.28 (d, *J* = 4.0 Hz, 1H), 4.21-3.92 (m, 4H), 3.80-3.60 (m, 2H), 2.92-2.70 (m, 1H), 2.69-2.55 (m, 1H). $^{31}$P NMR (162 MHz, DMSO-$d_6$) δ 56.96, 54.34. MS-ESI [M-H]$^-$: 722.9.

**Example 12: (2',3')-cyclo-(*Rp*,*Rp*)-[2'-O-phosphorothioate-diester-guanosine]-[3'-O-phosphorothioate-diester-2-chloro-2'-deoxy-adenosine] diammonium salt**

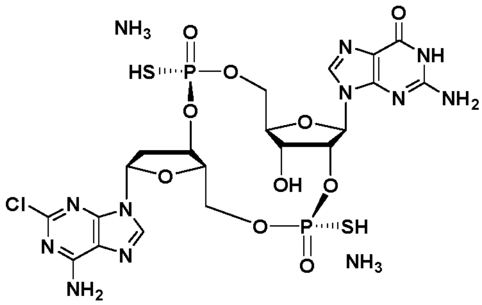

**Example 12** was obtained via the route of **Example 5** by reacting **Intermediate 77** with 7 M ammonia methanol solution, and then purifying with preparative liquid chromatography (10 mM ammonium bicarbonate as an additive). Analytical LCMS: Agilent 1100+G1946D LCMS, 4.6 x 150 mm Waters XBridge C18 3.5 μm analytical column, mobile phase A: 10 mM ammonium bicarbonate aqueous solution, B: acetonitrile, flow rate 1 ml/min, dual-wavelength UV absorption monitoring at 214 and 254 nm, gradient elution: 0-8 min, 5-95%B; 8-15 min, 95%B; the compound's retention time: 3.3 min.

$^1$H NMR (400 MHz, DMSO-$d_6$) δ 10.61 (brs, 1H), 8.39 (s, 1H), 8.20 (s, 1H), 7.82 (brs, 2H), 7.11 (t, *J* = 51.2 Hz, 8H), 6.70 (brs, 2H), 6.30-6.18 (m, 1H), 5.87 (d, *J* = 8.4 Hz, 1H), 5.25-5.13 (m, 2H), 4.96 (s, 1H), 4.50 (d, *J* = 4.4 Hz, 1H), 4.31-3.71 (m, 6H), 3.03-2.82 (m, 1H), 2.65-2.50 (m, 1H). $^{31}$P NMR (162 MHz, DMSO-$d_6$) δ 54.26, 49.51. MS-ESI [M-H]$^-$: 722.8.

**Example 13: (2',3')-cyclo-(*Rp*,*Rp*)-[2'-O-phosphorothioate-diester-guanosine]-[3'-O-phosphorothioate-diester-2'-deoxy-2',2'-difluorocytidine]diammonium salt**

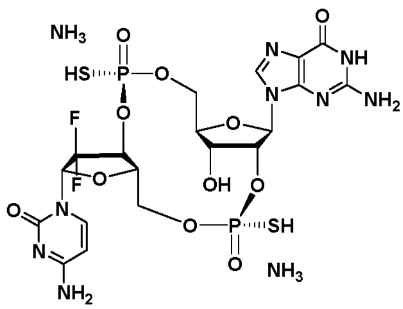

**Example 13** was obtained via the route of **Example 5** by reacting **Intermediate 78** and 28% ammonia water, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-25%B; 14-14.3 min, 25-95%B; 14.3-20 min, 95%B; the compound's retention time: 2 min).

$^1$H NMR (400 MHz, DMSO-$d_6$) δ 8.16 (s, 1H), 7.60-6.70 (m, 11H), 6.58 (brs, 2H), 6.35-6.20 (m, 1H), 5.90-5.70 (m, 2H), 5.22-5.10 (m, 1H), 5.00-4.80 (m, 2H), 4.52 (s, 1H), 4.32-4.15 (m, 2H), 4.11 (s, 1H), 4.00-3.82 (m, 2H), 3.78-3.65 (m, 1H). $^{31}$P NMR (162 MHz, DMSO-$d_6$) δ 53.24, 48.05. MS-ESI [(M-2H)/2]$^-$: 350.0.

**Example 14: (2',3')-cyclo-(*Rp*,*Rp*)-[2'-O-phosphorothioate-diester-guanosine]-[3'-O-phosphorothioate-diester-N4-(2-propylpentanoyl)-2'-deoxy-2',2'-difluoro-cytidine] diammonium salt**

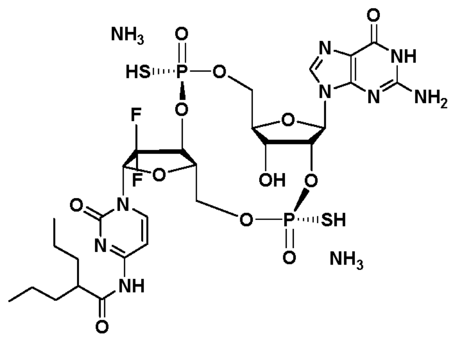

**Example 14** was obtained via the route of **Example** 5 by reacting **Intermediate 79** and 7 M ammonia-methanol solution, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-10%B; 3-14 min, 10-55%B; 14-14.3 min, 55-95%B; 14.3-20 min, 95%B; the compound's retention time: 11 min).

$^1H$ NMR (400 MHz, DMSO-$d_6$) δ 11.07 (brs, 1H), 10.62 (s, 1H), 8.68-7.97 (m, 2H), 7.40-6.97 (m, 9H), 6.59 (brs, 2H), 6.34-6.20 (m, 1H), 5.90-5.70 (m, 1H), 5.47-5.09 (m, 2H), 5.05-4.74 (m, 1H), 4.29 (d, *J* = 4.4 Hz, 1H), 4.20 (d, *J* = 9.6 Hz, 1H), 4.13-3.99 (m, 2H), 3.95-3.80 (m, 1H), 3.85-3.65 (m, 1H), 2.70-2.55 (m, 1H), 1.60-1.46 (m, 2H), 1.45-1.10 (m, 6H), 0.86 (t, *J* = 7.2 Hz, 6H). $^{31}P$ NMR (162 MHz, DMSO-$d_6$) δ 56.16, 53.39. MS-ESI [M-H]$^-$: 827.0.

**Example 15: (3',3')-cyclo-(*Sp*,*Rp*)-[3'-O-phosphorothioate-diester-guanosine]-[3'-O-phosphorothioate-diester-2-chloro-2'-deoxy-2'-fluoro-beta-adenosine] diammonium salt**

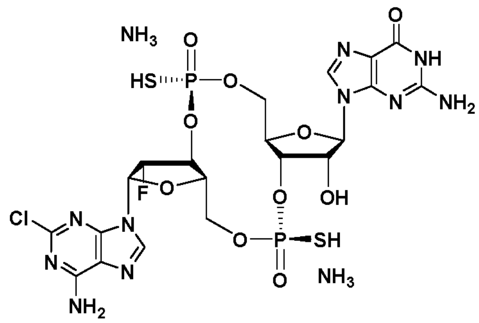

**Example 15** was obtained via the route of **Example** 5 by reacting **Intermediate 80** and 7 M ammonia-methanol solution, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-33%B; 14-14.3 min, 33-95%B; 14.3-20 min, 95%B; the compound's retention time: 11 min).

$^1H$ NMR (400 MHz, DMSO-$d_6$) δ 8.20 (d, *J* = 2.8 Hz, 1H), 8.04 (s, 1H), 8.03-6.87 (m, 10H), 6.60 (brs, 2H), 6.22 (dd, *J* = 24.0, 2.0 Hz, 1H), 5.85 (d, *J* = 8.8 Hz, 1H), 5.51-5.15 (m, 3H), 4.35-3.89 (m, 6H), 3.72 (d, *J* = 12.4 Hz, 1H). $^{31}P$ NMR (162 MHz, DMSO-$d_6$) δ 56.65, 54.03. MS-ESI [M-H]$^-$: 741.0.

**Example 16: (3',3')-cyclo-(*Rp*,*Rp*)-[3'-O-phosphorothioate-diester-guanosine]-[3'-O-phosphorothioate-diester-2-chloro-2'-deoxy-2'-fluoro-beta-adenosine] diammonium salt**

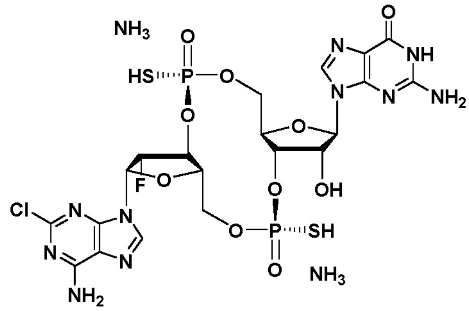

**Example 16** was obtained via the route of **Example 5** by reacting **Intermediate 81** and 7 M ammonia methanol solution, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-33%B; 14-14.3 min, 33-95%B; 14.3-20 min, 95%B; the compound's retention time: 13 min).

$^{1}H$ NMR (400 MHz, DMSO-$d_6$) δ 10.63 (brs, 1H), 8.22 (s, 1H), 8.09 (s, 1H), 7.94 (brs, 2H), 7.38-6.94 (m, 8H), 6.65 (brs, 2H), 6.28 (dd, *J* = 24.0, 2.4 Hz, 1H), 5.87 (d, *J* = 8.8 Hz, 1H), 5.42-5.11 (m, 3H), 5.00 (s, 1H), 4.56-4.42 (m, 1H), 4.39-4.21 (m, 2H), 4.13 (s, 1H), 4.05-3.94 (m, 1H), 3.89-3.71 (m, 2H). $^{31}P$ NMR (162 MHz, DMSO-$d_6$) δ 53.97, 49.08. MS-ESI [M-H]$^-$: 740.9.

**Example 17: (3',3')-cyclo-(*Rp*,*Rp*)-[3'-O-phosphorothioate-diester-guanosine]-[3'-O-phosphorothioate-diester-2-chloro-2'-deoxy-adenosine] diammonium salt**

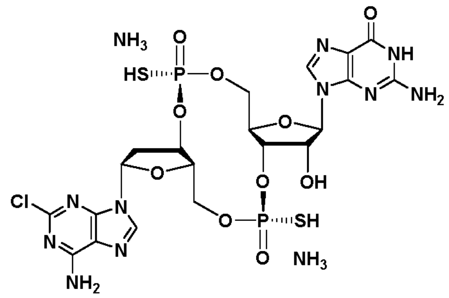

**Example 17** was obtained via the route of **Example 5** by reacting **Intermediate 82** and 7 M ammonia methanol solution, and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-3%B; 3-14 min, 3-33% B; 14-14.3 min, 33-95%B; 14.3-20 min, 95%B; the compound's retention time: 11 min).

$^{1}H$ NMR (400 MHz, DMSO-$d_6$) δ 10.59 (brs, 1H), 8.41 (s, 1H), 8.11 (s, 1H), 7.85 (brs, 2H), 7.15 (brs, 8H), 6.66 (brs, 2H), 6.25 (dd, *J* = 12.8, 6.0 Hz, 1H), 5.85 (d, *J* = 8.8 Hz, 1H), 5.35-5.11 (m, 2H), 4.91 (s, 1H), 4.59-4.50 (m, 1H), 4.31-3.71 (m, 6H), 3.03-2.87 (m, 1H). $^{31}P$NMR (162 MHz, DMSO-$d_6$) δ 53.46, 47.84. MS-ESI [M-H]$^-$: 740.9.

**Example 18: (3',3')-cyclo-(*Rp*,*Rp*)-[3'-O-phosphorothioate-diester-2'-deoxy-2'-fluoroadenosine]-[3'-O-phosphorothioate-diester-2-chloro-2'-deoxy-2'-fluoro-beta-adenosine]-0.5-ammonium-1.5-1,8-diazabicycloundec-7-ene-salt**

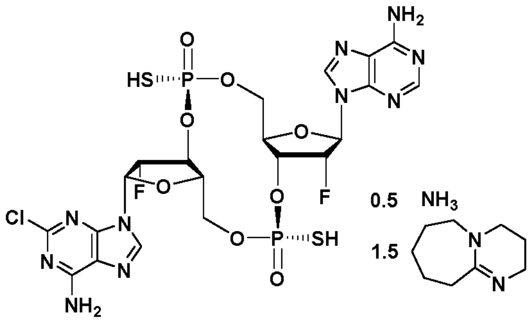

**Example 18** was obtained via the route of **Example 1** by reacting **Intermediate 67** and **(-)-PSI reagent,** and then purifying with preparative liquid chromatography (Gilson 281 Preparative HPLC, 19 x 250 mm Welch 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-3 min, 0-5%B; 3-14 min, 5-33%B; 14-14.3 min, 33-95%B; 14.3-20 min, 95%B; the compound's retention time: 11 min).

$^1$H NMR (400 MHz, DMSO-$d_6$) δ 9.62 (brs, 2H), 8.39 (s, 1H), 8.26 (d, *J* = 2.0 Hz, 1H), 8.18 (s, 1H), 7.36 (brs, 2H), 7.15 (t, *J* = 52.0 Hz, 2H), 6.31-6.20 (m, 2H), 5.46-5.25 (m, 2H), 5.15-5.00 (m, 1H), 4.95-4.83 (m, 1H), 4.42-4.25 (m, 1H), 4.10-3.98 (m, 2H), 3.80-3.60 (m, 2H), 3.60-3.40 (m, 6H), 3.30-3.20 (m, 3H), 2.70-2.55 (m, 3H), 1.96-1.85 (m, 3H), 1.73-1.50 (m, 9H). $^{31}$P NMR (162 MHz, DMSO-$d_6$) δ 53.37, 52.10. MS-ESI [M+H]$^+$: 729.0.

**Example 19: (3',3')-cyclo-(*Rp*,*Rp*)-[3'-O-phosphorothioate-diester-2'-deoxy-2'-fluoroadenosine]-[3'-O-phosphorothioate-diester-2-chloro-2'-deoxyadenosine] 0.5-1,8-diazabicycloundec-7-ene-salt**

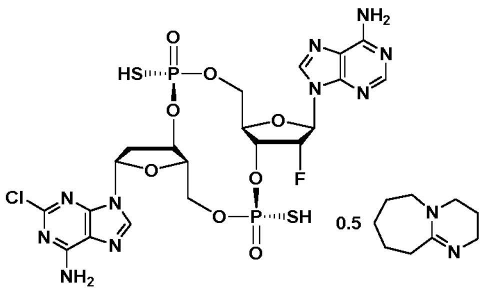

**Example 19** was obtained via the route of **Example 1** by reacting **Intermediate 68** and **(-)-PSI reagent,** and then purifying with preparative liquid chromatography (Gilson 281 Preparative HPLC, 19 x 250 mm Welch 10 μm preparative column, mobile phase A: 0.05% formic acid aqueous solution, B: 0.05% formic acid in acetonitrile, flow rate 25 ml/min, dual-wavelength UV absorption monitoring at 214 and 254 nm, gradient elution: 0-3 min, 0-10%B; 3-14 min, 10-50%B; 14-14.3 min, 50-95%B; 14.3-20 min, 95%B; the compound's retention time: 10 min).

$^1$H NMR (400 MHz, DMSO-$d_6$) δ 9.56 (s, 1H), 8.60 (s, 1H), 8.50-8.35 (m, 1H), 8.28 (s, 1H), 7.85 (brs, 2H), 6.40-6.20 (m, 2H), 5.70-5.40 (m, 1H), 5.18-4.80 (m, 2H), 4.60-3.95 (m, 6H), 3.65-3.45 (m, 2H), 3.30-3.20 (m, 1H), 2.85-2.71 (m, 1H), 2.70-2.55 (m, 2H), 1.96-1.85 (m, 1H), 1.73-1.50 (m, 3H). $^{31}$P NMR (162 MHz, DMSO-$d_6$) δ 54.78, 54.00. MS-ESI [M+H]$^+$: 710.8.

**Example 20: (3',3')-cyclo-(*Rp*,*Rp*)-[3'-O-phosphorothioate-diester-2'-deoxy-2'-fluoroguanosine]-[3'-O-phosphorothioate-diester-2-chloro-2'-deoxy-2'-fluoro-beta-adenosine] diammonium salt**

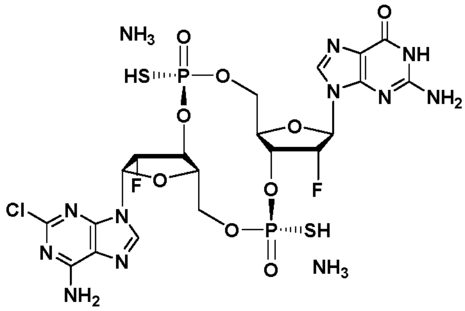

**Example 20** was obtained via the route of **Example 1** by reacting **Intermediate 69** and **(-)-PSI reagent,** and then purifying with preparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 5%B; 2-9 min, 5-15%B; 9-19 min, 15-25%B; 19-19.5 min, 25-95%B; 19.5-22.5 min, 95%B; the compound's retention time: 9 min).

$^1$H NMR (400 MHz, DMSO-$d_6$) δ 10.69 (brs, 1H), 8.26 (s, 1H), 7.98 (s, 1H), 7.92 (brs, 2H), 7.15 (t, *J* = 52.0 Hz, 6H), 6.64 (brs, 2H), 6.26 (dd, *J* = 17.2, 3.6 Hz, 1H), 6.08 (d, *J* = 16.8 Hz, 1H), 5.43-5.35 (m, 1H), 5.31-5.22 (m, 1H), 5.18-5.02 (m, 1H), 4.99-4.85 (m, 1H), 4.42-4.30 (m, 1H), 4.26 (d, *J* = 8.8 Hz, 1H), 4.15-4.00 (m, 2H), 3.90-3.70 (m, 2H). $^{31}$P NMR (162 MHz, DMSO-$d_6$) δ 54.20, 53.14. MS-ESI [M-H]$^-$: 743.2.

**Example 21: (3',3')-cyclo-*(Rp,Rp)*-[3'-O-phosphorothioate-diester-2'-deoxy-2'-fluoroguanosine]-[3'-O-phosphorothioate-diester-2-chloro-2'-deoxyadenosine] diammonium salt**

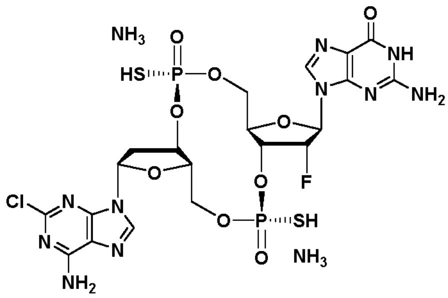

**Example 21** was obtained via the route of **Example 1** by reacting **Intermediate 70** and **(-)-PSI reagent,** and then purifying with preparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 5%B; 2-10 min, 5-20%B; 10-10.6 min, 20-95%B; 10.6-12.6 min, 95%B; the compound's retention time: 7.3 min).

$^1$H NMR (400 MHz, DMSO-$d_6$) δ 10.67 (brs, 1H), 8.49 (s, 1H), 7.92 (s, 1H), 7.82 (brs, 2H), 7.14 (t, *J* = 52.0 Hz, 6H), 6.62 (s, 2H), 6.25 (dd, *J* = 6.4, 4.8 Hz, 1H), 6.05 (d, *J* = 16.8 Hz, 1H), 5.44 (dd, *J* = 52.0, 4.0 Hz, 1H), 5.05-4.85 (m, 2H), 4.33 (d, *J* = 12.4 Hz, 1H), 4.23 (d, *J* = 9.2 Hz, 1H), 4.10-3.92 (m, 2H), 3.98-3.50 (m, 2H), 2.78-2.54 (m, 2H). $^{31}$P NMR (162 MHz, DMSO-$d_6$) δ 52.56, 52.51. MS-ESI [M-H]$^-$: 725.2.

**Example 22: (2',3')-cyclo-[2'-O-phosphodiester-guanosine]-[3'-O-phosphodiester-2-chloro-2'-deoxy-2'-fluoro-beta-adenosine]**

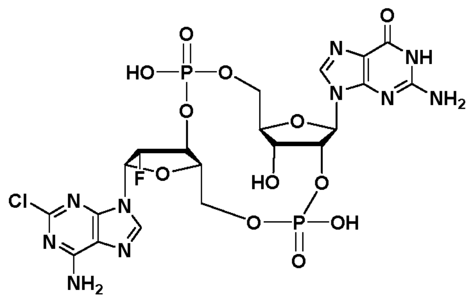

**Example 22** was obtained via the route of **Example 9** by reacting **Intermediate 95** and ammonium fluoride in methanol solution, and then purifying with preparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 5%B; 2-15.6 min, 5-20%B; 15.6-15.8 min, 20-95%B; 15.8-18 min, 95%B; the compound's retention time: 6.6 min).

$^1$H NMR (400 MHz, DMSO-*d*$_6$) δ 10.77 (s, 1H), 8.20 (d, *J* = 2.8 Hz, 1H), 7.99 (s, 1H), 7.93 (brs, 2H), 6.71 (brs, 2H), 6.33 (dd, *J* = 24.0, 2.4 Hz,1H), 5.90 (d, J = 8.0 Hz, 1H), 5.40 (d, J = 49.6 Hz, 1H), 5.20 - 5.00 (m, 2H), 4.42-4.30 (m, 2H), 4.20-3.80 (m, 6H). $^{31}$P NMR (162 MHz, DMSO-*d*$_6$) δ 1.61, -0.07. MS-ESI [M+H]$^+$: 710.9.

**Example 23: (2',3')-cyclo-[2'-O-Rp-phosphorothioate-diester-guanosine]-[3'-O-phosphodiester-2-chloro-2'-deoxy-2'-fluoro-beta-adenosine] diammonium salt**

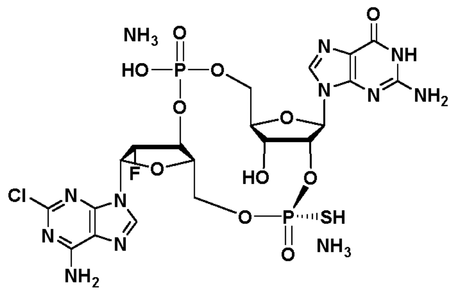

To **Intermediate 96** (75 mg, 0.074 mmol) was added 33% methylamine ethanol solution (3 mL) and stirred at room temperature for 3 hours. After the reaction solution was rotary evaporated to dry, ammonium fluoride (82 mg, 2.2 mmol) and methanol (3 mL) were added and stirred at 60°C for 16 hours. Purification with reparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate 25 ml/min, dual wavelength UV absorption monitoring at 214 and 254 nm, gradient elution: 0-2 min, 5%B; 2-18.9 min, 5-15%B; 18.9-19.4 min, 15-95%B; 19.4-22.4 min, 95%B; the compound's retention time: 8.5 min) and lyophilization gave a white solid (30.3 mg).

$^1$H NMR (400 MHz, DMSO-*d*$_6$) δ 10.60 (s, 1H), 8.21 (d, *J* = 2.8 Hz, 1H), 8.01 (s, 1H), 7.91 (s, 2H), 7.35-6.94 (m, 6H), 6.63 (s, 2H), 6.29 (dd, *J* = 24.4, 1.6 Hz,1H), 5.86 (d, *J* = 8.0 Hz, 1H), 5.42-5.17 (m, 2H), 5.09-4.90 (m, 2H), 4.48 (d, *J* = 4.0 Hz, 1H), 4.36 (dd, *J* = 10.8, 5.2 Hz, 1H), 4.28-4.17 (m, 1H), 4.09 (s, 1H), 3.98-3.73 (m, 3H). $^{31}$P NMR (162 MHz, DMSO-*d*$_6$) δ 48.92, -2.42. MS-ESI [M+H]$^+$: 726.8.

**Example 24: (2',3')-cyclo-[2'-O-phosphodiester-guanosine]-[3'-O-*Rp*-phosphorothioate-diester-2-chloro-2'-deoxy-2'-fluoro-beta-adenosine]**

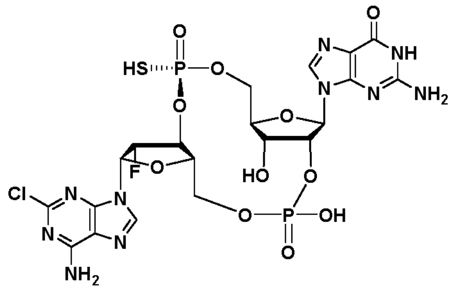

**Example 24** was obtained via the route of **Example 9** by reacting **Intermediate** 97 and ammonium fluoride in methanol solution, and then purifying with preparative liquid chromatography (Gilson 281 Preparative HPLC, 19 x 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 20%B; 2-14 min, 20-40%B; 14-14.2 min, 40-95%B; 14.2-18 min, 95%B; the compound's retention time: 2 min).

$^1H$ NMR (400 MHz, DMSO-$d_6$) δ 10.58 (s, 1H), 8.19 (d, *J* = 2.8 Hz, 1H), 8.04 - 7.82 (m, 2H), 7.37 - 7.00 (m, 4H), 6.73 - 6.53 (m, 1H), 6.27 (dd, *J* = 24.0, 2.4 Hz, 1H), 5.83 (d, *J* = 8.3 Hz, 1H), 5.44 - 5.19 (m, 2H), 5.18 - 5.06 (m, 1H), 5.04 - 4.95 (m, 1H), 4.36 - 4.27 (m, 2H), 4.12 (s, 1H), 4.09 - 3.96 (m, 2H), 3.79 - 3.65 (m, 2H). $^{31}P$ NMR (162 MHz, DMSO-$d_6$) δ 53.89 (s), -1.20 (s). $^{19}F$ NMR (376 MHz, DMSO-$d_6$) δ -195.45 (s). MS-ESI [M+H]$^+$: 726.

**Example 25: (2',3')-cyclo-(*Rp,Sp*)-[2'-O-phosphorothioate-diester-guanosine]-[3'-O-phosphorothioate-diester-2-chloro-2'-deoxy-2'-fluoro-beta-adenosine] diammonium salt**

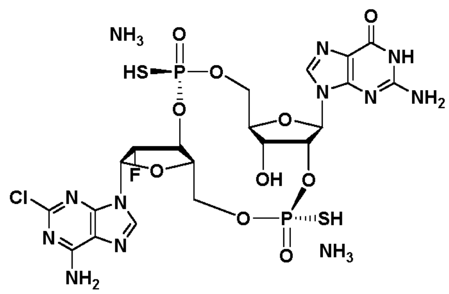

**Intermediate 90** (100 mg, crude product) was dissolved in 7 M ammonia methanol solution (3 mL), stirred at room temperature (28°C) for **4** hours and then rotary evaporated to dry. The resulting residue was dissolved in methanol (1 mL), added with ammonium fluoride (60.5 mg, 1.63 mmol), and stirred under heating (60°C) for 20 hours. After the completion of the reaction was monitored by LCMS and HPLC, the reaction solution was purified by preparative liquid chromatography (Gilson 281 Preparative HPLC, 19 × 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 5%B; 2-18.9 min, 5-15%B; 18.9-19.4 min, 15-95%B; 19.4-22.4 min, 95%B; the compound's retention time: 10 min), and then freeze-dried to give a white solid (21 mg).

$^1H$ NMR (400 MHz, DMSO-$d_6$) δ 10.71 (s, 1H), 8.44 (s, 1H), 8.23 (s, 1H), 7.92 (s, 2H), 7.11 (t, *J* = 52Hz, 6H), 6.56 (s, 2H), 6.29 (d, *J* = 28Hz, 1H), 5.95 (d, *J* = 8.4 Hz, 1H), 5.35 (d, *J* = 50.4 Hz, 1H), 5.20 (t, *J* = 11.8 Hz, 1H), 5.11-5.05 (m, 1H), 4.48 (d, *J* = 3.6 Hz, 1H), 4.39-4.28 (m, 2H), 4.14 (s, 1H), 4.02-3.96 (m, 1H), 3.88-3.83 (m, 1H), 3.81-3.76 (m, 1H). $^{31}P$ NMR (162 MHz, DMSO-$d_6$) δ 53.30, 50.07. $^{19}F$ NMR (376 MHz, DMSO-$d_6$) δ -196.87. MS-ESI [M+H]$^+$:742.8.

**Example 26: (2',3')-cyclo-(*Rp,Rp*)-[2'-O-phosphorothioate-diester-guanosine]-[3'-O-phosphorothioate-diester-2-methylamino-2'-deoxy-2'-fluoro-beta-adenosine] diammonium salt**

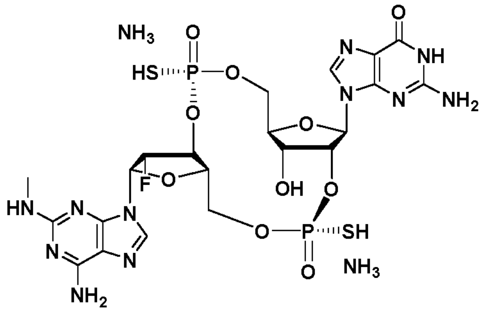

**Example 26** was obtained as a side product of the deprotection reaction between **Intermediate 98** and methylamine ethanol solution, via the route of **Example 25.** Purification with preparative liquid chromatography (Gilson 281 Preparative HPLC, 19 × 250 mm Waters XBridge C18 10 μm preparative column, mobile phase A: 10 mM ammonium bicarbonate aqueous solution, B: acetonitrile, flow rate 25 ml/min, dual-wavelength UV absorption monitoring at 214 and 254 nm, gradient elution: 0-2 min, 5%B; 2-15.6 min, 5-15%B; 15.6-15.8 min, 15-95%B; 15.8-18 min, 95%B; the compound's retention time: 14.1 min) and lyophilization gave a white solid.

$^1$H NMR (400 MHz, DMSO-*d6*) δ 10.69 (brs, 1H), 8.27 (s, 1H), 8.02 (s, 1H), 7.34-6.97 (m, 3H), 6.31 (brs, 1H), 6.28 (d, *J* = 2.4 Hz, 1H), 5.90 (d, *J* = 0.8 Hz, 1H), 5.44-5.15 (m, 3H), 4.44 (d, *J* = 0.4 Hz, 1H), 4.35-4.27 (m, 1H), 4.24-4.12 (m, 2H), 4.03-3.94 (m, 1H), 3.93-3.79 (m, 2H), 2.85 (s, 3H). $^{31}$P NMR (162 MHz, DMSO-$d_6$) δ 54.02, 51.06. MS+ESI--[M+H]: 738.0.

**Activity Examples**

**Example 1: Activation effect of the compounds of the present invention on IFN-β secretion by THP-1 cells**

THP-1 cell, a human monocytic leukemia cell line, has the STING phenotype of HAQ type, i.e., R71H-G230A-R293Q. This assay used the Human IFN-beta DuoSet ELISA Kit from R & D (R & D, Cat # DY814-05) and the DuoSet ELISA Ancillary Reagent Kit 2 (R & D, Cat # DY008) to evaluate the activation of IFN-β secretion from THP-1 cells by representative compounds of the invention.

In thawing the THP-1 cells (ATCC # TIB-202), a cell freezing tube was quickly shaken in a water bath at 37°C to thaw the cells within 1 min. The thawed cell suspension was taken and evenly mixed with an RPMI1640 medium (Hyclone, product # SH30027.01) containing 10% FBS (Life Technology, Cat #10099-141), centrifuged for 5 min at 1000 rpm, and the supernatant was discarded. The cell pellet was resuspended in 5 mL of the complete medium (RPMI 1640 medium containing 10% FBS), placed into a cell culture flask with a bottom area of 25 cm$^2$, and incubated in a cell incubator at 37°C, 5% $CO_2$ and 95% humidity. When the cell confluence reached about 80%, the cells were passaged. All the cells in the culture flask were transferred to a 15 mL centrifuge tube, centrifuged for 5 min at 1000 rpm, and the supernatant was discarded. 5 mL of fresh complete medium was added to re-suspend the cell pellet, 1 mL of which was taken to place in a cell culture flask with a bottom area of 25 cm$^2$, supplemented with 4 mL of fresh complete medium for further incubation. When the cell confluence reached about 80% again, plating was conducted following the procedure of cell passage, retaining 1/5 cell suspension for furher inculation and remaining 4/5 of the cell suspension in a 15 mL centrifuge tube. The cell suspension was centrifuged to discard the old culture medium, washed once using RPMI1640 culture medium (serum-free), and centrifuged to remove the supernatant. The cells were then re-suspended in RPMI1640 culture medium (serum-free). Cell viability was then detected by Trypan blue exclusion test and to be used for plating when the cell viability was confirmed to be more than 95%. A cell suspension with a density of 1.1 × 10$^6$ viable cells/mL was prepared using RPMI1640 culture medium (serum-free), and 180μL of the cell suspension was added to a 96-well cell culture plate (NUNC Company, Cat #167008) so that the cell density in the plate was 2×10$^5$ viable cells/well.

A 10 mM stock solution of the compounds in DMSO was first serially diluted at 3.16-fold with DMSO (Sigma, Cat # D2650) to the 5$^{th}$ concentration, and the 6$^{th}$ concentration was set as DMSO control (compound-free). Then, the DMSO solutions containing different concentrations of the compounds were diluted at 10-fold with PBS so that the DMSO content in each compound solution of various concentrations was 10%. Finally, 20μL of each of such solutions was added into corresponding cell culture plate to provide an initial compound concentration of 100μM, with the dilution fold of adjacent concentrations being 3.16 and the DMSO content in the cell culture plate being 1%. The cell plate was placed in a cell incubator for further 24 hours.

ELISA detection was conducted referring to the instructions from R & D System, Cat # DY814-05.

ELISA plate coating: PBS (R & D System Cat # DY006) was used to dilute the capture antibody (mouse anti-human IFN-β capture antibody PART#844508) to provide the working concentration, and 100 μL capture antibody working solution was added to a 96-well ELISA plate which was then sealed and incubated overnight at room temperature.

Upon discarding the capture antibody working solution, the cell plate was washed 3 times with a washing buffer (0.05% Tween-20 in PBS, pH 7.2-7.4, R & D System Cat #WA126) at 400 μL of the washing buffer per well, and the washing buffer was thoroughly removed after each washing, with the final washing to be completely removed of the wash buffer by inverting and claping the plate to a clean piece of paper. 300 μL of blocking buffer (1% BSA in PBS pH 7.2-7.4, R & D System Cat #DY995) was added to each well, incubated at room temperature for 1-2 hours. The above washing step was repeated to prepare each plate for sample addition.

Sample detection: Each cell was added with 100μL of the sample or standard (Recombinant human IFN-beta standard, PART #844510), sealed and incubated at room temperature for 2 hours, and the washing step of the above plate coating procedure was repeated. Then, 100 μL of detection antibody (Biotinylated mouse anti-human IFN-β detection antibody, PART #844509) was added to each well, the plates were sealed and left to incubate at room temperature for 2 hours, and the washing step of the above plate coating procedure was repeated. Subsequently, 100μL of Streptavidin-HRP (PART #893975) working solution was added to each well, the plates were sealed and left to incubate at room temperature for 20 min, the procedure was protected from light, and the washing step of the above plate coating procedure was repeated. Then, 100μL of a substrate solution (a 1:1 mixture of Color Reagent A ($H_2O_2$) and Color Reagent B (tetramethylbenzidine), R & D System Cat # DY999) was added to each well, the plates were sealed and left to incubate at room temperature for 20 min, and the process was protected from light. Finally, 50μL of a stop solution (2N $H_2SO_4$, R & D System Cat # DY994) was added to each well and the cell plate was tapped to ensure thorough mixing.

The OD450 of each well was measured using a multifunctional microplate reader (Molecular Devices, Spectramax M3), setting at 540 nm or 570 nm if a wavelength calibration function is available, and subtracting OD540 or OD570 from OD450 if the wavelength calibration function is unavailable. This was completed within 30 min after the addition of the stop solution.

2',3'-cGAMP (Invivogen, Cat # tlrl-nacga23) was used as a positive control compound and ADU-S100 (MCE, Cat # HY12885A) was used as a reference compound:

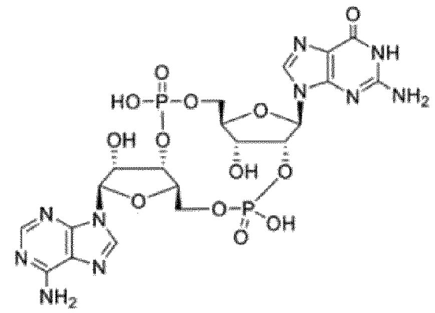

2',3'-cGAMP

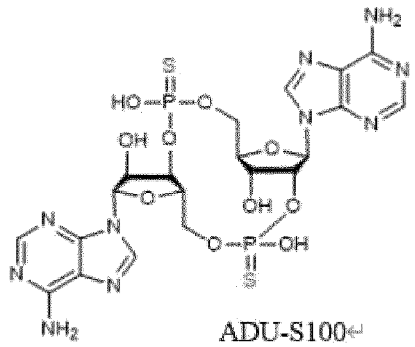

ADU-S100

Data were analyzed using GraphPad Prism 7.0 software and a standard curve for ELISA IFN-β content was developed using log-log plots. The OD value tested from each sample well was fitted into the standard curve equation to provide the corresponding IFN-beta concentration. The data were fitted by nonlinear sigmoidal regression to obtain a dose-response curve of IFN-β concentration versus compound concentration, and EC50 values were calculated.

Table 1-Activation effect of representative example compounds on IFN-β secretion by THP-1 cells (EC50, μM)

| Example | THP-1 Activity | Example | THP-1 Activity |
|---|---|---|---|
| 5 | B | 16 | A |
| 8 | B | 17 | A |
| 9 | B | 18 | B |
| 10 | A | 19 | B |
| 11 | B | 21 | B |
| 12 | A | 2',3'-cGAMP | B |
| 13 | B | ADU-S100 | A |
| 15 | B | 26 | A |

wherein: A: EC50 1~10 μM; B: EC50 10.1-100 μM

Experimental results show that, the example compounds have an activating effect on the secretion of IFN-beta by THP-1 cells. As can be seen from Table 1 and Figure 1, the compounds of Examples 10, 12, 16, 17 and 26 have, inter alia, significant STING agonist activity: their EC50s are greater than 2',3'-cGAMP, and comparable to ADU-S100; among them, the compounds of Examples 10, 16 and 17 stimulated THP-1 cells to secrete IFN-β, the highest concentrations of which are higher than that of ADU-S100.

**Example 2: Inhibitory activity of the compounds of the present invention on proliferation of CT26 cells**

CT26 cell is a mouse colorectal cancer cell line. This assay used the CellTiter-Glo Luminescence Cell Vitality Assay kit from Promega to evaluate the inhibitory activity of the compounds on the proliferation of CT26 cells.

In thawing the CT26 cells (ATCC # CRL-2638), a cell freezing tube was quickly shaken in a water bath at 37°C to thaw the cells within 1 min. The thawed cell suspension was taken and evenly mixed with DMEM medium (GE, Cat # SH30243.01) containing 10% FBS (GIBCO, Cat #10099-141), centrifuged for 5 min at 1000 rpm, and the supernatant was discarded. The cell pellet was resuspended in 5 mL of the complete medium (DMEM medium containing 10% FBS), placed into a cell culture flask with a bottom area of 25 $cm^2$, and incubated in a cell incubator at 37°C, 5% $CO_2$ and 95% humidity. When the cell confluence reached 70%~80%, the cells were passaged. All the cells in the culture flask were transferred to a 15 mL centrifuge tube, centrifuged for 5 min at 1000 rpm, and the supernatant was discarded. 5 mL of fresh complete medium was added to re-suspend the cell pellet, 1 mL of which was taken to place in a cell culture flask with a bottom area of 25 $cm^2$, supplemented with 4 mL of fresh complete medium for further incubation. When the cell confluence reached 70%~ 80% again, plating was conducted following the procedure of cell passage, retaining 1/5 cell suspension for furher inculation and remaining 4/5 of the cell suspension in a 15 mL centrifuge tube. The cell suspension was centrifuged to discard the old culture medium, washed once using DMEM medium (serum-free), and centrifuged to remove the supernatant. The cells were then re-suspended in DMEM medium (serum-free). Cell viability was then detected by Trypan blue exclusion test and to be used for plating when the cell viability was confimed to be more than 95%. A cell suspension with a density of $1.1\times10^4$ viable cells/mL was prepared using DMEM medium (serum-free), and 90μL of the cell suspension was added to a 96-well 96-well transparent flat-bottomed black-walled cell culture plate (Corning, Cat #3603) so that the cell density in the plate was 1000 viable cells/well. A control group containing no cells, no compound, and complete medium only (i.e., a culture medium control), and a control group containing no compound but cells (i.e., a cell control) were set. The cell plates were placed in a cell incubator overnight.

A 10 mM stock solution of the compounds in DMSO was first serially diluted at 3.16-fold with DMSO (Sigma, Cat # D2650) to the 9$^{th}$ concentration, and the 10$^{th}$ concentration was set as DMSO control (compound-free). Then, the DMSO solutions containing different concentrations of the compounds were diluted at 10-fold with PBS (Solarbio, Cat # P1020) so that the DMSO content in each compound solution of various concentrations was 10%. Finally, 10μL of each of such solutions was added into corresponding cell culture plate to provide an initial compound concentration of 100μM, with the dilution fold of adjacent concentrations being 3.16 and the DMSO content in the cell culture plate being 1%. The cell plate was placed in a cell incubator for further 120 hours.

After 120 hours, the CellTiter-Glo reagent (Promega, Cat # G7572) was thawed and the plates were equilibrated at room temperature for 30 min. 100 uL of CellTiter-Glo was added to each well of the plate, and the cells were lysed thoroughly by shaking on an orbital shaker for 5 min. The plates were placed at room temperature for 20 min to stabilize the luminescence signal, and the luminescence value of each well was scanned at full wavelength with a multifunctional microplate reader (Molecular Devices, Spectramax M3).

The following compounds with ADU-S100 (MCE, Cat # HY 12885A) were used as controls: Clofarabine (Clofarabine; Wuhu Huaren Science, Cat # HR-00701002), Cladribine (Cladribine; CSNpharm, Cat # CSN10004), Gemcitabine Hydrochloride (Gemcitabine Hydrochlorilide; ShaoYuan, Cat # SY014538), Gemcitabine prodrug LY2334737 (homemade, **Intermediate 7**).

Cell viability was calculated for each compound concentration using the following equation:

$$\text{Cell viability (\%)} = (\text{Lum}_{\text{Test drug}} - \text{Lum}_{\text{Culture medium control}})/(\text{Lum}_{\text{Cell control}} - \text{Lum}_{\text{Culture medium control}}) \times 100\%$$

Data were analyzed using GraphPad Prism 7.0 software, fitted with nonlinear S-curve regression to derive dose-response curves, and IC50 values were calculated.

Table 2-Inhibitory activity of Examples on proliferation of CT26 cells (EC50, μM)

| Example | CT26, IC50 (μM) | Example | CT26, IC50 (μM) |
|---|---|---|---|
| 1 | B | 13 | A |

(continued)

| Example | CT26, IC50 (μM) | Example | CT26, IC50 (μM) |
|---|---|---|---|
| 2 | B | 16 | B |
| 3 | A | 17 | B |
| 5 | B | Clofarabine_ | A |
| 7 | B | Cladribine | A |
| 10 | A | Gemcitabine | A |
| 12 | B | LY2334737 | B |
| | | ADU-S100 | NA |

wherein: A: IC50 <1 μM; B: IC50 1~10 μM; NA: IC50 > 10 μM

The experimental results show that, the representative Example compounds can inhibit the in vitro growth of CT26 tumor cells; among them, the compounds of Examples 3, 10 and 13 had particularly significant inhibitory potency, whereas ADU-S 100 had no significant tumor cell inhibitory potency. In combination with the results of Activity Example 1, the results indicate that, these compounds have multifunctional antitumor properties, i.e., tumor immunological activity of STING agonists and cytotoxic effects of antimetabolic anticancer drugs.

**Example 3: Anti-tumor activity of the compounds of the present invention in a bilateral transplantation tumor model of CT26 syngeneic mouse**

BALB/c mice of 6-8 weeks (purchased from Shanghai Ling Chang Biotech Co., Ltd.) were inoculated with $5\times10^5$ CT26 cells (supplied by Taicang Zexin Biotechnology Co., Ltd., ATCC # CRL-2638) subcutaneously on the left and right back dorsal, respectively, and the inoculation volume was 0.1 mL/side. When tumors grew to an average volume of 100 $mm^3$, they were randomized according to tumor size and mouse body weight and dosing was initiated. The day of the first dose was Day 0. Two doses were administered on Day 0 and Day 4, with 50μg of compound/mouse (i.e., 2.5mg/kg) injected intratumorally right side, with the same volume of PBS (Hyclone, Cat # SH30258.01) as a control. The tumor on the left side was not treated. The control mice were sacrificed 13 days and the treated mice were sacrificed 21 days, after dosing. During the experiment, tumor volumes on the left and right sides and the body weights were measured 3 times per week, and the tumor volume was calculated as $V = D\times d\times d/2$ (where D represents length of tumor and d represents width of tumor).

Data at Day 13 post-dosing indicate that, all Example compounds tested were able to significantly inhibit bilateral tumor growth. The right-side dosing group showed more significant effect, with regression for most of the tumors (Fig. 2-A). Among them, ADU-S100 treated group showed 99.1% inhibition of tumor growth, Example 17 showed 99.5%, and the remaining groups showed 100% inhibition of tumor growth without palpable tumors. At the same time, the untreated left side tumors showed slow growth (Fig. 2-B). On Day 13 post-dose, the growth inhibition rates for the left tumor groups were: ADU-S100, 64.2%; Example 10, 91.2%; Example 12, 75.3%; Example 16, 71.7%; Example 17, 67.4%. In addition, it was observed in this experiment that the post-dose body weight of individual mice decreased within 15%, recovered after discontinuation of administration (Fig. 2-C). The above results show that the compounds of the invention showed equivalent or superior tumor inhibition activity to ADU-S100 in the CT26 syngenic bilateral tumor model.

**Example 4: Anti-tumor activity of the compounds of the present invention in a transplantation tumor model of CT26 nude mouse**

BALB/c nude mice of 6-8 weeks (purchased from Shanghai Ling Chang Biotech Co., Ltd.) were inoculated with $5\times10^5$ CT26 cells (supplied by Taicang Zexin Biotechnology Co., Ltd., ATCC # CRL-2638) subcutaneously on the right back dorsal, respectively, and the inoculation volume was 0.1 mL. When tumors grew to an average volume of 100 $mm^3$, they were randomized according to tumor size and mouse body weight and dosed. The day of the first dose was Day 0. Two doses were administered on Day 0 and Day 4, with 50μg of compound/mouse (i.e., 2.5mg/kg) injected intratumorally right side, with the same volume of PBS (Hyclone, Cat # SH30258.01) as a control. The control mice were sacrificed 9 days, the ADU-S 100 treated mice were sacrificed 14 days, and Example 10 treated mice were sacrificed 16 days, after dosing, respectively. During the experiment, tumor volumes and the body weights were measured 3 times per week, and the tumor volume was calculated as $V = D\times d\times d/2$ (Ibid).

Data at Day 9 post-dosing indicate that, the compound of Example 10 as tested was able to significantly inhibit

tumor growth in T cell immunodeficient nude mice at a rate of 94.4%. In contrast, ADU-S 100 showed only partial tumor suppression with an inhibition rate of 61.4%, possibly due to its residual immune activity or other unknown reasons. Regardless of Example 10 or ADU-S 100, they showed better tumor growth inhibition in immuno-competent mice than in nude mice (Fig. 2D). This experiment verifies that, ADU-S 100 acts primarily by activating T cell-mediated immunity; while the compound of Example 10 inhibits the tumor via its multi-functional mechanism, which has been demonstrated in this case that it shows superior anti-tumor ability to that of the STING agonist ADU-S100.

**Example 5: Immunological memory function of the compounds of the present invention in transplantation tumor model in CT26 syngeneic or nude mouse**

BALB/c immune-competent or nude mice of 6-8 weeks (purchased from Shanghai Ling Chang Biotech Co., Ltd.) were inoculated with $5\times10^5$ CT26 cells (supplied by Taicang Zexin Biotechnology Co., Ltd., ATCC # CRL-2638) subcutaneously on the right back dorsal, respectively, and the inoculation volume was 0.1 mL. When tumors grew to an average volume of 100 $mm^3$, they were randomized according to tumor size and mouse body weight and dosed. The day of the first dose was Day 0. Two doses were administered on Day 0 and Day 4, with 50μg of compound/mouse (i.e., 2.5mg/kg) injected intratumorally, with the same volume of PBS (Hyclone, Cat # SH30258.01) as a control. Mice in the nude or BALB/c mice groups were sacrificed on Day 9 or Day 13 post-dosing, respectively. On Day 21 post-dosing, mice were inoculated again subcutaneously on the left back dorsal with $5\times10^5$ CT26 cells in a volume of 0.1mL. Blank BALB/c or nude mice without any treatment were also inoculated as controls. During the experiment, tumor volumes and the body weights were measured 3 times per week, and the tumor volume was calculated as $V = D\times d\times d/2$ (Ibid).

The results show that, the compound of Example 10 as tested significantly inhibited CT26 tumor growth in both immunocompetent and immunodeficient mice. The effect on the immunocompetent mice was more prominent (Fig 3-A). On Day 5 post-dosing, the tumors completely disappeared in the mice treated with Example 10. On Day 21, CT26 cells were re-inoculated in Compound 10-treated groups, respectively. After 7 days (Day 30), the average tumor volume of the uninmmunized blank BALB/c mice was 75 $mm^3$, while the average tumor volume of the mice previously treated with Example 10 was 29 $mm^3$. By the end of the experiment on Day 33, the average tumor volumes of mice in the control group and in the Example 10-immunized group were 648 $mm^3$ and 101 $mm^3$, respectively. This experiment demonstrates that, following treatment with the compound of Example 10, immunocompetent mice developed an immunological memory and produced strong immunological rejection to the re-inoculated allogeneic cells, thereby effectively preventing tumor recurrence.

On the other hand, in immunodeficient nude mice, continued growth of tumors occurred in individual mice after the administration was stopped (Fig. 3-B). Finally, 3 mice with smaller tumor volumes were selected to be re-inoculated with CT26 cells on Day 21. The results show that, the re-inoculated CT26 tumor cells exhibited similar growth rate to that in the blank mouse. The results indicate that, no immunological memory has been generated in the nude mouse experiment, and further verify that the compound of Example 10 has a cytotoxic tumor-inhibiting ability even without the involvement of immune system in this model.

**Example 6: Hepatocyte metabolic stability assay for the compounds of the invention**

The compounds of the invention were similarly tested for hepatocyte metabolic stability in 5 species (mouse, rat, dog, monkey, human) according to standard methods of in vitro metabolic stability studies conventional in the art, for example (Kerns, Edward H. and Di Li (2008). Drug-like Properties: Concepts, Structure Design and Methods: From ADME to Toxicity Optimization, San Diego: Academic Press; Di, Li et al, Optimization of a High Throughput Microsomal Stability Screening Assay for Profiling Drug Discovery Candidates, J Biomol Screen. 2003, 8(4), 453).

The hepatocytes used in the experiment: human hepatocytes (SHQY, Lot # HEP190006), dog hepatocytes (IVT, Lot # ZMB); monkey hepatocytes (Xenotech, Lot #2010022), rat hepatocytes (SHQY, Lot # HEP134045), and mouse hepatocytes (BioIVT, Cat. # M005052, Lot. # MEO).

Cryopreserved hepatocyte tubes were removed from a liquid nitrogen tank and immediately placed in a shaking water bath at 37 ± 1°C for 2 minutes ± 15 seconds. The hepatocytes were transferred to 50 mL hepatocyte thawing medium (composition: Williams E medium, 35 mL, Invitrogen, Cat # A1217601; isotonic Percoll solution, 13.5 mL, GE, Cat # 17-0891-01; Dulbecco's phosphate buffer, 1.5 mL, Invitrogen, Cat # 14200-075; Glutamax, 500 μL, Invitrogen, Cat # 35050061; HEPES, 750 μL, Invitrogen, Cat # 15630106; fetal bovine serum, 2.5 mL, Invitrogen, Cat # 10091130; recombinant human insulin, 50 μL, Invitrogen, Cat # 12585014; dexamethasone (formulated as 10mM DMSO solution), 5 μL, Sigma, Cat # D1756), mixed gently and centrifugated at 500 rpm for 3 min. After centrifugation, the supernatant was carefully aspirated (without disturbing the cell pellet), 10× volumes of pre-warmed KHB buffer (Krebs-Henseleit buffer, Sigma, Cat # K3753-10X1L) and 5.6 g/L HEPES were added, the cell pellet was resuspended, mixed gently, and centrifuged at 500 rpm for 3 min. The supernatant was aspirated without touching the cell pellet, and cell viability and number were determined. The hepatocytes were counted and then the cell suspension was diluted in KHB buffer to the

appropriate density (viable cell density=2$\times 10^6$ cells/mL). The hepatocyte solution was placed on ice until use.

A 2$\times$ medicated solution was prepared in pre-warmed KHB (1% dimethyl sulfoxide) and centrifuged at 5594 g for 15 min (Thermo Multifuge$\times$3R), with 200 $\mu$M spiking solution: adding 20 $\mu$L of compound stock solution (10 mM in DMSO) to 980 $\mu$L of dimethyl sulfoxide; 2$\times$ medicated solution: adding 10 $\mu$L of 200 $\mu$M spiking solution to 990 $\mu$L KHB (2 $\mu$M after dilution).

To wells designated for different time points was added 50 $\mu$L of pre-warmed 2$\times$ medicated solution. 50 $\mu$L of pre-warmed hepatocyte solution (2$\times 10^6$ cells/mL) was added to designated wells for the detection at 15 min, 30 min, 60 min and 120 min, and then the timing was started, and the reaction plates were placed in a 37°C incubator.

100 $\mu$L of IS (Osalmide or Imipramine) in acetonitrile (Merck, Cat. # CN34854-4L) was added to a well designed for 0 min, mixed gently, then 50 $\mu$L of pre-warmed hepatocyte solution (2$\times 10^6$ cells/mL) was added and the plates were blocked. At 15 min, 30 min, 60 min and 120 min, 100 $\mu$L of IS-containing acetonitrile was added to the wells, respectively, and then blocked. After quenching, the plates were shaken on a shaker (IKA, MTS 2/4) for 10 min (600 rpm). The plates were sonicated for 2 min and then centrifuged at 5594 g for 15 min (Thermo Multifuge$\times$3R). 50 $\mu$L of supernatant per well was transferred to a 96 well sample plate containing 50 $\mu$L of ultrapure water (Millicore, ZMQS 50F01) for LC/MS analysis.

The concentration of the test compound at the time point of T0 was taken as 100% (C0), and the concentrations of other incubation time points were converted into residual percentages, the natural logarithm of which was then treated against the incubation time by linear regression to obtain a slope K. The hepatocyte clearance rate (Clint) and the in vitro half-life (T1/2) were then calculated according to the following equations:

$$T1/2 = 1n2/K = 0.693/K$$

$$Cl_{int} = (0.693/T1/2) \times (1/\text{Hepatocyte density}) \times \text{Scaling factor}$$

wherein, the hepatocyte density is the final concentration of hepatocyte in the incubation system of the experiment: 1$\times 10^6$/mL. Scaling factor = hepatocyte number $\times$ liver weight (for 5 species of hepatocytes, 11812.5$\times 10^6$/kg mouse, 4680$\times 10^6$/kg rat, 6880$\times 10^6$/kg dog, 3900$\times 10^6$/kg monkey, 2544.3$\times 10^6$/kg human, respectively).

As can be seen from Fig. 4A, the compounds of the present invention, such as the compound of Example 10, showed good metabolic stability in 5 species of hepatocytes, showing a long metabolic half-life and a low clearance rate.

**Example 7: Discovery and Identification of metabolites of the compounds of the invention**

The main metabolites of the compounds of the invention in 5 species (mouse, rat, dog, monkey, human) of hepatocytes were similarly identified according to standard methods of in vitro metabolic stability studies conventional in the art, for example (Kerns, Edward H. and Di Li (2008). Drug-like Properties: Concepts, Structure Design and Methods: From ADME to Toxicity Optimization, San Diego: Academic Press; Di, Li et al, Optimization of a High Throughput Microsomal Stability Screening Assay for Profiling Drug Discovery Candidates, J Biomol Screen. 2003, 8(4), 453).

The hepatocytes used in the experiment: human hepatocytes (SHQY, Cat. # BQHPCH10, Lot. # HEP190006-TA05), dog hepatocytes (BioIVT, Cat. # M00205, Lot. # ZMB); monkey hepatocytes (XENOTECH, Cat. # PPCH2000, Lot. # 2010022), rat hepatocytes (SHQY, Cat. # BQR1000 • H15, Lot. # HEP134049), and mouse hepatocytes (BioIVT, Cat. # M005052, Lot. # MEO).

A HI hepatocyte maintenance medium (BIOIVT, Cat. # Z99009; Lot. # C02060C) was pre-warmed to 37°C. Cryopreserved hepatocyte tubes were removed from a liquid nitrogen tank and immediately placed in a shaking water bath at 37 $\pm$ 1°C for 2 minutes $\pm$ 15 seconds. The hepatocytes were transferred to 50 mL HI medium, mixed gently and centrifugated at 500 rpm for 3 min. After centrifugation, the supernatant was carefully aspirated (without disturbing the cell pellet), 10$\times$ volumes of pre-warmed HI medium were added, and the cell pellet was resuspended, mixed gently, and centrifuged at 500 rpm for 3 min. The supernatant was aspirated and discarded without touching the cell pellet. The hepatocytes were counted and then the cell suspension was diluted in HI medium to the appropriate density (viable cell density=2$\times 10^6$ cells/mL). The hepatocyte solution was placed on ice until use.

A2$\times$ medicated solution (20 $\mu$M) was prepared in pre-warmed HI medium, by adding 8 $\mu$L of 10 mM compound stock solution to 3992 $\mu$L of HI medium (20 $\mu$M, 0.2% dimethyl sulfoxide after dilution).

The hepatocyte solution and the 2$\times$ medicated solution were pre-warmed, and 200 $\mu$L of the pre-warmed 2$\times$ medicated solution was added to wells designated for $T_{240}$ and $T_{240\text{-w/o}}$. For T0, 1200 $\mu$L acetonitrile (Merck, Cat. # CN34854-4L) and 200 $\mu$L hepatocyte solution (2$\times$ $10^6$ cells/mL) were added to the wells, followed by 200 $\mu$L pre-warmed 2$\times$ medicated solution, and the plates were blocked. 200 $\mu$L of pre-warmed hepatocyte solution (2$\times$ $10^6$ cells/mL) was added to wells designated for $T_{240}$; 200 $\mu$L of pre-warmed HI medium was added to wells designated for $T_{240\text{-w/o}}$, and

the timing was started. The reaction plates were placed in a 37°C $CO_2$ incubator.

At 240 min, 1200 μL of acetonitrile was added to each designated well, and the plates were then blocked. After quenching, the plates were sonicated for 2 min and then centrifuged at 1400 rpm for 5 min. 1200 μL of the supernatant was evaporated under a nitrogen flow until dry. The dried extract was then re-solubilized with 200 μL acetonitrile: water (1:3 v/v), vortexed for 2 min, and centrifuged at 14000 rpm for 5 min. 2/5 μL of the supernatant was injected into LC-UV-MS for analysis.

As can be seen from Fig. 4-B, the cytotoxic small molecule Clofarabine (Clofarabine) produced by the decomposition the compounds of the present invention such as the compound of Example 10 can be detected in 5 species of hepatocytes, and this is the main metabolite of the compound of Example 10. In combination with the results of Activity Example 6, it is highly likely that both the CDN molecule with STING agonistic activity and the small cytotoxic molecule were present over a period of time in vivo, thereby achieving a combination at the molecular level, providing an enhanced, even synergistic anti-tumor effect. The results also explain why it worked so well in the mouse tumor suppression experiments.

**Example 8: Effect comparison of anti-tumor activities between the compounds of the invention and the simple combination of a CDN STING agonist and a cytotoxic drug in a bilateral transplantation tumor model of CT26 syngeneic mouse**

BALB/c mice of 6-8 weeks (purchased from Shanghai Ling Chang Biotech Co., Ltd.) were inoculated with $5\times10^5$ CT26 cells (supplied by Taicang Zexin Biotechnology Co., Ltd., ATCC # CRL-2638) subcutaneously on the left and right back dorsal, respectively, and the inoculation volume was 0.1 mL/side. When tumors grew to an average volume of 100 $mm^3$, they were randomized according to tumor size and mouse body weight (Inventive compound group; single CDN STING agonist group; single cytotoxic drug group; simple combination of CDN STING agonist and cytotoxic drug group) and dosing was initiated. The day of the first dose was Day 0. Three doses were administered on Day 0, Day 4 and Day 7, injected intratumorally right side, with the same volume of PBS (Hyclone, Cat # SH30258.01) as a control. The tumor on the left side was not treated. The control mice were sacrificed 13 days and the treated mice were sacrificed 21 days, after dosing. During the experiment, tumor volumes on the left and right sides and the body weights were measured 3 times per week, and the tumor volume was calculated as $V = D\times d\times d/2$ (Ibid).

The experimental results show that, the tumor growth inhibition rate of the inventive compound group was obviously higher than that of the single CDN STING group and the single cytotoxic drug group, the activity of the inventive compound group was ecomparable to, or higher than the additive activity of the latter two groups, producing improved and even synergistic tumor inhibition activity.

General or preferred definitions of specified features in the various enumerated embodiments of the present invention may be combined with general or preferred definitions of other specified features to yield additional embodiments of the present invention. As if such combinations were specifically and individually set forth herein, unless the context clearly indicates otherwise.

In this specification, several prior publications are referenced. These publications, while not considered to be relevant to the patentability of the invention, are incorporated herein by reference in their entirety. The reference in this specification to any prior publication (or information derived from it), is not, and should not be taken as, an acknowledgment or admission or any form of suggestion that the corresponding prior publication (or information derived from it) forms part of the common general knowledge in the field of technology to which this specification relates.

## Claims

1. A cyclic dinucleotide compound of formula (II),

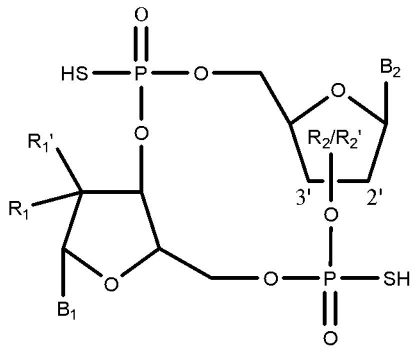

(II)

wherein:

$B_1$ is adenine

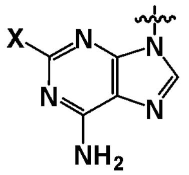

substituted by X, wherein X is selected from Cl, F or $-NHC_{1-6}$ alkyl; or cytosine

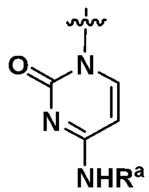

optionally substituted by $R^a$, wherein $R^a$ is selected from H or $-C(O)-C_{1-14}$ alkyl;
$R_1$ and $R_1$' are each independently selected from H, F or -OH;
$B_2$ is selected from adenine

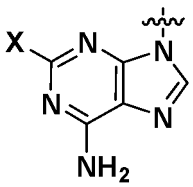

optionally substituted by X, wherein X is selected from H, F or Cl; cytosine

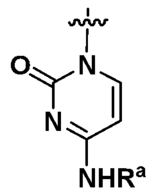

optionally substituted by $R^a$, wherein $R^a$ is selected from H or $-C(O)-C_{1-14}$ alkyl; or guanine

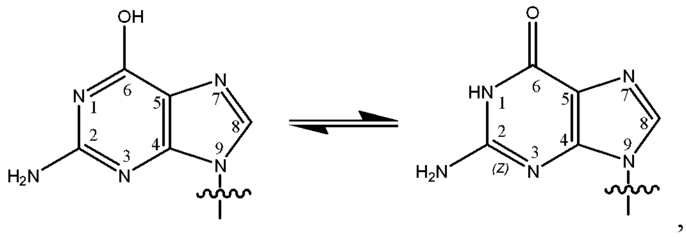

wherein OH is optionally substituted by $C_{1-6}$ alkyl;

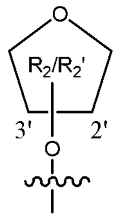

denotes that the phosphate linkage is connected to the 2' position or the 3' position of the pentose, and the position not cyclized with the phosphate is substituted by $R^2$ and $R_2$'; and
$R^2$ and $R_2$' are each independently selected from H, -OH or F;
with the proviso that when one of $B_1$ or $B_2$ is cytosine optionally substituted by $R^a$, the carbon atom adjacent to it on the pentose ring to which it is attached is substituted by two F;
or a stereoisomer, a tautomer, a stable isotopic variant, a pharmaceutically acceptable salt, a prodrug or a solvate thereof.

2. The compound of formula (II) according to claim 1, a stereoisomer, a tautomer, a stable isotopic variant, a pharmaceutically acceptable salt, a prodrug or a solvate thereof, wherein the

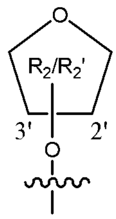

denotes that the phosphate linkage is connected to the 3' position of the pentose, and formula (II) has the following formula,

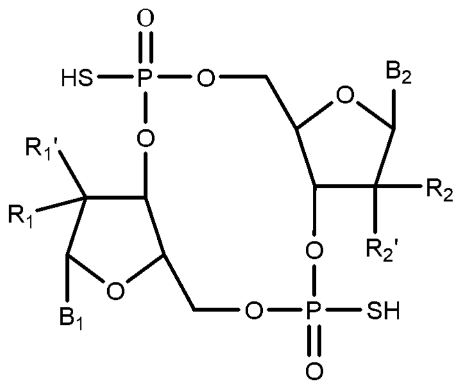

(II-a).

3. The compound according to claim 2, wherein $B_1$ is

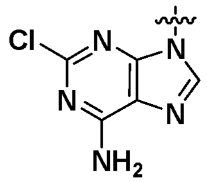

,

$R_1$ and $R_1$' are both H, or one of $R_1$ and $R_1$' is H and the other is F.

**4.** The compound according to claim 2, wherein $B_1$ is adenine

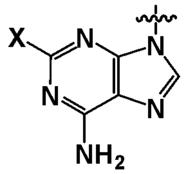

substituted by X, wherein X is selected from $-NHC_{1-6}$ alkyl, preferably $-NHCH_3$, $R_1$ and $R_1$' are both H, or one of $R_1$ and $R_1$' is H and the other is F.

**5.** The compound according to claim 2, wherein $B_1$ is cytosine

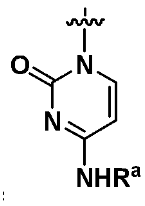

:

optionally substituted by $R^a$, wherein $R^a$ is selected from H or $-C(O)-C_{1-14}$ alkyl, $R_1$ and $R_1$' are both F.

**6.** The compound according to any one of claims 2-5, wherein $B_2$ is guanine

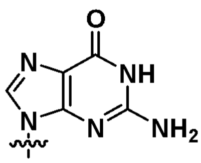

or adenine

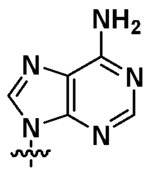

,

one of $R_2$ and $R_2$' is H, and the other is selected from -OH or F.

**7.** The compound according to any one of claims 2-5, wherein $B_2$ is adenine

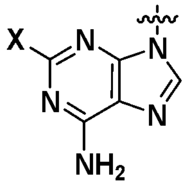

substituted by X, wherein X is Cl, one of $R_2$ and $R_2$' is H, the other is F, or $R_2$ and $R_2$' are both H.

**8.** The compound according to any one of claims 2-5, wherein $B_2$ is cytosine

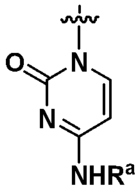

optionally substituted by $R^a$, wherein $R^a$ is selected from H or -C(O)-$C_{1-14}$ alkyl, $R_2$ and $R_2$' are both F.

**9.** The compound of formula (II) according to claim 1, a stereoisomer, a tautomer, a stable isotopic variant, a pharmaceutically acceptable salt, a prodrug or a solvate thereof, wherein the

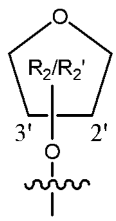

denotes that the phosphate linkage is connected to the 2' position of the pentose, and formula (II) has the following formula,

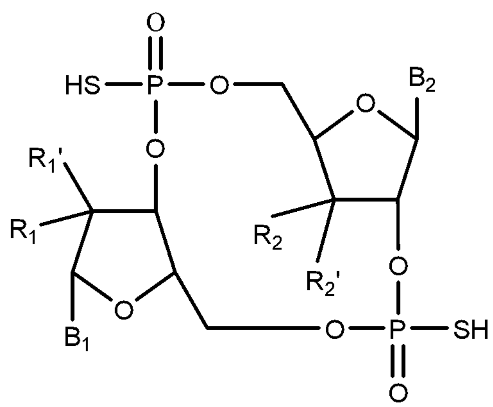

(II-b).

**10.** The compound according to claim 9, wherein $B_1$ is or one of $R_1$ and $R_1$' is H and the other is F.

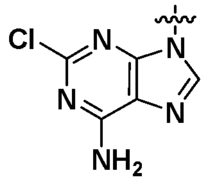

,

$R_1$ and $R_1$' are both H,

**11.** The compound according to claim 9, wherein $B_1$ is adenine

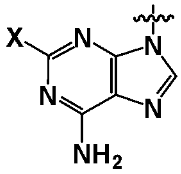

substituted by X, wherein X is $-NHC_{1-6}$ alkyl, preferably $-NHCH_3$, $R_1$ and $R_1$' are both H, or one of $R_1$ and $R_1$' is H and the other is F.

**12.** The compound according to claim 9, wherein $B_1$ is cytosine

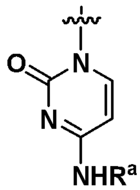

optionally substituted by $R^a$, wherein $R^a$ is selected from H or $-C(O)-C_{1-14}$ alkyl, $R_1$ and $R_1$' are both F.

**13.** The compound according to any one of claims 9-12, wherein $B_2$ is guanine

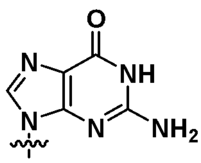

or adenine

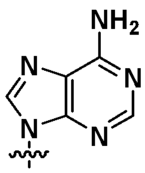

,

one of $R_2$ and $R_2$' is H, and the other is -OH.

**14.** The compound according to claim 1, 2 or 9 which is

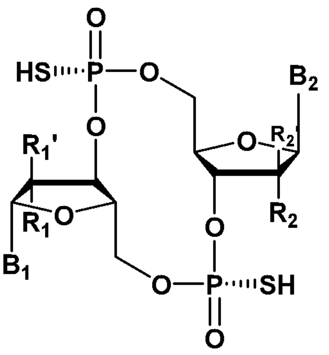

or

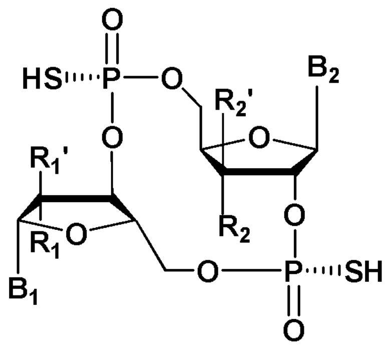

(II-a') (II-b'),

or a pharmaceutically acceptable salt or a solvate thereof.

**15.** The compound according to claim 14, wherein $B_1$ is

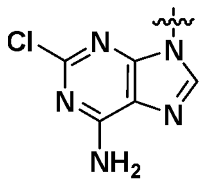

,

$R_1$ and $R_1$' are both H, or $R_1$ is F and $R_1$' is H.

**16.** The compound according to claim 14, wherein $B_1$ is adenine

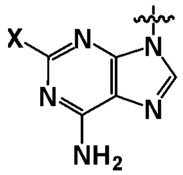

substituted by X, wherein X is $-NHC_{1-6}$ alkyl, preferably $-NHCH_3$, $R_1$ and $R_1$' are both H, or $R_1$ is F and $R_1$' is H.

**17.** The compound according to claim 14, wherein $B_1$ is cytosine

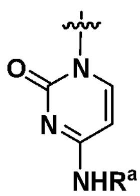

optionally substituted by $R^a$, wherein $R^a$ is selected from H or $-C(O)-C_{1-14}$ alkyl, $R_1$ and $R_1$' are both F.

**18.** The compound according to any one of claims 14-17, wherein in formula (II-a'), $B_2$ is guanine

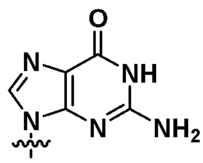

or adenine

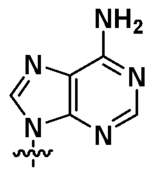

,

$R_2$' is H, and $R^2$ is selected from -OH or F.

**19.** The compound according to any one of claims 14-17, wherein in formula (II-a'), $B_2$ is adenine

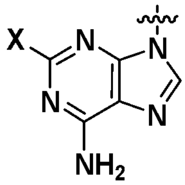

substituted by X, wherein X is Cl, $R_2$ is H, and $R_2$' is F, or $R_2$ and $R_2$' are both H.

**20.** The compound according to any one of claims 14-17, wherein in formula (II-b'), $B_2$ is guanine

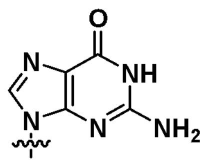

or adenine

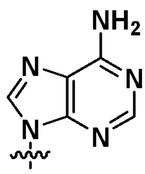

,

$R_2$' is H, $R_2$ is -OH.

**21.** A cyclic dinucleotide compound selected from the group consisting of

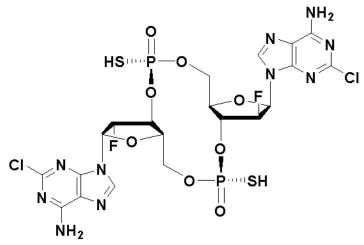

compound 1

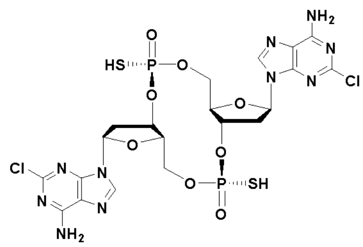

compound 2

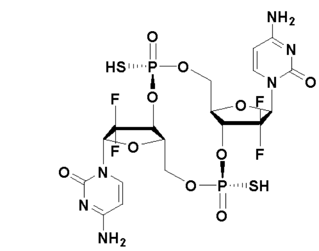

compound 3

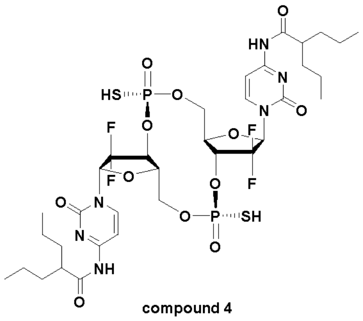

compound 4

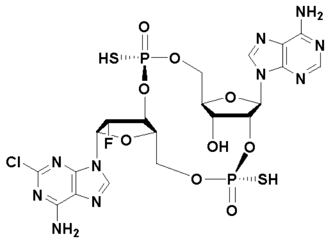

compound 5

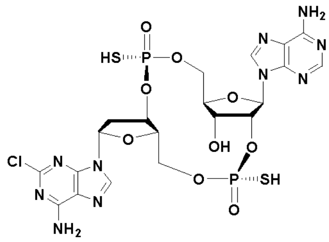

compound 6

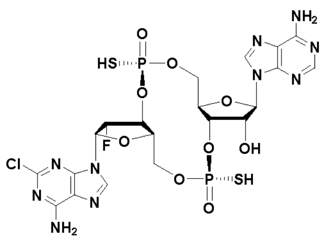

compound 7

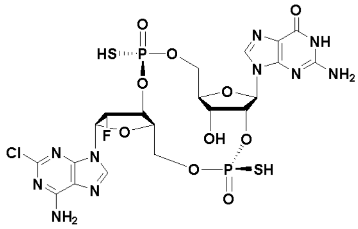

compound 8

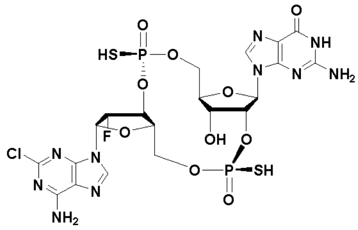

compound 9

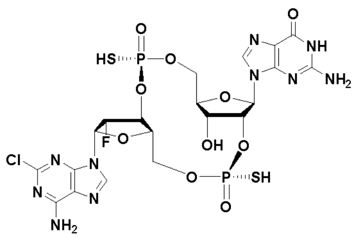

compound 10

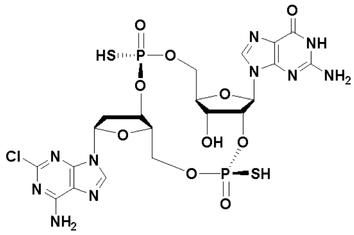

compound 11

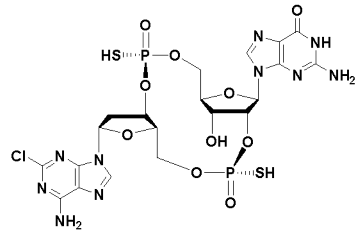

compound 12

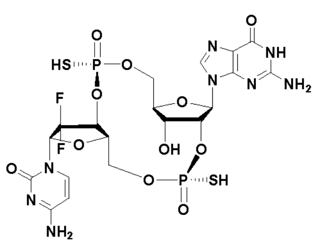

compound 13

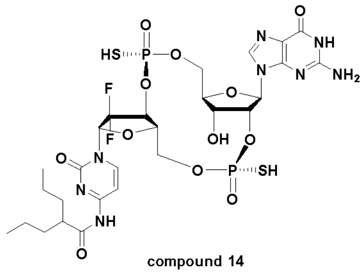

compound 14

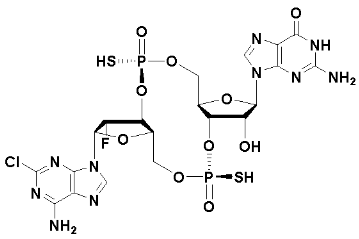

compound 15

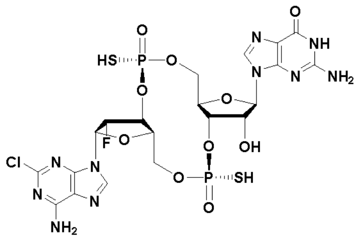

compound 16

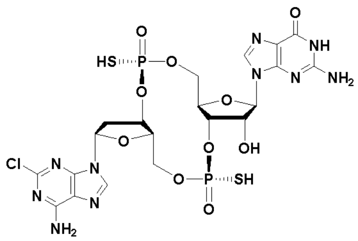

compound 17

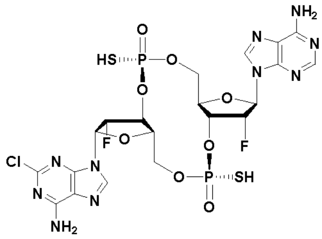

compound 18

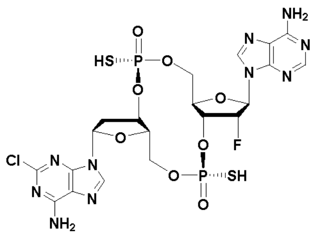

compound 19

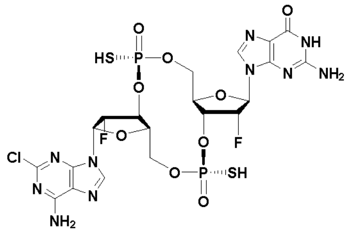

compound 20

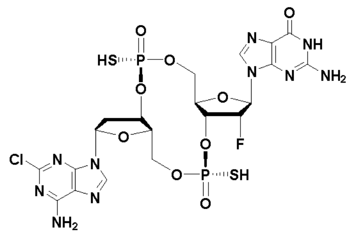

compound 21

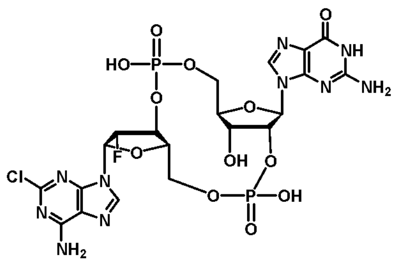

compound 22

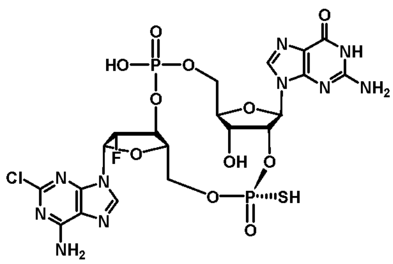

compound 23

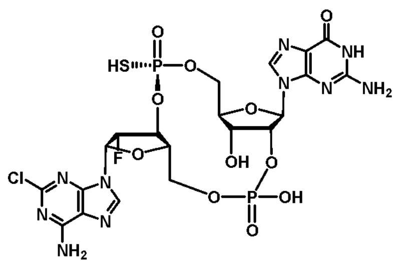

compound 24

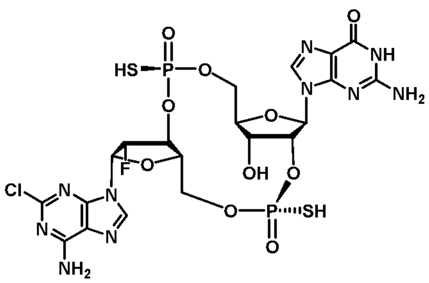

compound 25

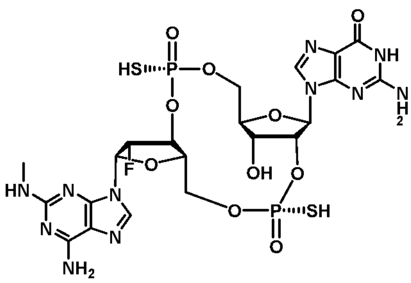

compound 26

or a pharmaceutically acceptable salt or solvate thereof.

**22.** A pharmaceutical composition comprising a therapeutically effective amount of a compound according to any one of claims 1-21 together with a pharmaceutically acceptable excipient.

**23.** The pharmaceutical composition according to claim 22 in the form of topical administration.

**24.** A method for the treatment or prevention of diseases associated with or mediated by STING, especially viral infections or tumors in a mammal, especially human, comprising administering an effective amount of a compounds according to any one of claims 1 to 21 or a pharmaceutical composition according to any one of claims 22-23.

**25.** Use of a compound according to any one of claims 1 to 21 or a pharmaceutical composition according to any one of claims 22-23 as a STING agonist for the treatment or prevention of diseases associated with or mediated by STING, especially viral infections or tumors.

**26.** Use of a compound according to any one of claims 1 to 21 or a pharmaceutical composition according to any one

of claims 22-23 as a cytotoxic agent for the treatment or prophylaxis of viral infections or tumors.

**27.** Use of a compound according to any one of claims 1 to 21 or a pharmaceutical composition according to any one of claims 22-23 as a multifunctional active agent for immunotherapy and cytotoxic therapy.

**28.** The use according to claim 27, wherein the multifunctional active agent is used to activate the immune system by activating the STING signaling pathway so as to exert the functions of anti-tumor and anti-viral replication, to induce tumor cell death or prevent viral replication by releasing cytotoxic agents, to kill tumor cells by persistently activation of STING through releasing tumor DNA, and to provide the ability of "immunological memory" or persistent immunity to tumors by releasing tumor neoantigens to generate an antibody-antigen response.

**29.** Use of a compound according to any one of claims 1 to 21 or a pharmaceutical composition according to any one of claims 22-23 in the manufacture of a medicament for the treatment or prevention of diseases associated with or mediated by STING, especially viral infections or tumors.

**30.** Use of a compound according to any one of claims 1 to 21 or a pharmaceutical composition according to any one of claims 22-23 in the manufacture of a cytotoxic agent for the treatment or prevention of viral infections or tumors.

**31.** The method or use according to any one of claims 24-26 and 28-30, wherein the tumor is selected from brain cancer, head and neck cancer, skin cancer, melanoma, bladder cancer, ovarian cancer, breast cancer, gastric cancer, pancreatic cancer, prostate cancer, colon cancer, blood cancer, lung cancer, small cell lung cancer, non-small cell lung cancer, bone cancer, colorectal cancer, liver cancer, renal cell cancer, pancreatic cancer, Hodgkin lymphoma, or leukemia.

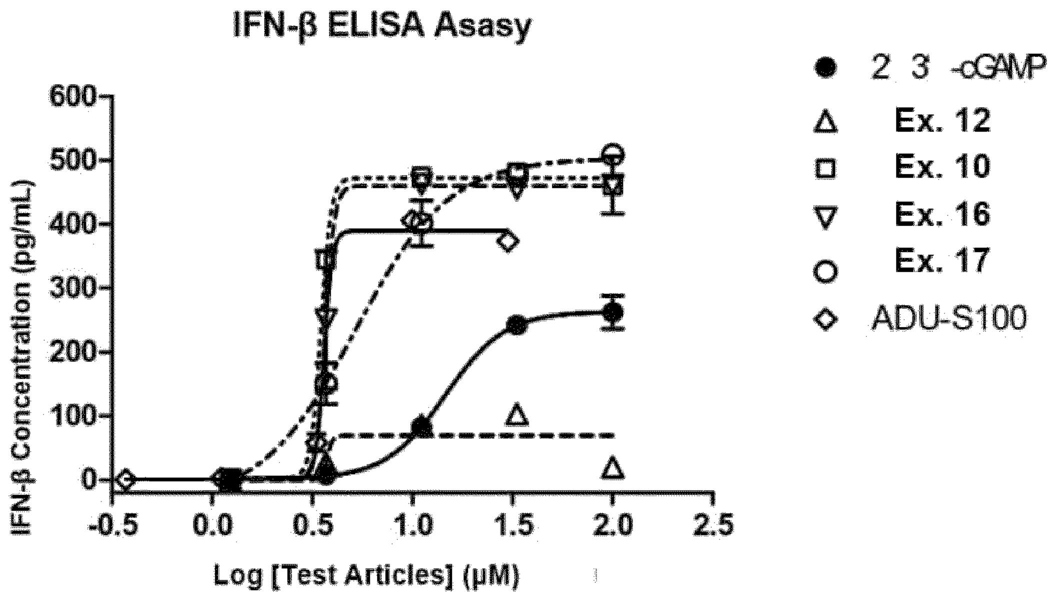
IFN-β ELISA Asasy
IFN-β Concentration (pg/mL)
Log [Test Articles] (μM)
2' 3' -cGAMP
Ex. 12
Ex. 10
Ex. 16
Ex. 17
ADU-S100

Fig. 1

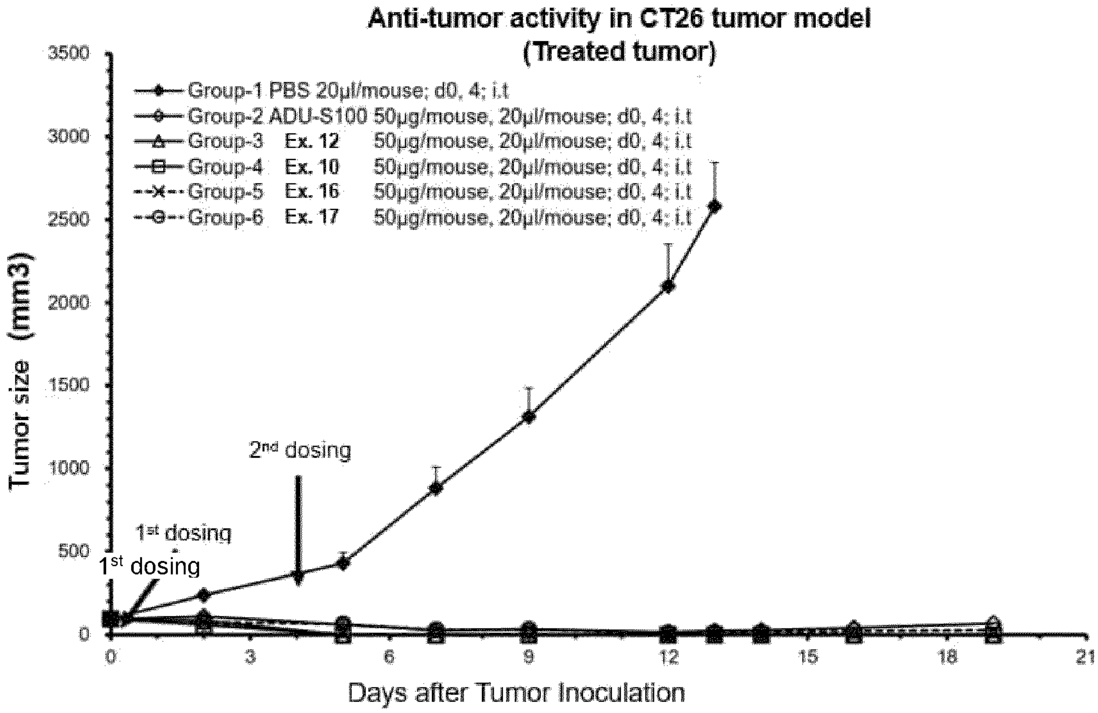
Anti-tumor activity in CT26 tumor model
(Treated tumor)
Group-1 PBS 20μl/mouse; d0, 4; i.t
Group-2 ADU-S100 50μg/mouse, 20μl/mouse; d0, 4; i.t
Group-3 Ex. 12 50μg/mouse, 20μl/mouse; d0, 4; i.t
Group-4 Ex. 10 50μg/mouse, 20μl/mouse; d0, 4; i.t
Group-5 Ex. 16 50μg/mouse, 20μl/mouse; d0, 4; i.t
Group-6 Ex. 17 50μg/mouse, 20μl/mouse; d0, 4; i.t
Tumor size (mm3)
2nd dosing
1st dosing
1st dosing
Days after Tumor Inoculation

Fig. 2-A

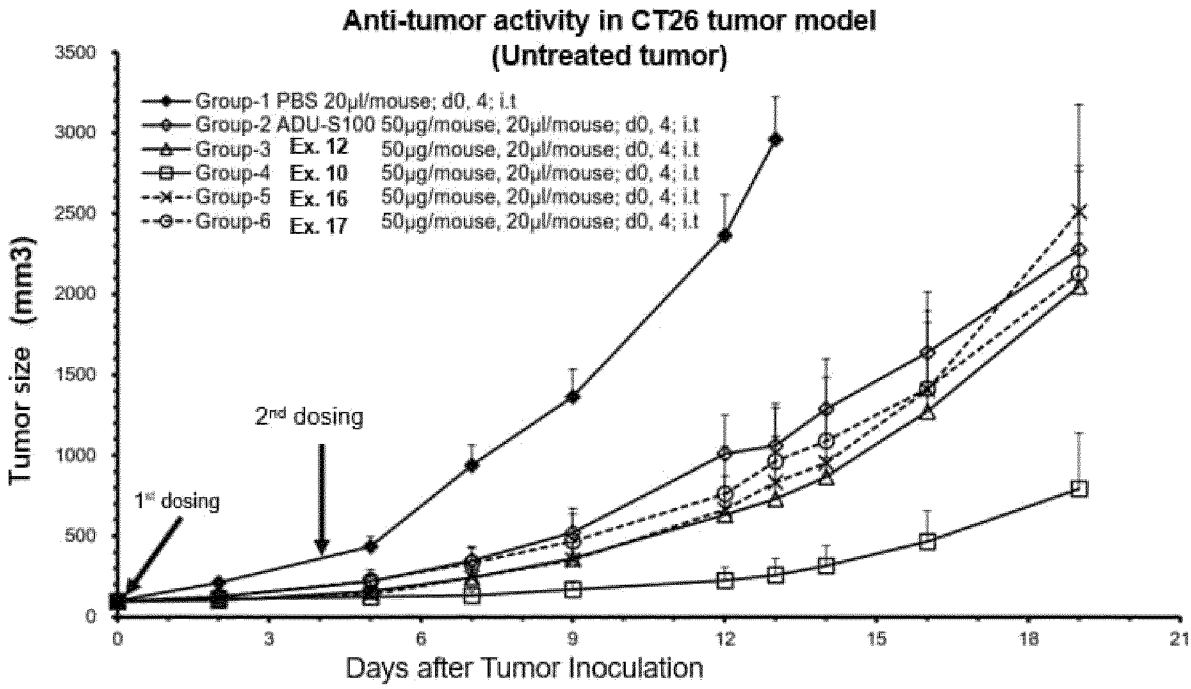
Anti-tumor activity in CT26 tumor model
(Untreated tumor)
Group-1 PBS 20µl/mouse; d0, 4; i.t
Group-2 ADU-S100 50µg/mouse, 20µl/mouse; d0, 4; i.t
Group-3 Ex. 12 50µg/mouse, 20µl/mouse; d0, 4; i.t
Group-4 Ex. 10 50µg/mouse, 20µl/mouse; d0, 4; i.t
Group-5 Ex. 16 50µg/mouse, 20µl/mouse; d0, 4; i.t
Group-6 Ex. 17 50µg/mouse, 20µl/mouse; d0, 4; i.t
Tumor size (mm3)
2nd dosing
1st dosing
Days after Tumor Inoculation

**Fig. 2-B**

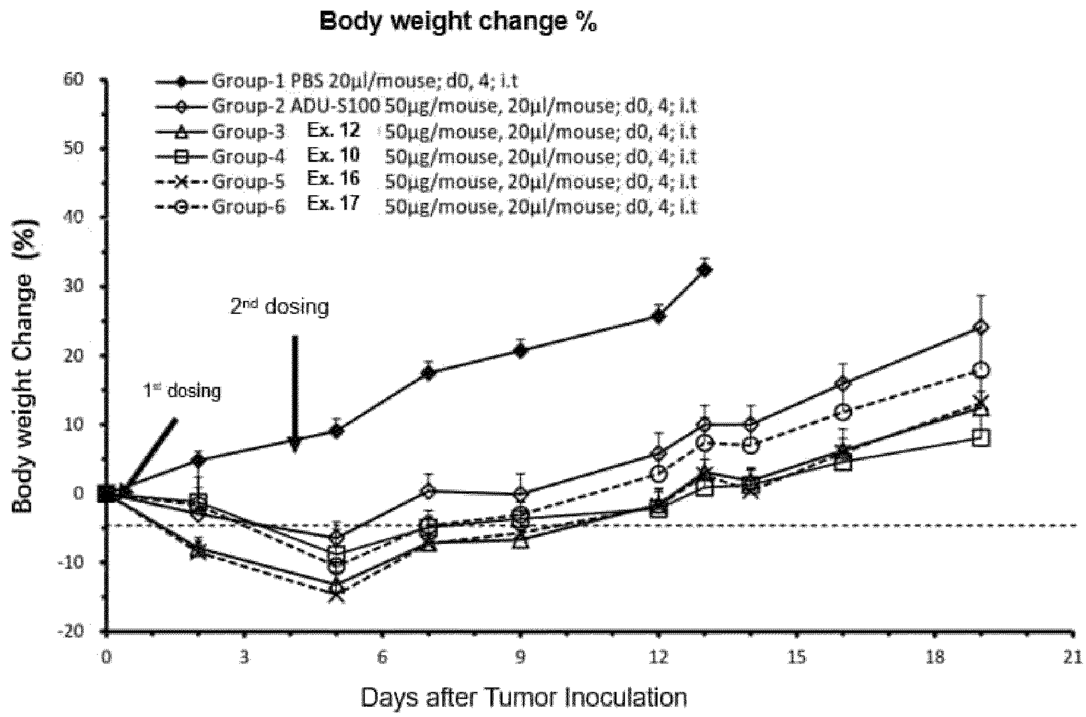
Body weight change %
Group-1 PBS 20µl/mouse; d0, 4; i.t
Group-2 ADU-S100 50µg/mouse, 20µl/mouse; d0, 4; i.t
Group-3 Ex. 12 50µg/mouse, 20µl/mouse; d0, 4; i.t
Group-4 Ex. 10 50µg/mouse, 20µl/mouse; d0, 4; i.t
Group-5 Ex. 16 50µg/mouse, 20µl/mouse; d0, 4; i.t
Group-6 Ex. 17 50µg/mouse, 20µl/mouse; d0, 4; i.t
Body weight Change (%)
2nd dosing
1st dosing
Days after Tumor Inoculation

**Fig. 2-C**

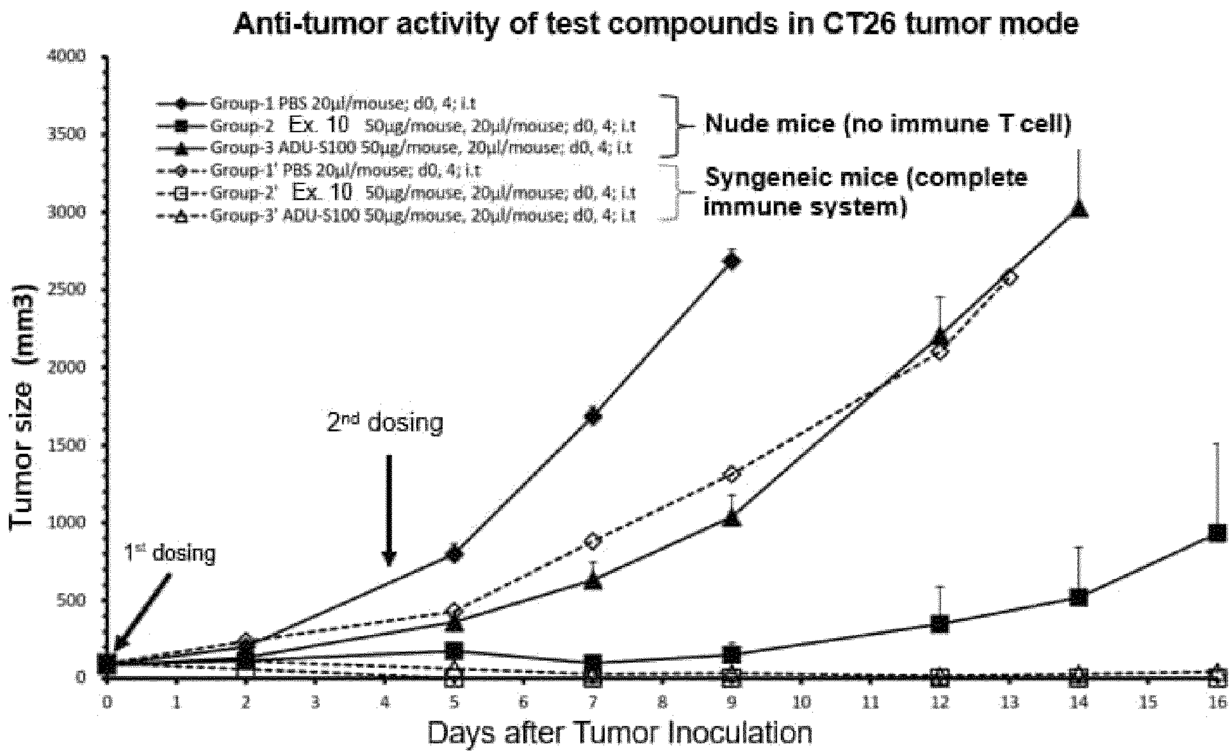
Anti-tumor activity of test compounds in CT26 tumor mode
Group-1 PBS 20µl/mouse; d0, 4; i.t
Group-2 Ex. 10 50µg/mouse, 20µl/mouse; d0, 4; i.t
Group-3 ADU-S100 50µg/mouse, 20µl/mouse; d0, 4; i.t
Group-1' PBS 20µl/mouse; d0, 4; i.t
Group-2' Ex. 10 50µg/mouse, 20µl/mouse; d0, 4; i.t
Group-3' ADU-S100 50µg/mouse, 20µl/mouse; d0, 4; i.t
Nude mice (no immune T cell)
Syngeneic mice (complete immune system)
Tumor size (mm3)
2nd dosing
1st dosing
Days after Tumor Inoculation

Fig. 2-D

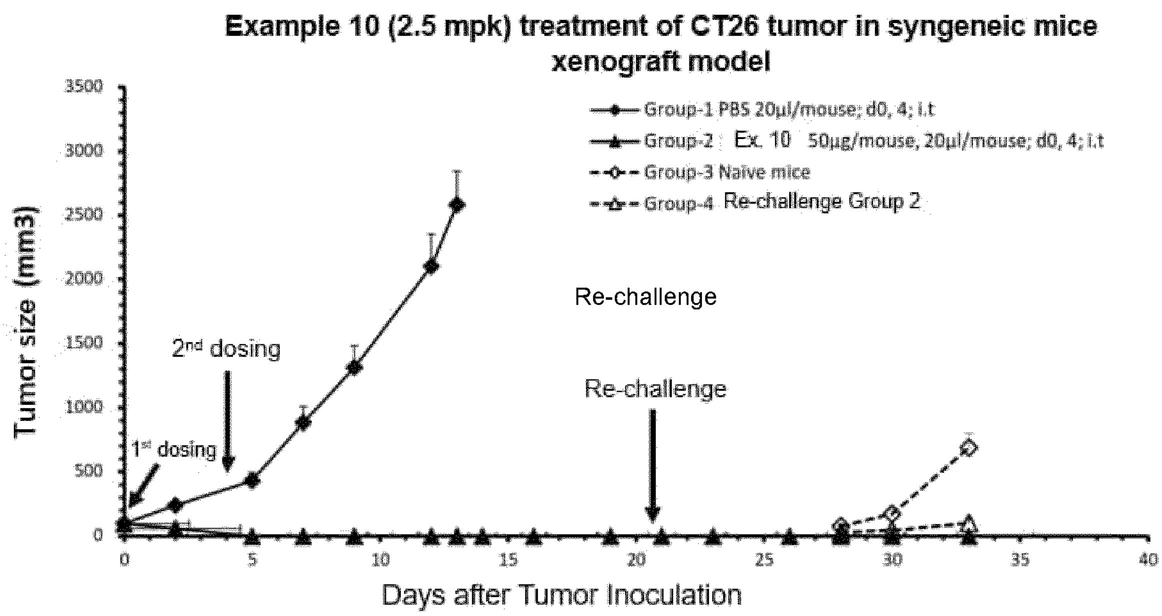
Example 10 (2.5 mpk) treatment of CT26 tumor in syngeneic mice xenograft model
Group-1 PBS 20µl/mouse; d0, 4; i.t
Group-2 Ex. 10 50µg/mouse, 20µl/mouse; d0, 4; i.t
Group-3 Naïve mice
Group-4 Re-challenge Group 2
Re-challenge
Re-challenge
2nd dosing
1st dosing
Tumor size (mm3)
Days after Tumor Inoculation

Fig. 3-A

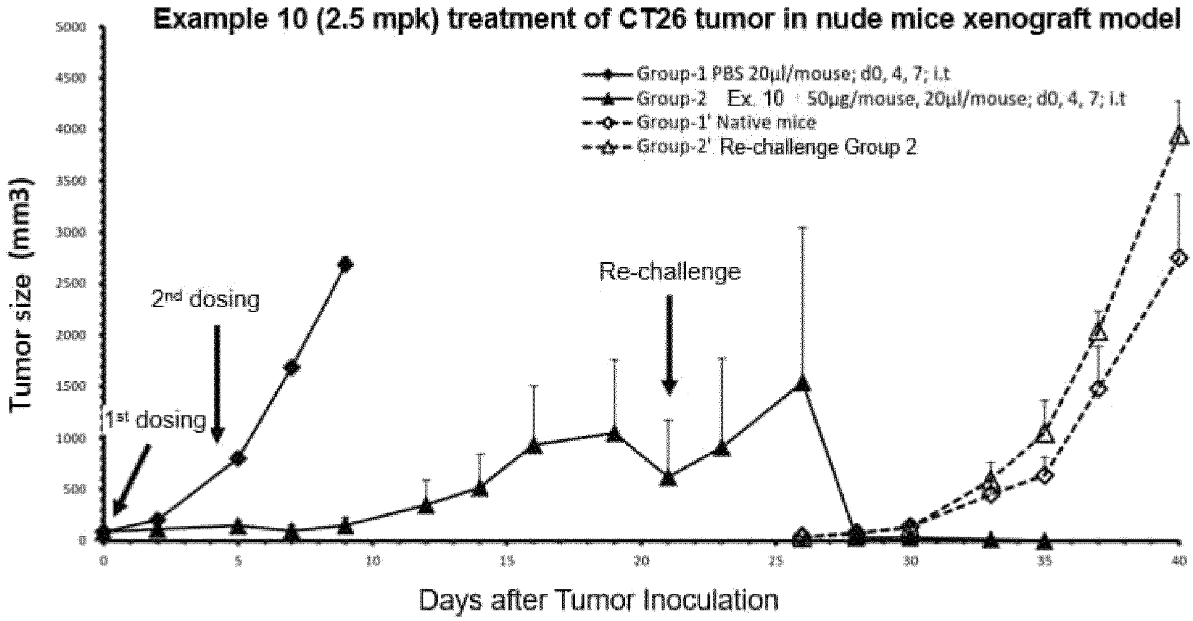


Fig. 3-B

| | T1/2 (minute) | | | | | $Cl_{int}$ (mL/min/kg) | | | | |
|---|---|---|---|---|---|---|---|---|---|---|
| | human | mouse | rat | dog | monkey | human | mouse | rat | dog | monkey |
| **Example 10** | 689.84 | 1466.5 | 543.75 | 524.80 | 475.31 | 2.56 | 5.58 | 5.96 | 9.09 | 5.69 |

Fig. 4-A

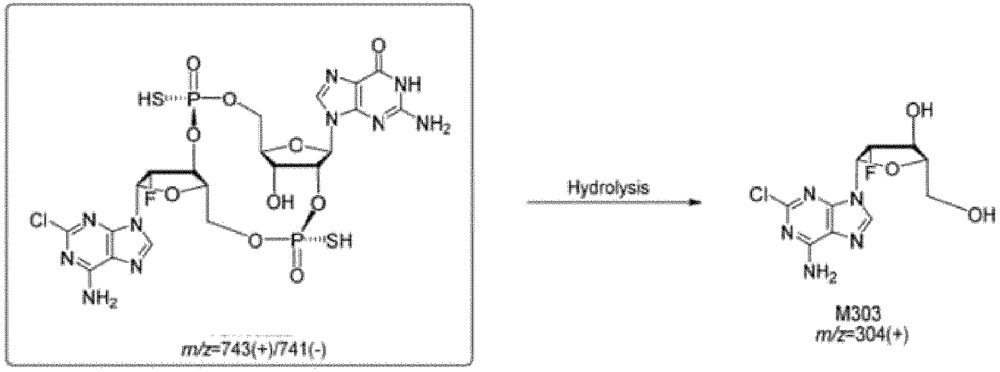


m/z=743(+)/741(-)

M303
m/z=304(+)

## Example 10

## Clofarabine

| Peak ID | Found m/z | Mass Shift | Bio-transform. | R.T. (min) | Mice Hepatocytes (240min) | | Human Hepatocytes (240min) | | Rat Hepatocytes (240min) | | Dog Hepatocytes (240min) | | Monkey Hepatocytes (240min) | |
|---|---|---|---|---|---|---|---|---|---|---|---|---|---|---|
| | | | | | Relative UV Abundance* | MS peak area | Relative UV Abundance* | MS peak area | Relative UV Abundance* | MS peak area | Relative UV Abundance* | MS peak area | Relative UV Abundance* | MS peak area |
| Parent (T0) | 743.0188 [M+H]+ | 0.0000 | N/A | 4.32 | **100.00%** | 4.09E+03 | **100.00%** | 3.06E+03 | **100.00%** | 2.23E+03 | **100.00%** | 1.52E+03 | **100.00%** | 2.51E+03 |
| | 741.0031 [M-H]- | 0.0000 | N/A | 4.5~4.7 | | 6.54E+03 | | 4.24E+03 | | 6.23E+03 | | 7.92E+03 | | 7.18E+03 |
| Parent (T240) | 743.0188 [M+H]+ | 0.0000 | N/A | 4.32 | **89.43%** | 3.42E+03 | **84.83%** | 2.68E+03 | **76.19%** | 1.81E+03 | **77.69%** | 8.51E+02 | **88.32%** | 2.46E+03 |
| | 741.0031 [M-H]- | 0.0000 | N/A | 4.5~4.7 | | 5.00E+03 | | 4.19E+03 | | 5.13E+03 | | 5.78E+03 | | 4.41E+03 |
| M303 | 304.0607 [M+H]+ | -438.9581 | Hydrolysis | 5.60 | + | 4.36E+02 | **4.04%** | 5.32E+02 | **11.54%** | 1.81E+03 | **8.43%** | 1.27E+03 | **7.21%** | 2.20E+03 |

Fig. 4-B

**INTERNATIONAL SEARCH REPORT**

International application No.
**PCT/CN2021/124704**

**A. CLASSIFICATION OF SUBJECT MATTER**

C07H 21/04(2006.01)i; A61K 31/7084(2006.01)i; A61P 35/00(2006.01)i; A61P 31/12(2006.01)i

According to International Patent Classification (IPC) or to both national classification and IPC

**B. FIELDS SEARCHED**

Minimum documentation searched (classification system followed by classification symbols)

C07H; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, PUBMED, ISI_Web of Science, Science Direct, STNext: 泰励生物, 张彦涛, 屈粒, 结构检索, 环状二核苷酸, 癌症, 病毒, CDN, sting, structure, cyclic dinucleotide, cancer, virus

**C. DOCUMENTS CONSIDERED TO BE RELEVANT**

| Category* | Citation of document, with indication, where appropriate, of the relevant passages | Relevant to claim No. |
|---|---|---|
| X | CN 107148424 A (INVIVOGEN) 08 September 2017 (2017-09-08)<br>abstract, description, paragraphs 0065, 0091-0094, 0124, 0126, 0129, and 0137-0138, and claims 1-12 | 1-31 |
| X | WO 2017123657 A1 (GARY, G. et al.) 20 July 2017 (2017-07-20)<br>description, table 1, and claims 1-145 | 1-31 |
| X | WO 2020117624 A1 (MERCK SHARP & DOHME CORP.) 11 June 2020 (2020-06-11)<br>claims 1-20 | 1-31 |
| X | WO 2016145102 A1 (ADURO BIOTECH, INC. et al.) 15 September 2016 (2016-09-15)<br>description, paragraphs 0025, 0031, 0039, and 0061, claims 1-16, and abstract | 1-31 |
| X | WO 2020057546 A1 (SHANGHAI DE NOVO PHARMATECH CO., LTD.) 26 March 2020 (2020-03-26)<br>description, page 23, line 11 to page 31, line 13 | 1-31 |
| X | EP 3505527 A1 (INVIVOGEN) 03 July 2019 (2019-07-03)<br>abstract, description, paragraphs 0009-0055, and claims 1-20 | 1-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 January 2022** | **19 January 2022** |

Name and mailing address of the ISA/CN
**China National Intellectual Property Administration (ISA/CN)**
**No. 6, Xitucheng Road, Jimenqiao, Haidian District, Beijing 100088, China**
Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2021/124704**

| C. DOCUMENTS CONSIDERED TO BE RELEVANT | | |
|---|---|---|
| Category* | Citation of document, with indication, where appropriate, of the relevant passages | Relevant to claim No. |
| X | WO 2019043634 A2 (BEIJING XUANYI PHARMASCIENCES CO., LTD.) 07 March 2019 (2019-03-07)<br>description, table A and table B, and claims 1-79 | 1-31 |
| X | CN 107849084 A (GLAXOSMITHKLINE INTELLECTUAL PROPERTY DEVELOPMENT LIMITED) 27 March 2018 (2018-03-27)<br>abstract, and claims 1-53 | 1-31 |
| X | WO 2017106740 A1 (ADURO BIOTECH, INC.) 22 June 2017 (2017-06-22)<br>description, paragraphs 0014-0023 and 00187, and claims 1-49 | 1-31 |
| X | WO 2018156625 A1 (BOARD OF REGENTS, UNIVERSITY OF TEXAS SYSTEM) 30 August 2018 (2018-08-30)<br>description, paragraphs 045-052, and claims 1-79 | 1-31 |
| X | WO 2014093936 A1 (ADURO BIOTECH, INC.) 19 June 2014 (2014-06-19)<br>abstract, claims 1-30, and figures 1-5 | 1-31 |
| X | WO 2017075477 A1 (ADURO BIOTECH, INC. et al.) 04 May 2017 (2017-05-04)<br>description, paragraphs 0020, 0028, and 00433, and claims 1-38 | 1-31 |
| X | WO 2017027646 A1 (MERCK SHARP & DOHME CORP.) 16 February 2017 (2017-02-16)<br>description, page 48, line 11 to page 100, line 4, and claims 1-33 | 1-31 |
| X | WO 2014189806 A1 (ADURO BIOTECH, INC.) 27 November 2014 (2014-11-27)<br>claims 1-29 | 1-31 |
| X | US 5547941 A (BATTISTINI, C. et al.) 20 August 1996 (1996-08-20)<br>abstract and claims 1-6 | 1-31 |
| X | WO 2018098203 A1 (JANSSEN BIOTECH, INC.) 31 May 2018 (2018-05-31)<br>description, pages 19-27, compounds 1-23, and claims 1-18 | 1-31 |
| X | LAUNER-FELTY, K. D. et al. "Enzymatic synthesis of cyclic dinucleotide analogs by a promiscuous cyclic-AMP-GMP synthetase and analysis of cyclic dinucleotide responsive riboswitches,"<br>*Nucleic Acids Research,* Vol. 46, No. 6, 05 March 2018 (2018-03-05),<br>pages 2765-2776 | 1-31 |
| X | LI, Lingyin et al. "Hydrolysis of 2′3′-cGAMP by ENPP1 and design of non-hydrolyzable analogs,"<br>*Nat. Chem. Biol.,* Vol. 10, No. 12, 31 December 2014 (2014-12-31),<br>pages 1043-1048 | 1-31 |
| X | BATTISTINI, C. et al. "STEREOSELECTIVE SYNTHESIS OF CYCLIC DINUCLEOTIDE PHOSPHOROTHIOATES,"<br>*Tetrahedron,* Vol. 49, No. 5, 31 December 1993 (1993-12-31),<br>pages 1115-1132 | 1-31 |

Form PCT/ISA/210 (second sheet) (January 2015)

**INTERNATIONAL SEARCH REPORT**

International application No.
**PCT/CN2021/124704**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24-28**，**31**（部分）
because they relate to subject matter not required to be searched by this Authority, namely:

[1] Claim 24 sets forth a use in prevention or treatment of STING related or mediated diseases in mammals, especially humans; claims 25-28 and 31 (in part) set forth a use of the compound according to any one of claims 1-21 or the pharmaceutical composition according to any one of claims 22-23 in treatment or prevention of STING related or mediated diseases as a STING agonist or in treatment or prevention of viral infections or tumors as a cytotoxic agent, or a multifunctional active agent used in immunologic therapy and cytotoxic therapy, comprise a solution of treating a human body or animal body, and do not comply with PCT Rule 39.1(iv). The search on claims 24-28 and 31 (in part) is made on the following amendments: a use of the compound according to any one of claims 1-21 or the pharmaceutical composition according to any one of claims 22-23 in the preparation of a drug used as a STING agonist for treatment or prevention of STING related or mediated diseases, a drug used as a cytotoxic agent for treatment or prevention of viral infections or tumors, or a drug used in immunologic therapy and cytotoxic therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/124704**

| Patent document cited in search report | Publication date (day/month/year) | Patent family member(s) | Publication date (day/month/year) |
|---|---|---|---|
| CN 107148424 A | 23 June 2016 | PL 3233882 T3 | 30 April 2020 |
| | | DK 3233882 T3 | 27 January 2020 |
| | | EP 3233882 A1 | 25 October 2017 |
| | | EP 3233882 B1 | 30 October 2019 |
| | | WO 2016096174 A8 | 29 September 2016 |
| | | US 2016362441 A1 | 15 December 2016 |
| | | US 10011630 B2 | 03 July 2018 |
| | | EP 3546473 A1 | 02 October 2019 |
| | | CN 107148424 A | 08 September 2017 |
| | | PT 3233882 T | 21 January 2020 |
| | | US 2020255469 A1 | 13 August 2020 |
| | | US 11053272 B2 | 06 July 2021 |
| | | ES 2764178 T3 | 02 June 2020 |
| | | US 2018354983 A1 | 13 December 2018 |
| | | US 10562929 B2 | 18 February 2020 |
| | | WO 2016096174 A1 | 23 June 2016 |
| CN 107148424 A | 08 September 2017 | PL 3233882 T3 | 30 April 2020 |
| | | DK 3233882 T3 | 27 January 2020 |
| | | EP 3233882 A1 | 25 October 2017 |
| | | EP 3233882 B1 | 30 October 2019 |
| | | WO 2016096174 A8 | 29 September 2016 |
| | | US 2016362441 A1 | 15 December 2016 |
| | | US 10011630 B2 | 03 July 2018 |
| | | EP 3546473 A1 | 02 October 2019 |
| | | PT 3233882 T | 21 January 2020 |
| | | US 2020255469 A1 | 13 August 2020 |
| | | US 11053272 B2 | 06 July 2021 |
| | | ES 2764178 T3 | 02 June 2020 |
| | | US 2018354983 A1 | 13 December 2018 |
| | | US 10562929 B2 | 18 February 2020 |
| | | WO 2016096174 A1 | 23 June 2016 |
| WO 2017123657 A1 | 20 July 2017 | US 2019031708 A1 | 31 January 2019 |
| | | US 10723756 B2 | 28 July 2020 |
| WO 2020117624 A1 | 11 June 2020 | EP 3891166 A1 | 13 October 2021 |
| WO 2016145102 A1 | 15 September 2016 | CO 2017009104 A2 | 05 January 2018 |
| | | MX 2017011597 A | 11 May 2018 |
| | | JO 3746 B1 | 31 January 2021 |
| | | AU 2016229146 A1 | 07 September 2017 |
| | | BR 112017018908 A2 | 17 April 2018 |
| | | CA 2979215 A1 | 15 September 2016 |
| | | JP 2018509409 A | 05 April 2018 |
| | | JP 6692826 B2 | 13 May 2020 |
| | | EC SP17064523 A | 29 March 2019 |
| | | GT 201700200 A | 10 June 2019 |
| | | KR 20170129802 A | 27 November 2017 |
| | | SV 2017005529 A | 20 August 2018 |
| | | DO P2017000205 A | 15 November 2017 |
| | | MA 42146 A | 21 April 2021 |
| | | CR 10 April 2017 A | 08 November 2017 |
| | | IL 254047 D0 | 31 October 2017 |

Form PCT/ISA/210 (patent family annex) (January 2015)

**INTERNATIONAL SEARCH REPORT**
**Information on patent family members**

International application No.
**PCT/CN2021/124704**

| Patent document cited in search report | Publication date (day/month/year) | Patent family member(s) | Publication date (day/month/year) |
|---|---|---|---|
| | | US 2018064745 A1 | 08 March 2018 |
| | | US 10449211 B2 | 22 October 2019 |
| | | SG 11201706756 V A | 28 September 2017 |
| | | EA 201791999 A1 | 28 February 2018 |
| | | EA 035817 B1 | 14 August 2020 |
| | | TN 2017000375 A1 | 16 January 2019 |
| | | HK 1248603 A1 | 19 October 2018 |
| | | EP 3268035 A1 | 17 January 2018 |
| | | EP 3268035 A4 | 31 October 2018 |
| | | CN 107530415 A | 02 January 2018 |
| | | CL 2017002272 A1 | 23 March 2018 |
| | | TW 201639866 A | 16 November 2016 |
| | | TW I706958 B | 11 October 2020 |
| | | PE 20171448 A1 | 02 October 2017 |
| | | US 2020179431 A1 | 11 June 2020 |
| | | US 11040053 B2 | 22 June 2021 |
| | | UY 36579 A | 31 October 2016 |
| | | PH 12017501506 A1 | 05 February 2018 |
| | | AR 103894 A1 | 14 June 2017 |
| | | HK 1247089 A1 | 21 September 2018 |
| WO 2020057546 A1 | 26 March 2020 | EP 3854799 A1 | 28 July 2021 |
| | | CA 3113425 A1 | 26 March 2020 |
| | | SG 11202102742 R A | 29 April 2021 |
| | | BR 112021005208 A2 | 08 June 2021 |
| | | KR 20210062668 A | 31 May 2021 |
| | | PH 12021550617 A1 | 04 October 2021 |
| | | AU 2019344398 A1 | 20 May 2021 |
| | | CN 110938104 A | 31 March 2020 |
| | | TW 202023572 A | 01 July 2020 |
| EP 3505527 A1 | 03 July 2019 | None | |
| WO 2019043634 A2 | 07 March 2019 | SG 11202001847 W A | 30 March 2020 |
| | | RU 2020112502 A | 01 October 2021 |
| | | RU 2020112502 A3 | 08 October 2021 |
| | | IL 272928 D0 | 30 April 2020 |
| | | WO 2019043634 A3 | 11 April 2019 |
| | | KR 20200058411 A | 27 May 2020 |
| | | CN 111263767 A | 09 June 2020 |
| | | EP 3692048 A2 | 12 August 2020 |
| | | EP 3692048 A4 | 20 October 2021 |
| | | BR 112020004047 A2 | 01 September 2020 |
| | | CA 3073919 A1 | 07 March 2019 |
| | | JP 2020532585 A | 12 November 2020 |
| | | TW 201919650 A | 01 June 2019 |
| | | US 2020331957 A1 | 22 October 2020 |
| | | AU 2018323053 A1 | 19 March 2020 |
| CN 107849084 A | 27 March 2018 | CO 2017013310 A2 | 20 March 2018 |
| | | CA 3006930 A1 | 08 June 2017 |
| | | PH 12017502332 A1 | 11 June 2018 |
| | | US 2017158724 A1 | 08 June 2017 |
| | | US 9718848 B2 | 01 August 2017 |

Form PCT/ISA/210 (patent family annex) (January 2015)

**INTERNATIONAL SEARCH REPORT**
**Information on patent family members**

International application No.
**PCT/CN2021/124704**

| Patent document cited in search report | Publication date (day/month/year) | Patent family member(s) | Publication date (day/month/year) |
|---|---|---|---|
| | | TW 201731862 A | 16 September 2017 |
| | | TW I704154 B | 11 September 2020 |
| | | NZ 738202 A | 26 July 2019 |
| | | PE 20181297 A1 | 07 August 2018 |
| | | KR 20180009812 A | 29 January 2018 |
| | | KR 101949108 B1 | 15 February 2019 |
| | | ES 2863225 T3 | 11 October 2021 |
| | | CR 20180286 A | 16 July 2018 |
| | | EP 3322713 A1 | 23 May 2018 |
| | | EP 3322713 B1 | 20 January 2021 |
| | | RU 2020113165 A | 09 June 2020 |
| | | HK 1250032 A1 | 23 November 2018 |
| | | EP 3366691 A1 | 29 August 2018 |
| | | US 2018258132 A1 | 13 September 2018 |
| | | US 10364266 B2 | 30 July 2019 |
| | | IL 255525 D0 | 31 January 2018 |
| | | IL 255525 A | 31 October 2019 |
| | | US 2017233430 A1 | 17 August 2017 |
| | | US 9994607 B2 | 12 June 2018 |
| | | US 2020002370 A1 | 02 January 2020 |
| | | US 10730907 B2 | 04 August 2020 |
| | | BR 112018011302 A2 | 27 November 2018 |
| | | AU 2016362697 A1 | 04 January 2018 |
| | | AU 2016362697 B2 | 12 July 2018 |
| | | MA 52157 A | 17 February 2021 |
| | | UY 37007 A | 30 June 2017 |
| | | CL 2018001484 A1 | 20 July 2018 |
| | | JP 2018516903 A | 28 June 2018 |
| | | JP 6411676 B2 | 24 October 2018 |
| | | RU 2018103881 A | 02 August 2019 |
| | | RU 2018103881 A3 | 02 August 2019 |
| | | RU 2722019 C2 | 26 May 2020 |
| | | MX 2017015299 A | 15 March 2018 |
| | | MX 363780 B | 03 April 2019 |
| | | ZA 201801631 B | 27 November 2019 |
| | | WO 2017093933 A1 | 08 June 2017 |
| WO 2017106740 A1 | 22 June 2017 | US 2018369268 A1 | 27 December 2018 |
| WO 2018156625 A1 | 30 August 2018 | EP 3585379 A1 | 01 January 2020 |
| | | EP 3585379 A4 | 02 December 2020 |
| | | JP 2020508310 A | 19 March 2020 |
| | | CA 3053568 A1 | 30 August 2018 |
| | | US 2021236531 A1 | 05 August 2021 |
| | | US 2019358254 A1 | 28 November 2019 |
| | | US 10933078 B2 | 02 March 2021 |
| WO 2014093936 A1 | 19 June 2014 | HK 1212707 A1 | 17 June 2016 |
| | | PT 2931738 T | 10 April 2019 |
| | | AU 2013358892 A1 | 21 May 2015 |
| | | AU 2013358892 B2 | 21 June 2018 |
| | | US 2014205653 A1 | 24 July 2014 |
| | | US 9695212 B2 | 04 July 2017 |

Form PCT/ISA/210 (patent family annex) (January 2015)

**INTERNATIONAL SEARCH REPORT**
**Information on patent family members**

International application No.
**PCT/CN2021/124704**

| Patent document cited in search report | Publication date (day/month/year) | Patent family member(s) | Publication date (day/month/year) |
|---|---|---|---|
| | | SG 11201502796 R A | 28 May 2015 |
| | | CN 108542913 A | 18 September 2018 |
| | | JP 2018135367 A | 30 August 2018 |
| | | JP 6704012 B2 | 03 June 2020 |
| | | EP 3527579 A1 | 21 August 2019 |
| | | AU 2018203682 A1 | 14 June 2018 |
| | | AU 2018203682 B2 | 02 July 2020 |
| | | BR 112015013440 A2 | 11 July 2017 |
| | | BR 112015013440 B1 | 08 December 2020 |
| | | MX 2015007447 A | 09 December 2015 |
| | | MX 361680 B | 13 December 2018 |
| | | JP 2016503029 A | 01 February 2016 |
| | | JP 6333843 B2 | 30 May 2018 |
| | | KR 20150095668 A | 21 August 2015 |
| | | SG 10201704611 W A | 28 July 2017 |
| | | DK 2931738 T3 | 15 April 2019 |
| | | PL 2931738 T3 | 31 July 2019 |
| | | CN 105008381 A | 28 October 2015 |
| | | EP 2931738 A1 | 21 October 2015 |
| | | EP 2931738 A4 | 27 April 2016 |
| | | EP 2931738 B1 | 06 February 2019 |
| | | ES 2718910 T3 | 05 July 2019 |
| | | CA 2886456 A1 | 19 June 2014 |
| | | EA 201590396 A1 | 30 December 2015 |
| | | NZ 707561 A | 27 November 2020 |
| | | SI 2931738 T1 | 31 May 2019 |
| | | US 2017283454 A1 | 05 October 2017 |
| | | US 10414789 B2 | 17 September 2019 |
| | | HU E043262 T2 | 28 August 2019 |
| WO 2017075477 A1 | 04 May 2017 | EP 3368072 A1 | 05 September 2018 |
| | | EP 3368072 A4 | 03 July 2019 |
| | | AU 2016343993 A1 | 10 May 2018 |
| | | CN 108430503 A | 21 August 2018 |
| | | RU 2018119306 A | 28 November 2019 |
| | | US 2019062365 A1 | 28 February 2019 |
| | | US 10906930 B2 | 02 February 2021 |
| | | CA 3002236 A1 | 04 May 2017 |
| | | BR 112018008339 A2 | 30 October 2018 |
| | | UY 36969 A | 31 May 2017 |
| | | JP 2018534295 A | 22 November 2018 |
| | | TW 201726700 A | 01 August 2017 |
| | | WO 2017075477 A8 | 26 April 2018 |
| | | MX 2018005299 A | 18 March 2019 |
| | | KR 20180066241 A | 18 June 2018 |
| WO 2017027646 A1 | 16 February 2017 | EA 201890450 A1 | 31 July 2018 |
| | | EA 034786 B1 | 20 March 2020 |
| | | SV 2018005632 A | 19 October 2018 |
| | | CL 2018000385 A1 | 17 August 2018 |
| | | US 2018237469 A1 | 23 August 2018 |
| | | US 10766919 B2 | 08 September 2020 |

Form PCT/ISA/210 (patent family annex) (January 2015)

**INTERNATIONAL SEARCH REPORT**
**Information on patent family members**

International application No.
**PCT/CN2021/124704**

| Patent document cited in search report | | | Publication date (day/month/year) | Patent family member(s) | | | Publication date (day/month/year) |
|---|---|---|---|---|---|---|---|
| | | | | GE | AP201614726 | A | 27 July 2020 |
| | | | | GE | P20207182 | B | 25 November 2020 |
| | | | | ZA | 201800442 | B | 19 December 2018 |
| | | | | PE | 20180688 | A1 | 23 April 2018 |
| | | | | EP | 3344644 | A1 | 11 July 2018 |
| | | | | EP | 3344644 | A4 | 27 February 2019 |
| | | | | CR | 01.01.2018 | A | 12 April 2018 |
| | | | | US | 2018230178 | A1 | 16 August 2018 |
| | | | | US | 10738074 | B2 | 11 August 2020 |
| | | | | KR | 20200116537 | A | 12 October 2020 |
| | | | | KR | 102271750 | B1 | 30 June 2021 |
| | | | | SG | 10202010609 Q | A | 27 November 2020 |
| | | | | MX | 2018001814 | A | 07 May 2018 |
| | | | | PH | 12018500299 | A1 | 29 August 2018 |
| | | | | EP | 3334745 | A1 | 20 June 2018 |
| | | | | JO | P20200224 | A1 | 16 June 2017 |
| | | | | TW | 201718619 | A | 01 June 2017 |
| | | | | TW | I696629 | B | 21 June 2020 |
| | | | | MA | 42608 | A | 20 June 2018 |
| | | | | CN | 108137641 | A | 08 June 2018 |
| | | | | UA | 123701 | C2 | 19 May 2021 |
| | | | | CA | 2995365 | A1 | 16 February 2017 |
| | | | | CA | 2995365 | C | 12 October 2021 |
| | | | | CO | 2018001425 | A2 | 10 May 2018 |
| | | | | NI | 201800025 | A | 12 June 2018 |
| | | | | US | 2017044206 | A1 | 16 February 2017 |
| | | | | US | 10106574 | B2 | 23 October 2018 |
| | | | | DO | P2018000046 | A | 15 April 2018 |
| | | | | US | 2018244712 | A1 | 30 August 2018 |
| | | | | US | 10759825 | B2 | 01 September 2020 |
| | | | | AU | 2016304899 | A1 | 01 February 2018 |
| | | | | AU | 2016304899 | B2 | 08 November 2018 |
| | | | | WO | 2017027645 | A1 | 16 February 2017 |
| | | | | KR | 20180030926 | A | 26 March 2018 |
| | | | | KR | 102222186 | B1 | 03 March 2021 |
| | | | | TN | 2018000023 | A1 | 08 July 2019 |
| | | | | HK | 1249109 | A1 | 26 October 2018 |
| | | | | IL | 257142 | D0 | 29 March 2018 |
| | | | | IL | 257142 | A | 29 April 2021 |
| | | | | JP | 2018522914 | A | 16 August 2018 |
| | | | | JP | 6596146 | B2 | 23 October 2019 |
| WO | 2014189806 | A1 | 27 November 2014 | EP | 2996472 | A1 | 23 March 2016 |
| | | | | EP | 2996472 | A4 | 28 December 2016 |
| | | | | EP | 2996472 | B1 | 27 March 2019 |
| | | | | CN | 105188373 | A | 23 December 2015 |
| | | | | HK | 1217606 | A1 | 20 January 2017 |
| | | | | JP | 2016531842 | A | 13 October 2016 |
| | | | | JP | 6400082 | B2 | 03 October 2018 |
| US | 5547941 | A | 20 August 1996 | DE | 4223438 | A1 | 21 January 1993 |
| | | | | JP | H05186495 | A | 27 July 1993 |

Form PCT/ISA/210 (patent family annex) (January 2015)

**INTERNATIONAL SEARCH REPORT**
**Information on patent family members**

International application No.
**PCT/CN2021/124704**

| Patent document cited in search report | Publication date (day/month/year) | Patent family member(s) | Publication date (day/month/year) |
|---|---|---|---|
| | | GB 9115586 D0 | 04 September 1991 |
| | | GB 2257704 A | 20 January 1993 |
| | | GB 2257704 B | 01 March 1995 |
| | | IT MI921720 D0 | 15 July 1992 |
| | | IT MI921720 A1 | 15 January 1994 |
| | | IT 1255222 B | 20 October 1995 |
| WO 2018098203 A1 | 31 May 2018 | US 2018162899 A1 | 14 June 2018 |
| | | UY 37495 A | 31 May 2018 |
| | | KR 20190085107 A | 17 July 2019 |
| | | TW 201831192 A | 01 September 2018 |
| | | AU 2017365158 A1 | 06 June 2019 |
| | | MX 2019006082 A | 12 November 2019 |
| | | BR 112019010606 A2 | 17 September 2019 |
| | | JP 2020500862 A | 16 January 2020 |
| | | EP 3544989 A1 | 02 October 2019 |
| | | CN 110291096 A | 27 September 2019 |
| | | CA 3044693 A1 | 31 May 2018 |
| | | JO P20170188 A1 | 30 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014189805 A
- WO 2017027645 A
- WO 2018060323 A
- WO 2016145102 A


### Non-patent literature cited in the description


- **JIANG et al.** cGAS-STING, an antigenic pathway in cancer immunology. *Journal of Hematology & Oncology,* 2020, vol. 13, 81
- **XIANGLING CUI et al.** STING modulatories: Predictive significance in drug discovery. *European Journal of chemical Chemistry,* 2019, vol. 182, 111591
- **BERGE et al.** *Pharm ScL,* 1977, vol. 66, 1-19
- Prodrugs and Targeted Delivery. John Wiley & Sons, 2011
- **ZHAO et al.** *Nucleosides, Nucleotides and Nucleic Acids,* 2009, vol. 289, 352-378
- **KNOUSE et al.** *Science,* 2018, (361), 1234
- **T.W. GREENE ; P.G.M. WUTS.** Greene's protective groups in organic synthesis. John Wiley & Sons., Inc, 2014
- **GENNARO AR et al.** Remington: The Science and Practice of Pharmacy. Lippincott, Williams & Wilkins, 2000
- **EL ELIEL ; SH WILEN ; LN MANDER.** Stereochemistry of Organic Compounds. Wiley-Interscience, 1994
- *CHEMICAL ABSTRACTS,* 2245335-71-9
- *CHEMICAL ABSTRACTS,* 2245335-70-8
- **KERNS ; EDWARD H. ; DI LI.** Drug-like Properties: Concepts, Structure Design and Methods: From ADME to Toxicity Optimization. Academic Press, 2008
- **DI, LI et al.** Optimization of a High Throughput Microsomal Stability Screening Assay for Profiling Drug Discovery Candidates. *J Biomol Screen.,* 2003, vol. 8 (4), 453
- **KERNS, EDWARD H. ; DI LI.** Drug-like Properties: Concepts, Structure Design and Methods: From ADME to Toxicity Optimization. Academic Press, 2008